# EUROPEAN PATENT SPECIFICATION

(11) **EP 3 956 323 B1**
(45) Date of publication and mention of the grant of the patent: **05.03.2025**
(21) Application number: 19724354.6
(22) Date of filing: 18.04.2019
(51) Int. Cl.: C07D 413/12, C07D 413/14, A61P 3/10, C07D 417/14, C07D 471/04, C07D 495/04, A61K 31/506, A61K 31/53

(54) **OXAZOLE AND OXADIAZOLE DERIVATIVES USEFUL AS AGONISTS OF FREE FATTY ACID RECEPTOR 1**
OXAZOL- UND OXADIAZOLDERIVATE ALS AGONISTEN DES FREIE-FETTSÄURE-REZEPTORS 1
DÉRIVÉS D'OXAZOLE ET D'OXADIAZOLE UTILES EN TANT QU'AGONISTES DU RÉCEPTEUR 1 D'ACIDES GRAS LIBRES

(43) Date of publication of application: 23.02.2022
(73) Proprietor: Halo Therapeutics Ltd, Caerphilly, Wales CF83 3GG (GB)
(72) Inventor: SHAFEEV, Mikhail, Kyiv, 02100 (UA); FITZGERALD, Daniel Josef, 1227 Carouge (CH); SCHELSHORN, Dominik Wolfgang, 1205 Geneva (CH); PERVAK, Igor I., Kyiv, 01283 (UA)
(74) Representative: Habermann, Hruschka & Schnabel
(86) International application number: PCT/EP2019/060211
(87) International publication number: WO 2020/211956

(56) References cited:
- US-A1- 2004 006 011
- XU LI-LI ET AL: "Molecular similarity guided optimization of novel Nrf2 activators with 1,2,4-oxadiazole core", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 24, no. 16, 28 May 2016 (2016-05-28), pages 3540 - 3547, XP029642287, ISSN: 0968-0896, DOI: 10.1016/J.BMC.2016.05.056
- DANIEL F. KAWANO ET AL: "Prospecting for New Inhibitors of Anaplastic Lymphoma Kinase, A Clinically Relevant Oncogenic Drug Target", CURRENT BIOACTIVE COMPOUNDS, vol. 13, no. 3, 24 July 2017 (2017-07-24), XP055640633, ISSN: 1573-4072, DOI: 10.2174/1573407212666160607092819
- DUFFY MARGARET R ET AL: "Identification of novel small molecule inhibitors of adenovirus gene transfer using a high throughput screening approach", JOURNAL OF CONTROLLED RELEASE, ELSEVIER, AMSTERDAM, NL, vol. 170, no. 1, 20 May 2013 (2013-05-20), pages 132 - 140, XP028574945, ISSN: 0168-3659, DOI: 10.1016/J.JCONREL.2013.05.007
- JUXIAN WANG ET AL: "Pharmacophore-Based Virtual Screening and Biological Evaluation of Small Molecule Inhibitors for Protein Arginine Methylation", JOURNAL OF MEDICINAL CHEMISTRY, vol. 55, no. 18, 12 September 2012 (2012-09-12), US, pages 7978 - 7987, XP055462499, ISSN: 0022-2623, DOI: 10.1021/jm300521m
- JUXIAN WANG ET AL: "Supporting Information, Pharmacophore-Based Virtual Screening and Biological Evaluation of Small Molecule Inhibitors for Protein Arginine Methylation", 28 August 2012 (2012-08-28), XP055640596, Retrieved from the Internet <URL:https://pubs.acs.org/doi/suppl/10.1021/jm300521m/suppl_file/jm300521m_si_001.pdf> [retrieved on 20191108]
- YI-YOU HUANG ET AL: "Validation of Phosphodiesterase-10 as a Novel Target for Pulmonary Arterial Hypertension via Highly Selective and Subnanomolar Inhibitors", JOURNAL OF MEDICINAL CHEMISTRY, vol. 62, no. 7, 19 March 2019 (2019-03-19), US, pages 3707 - 3721, XP055640628, ISSN: 0022-2623, DOI: 10.1021/acs.jmedchem.9b00224
- YI-YOU HUANG ET AL: "Supporting Information Validation of Phosphodiesterase-10 as a Novel Target for Pulmonary Arterial Hypertension via Highly Selective and Subnanomolar Inhibitors", 19 March 2019 (2019-03-19), XP055640624, Retrieved from the Internet <URL:https://pubs.acs.org/doi/suppl/10.1021/acs.jmedchem.9b00224/suppl_file/jm9b00224_si_001.pdf> [retrieved on 20191108]
- JONAS BOSTR?M ET AL: "Oxadiazoles in Medicinal Chemistry", JOURNAL OF MEDICINAL CHEMISTRY, vol. 55, no. 5, 8 March 2012 (2012-03-08), pages 1817 - 1830, XP055050959, ISSN: 0022-2623, DOI: 10.1021/jm2013248
- DATABASE CHEMCATS Chemical Abstracts Service, Columbus, Ohio, USA; XP002795546
- "Aurora Screening Compounds 1", 21 February 2019, AURORA FINE CHEMICALS LLC, San Diego
- DATABASE CHEMCATS Chemical Abstracts Service, Columbus, Ohio, USA; XP002795547
- "AKos Out of Stock Catalog", 4 May 2015, AKOS-CONSULTING AND SOLUTIONS DEUTSCHLAND GMBH, Steinen
- IHOR ZAHANICH ET AL: "Phenoxymethyl 1,3-oxazoles and 1,2,4-oxadiazoles as potent and selective agonists of free fatty acid receptor 1 (GPR40)", BIOORGANIC & MEDICINAL CHEMISTRY LETTERS, vol. 25, no. 16, 1 August 2015 (2015-08-01), AMSTERDAM, NL, pages 3105 - 3111, XP055641161, ISSN: 0960-894X, DOI: 10.1016/j.bmcl.2015.06.018

## Description

The present invention relates to novel free fatty acid receptor (FFAR) agonists, in particular agonists of FFAR1 as defined in claim 1. The invention also relates to compounds as defined in claim 20 for use as FFAR agonists in the treatment and/or prevention of type 2 diabetes mellitus (T2DM) and/or prediabetic conditions.

The free fatty acid receptors are G-protein coupled receptors which bind free fatty acids [1]. Free fatty acid receptors have broad tissue distribution, e.g., they are expressed in mouth (possibly for sensing fatty taste), digestive system (as energy sensors, and eating sensors), pancreatic Beta cells (to sense feeding), and even in CNS (of yet unknown function). There are at least four different FFARs, each encoded by a separate gene (FFAR1, FFAR2, FFAR3, FFAR4). Preliminary findings suggest that FFAR2 and FFAR3 may interact to form a FFAR2-FFAR3 receptor heteromer. [2] Free fatty acid receptors (FFA, nomenclature as agreed by the NC-IUPHAR Subcommittee on free fatty acid receptors [3,4]) are activated by free fatty acids. Long-chain saturated and unsaturated fatty acids (C14.0 (myristic acid), C16:0 (palmitic acid), C18:1 (oleic acid), C18:2 (linoleic acid), C18:3, (α-linolenic acid), C20:4 (arachidonic acid), C20:5,n-3 (EPA) and C22:6,n-3 (docosahexaenoic acid)) activate FFAR1 [5, 6, 7] and FFAR4 receptors [8, 9, 10]. while short chain fatty acids (C2 (acetic acid), C3 (propanoic acid), C4 (butyric acid) and C5 (pentanoic acid)) activate FFAR2 [11, 12, 13] and FFAR3 [11, 12] receptors. The crystal structure for agonist bound FFAR1 has been described in [14].

FFAR1 is also known as GPR40, FFAR4 is known as GPR120.

Several known FFAR1 (GPR40) agonists are under development as Type 2 diabetic drugs. An example is Fasiglifam (TAK-875) is a highly potent GPR40 agonist (low nanomolar EC₅₀ on human GPR40) with marked selectivity over other FFA family receptors (i.e., GPR120). It stimulates insulin secretion independently of blood glucose levels, which led to the expectation that TAK-875, in contrast to other anti-diabetic medicines, would not induce hypoglycemia, while also causing less weight gain [15]. Although very promising, the development of TAK-875 was terminated in 2013 due to (liver) toxicity issues in phase III clinical trials. Also the clinical development of other FFAR1 (GPR40) agonists such as LY2881835 [16] and AMG 837 [17] was stopped because of toxicity issues.

Several agonists for FFAR4 (GPR120), are undergoing preliminary development. In the search for compounds binding and activating FFAR4 (GPR120): for example, GW9508, initially identified as a GPR40 agonist, was shown to also moderately activate GPR120 [17]. However, the dual specificity of GW9508 for GPR40 and GPR120 represents a confounding variable in the interpretation of results in studies using GW9508 as a result of off-target effects at GPR40. Further research identified several other potential agonists for GPR120 including the plant-derived compound grifolic acid which acts as a partially selective GPR120 agonist [18], and NCG21 [19] as well as GSK-137647A [20], which are reported to be selective for GPR120. Recently, TUG891 has been made commercially available as a GPR120 agonist. TUG891 is reported to be potent and selective for GPR120 demonstrating greater selectivity and potency to GPR120 than GPR40 [21].

The broad tissue distribution of FFARs and their apparently diverse physiological functions in the body has resulted in the target class being investigated for several types of diseases. The FFAR family's broad tissue distribution and involvement in multiple physiological processes also mean that target selectivity within the FFAR family is a major issue to consider in the context of development of therapeutic molecules. FFAR1/GPR40 has emerged as a target for treatment of (T2DM). T2DM is a disease in which blood sugar homeostasis is regulated improperly by insulin. Insulin is secreted from pancreatic b cells in response to elevated plasma glucose, with several additional types of signals combining to modify its insulin secretion rate from pancreatic b cells. One of those signals comes from free fatty acids circulating in the bloodstream, which typically accompany elevated blood glucose following feeding. In 2003, it was demonstrated [6] that FFAR1/GPR40 is abundantly expressed on the surface of pancreatic beta cells and functions as a receptor for long-chain FFAs, and that these FFAs amplify glucose-stimulated insulin secretion. Agonism of FFAR1/GPR40 in pancreatic beta cells was demonstrated to amplify insulin secretion over a period spanning hours, and even full agonism of the receptor did not appear to result in secretion of insulin levels sufficient to drive hypoglycemia. Hypoglycemia means low blood glucose, and severe hypoglycemia occurs when the blood glucose level becomes so low that a patient is unable to maintain normal activity, and it can result in loss of consciousness and be life threatening (due to oxygen deprivation in the brain which function crucially depends on appropriate glucose supply). Critically, available insulin stimulation drugs for treating T2DM generally have the potential to result in inappropriately high insulin secretion and trigger severe hypoglycemia. Since severe hypoglycemia cannot be triggered by agonizing GPR40, GPR40 agonists therefore have a high potential for the treatment of T2DM.

Zahanich et al. (Bioorganic & Medicinal Chemistry Letters Vol. 25, No. 16, 2015) disclose certain phenoxymethyl 1,3-oxazoles and 1,2,4-oxadiazoles as agonists of FFAR1.

The technical problem underlying the present invention is to provide agonists of FFARs, in particular agonists of FFAR1.

The above technical problem is provided by the embodiments of the present invention as characterized in the claims, the present description and the drawings.

In particular, the present invention provides a compound of general formula (I)

R¹-S-CH₂-OXA-R² (I)

including enantiomers, diastereomers, hydrates, solvates, pharmaceutically acceptable cocrystals or salts, and complexes thereof;
wherein
   OXA is 1,3-oxazolyl;
   the group R¹-S-CH₂ is bound to C² of the 1,3-oxazolyl and the group R² is bound to C⁴ of the 1,3-oxazolyl;
   R¹ is a 6 membered heteroaryl group selected from the group consisting of 1,3,5-triazinyl and pyrimidinyl being independently substituted with one or more substituents selected from the group consisting of hydroxyl, amino, C₁-C₆-alkyl, C₃-C₆-cycloalkyl, C₁-C₄-alkoxy, N-mono- or N,N-di-substituted C₁-C₃-alkylamino, non-aromatic 5- to 6-membered heterocyclyl, 6-membered aryl and 5- to 6-membered heteroaryl which substituents may be unsubstituted or substituted with one or more groups selected from the group consisting of halide, cyano and C₁-C₆-alkyl, wherein the 6-membered aryl and 5- to 6-membered heteroaryl group, respectively, may be fused to said 1,3,5-triazinyl or pyrimidyl group, respectively, and
   R² is phenyl being unsubstituted or being substituted with one or more substituents selected from the group consisting of halide, cyano, amino, C₁-C₆-alkyl, C₃-C₆-cycloalkyl which may be optionally substituted with one or more halides, C₁-C₄-alkoxy which may optionally substituted with one or more halides, hydroxy-C₁-C₆-alkyl, sulfonyl-C₁-C₆-alkyl, sulphamidyl-N-C₁-C6-alkyl and carboxamidyl-N-mono- or - N,N-di-C₁-C₆-alkyl;
   with the proviso that the following compounds are excluded:

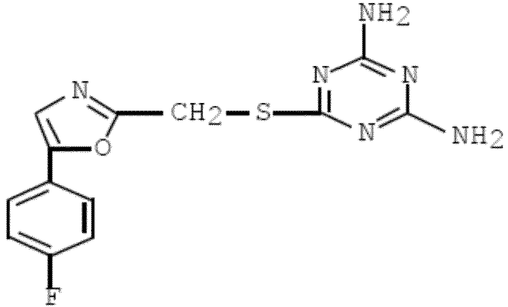

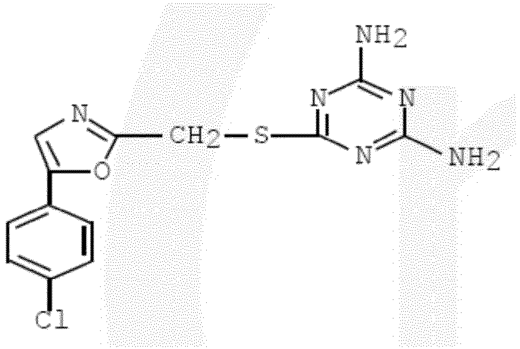

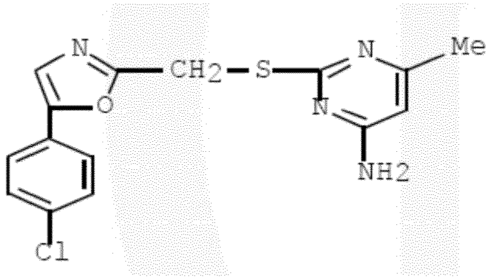

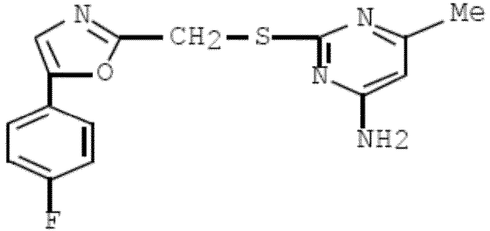

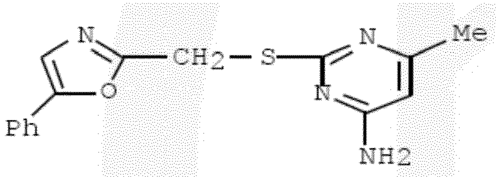

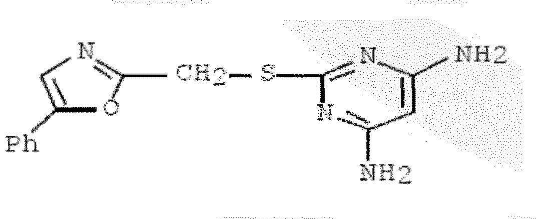

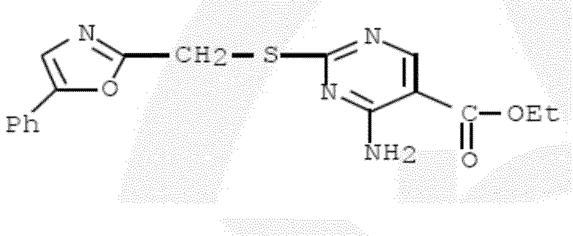

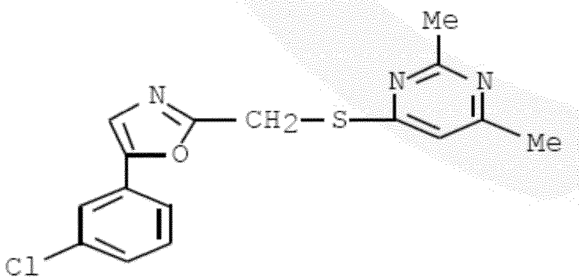

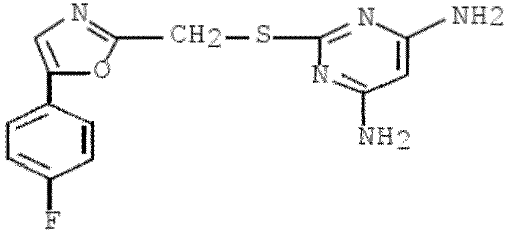

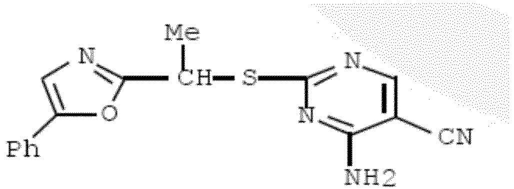

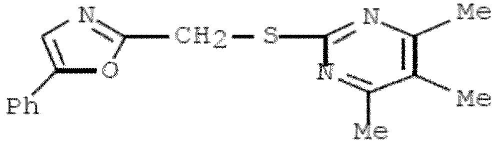

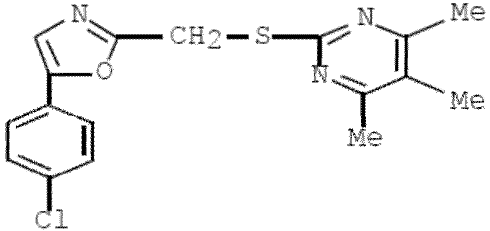

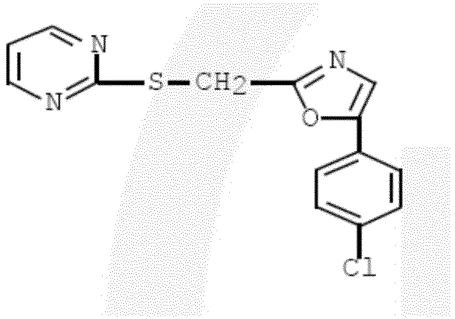

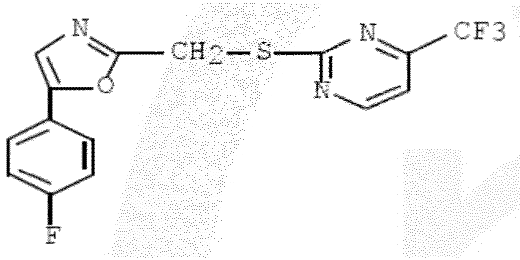

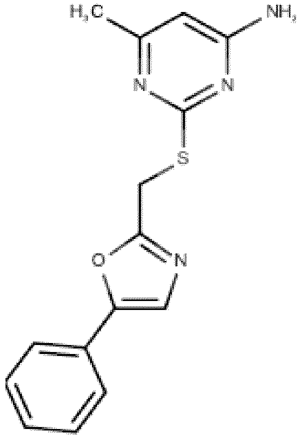

Preferred halide substituents of the invention are selected from CI, Br and F.

In the context of the present invention, the "C₁-C₆ alkyl (group)" means, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbuty, or the like. C₁-C₅₋alkyl groups, more preferably those mentioned before, are preferred. It is also to be understood that the above examples and preferred embodiments of "C₁-C₆ alkyl" also relate to substituents in which such ""C₁-C₆ alkyl" is present. Any alkyl group as referred to herein having more than 2 carbons may be a linear or branched chain.

In the context of the present invention, the "C₁-C₄-alkoxy (group)" means, for example, methoxy, ethoxy, propoxy, isopropoxy, butoxy, iso-butoxy, sec-butoxy, tert.-butoxy or the like. C₁-C₃₋alkoxy groups, more preferably those mentioned before, are preferred. Any alkoxy group as referred to herein having more than 2 carbons may be a linear or branched chain.

In the context of the present invention, a "N-mono-C₁-C₃-alkylamino (group)" means, for example, methylamino, ethylamino, propylamino, isopropylamino or the like.

In the context of the present invention, a "N,N-di-C₁-C₃-alkylamino (group)" means, for example, dimethylamino, diethylamino, dipropylamino, diisopropylamino, or the like. It is to be understood that the C₁-C₃-alkyl groups of the N,N-di-C₁-C₃-alkylamino (group) may also be different from one another.

In the context of the present invention, a "C₃-C₆-cycloalkyl (group)" means, for example, cyclopropyl, cyclobutyl, cyclopentyl, cyclohexyl or the like. C₃-C₅-cycloalkyl groups, more preferably those mentioned before, are preferred.

In the context of the present invention, the 6-membered aryl substituent of 1,2,4-triazine or pyrimidine in the definition of R² of general formula (I) means phenyl.

In the context of the present invention, examples of the "5- or 6-membered heteraryl (group)" means a 5- or 6-membered monocyclic aromatic heterocyclic group containing, as a ring-constituting atom besides carbon atoms, 1 to 4 hetero atoms selected from an oxygen atom, a sulfur atom (optionally oxidized) and a nitrogen atom (optionally oxidized). Examples thereof include furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), and the like.

In the context of the present invention, a "non-aromatic 5-to 6-membered heterocyclic group" means a 5- or 6-membered monocyclic non-aromatic heterocyclic group containing, as a ring-constituting atom besides carbon atoms, 1 to 4 hetero atoms selected from an oxygen atom, a sulfur atom (optionally oxidized) and a nitrogen atom (optionally oxidized). Examples thereof include azetidinyl (e.g., 1-azetidinyl, 2-azetidinyl), pyrrolidinyl (e.g., 1-pyrrolidinyl, 2-pyrrolidinyl), piperidyl (e.g., piperidino, 2-piperidyl, 3-piperidyl, 4-piperidyl), morpholinyl (e.g., morpholino), thiomorpholinyl (e.g., thiomorpholino), piperazinyl (e.g., 1-piperazinyl, 2-piperazinyl, 3-piperazinyl), oxazolidinyl (e.g., oxazolidin-2-yl), thiazolidinyl (e.g., thiazolidin-2-yl), dihydrothiopyranyl (e.g., dihydrothiopyran-3-yl, dihydrothiopyran-4-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), pyranyl (e.g., 2-pyranyl, 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), oxetanyl (e.g., oxetan-2-yl, oxetan-3-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl, dihydropyridyl (e.g., dihydropyridin-1-yl, dihydropyridin-2-yl, dihydropyridin-3-yl, dihydropyridin-4-yl), tetrahydropyridyl (e.g., 1,2,3,4-tetrahydropyridin-1-yl, 1,2,3,4-tetrahydropyridin-2-yl, 1,2,3,4-tetrahydropyridin-3-yl, 1,2,3,4-tetrahydropyridin-4-yl) and the like.

In preferred embodiments of the invention R¹ is 1,3,5-triazinyl substituted as defined above in general formula (I). It is further preferred that, if the 1,3,5-triazinyl group is substituted with more than one substituent, the substituents are different. According to further preferred embodiments, the 1,3,5 triazinyl group is independently substituted with one or two substituents selected from the group consisting of amino, methyl, ethyl, isopropyl and tert.-butyl, which may be in turn substituted with one or more halide, preferably Cl, F and/or Br..

According to particularly preferred compounds of the inventions R¹ is selected from the group consisting of 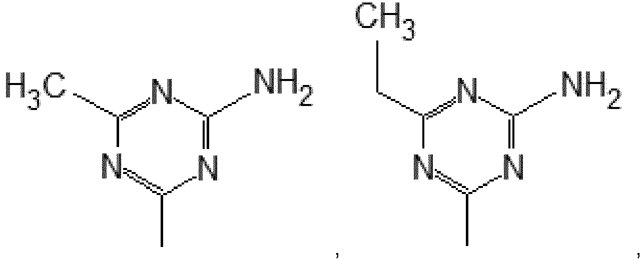 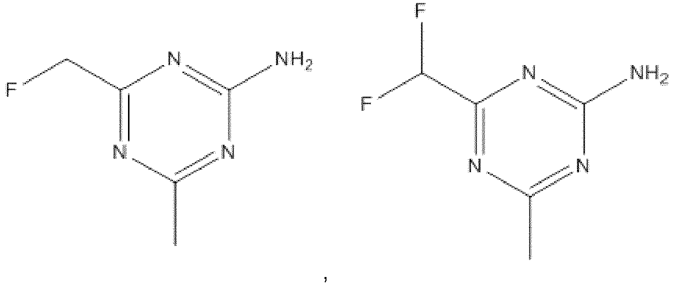 and

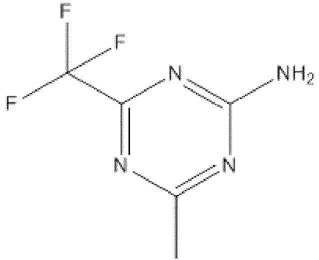

In other preferred embodiments of the invention, R¹ is pyrimidinyl substituted as defined above in general formula (I). It is further preferred that, if the pyrimidinyl group is substituted with more than one substituent, the substituents are different. According to further preferred embodiments the pyrimidinyl group is independently substituted by one or more substituents selected from the group consisting of amino (preferably mono- or di-substituted with methyl, more preferably methylamino), methyl and ethyl wherein the latter two groups are substituted with one or more halides as defined above.

According to particular preferred embodiments of the compound of the invention R¹ is selected from the group consisting of

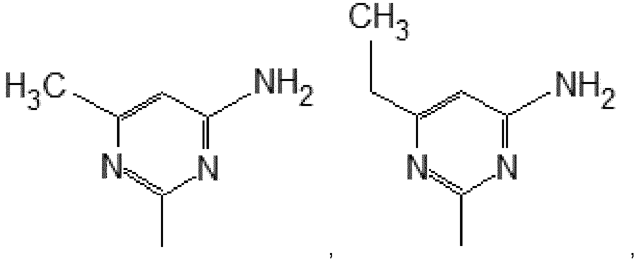

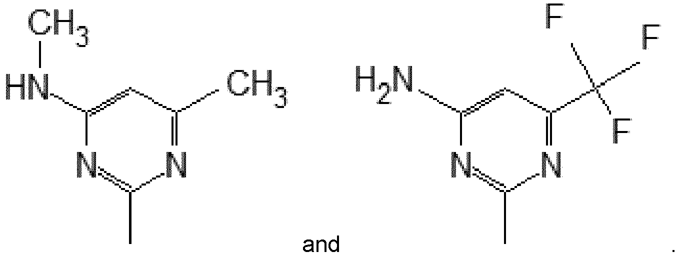

According to further preferred embodiments of the invention are compounds of general formula (I) wherein R² is independently substituted with one more substituents selected from the group consisting of Cl, Br, F, methyl, triflourmethyl, methoxy and ethoxy. Furthermore, it is preferred that R² is substituted in at least one meta position and/or at least one ortho position.

According to particularly preferred embodiments of the invention R² is selected from the group consisting of

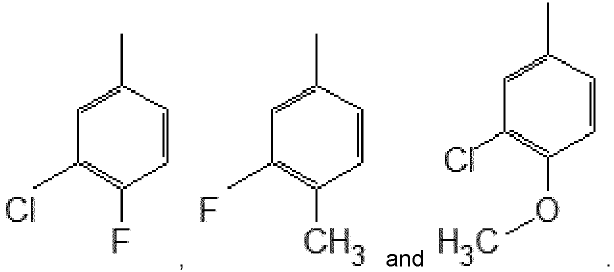

Highly active agonists of GPR40 according to the invention are compounds wherein R² is not substituted in the para position.

Particularly preferred groups for R² are selected from the group consisting of 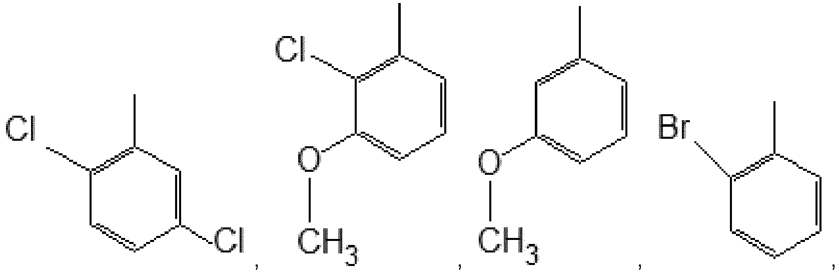 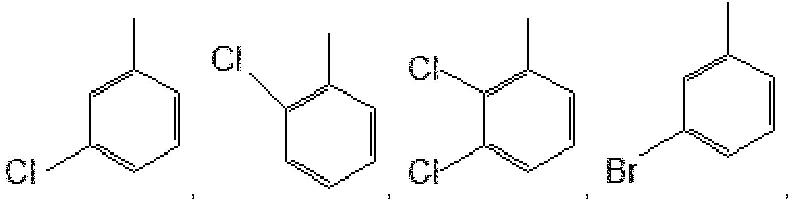 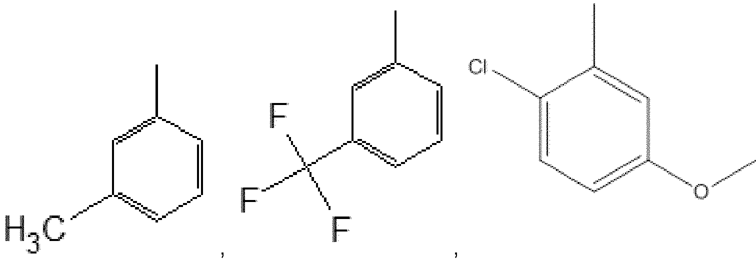 and

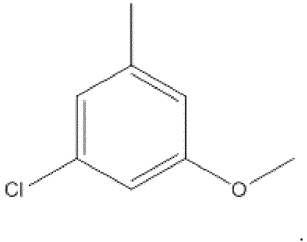

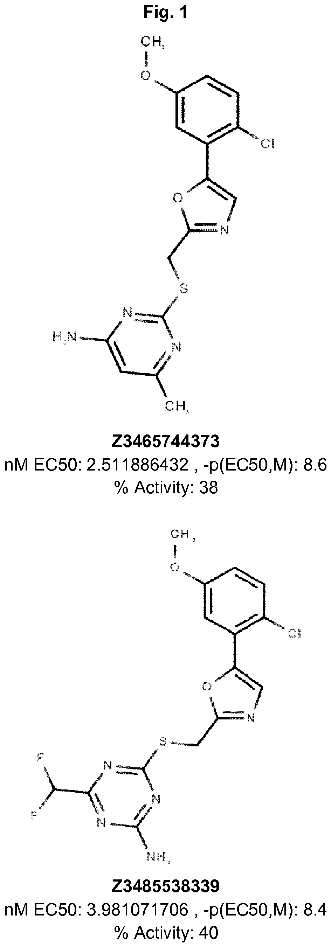

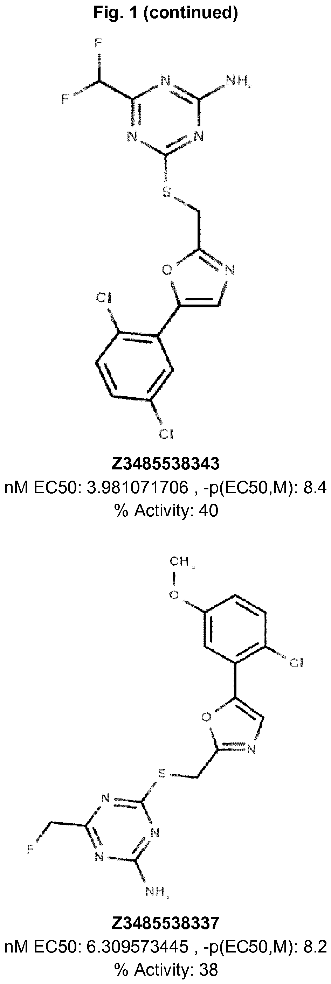

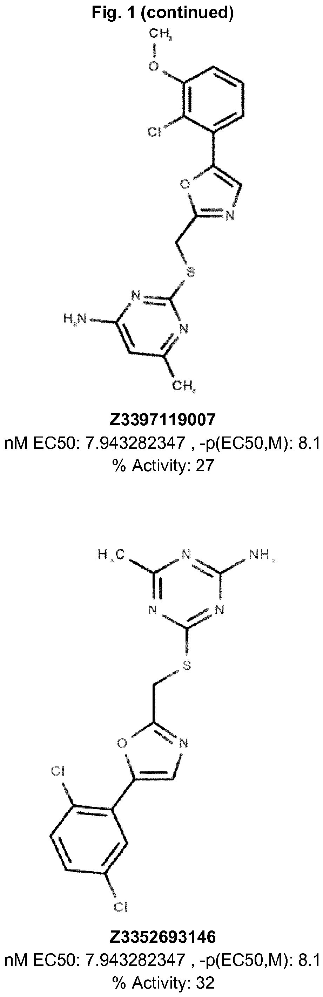

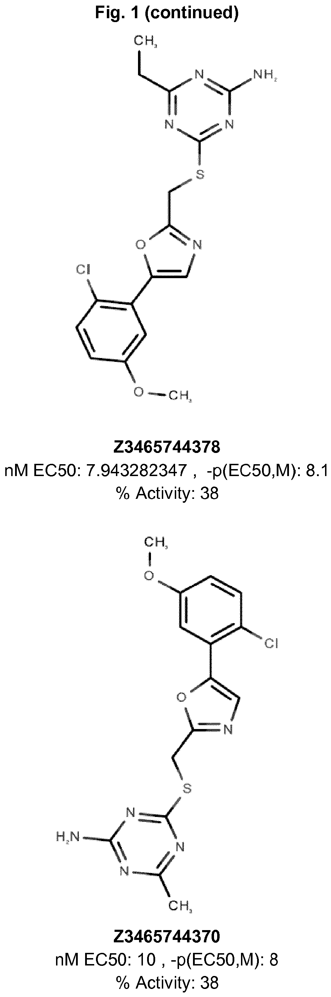

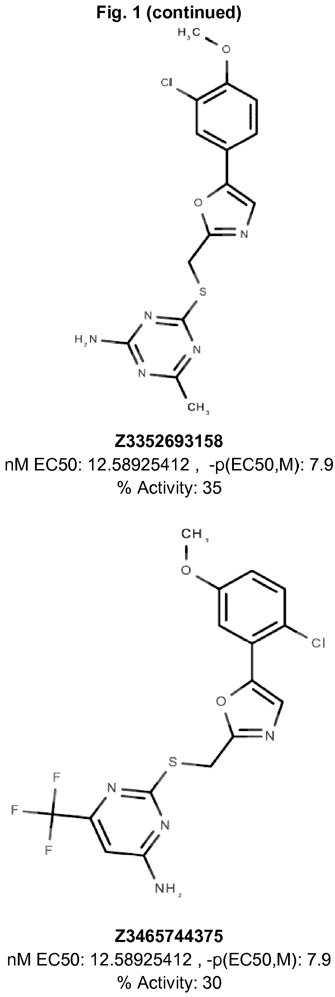

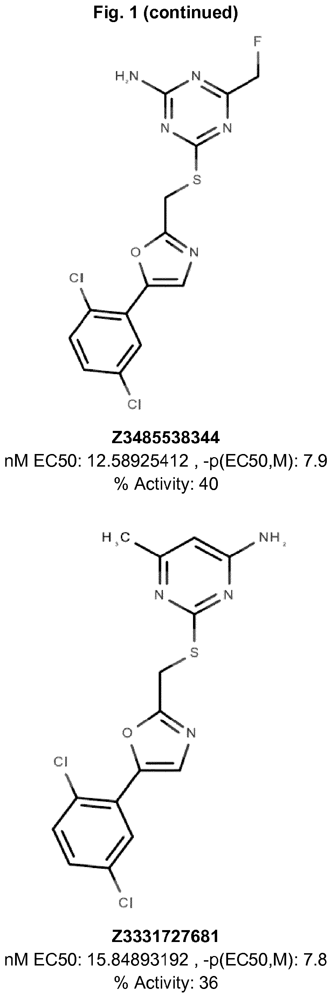

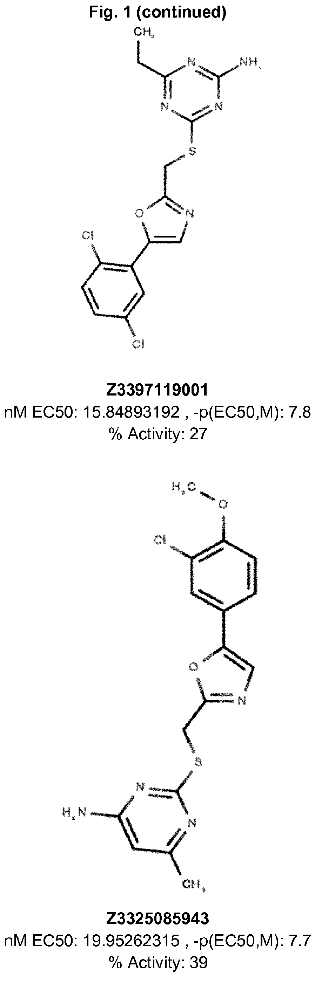

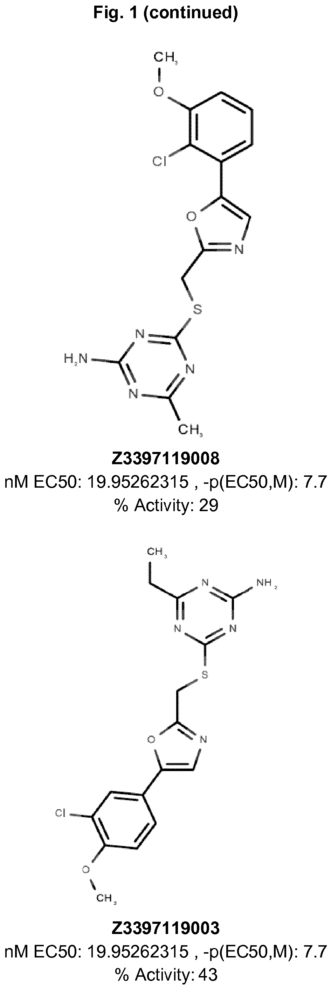

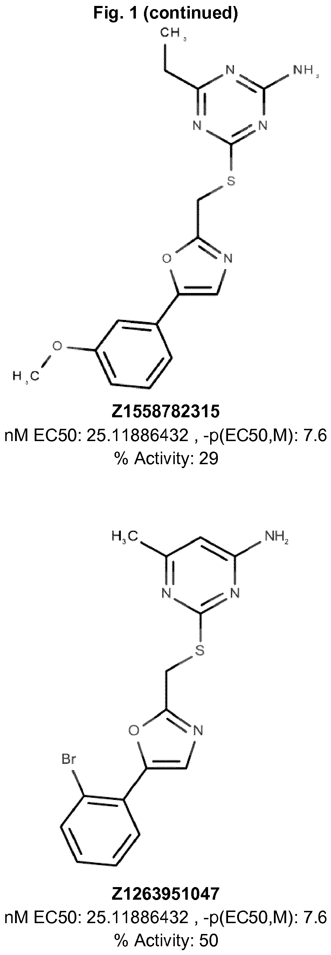

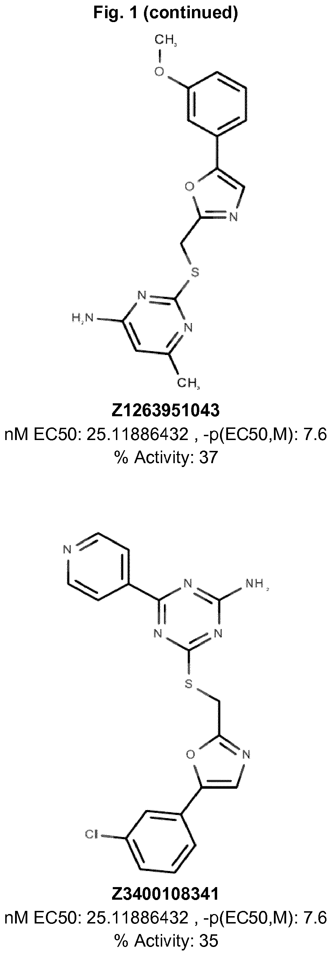

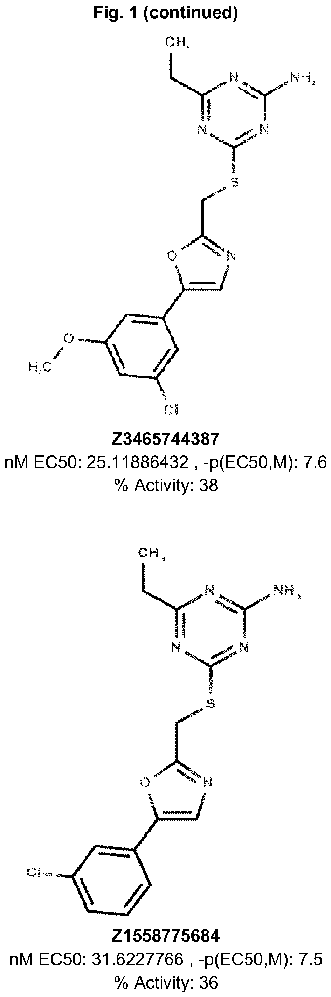

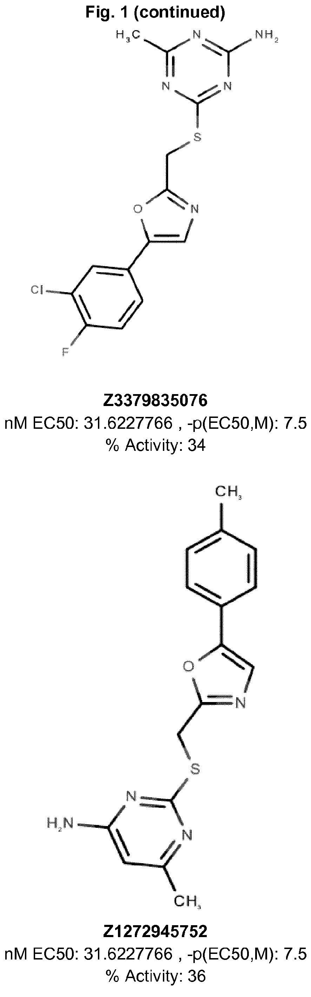

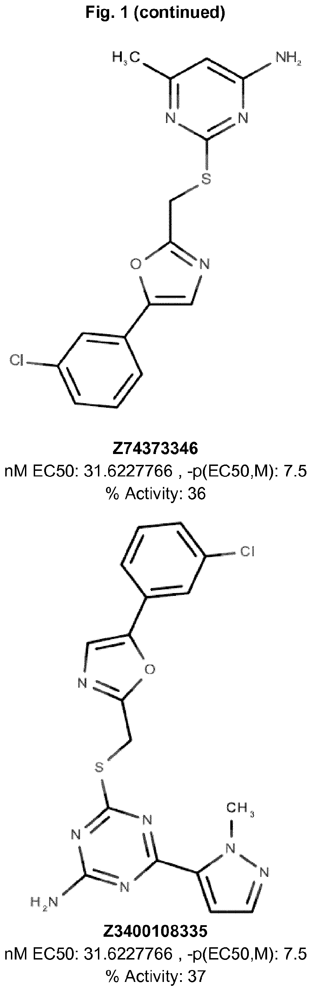

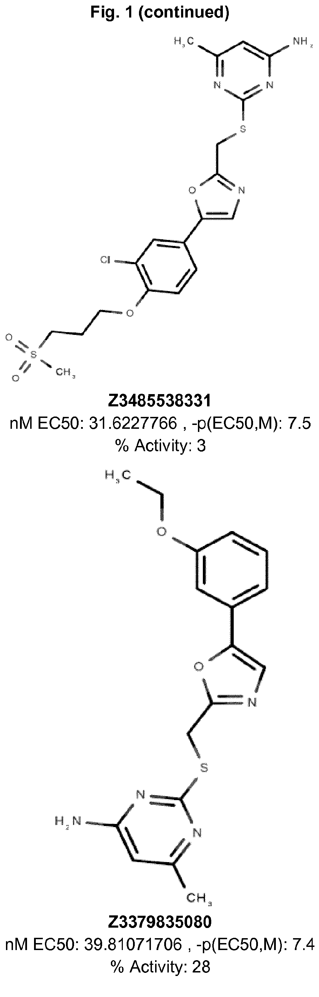

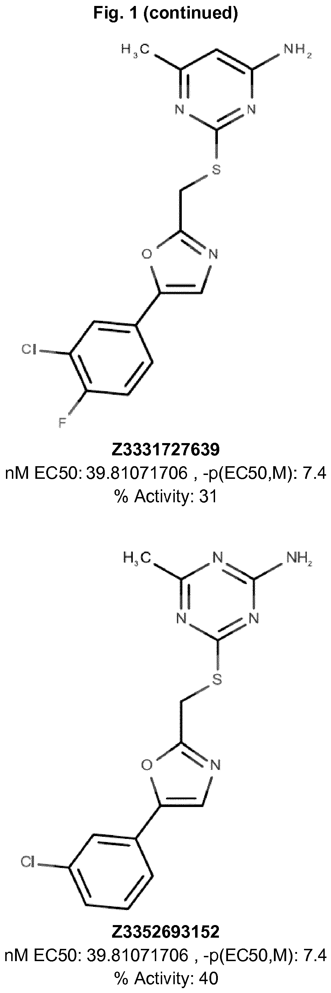

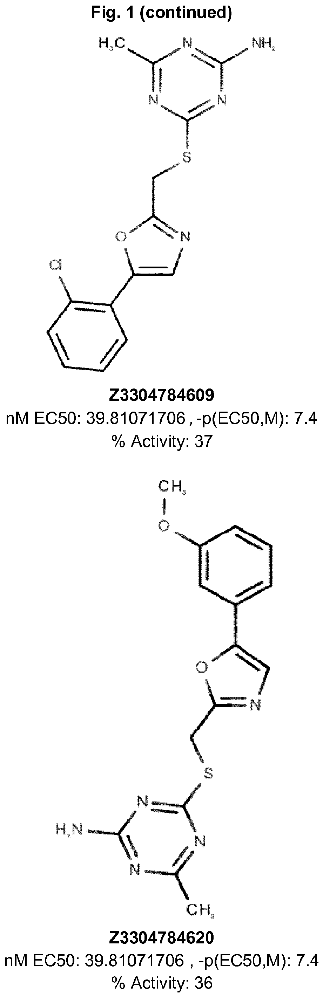

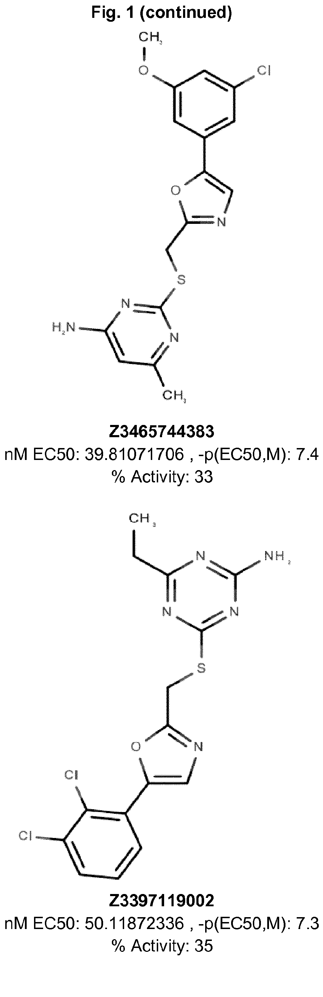

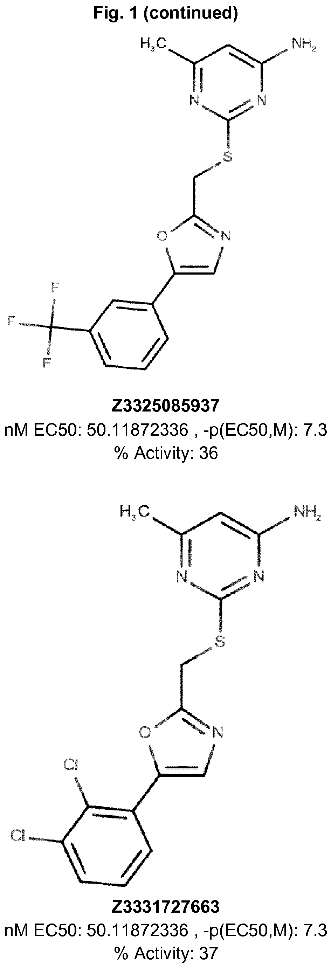

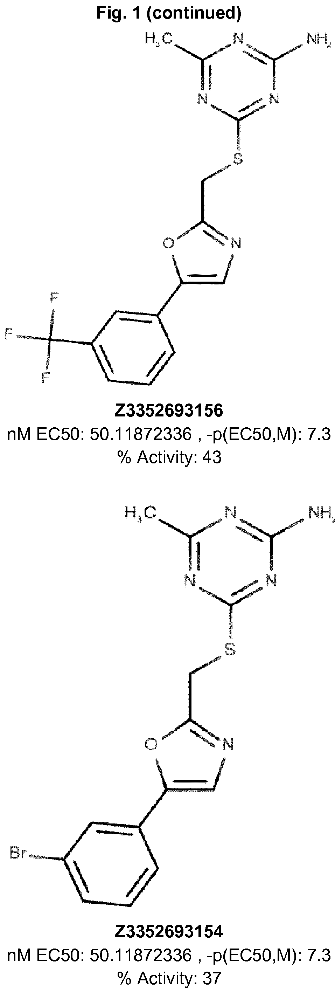

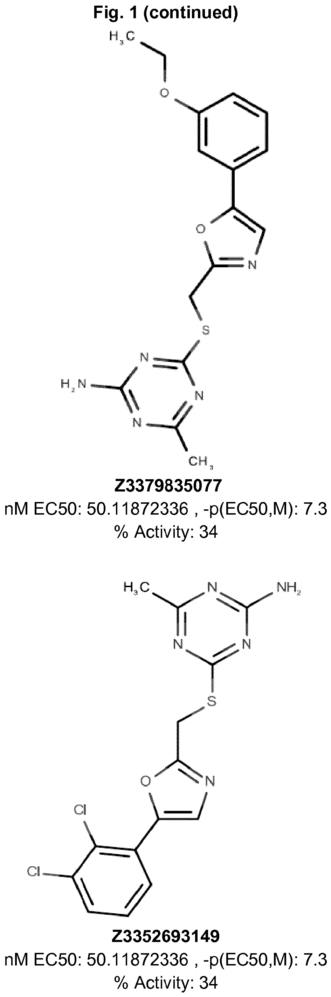

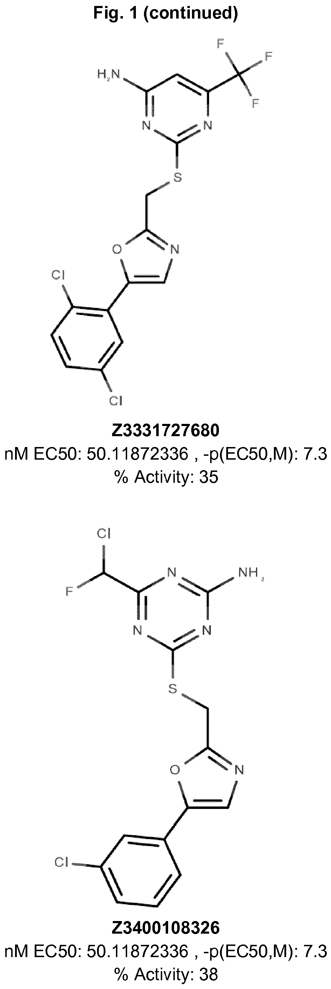

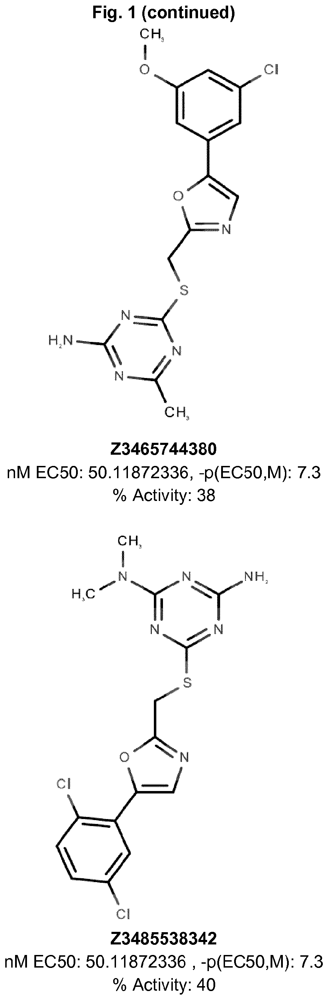

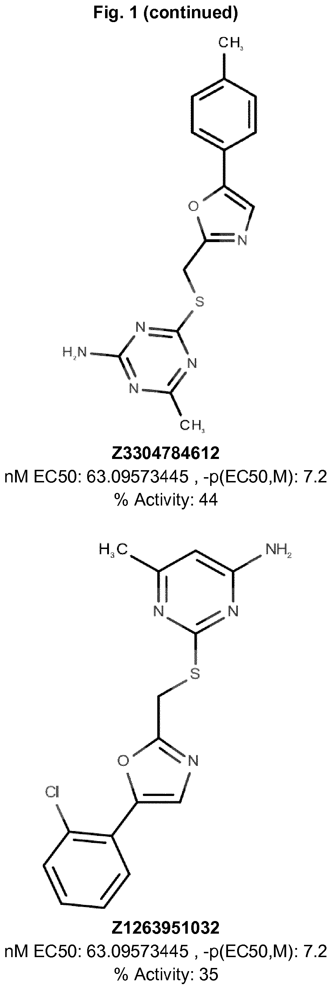

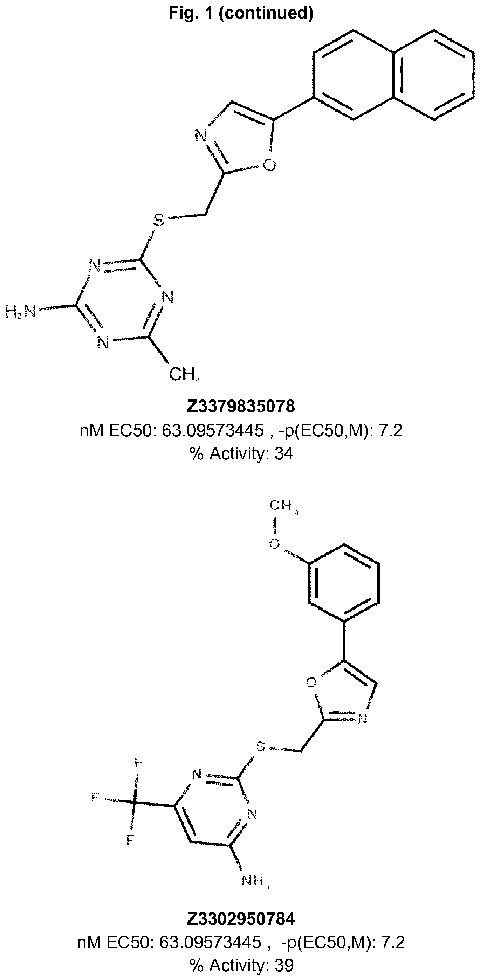

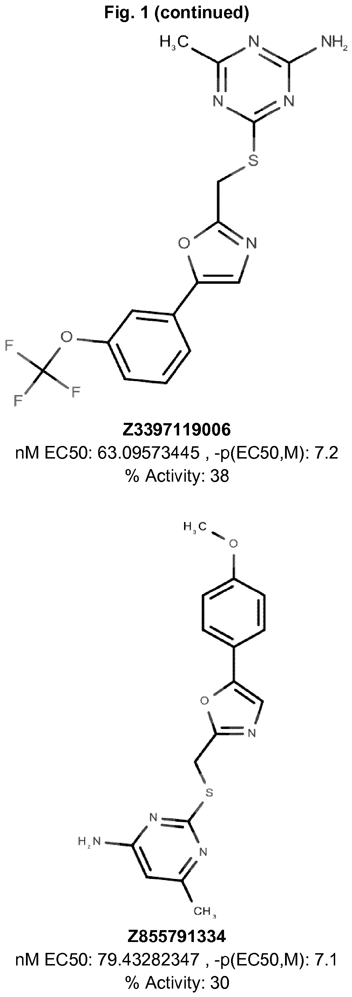

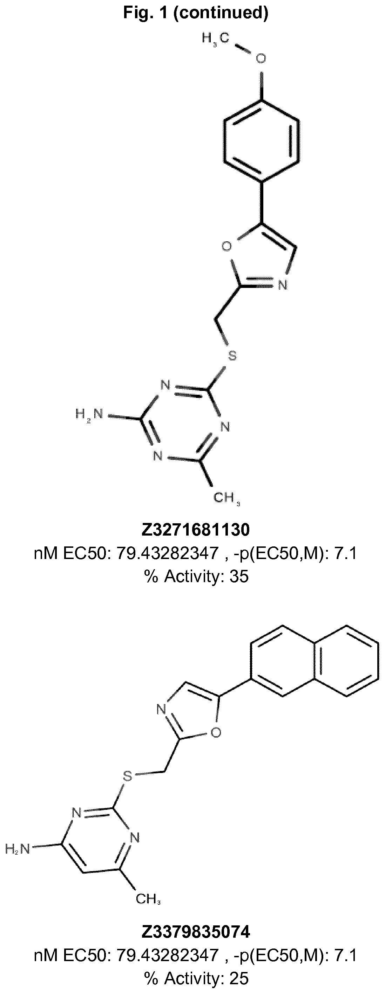

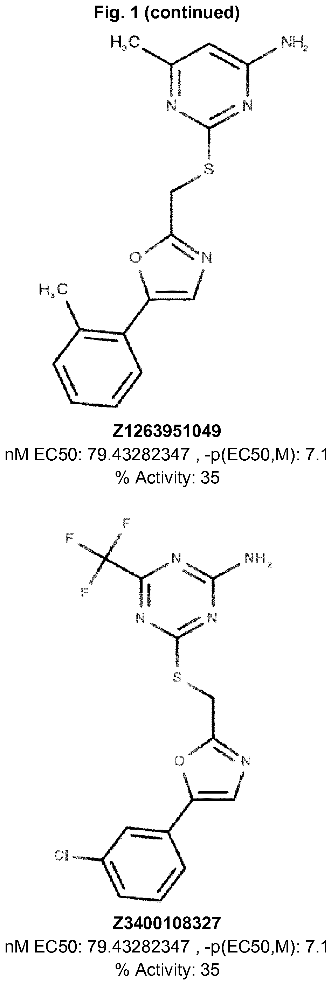

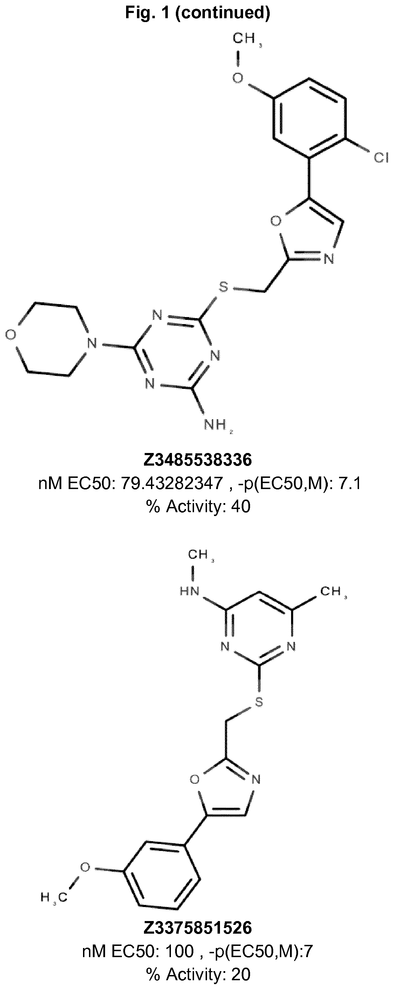

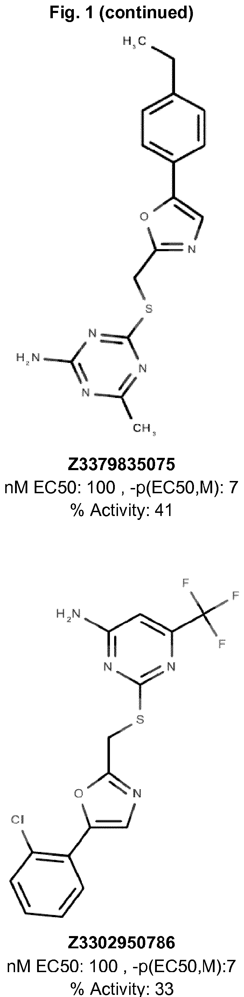

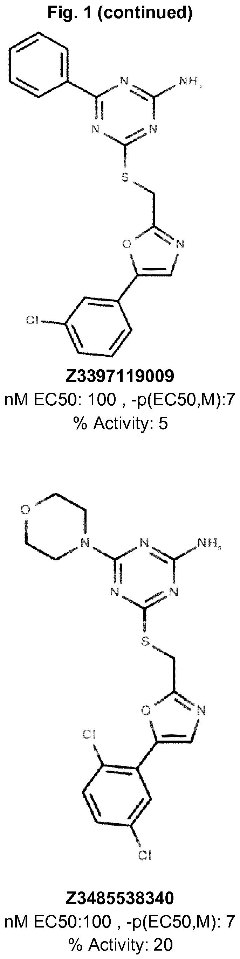

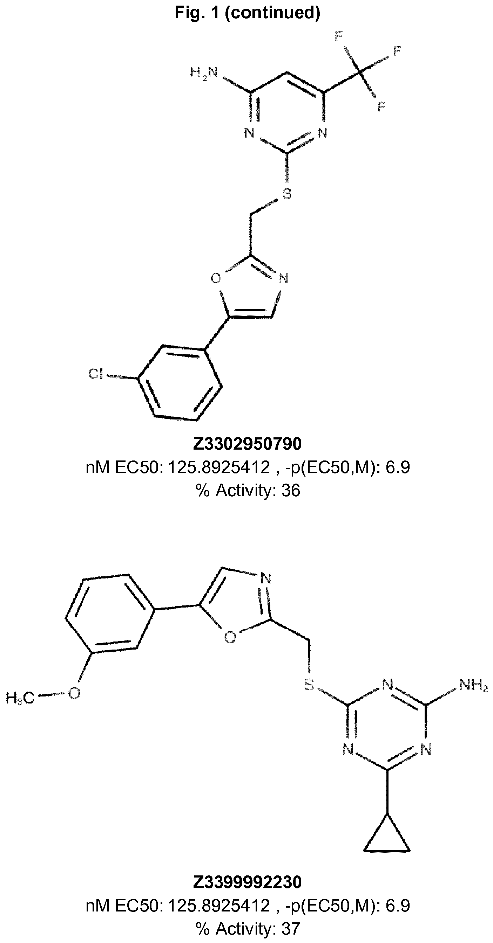

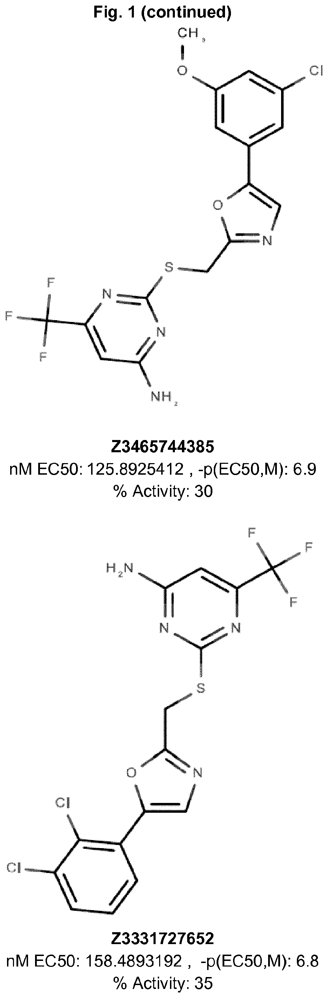

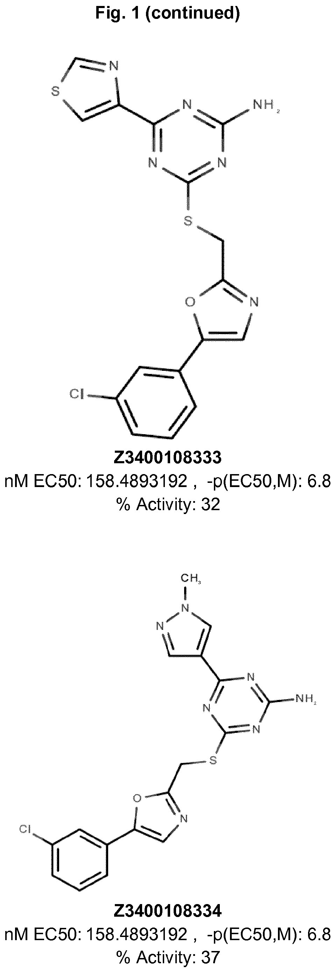

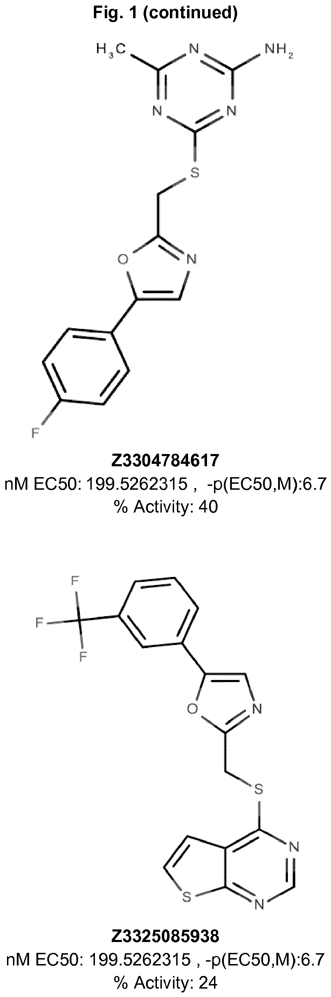

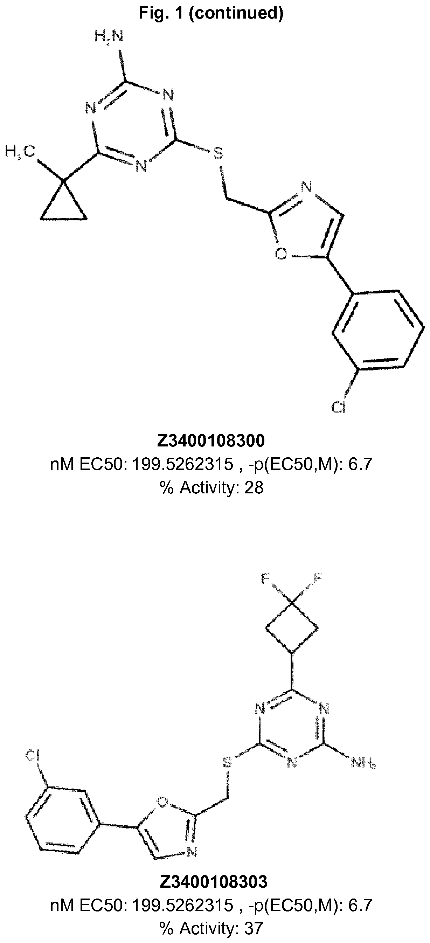

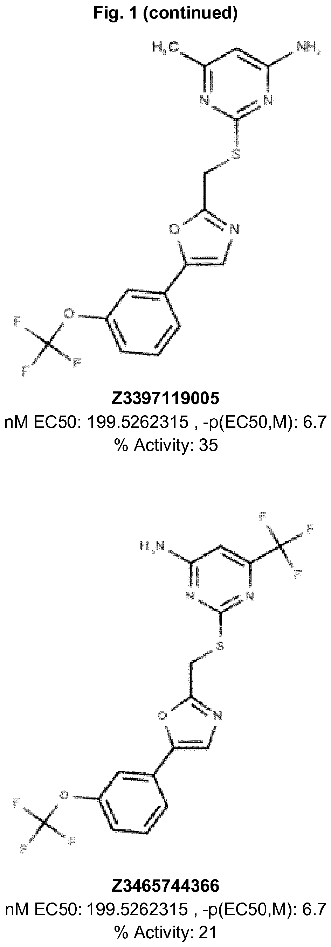

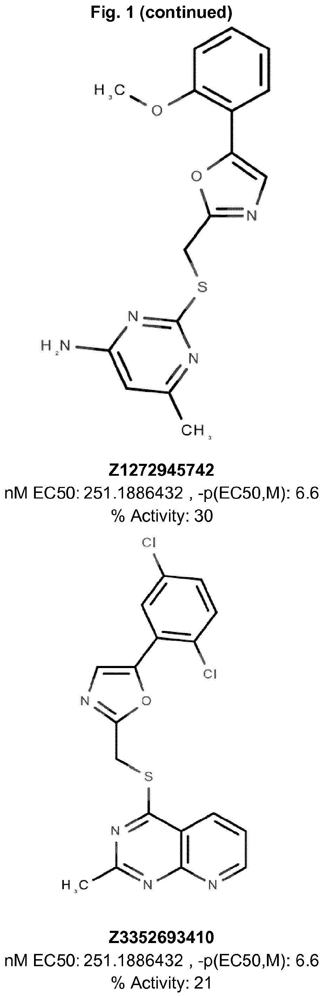

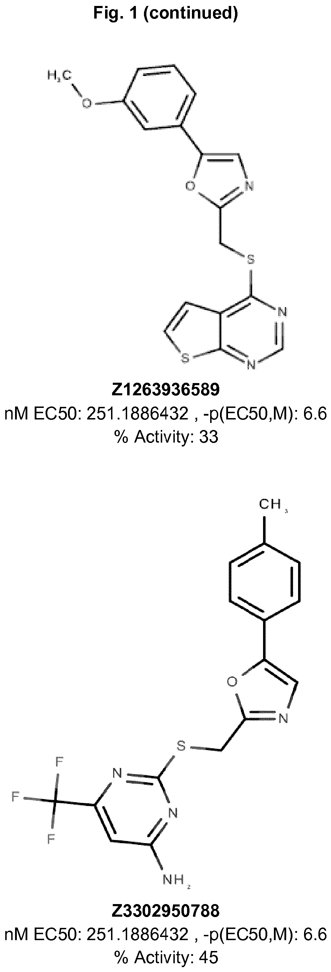

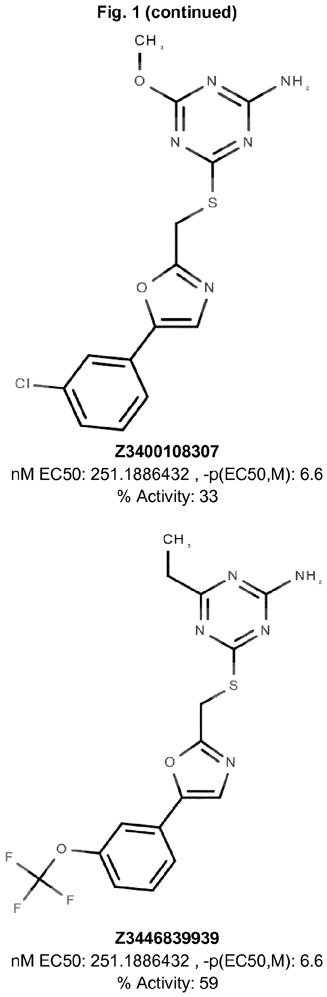

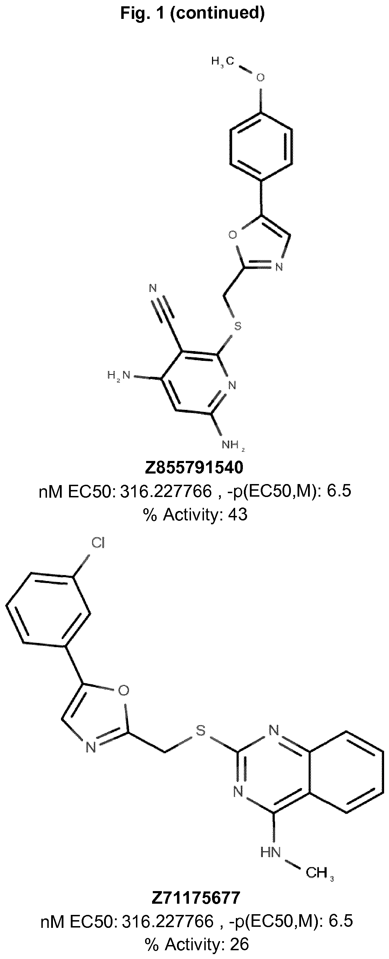

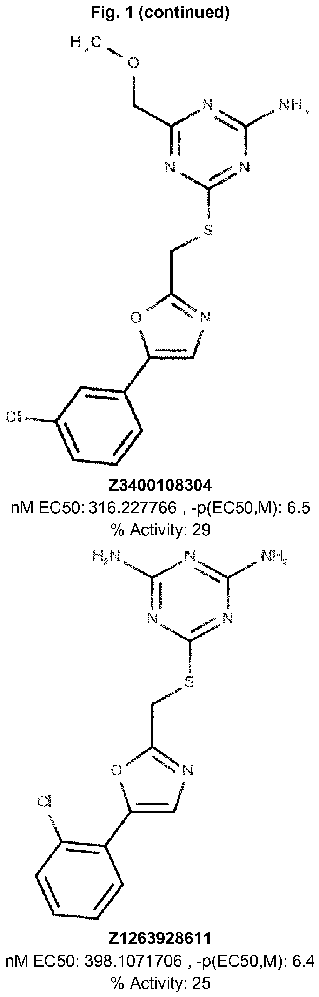

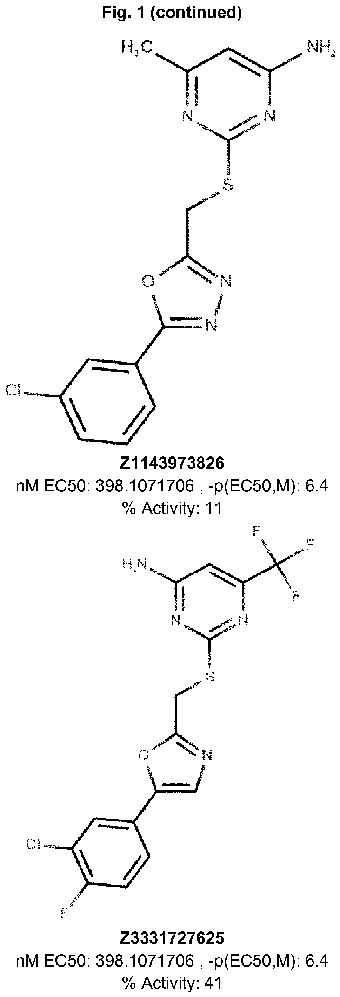

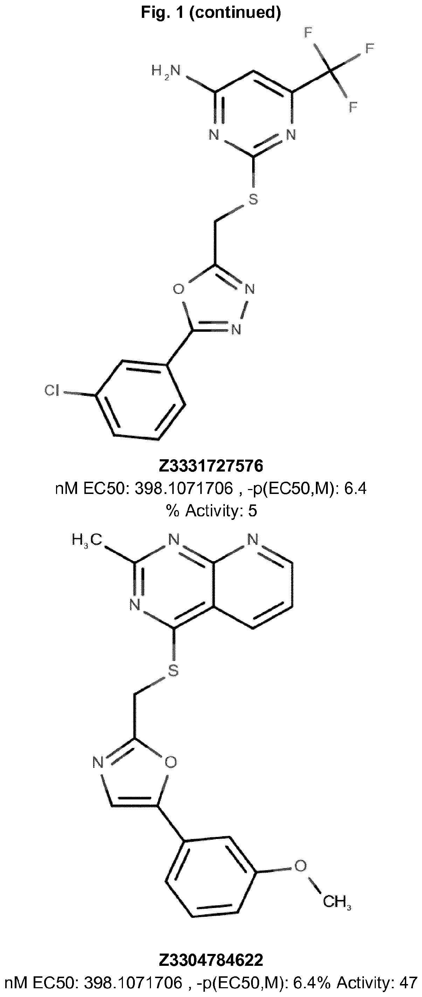

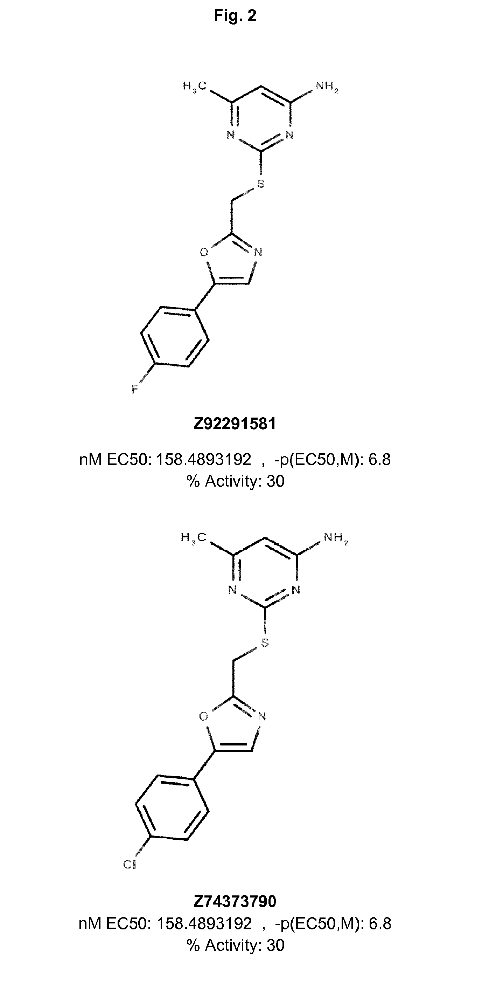

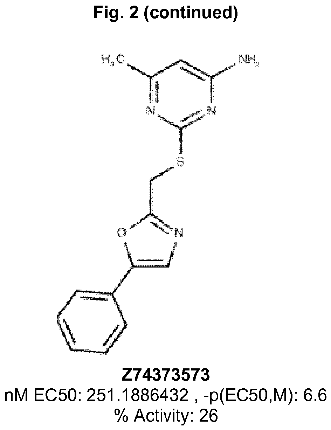

Highly preferred compounds of the invention are compounds shown in Fig. 1. Fig. 2 shows additional compounds specifically preferred for the use as medicaments, in particular for the uses according to the invention as further described below.

Particularly preferred compounds according to the invention are shown in the following Table 1 whereby it is to be understood that 1,2,4-oxadiazolyl and 1,3,4-oxadiazolyl, respectively, compounds presented in Table 1 are only disclosed as far as they relate to the compound for use according to the invention as defined in claim 20.

**Table 1:**

| **Compound ID** | **Name** |
|---|---|
| Z3352693410 | 5-(2,5-dichlorophenyl)-2-[({2-methylpyrido[2,3-d]pyrimidin-4-yl}sulfanyl)methyl]-1,3-oxazole |
| Z3331727678 | 2-({[5-(2,3-dichlorophenyl)-1,3-oxazol-2-yl]methyl}sulfanyl)-6-(trifluoromethyl)pyrimidin-4-ol |
| Z3331727680 | 2-({[5-(2,5-dichlorophenyl)-1,3-oxazol-2-yl]methyl}sulfanyl)-6-(trifluoromethyl)pyrimidin-4-amine |
| Z3304784611 | 5-(2-chlorophenyl)-2-[({2-methylpyrido[2,3-d]pyrimidin-4-yl}sulfanyl)methyl]-1,3-oxazole |
| Z3304784618 | 4-({[5-(4-fluorophenyl)-1,3-oxazol-2-yl]methyl}sulfanyl)-1,3,5-triazin-2-amine |
| Z3304784621 | 4-({[5-(3-methoxyphenyl)-1,3-oxazol-2-yl]methyl}sulfanyl)-1,3,5-triazin-2-amine |
| Z3397119005 | 6-methyl-2-[({5-[3-(trifluoromethoxy)phenyl]-1,3-oxazol-2-yl}methyl)sulfanyl]pyrimidin-4-amine |
| Z3352693156 | 4-methyl-6-[({5-[3-(trifluoromethyl)phenyl]-1,3-oxazol-2-yl}methyl)sulfanyl]-1,3,5-triazin-2-amine |
| Z3331727682 | 2-({[5-(2,5-dichlorophenyl)-1,3-oxazol-2-yl]methyl}sulfanyl)-6-(trifluoromethyl)pyrimidin-4-ol |
| Z1263986196 | 2-({[5-(2-chlorophenyl)-1,3-oxazol-2-yl]methyl}sulfanyl)-6-(trifluoromethyl)pyrimidin-4-ol |
| Z3304784610 | 4-({[5-(2-chlorophenyl)-1,3-oxazol-2-yl]methyl}sulfanyl)-1,3,5-triazin-2-amine |
| Z3325085929 | 6-(difluoromethyl)-2-({[5-(3-phenylphenyl)-1,3-oxazol-2-yl]methyl}sulfanyl)pyrimidin-4-ol |
| Z3325085931 | 6-methyl-2-({[5-(3-phenylphenyl)-1,3-oxazol-2-yl]methyl}sulfanyl)pyrimidin-4-amine |
| Z1558782315 | 4-ethyl-6-({[5-(3-methoxyphenyl)-1,3-oxazol-2-yl]methyl}sulfanyl)-1,3,5-triazin-2-amine |
| Z3397119007 | 2-({[5-(2-chloro-3-methoxyphenyl)-1,3-oxazol-2-yl]methyl}sulfanyl)-6-methylpyrimidin-4-amine |
| Z3397119008 | 4-({[5-(2-chloro-3-methoxyphenyl)-1,3-oxazol-2-yl]methyl}sulfanyl)-6-methyl-1,3,5-triazin-2-amine |
| Z71175677 | 2-({[5-(3-chlorophenyl)-1,3-oxazol-2-yl]methyl}sulfanyl)-N-methylquinazolin-4-amine |
| Z74373205 | 2-({[5-(2-chlorophenyl)-1,3,4-oxadiazol-2-yl]methyl}sulfanyl)-6-methylpyrimidin-4-amine |
| Z1231014532 | 2-({[5-(3-chlorophenyl)-1,3,4-oxadiazol-2-yl]methyl}sulfanyl)-6-(trifluoromethyl)pyrimidin-4-ol |
| Z359955408 | 6-methyl-2-({[5-(4-methylphenyl)-1,3,4-oxadiazol-2-yl]methyl}sulfanyl)pyrimidin-4-amine |
| Z3331727647 | 2-({[5-(3-chloro-4-fluorophenyl)-1,3-oxazol-2-yl]methyl}sulfanyl)-6-(trifluoromethyl)pyrimidin-4-ol |
| Z1882973046 | 6-cyclopropyl-2-({[5-(4-methylphenyl)-1,3-oxazol-2-yl]methyl}sulfanyl)pyrimidin-4-ol |
| Z1263986208 | 2-({[5-(3-methoxyphenyl)-1,3-oxazol-2-yl]methyl}sulfanyl)-6-(trifluoromethyl)pyrimidin-4-ol |
| Z2169241286 | 2-({[5-(3-chlorophenyl)-1,3-oxazol-2-yl]methyl}sulfanyl)-6-(difluoromethyl)pyrimidin-4-ol |
| Z3304784619 | 5-(4-fluorophenyl)-2-[({2-methylpyrido[2,3-d]pyrimidin-4-yl}sulfanyl)methyl]-1,3-oxazole |
| Z3325085934 | 6-cyclopropyl-2-[({5-[3-(trifluoromethyl)phenyl]-1,3-oxazol-2-yl}methyl)sulfanyl]pyrimidin-4-ol |
| Z3325085938 | 2-({thieno[2,3-d]pyrimidin-4-ylsulfanyl}methyl)-5-[3-(trifluoromethyl)phenyl]-1,3-oxazole |
| Z3325085930 | 6-methyl-2-({[5-(3-phenylphenyl)-1,3-oxazol-2-yl]methyl}sulfanyl)pyrimidin-4-ol |
| Z3325085942 | 2-({[5-(3-chloro-4-methoxyphenyl)-1,3-oxazol-2-yl]methyl}sulfanyl)-6-methylpyrimidin-4-ol |
| Z3325085928 | 6-cyclopropyl-2-({[5-(3-phenylphenyl)-1,3-oxazol-2-yl]methyl}sulfanyl)pyrimidin-4-ol |
| Z3465744370 | 4-({[5-(2-chloro-5-methoxyphenyl)-1,3-oxazol-2-yl]methyl}sulfanyl)-6-methyl-1,3,5-triazin-2-amine |
| Z3465744375 | 2-({[5-(2-chloro-5-methoxyphenyl)-1,3-oxazol-2-yl]methyl}sulfanyl)-6-(trifluoromethyl)pyrimidin-4-amine |
| Z3446839939 | 4-ethyl-6-[({5-[3-(trifluoromethoxy)phenyl]-1,3-oxazol-2-yl}methyl)sulfanyl]-1,3,5-triazin-2-amine |
| Z3397119003 | 4-({[5-(3-chloro-4-methoxyphenyl)-1,3-oxazol-2-yl]methyl}sulfanyl)-6-ethyl-1,3,5-triazin-2-amine |
| Z3352693152 | 4-({[5-(3-chlorophenyl)-1,3-oxazol-2-yl]methyl}sulfanyl)-6-methyl-1,3,5-triazin-2-amine |
| Z3352693158 | 4-({[5-(3-chloro-4-methoxyphenyl)-1,3-oxazol-2-yl]methyl}sulfanyl)-6-methyl-1,3,5-triazin-2-amine |
| Z3352693149 | 4-({[5-(2,3-dichlorophenyl)-1,3-oxazol-2-yl]methyl}sulfanyl)-6-methyl-1,3,5-triazin-2-amine |
| Z1143973826 | 2-({[5-(3-chlorophenyl)-1,3,4-oxadiazol-2-yl]methyl}sulfanyl)-6-methylpyrimidin-4-amine |
| Z215331784 | 2-({[5-(4-chlorophenyl)-1,3,4-oxadiazol-2-yl]methyl}sulfanyl)-6-methylpyrimidin-4-amine |
| Z1171240450 | 2-({[5-(3-chlorophenyl)-1,3-oxazol-2-yl]methyl}sulfanyl)-6-(trifluoromethyl)pyrimidin-4-ol |
| Z3272467650 | 6-tert-butyl-2-({[5-(4-methylphenyl)-1,3-oxazol-2-yl]methyl}sulfanyl)pyrimidin-4-ol |
| Z3302950788 | 2-({[5-(4-methylphenyl)-1,3-oxazol-2-yl]methyl}sulfanyl)-6-(trifluoromethyl)pyrimidin-4-amine |
| Z1607529540 | 2-({[5-(4-fluorophenyl)-1,3-oxazol-2-yl]methyl}sulfanyl)-5H,6H,7H-cyclopenta[d]pyrimidin-4-ol |
| Z3304784609 | 4-({[5-(2-chlorophenyl)-1,3-oxazol-2-yl]methyl}sulfanyl)-6-methyl-1,3,5-triazin-2-amine |
| Z3304784620 | 4-({[5-(3-methoxyphenyl)-1,3-oxazol-2-yl]methyl}sulfanyl)-6-methyl-1,3,5-triazin-2-amine |
| Z1607530768 | 2-({[5-(3-methoxyphenyl)-1,3-oxazol-2-yl]methyl}sulfanyl)-5H,6H,7H-cyclopenta[d]pyrimidin-4-ol |
| Z1607539139 | 2-({[5-(2-chlorophenyl)-1,3-oxazol-2-yl]methyl}sulfanyl)-5H,6H,7H-cyclopenta[d]pyrimidin-4-ol |
| Z1272915868 | 6-({[5-(4-methylphenyl)-1,3-oxazol-2-yl]methyl}sulfanyl)-1,3,5-triazine-2,4-diamine |
| Z3465744373 | 2-({[5-(2-chloro-5-methoxyphenyl)-1,3-oxazol-2-yl]methyl}sulfanyl)-6-methylpyrimidin-4-amine |
| Z1955117405 | 5-(3-chlorophenyl)-2-[({7-ethyl-[1,2,4]triazolo[4,3-c]pyrimidin-5-yl}sulfanyl)methyl]-1,3-oxazole |
| Z1272945711 | 2-({[5-(3-methoxyphenyl)-1,3,4-oxadiazol-2-yl]methyl}sulfanyl)-6-methylpyrimidin-4-amine |
| Z1272988076 | 2-({[5-(3-methoxyphenyl)-1,3,4-oxadiazol-2-yl]methyl}sulfanyl)-6-(trifluoromethyl)pyrimidin-4-ol |
| Z3331727556 | 2-({[5-(2-chlorophenyl)-1,3,4-oxadiazol-2-yl]methyl}sulfanyl)-6-(trifluoromethyl)pyrimidin-4-amine |
| Z3331727606 | 2-({[5-(3-methoxyphenyl)-1,3,4-oxadiazol-2-yl]methyl}sulfanyl)-6-(trifluoromethyl)pyrimidin-4-amine |
| Z3331727652 | 2-({[5-(2,3-dichlorophenyl)-1,3-oxazol-2-yl]methyl}sulfanyl)-6-(trifluoromethyl)pyrimidin-4-amine |
| Z3331727576 | 2-({[5-(3-chlorophenyl)-1,3,4-oxadiazol-2-yl]methyl}sulfanyl)-6-(trifluoromethyl)pyrimidin-4-amine |
| Z1171240167 | 2-({[5-(4-methylphenyl)-1,3,4-oxadiazol-2-yl]methyl}sulfanyl)-6-(trifluoromethyl)pyrimidin-4-ol |
| Z3272467647 | 6-tert-butyl-2-({[5-(2-chlorophenyl)-1,3-oxazol-2-yl]methyl}sulfanyl)pyrimidin-4-ol |
| Z3272467633 | 6-tert-butyl-2-({[5-(3-chlorophenyl)-1,3-oxazol-2-yl]methyl}sulfanyl)pyrimidin-4-ol |
| Z1882978995 | 6-cyclopropyl-2-({[5-(3-methoxyphenyl)-1,3-oxazol-2-yl]methyl}sulfanyl)pyrimidin-4-ol |
| Z1272925835 | 5-(4-methylphenyl)-2-({thieno[2,3-d]pyrimidin-4-ylsulfanyl}methyl)-1,3-oxazole |
| Z1263936576 | 5-(2-chlorophenyl)-2-({thieno[2,3-d]pyrimidin-4-ylsulfanyl}methyl)-1,3-oxazole |
| Z1607528727 | 2-({[5-(4-methylphenyl)-1,3-oxazol-2-yl]methyl}sulfanyl)-5H,6H,7H-cyclopenta[d]pyrimidin-4-ol |
| Z3304784617 | 4-({[5-(4-fluorophenyl)-1,3-oxazol-2-yl]methyl}sulfanyl)-6-methyl-1,3,5-triazin-2-amine |
| Z3325085936 | 6-methyl-2-[({5-[3-(trifluoromethyl)phenyl]-1,3-oxazol-2-yl}methyl)sulfanyl]pyrimidin-4-ol |
| Z3325085943 | 2-({[5-(3-chloro-4-methoxyphenyl)-1,3-oxazol-2-yl]methyl}sulfanyl)-6-methylpyrimidin-4-amine |
| Z3325085937 | 6-methyl-2-[({5-[3-(trifluoromethyl)phenyl]-1,3-oxazol-2-yl}methyl)sulfanyl]pyrimidin-4-amine |
| Z3465744378 | 4-({[5-(2-chloro-5-methoxyphenyl)-1,3-oxazol-2-yl]methyl}sulfanyl)-6-ethyl-1,3,5-triazin-2-amine |
| Z3465744385 | 2-({[5-(3-chloro-5-methoxyphenyl)-1,3-oxazol-2-yl]methyl}sulfanyl)-6-(trifluoromethyl)pyrimidin-4-amine |
| Z3397119002 | 4-({[5-(2,3-dichlorophenyl)-1,3-oxazol-2-yl]methyl}sulfanyl)-6-ethyl-1,3,5-triazin-2-amine |
| Z3331727663 | 2-({[5-(2,3-dichlorophenyl)-1,3-oxazol-2-yl]methyl}sulfanyl)-6-methylpyrimidin-4-amine |
| Z3331727527 | 2-({[5-(4-fluorophenyl)-1,3,4-oxadiazol-2-yl]methyl}sulfanyl)-6-(trifluoromethyl)pyrimidin-4-amine |
| Z74373206 | 2-({[5-(4-fluorophenyl)-1,3,4-oxadiazol-2-yl]methyl}sulfanyl)-6-methylpyrimidin-4-amine |
| Z3331727681 | 2-({[5-(2,5-dichlorophenyl)-1,3-oxazol-2-yl]methyl}sulfanyl)-6-methylpyrimidin-4-amine |
| Z3331727540 | 2-({[5-(4-chlorophenyl)-1,3,4-oxadiazol-2-yl]methyl}sulfanyl)-6-(trifluoromethyl)pyrimidin-4-amine |
| Z3304784614 | 4-({[5-(4-methylphenyl)-1,3-oxazol-2-yl]methyl}sulfanyl)-1,3,5-triazin-2-amine |
| Z213830254 | 6-({[5-(4-fluorophenyl)-1,3-oxazol-2-yl]methyl}sulfanyl)-1,3,5-triazine-2,4-diamine |
| Z3304784612 | 4-methyl-6-({[5-(4-methylphenyl)-1,3-oxazol-2-yl]methyl}sulfanyl)-1,3,5-triazin-2-amine |
| Z1263936589 | 5-(3-methoxyphenyl)-2-({thieno[2,3-d]pyrimidin-4-ylsulfanyl}methyl)-1,3-oxazole |
| Z3304784622 | 5-(3-methoxyphenyl)-2-[({2-methylpyrido[2,3-d]pyrimidin-4-yl}sulfanyl)methyl]-1,3-oxazole |
| Z3325085933 | 6-(trifluoromethyl)-2-[({5-[3-(trifluoromethyl)phenyl]-1,3-oxazol-2-yl}methyl)sulfanyl]pyrimidin-4-ol |
| Z3325085944 | 5-(3-chloro-4-methoxyphenyl)-2-({thieno[2,3-d]pyrimidin-4-ylsulfanyl}methyl)-1,3-oxazole |
| Z3465744380 | 4-({[5-(3-chloro-5-methoxyphenyl)-1,3-oxazol-2-yl]methyl}sulfanyl)-6-methyl-1,3,5-triazin-2-amine |
| Z3465744387 | 4-({[5-(3-chloro-5-methoxyphenyl)-1,3-oxazol-2-yl]methyl}sulfanyl)-6-ethyl-1,3,5-triazin-2-amine |
| Z3397119006 | 4-methyl-6-[({5-[3-(trifluoromethoxy)phenyl]-1,3-oxazol-2-yl}methyl)sulfanyl]-1,3,5-triazin-2-amine |
| Z3352693408 | 5-(3-bromophenyl)-2-[({2-methylpyrido[2,3-d]pyrimidin-4-yl}sulfanyl)methyl]-1,3-oxazole |
| Z3331727625 | 2-({[5-(3-chloro-4-fluorophenyl)-1,3-oxazol-2-yl]methyl}sulfanyl)-6-(trifluoromethyl)pyrimidin-4-amine |
| Z3331727639 | 2-({[5-(3-chloro-4-fluorophenyl)-1,3-oxazol-2-yl]methyl}sulfanyl)-6-methylpyrimidin-4-amine |
| Z1231014130 | 2-({[5-(4-fluorophenyl)-1,3,4-oxadiazol-2-yl]methyl}sulfanyl)-6-(trifluoromethyl)pyrimidin-4-ol |
| Z3272467645 | 6-tert-butyl-2-({[5-(3-methoxyphenyl)-1,3-oxazol-2-yl]methyl}sulfanyl)pyrimidin-4-ol |
| Z2169232902 | 6-(difluoromethyl)-2-({[5-(3-methoxyphenyl)-1,3-oxazol-2-yl]methyl}sulfanyl)pyrimidin-4-ol |
| Z2169235138 | 6-(difluoromethyl)-2-({[5-(4-methylphenyl)-1,3-oxazol-2-yl]methyl}sulfanyl)pyrimidin-4-ol |
| Z1272988122 | 2-({[5-(4-methylphenyl)-1,3-oxazol-2-yl]methyl}sulfanyl)-6-(trifluoromethyl)pyrimidin-4-ol |
| Z3302950790 | 2-({[5-(3-chlorophenyl)-1,3-oxazol-2-yl]methyl}sulfanyl)-6-(trifluoromethyl)pyrimidin-4-amine |
| Z3304784616 | 5-(4-methylphenyl)-2-[({2-methylpyrido[2,3-d]pyrimidin-4-yl}sulfanyl)methyl]-1,3-oxazole |
| Z1263928611 | 6-({[5-(2-chlorophenyl)-1,3-oxazol-2-yl]methyl}sulfanyl)-1,3,5-triazine-2,4-diamine |
| Z3325085940 | 2-({[5-(3-chloro-4-methoxyphenyl)-1,3-oxazol-2-yl]methyl}sulfanyl)-6-cyclopropylpyrimidin-4-ol |
| Z3325085932 | 5-(3-phenylphenyl)-2-({thieno[2,3-d]pyrimidin-4-ylsulfanyl}methyl)-1,3-oxazole |
| Z3325085939 | 2-({[5-(3-chloro-4-methoxyphenyl)-1,3-oxazol-2-yl]methyl}sulfanyl)-6-(trifluoromethyl)pyrimidin-4-ol |
| Z3325085927 | 2-({[5-(3-phenylphenyl)-1,3-oxazol-2-yl]methyl}sulfanyl)-6-(trifluoromethyl)pyrimidin-4-ol |
| Z3465744366 | 2-[({5-[3-(trifluoromethoxy)phenyl]-1,3-oxazol-2-yl}methyl) sulfanyl]-6-(trifluoromethyl)pyrimidin-4-amine |
| Z3397119004 | 5,6-diamino-2-({[5-(3-chlorophenyl)-1,3-oxazol-2-yl]methyl}sulfanyl)pyrimidin-4-ol |
| Z1558775684 | 4-({[5-(3-chlorophenyl)-1,3-oxazol-2-yl]methyl}sulfanyl)-6-ethyl-1,3,5-triazin-2-amine |
| Z3352693146 | 4-({[5-(2,5-dichlorophenyl)-1,3-oxazol-2-yl]methyl}sulfanyl)-6-methyl-1,3,5-triazin-2-amine |
| Z3352693154 | 4-({[5-(3-bromophenyl)-1,3-oxazol-2-yl]methyl}sulfanyl)-6-methyl-1,3,5-triazin-2-amine |
| Z1171239933 | 2-({[5-(2-chlorophenyl)-1,3,4-oxadiazol-2-yl]methyl}sulfanyl)-6-(trifluoromethyl)pyrimidin-4-ol |
| Z1171240290 | 2-({[5-(4-chlorophenyl)-1,3,4-oxadiazol-2-yl]methyl}sulfanyl)-6-(trifluoromethyl)pyrimidin-4-ol |
| Z3331727590 | 2-({[5-(4-methylphenyl)-1,3,4-oxadiazol-2-yl]methyl}sulfanyl)-6-(trifluoromethyl)pyrimidin-4-amine |
| Z2169238509 | 2-({[5-(2-chlorophenyl)-1,3-oxazol-2-yl]methyl}sulfanyl)-6-(difluoromethyl)pyrimidin-4-ol |
| Z1882973222 | 2-({[5-(2-chlorophenyl)-1,3-oxazol-2-yl]methyl}sulfanyl)-6-cyclopropylpyrimidin-4-ol |
| Z3302950786 | 2-({[5-(2-chlorophenyl)-1,3-oxazol-2-yl]methyl}sulfanyl)-6-(trifluoromethyl)pyrimidin-4-amine |
| Z3302950784 | 2-({[5-(3-methoxyphenyl)-1,3-oxazol-2-yl]methyl}sulfanyl)-6-(trifluoromethyl)pyrimidin-4-amine |
| Z3272467625 | 2-({[5-(3-chlorophenyl)-1,3-oxazol-2-yl]methyl}sulfanyl)-6-cyclopropylpyrimidin-4-ol |
| Z1263928626 | 6-({[5-(3-methoxyphenyl)-1, 3-oxazol-2-yl] methyl}sulfanyl)-1, 3, 5-triazi ne-2,4-diamine |
| Z92097091 | 5-(4-fluorophenyl)-2-({thieno[2,3-d]pyrimidin-4-ylsulfanyl}methyl)-1,3-oxazole |
| Z3325085935 | 6-(difluoromethyl)-2-[({5-[3-(trifluoromethyl)phenyl]-1,3-oxazol-2-yl}methyl)sulfanyl]pyrimidin-4-ol |
| Z3325085941 | 2-({[5-(3-chloro-4-methoxyphenyl)-1,3-oxazol-2-yl]methyl}sulfanyl)-6-(difluoromethyl)pyrimidin-4-ol |
| Z3465744383 | 2-({[5-(3-chloro-5-methoxyphenyl)-1,3-oxazol-2-yl]methyl}sulfanyl)-6-methylpyrimidin-4-amine |
| Z167994154 | 5-(1-benzofuran-2-yl)-3-{[5-(4-methoxyphenyl)-1,3,4-oxadiazol-2-yl]methyl}-5-methylimidazolidine-2,4-dione |
| Z153632018 | 6-methyl-2-{[(3-phenyl-1,2,4-oxadiazol-5-yl)methyl]sulfanyl}pyrimidin-4-amine |
| Z92291585 | 2-({[3-(4-ethylphenyl)-1,2,4-oxadiazol-5-yl]methyl}sulfanyl)-6-methylpyrimidin-4-amine |
| Z224417008 | 6-[({3-[3-(trifluoromethyl)phenyl]-1,2,4-oxadiazol-5-yl}methyl)sulfanyl]-1,3,5-triazine-2,4-diamine |
| Z74374081 | 2-({[3-(4-methoxyphenyl)-1,2,4-oxadiazol-5-yl]methyl}sulfanyl)-6-methylpyrimidin-4-amine |
| Z1029501714 | 2-({[3-(2H-1,3-benzodioxol-5-yl)-1,2,4-oxadiazol-5-yl]methyl}sulfanyl)-6-methylpyrimidin-4-amine |
| Z1198273125 | 6-methyl-2-{[2-(5-phenyl-1,3-oxazol-2-yl)ethyl]sulfanyl}pyrimidin-4-amine |
| Z1209503309 | 3-({[5-(4-methoxyphenyl)-1,3-oxazol-2-yl]methyl}sulfanyl)-6-methyl-[1,2,4]triazolo[4,3-b][1,2,4]triazin-7-ol |
| Z1231012460 | 2-({3-[3-(4-chlorophenyl)-1,2,4-oxadiazol-5-yl]propyl}sulfanyl)-6-(trifluoromethyl)pyrimidin-4-ol |
| Z1231014625 | 2-({[5-(4-methoxyphenyl)-1,3-oxazol-2-yl]methyl}sulfanyl)-6-(trifluoromethyl)pyrimidin-4-ol |
| Z1231014829 | 2-{[3-(3-phenyl-1,2,4-oxadiazol-5-yl)propyl]sulfanyl}-6-(trifluoromethyl)pyrimidin-4-ol |
| Z1231014839 | 2-({[5-(4-methoxyphenyl)-1,3,4-oxadiazol-2-yl]methyl}sulfanyl)-6-(trifluoromethyl)pyrimidin-4-ol |
| Z1231014954 | 2-{[2-(5-phenyl-1,3-oxazol-2-yl)ethyl]sulfanyl}-6-(trifluoromethyl)pyrimidin-4-ol |
| Z1263951032 | 2-({[5-(2-chlorophenyl)-1,3-oxazol-2-yl]methyl}sulfanyl)-6-methylpyrimidin-4-amine |
| Z1263951043 | 2-({[5-(3-methoxyphenyl)-1,3-oxazol-2-yl]methyl}sulfanyl)-6-methylpyrimidin-4-amine |
| Z1263951047 | 2-({[5-(2-bromophenyl)-1,3-oxazol-2-yl]methyl}sulfanyl)-6-methylpyrimidin-4-amine |
| Z1263951049 | 6-methyl-2-({[5-(2-methylphenyl)-1,3-oxazol-2-yl]methyl}sulfanyl)pyrimidin-4-amine |
| Z1272945742 | 2-({[5-(2-methoxyphenyl)-1,3-oxazol-2-yl]methyl}sulfanyl)-6-methylpyrimidin-4-amine |
| Z1272945752 | 6-methyl-2-({[5-(4-methylphenyl)-1,3-oxazol-2-yl]methyl}sulfanyl)pyrimidin-4-amine |
| Z153632138 | 2-({3-[3-(4-chlorophenyl)-1,2,4-oxadiazol-5-yl]propyl}sulfanyl)-6-methylpyrimidin-4-amine |
| Z1729866458 | 8-({[5-(4-methoxyphenyl)-1,3-oxazol-2-yl]methyl}sulfanyl)-7H-purine |
| Z1882969362 | 6-cyclopropyl-2-({[5-(4-methoxyphenyl)-1,3-oxazol-2-yl]methyl}sulfanyl)pyrimidin-4-ol |
| Z1882977097 | 6-cyclopropyl-2-({[5-(4-methoxyphenyl)-1,3,4-oxadiazol-2-yl]methyl}sulfanyl)pyrimidin-4-ol |
| Z2067312306 | 6-tert-butyl-2-({[5-(4-methoxyphenyl)-1,3,4-oxadiazol-2-yl]methyl}sulfanyl)pyrimidin-4-ol |
| Z213832006 | 6-({[5-(4-methoxyphenyl)-1,3,4-oxadiazol-2-yl]methyl}sulfanyl)-1,3,5-triazine-2,4-diamine |
| Z2169237989 | 6-(difluoromethyl)-2-({[5-(4-methoxyphenyl)-1,3,4-oxadiazol-2-yl]methyl}sulfanyl)pyrimidin-4-ol |
| Z2169243043 | 6-(difluoromethyl)-2-({[5-(4-methoxyphenyl)-1,3-oxazol-2-yl]methyl}sulfanyl)pyrimidin-4-ol |
| Z291790796 | 2-({[5-(4-methoxyphenyl)-1,3,4-oxadiazol-2-yl]methyl}sulfanyl)pyrimidin-4-ol |
| Z3271681128 | 4-({[5-(4-methoxyphenyl)-1,3-oxazol-2-yl]methyl}sulfanyl)-1,3,5-triazin-2-amine |
| Z3271681130 | 4-({[5-(4-methoxyphenyl)-1,3-oxazol-2-yl]methyl}sulfanyl)-6-methyl-1,3,5-triazin-2-amine |
| Z3271681135 | 2-({[5-(4-methoxyphenyl)-1,3-oxazol-2-yl]methyl}sulfanyl)-5-nitropyrimidin-4-amine |
| Z3271681140 | 4-hydroxy-2-({[5-(4-methoxyphenyl)-1,3-oxazol-2-yl]methyl}sulfanyl)pyrimidine-5-carbonitrile |
| Z3271681148 | 4-({[5-(4-methoxyphenyl)-1,3,4-oxadiazol-2-yl]methyl}sulfanyl)-1,3,5-triazin-2-amine |
| Z3271681150 | 4-({[5-(4-methoxyphenyl)-1,3,4-oxadiazol-2-yl]methyl}sulfanyl)-6-methyl-1,3,5-triazin-2-amine |
| Z3271681155 | 2-({[5-(4-methoxyphenyl)-1,3,4-oxadiazol-2-yl]methyl}sulfanyl)-5-nitropyrimidin-4-amine |
| Z3271681160 | 4-hydroxy-2-({[5-(4-methoxyphenyl)-1,3,4-oxadiazol-2-yl]methyl}sulfanyl)pyrimidine-5-carbonitrile |
| Z3271681169 | 6-tert-butyl-2-({[5-(4-methoxyphenyl)-1,3-oxazol-2-yl]methyl}sulfanyl)pyrimidin-4-ol |
| Z3271747780 | 2-({2-[3-(4-methylphenyl)-1,2,4-oxadiazol-5-yl]ethyl}sulfanyl)-6-(trifluoromethyl)pyrimidin-4-ol |
| Z3271747781 | 6-tert-butyl-2-({2-[3-(4-methylphenyl)-1,2,4-oxadiazol-5-yl]ethyl}sulfanyl)pyrimidin-4-ol |
| Z3271747785 | 6-tert-butyl-2-{[3-(3-phenyl-1,2,4-oxadiazol-5-yl)propyl]sulfanyl}pyrimidin-4-ol |
| Z3271747786 | 6-tert-butyl-2-({3-[3-(4-chlorophenyl)-1,2,4-oxadiazol-5-yl]propyl}sulfanyl)pyrimidin-4-ol |
| Z3271747787 | 6-tert-butyl-2-[(3-{3-[3-(trifluoromethyl)phenyl]-1,2,4-oxadiazol-5-yl}propyl)sulfanyl]pyrimidin-4-ol |
| Z3271747788 | 6-(trifluoromethyl)-2-[(3-{3-[3-(trifluoromethyl)phenyl]-1,2,4-oxadiazol-5-yl}propyl)sulfanyl]pyrimidin-4-ol |
| Z3271747791 | 3-({[5-(4-methoxyphenyl)-1,3-oxazol-2-yl]methyl}sulfanyl)-1,2,4-triazin-5-ol |
| Z3271747792 | 3-({[3-(4-methoxyphenyl)-1,2,4-oxadiazol-5-yl]methyl}sulfanyl)-1,2,4-triazin-5-ol |
| Z3271747795 | 5-(4-methoxyphenyl)-2-({7H-[1,2,4]triazolo[4,3-b][1,2,4]triazol-3-ylsulfanyl}methyl)-1,3-oxazole |
| Z3271747796 | 3-(4-methoxyphenyl)-5-({7H-[1,2,4]triazolo[4,3-b][1,2,4]triazol-3-ylsulfanyl}methyl)-1,2,4-oxadiazole |
| Z335843894 | 6-methyl-2-[(3-{3-[3-(trifluoromethyl)phenyl]-1,2,4-oxadiazol-5-yl}propyl)sulfanyl]pyrimidin-4-amine |
| Z3379835074 | 6-methyl-2-({[5-(naphthalen-2-yl)-1,3-oxazol-2-yl]methyl}sulfanyl)pyrimidin-4-amine |
| Z3379835075 | 4-({[5-(4-ethylphenyl)-1,3-oxazol-2-yl]methyl}sulfanyl)-6-methyl-1,3,5-triazin-2-amine |
| Z3379835076 | 4-({[5-(3-chloro-4-fluorophenyl)-1,3-oxazol-2-yl]methyl}sulfanyl)-6-methyl-1,3,5-triazin-2-amine |
| Z3379835077 | 4-({[5-(3-ethoxyphenyl)-1,3-oxazol-2-yl]methyl}sulfanyl)-6-methyl-1,3,5-triazin-2-amine |
| Z3379835078 | 4-methyl-6-({[5-(naphthalen-2-yl)-1,3-oxazol-2-yl]methyl}sulfanyl)-1,3,5-triazin-2-amine |
| Z3379835080 | 2-({[5-(3-ethoxyphenyl)-1,3-oxazol-2-yl]methyl}sulfanyl)-6-methylpyrimidin-4-amine |
| Z74373346 | 2-({[5-(3-chlorophenyl)-1,3-oxazol-2-yl]methyl}sulfanyl)-6-methylpyrimidin-4-amine |
| Z74373790 | 2-({[5-(4-chlorophenyl)-1,3-oxazol-2-yl]methyl}sulfanyl)-6-methylpyrimidin-4-amine |
| Z855789072 | 2-({[5-(4-methoxyphenyl)-1,3-oxazol-2-yl]methyl}sulfanyl)pyrimidin-4-ol |
| Z92291581 | 2-({[5-(4-fluorophenyl)-1,3-oxazol-2-yl]methyl}sulfanyl)-6-methylpyrimidin-4-amine |
| Z97827966 | 6-methyl-2-{[3-(3-phenyl-1,2,4-oxadiazol-5-yl)propyl]sulfanyl}pyrimidin-4-amine |
| Z1029494972 | 3-(2H-1,3-benzodioxol-5-yl)-5-{[(1-methyl-1H-imidazol-2-yl)sulfanyl]methyl}-1,2,4-oxadiazole |
| Z1029495534 | 2-({[3-(2H-1,3-benzodioxol-5-yl)-1,2,4-oxadiazol-5-yl]methyl}sulfanyl)-5-chloro-1,3-benzoxazole |
| Z1029496480 | 3-(2H-1,3-benzodioxol-5-yl)-5-({thieno[2,3-d]pyrimidin-4-ylsulfanyl}methyl)-1,2,4-oxadiazole |
| Z1029496618 | 4-[5-({[3-(2H-1,3-benzodioxol-5-yl)-1,2,4-oxadiazol-5-yl]methyl}sulfanyl)-1,3,4-oxadiazol-2-yl]pyridine |
| Z1029497206 | 2-({[3-(2H-1,3-benzodioxol-5-yl)-1,2,4-oxadiazol-5-yl]methyl}sulfanyl)-3-methyl-3,4-dihydroquinazolin-4-one |
| Z1029497420 | 2-({[3-(2H-1,3-benzodioxol-5-yl)-1,2,4-oxadiazol-5-yl]methyl}sulfanyl)-3-methyl-3H,4H-thieno[2,3-d]pyrimidin-4-one |
| Z1029502292 | 3-({[3-(2H-1,3-benzodioxol-5-yl)-1,2,4-oxadiazol-5-yl]methyl}sulfanyl)-5-(thiophen-2-yl)-1,2,4-triazine |
| Z1029503244 | 2-({[3-(2H-1,3-benzodioxol-5-yl)-1,2,4-oxadiazol-5-yl]methyl}sulfanyl)-5-(ethanesulfonyl)-1,3-benzoxazole |
| Z1029503342 | 2-({[3-(2H-1,3-benzodioxol-5-yl)-1,2,4-oxadiazol-5-yl]methyl}sulfanyl)-4-methyl-6-phenylpyrimidine |
| Z1029503770 | 2-({[3-(2H-1,3-benzodioxol-5-yl)-1,2,4-oxadiazol-5-yl]methyl}sulfanyl)pyridine-3-carbonitrile |
| Z1029506360 | 3-(2H-1,3-benzodioxol-5-yl)-5-{[(1-cyclopropyl-1H-imidazol-2-yl)sulfanyl]methyl}-1,2,4-oxadiazole |
| Z1029506602 | 3-(2H-1,3-benzodioxol-5-yl)-5-{[(4,5-dimethyl-1,3-oxazol-2-yl)sulfanyl]methyl}-1,2,4-oxadiazole |
| Z1029507470 | 2-({[3-(2H-1,3-benzodioxol-5-yl)-1,2,4-oxadiazol-5-yl]methyl}sulfanyl)-3-methylquinoxaline |
| Z1029507482 | 1-({[3-(2H-1,3-benzodioxol-5-yl)-1,2,4-oxadiazol-5-yl]methyl}sulfanyl)phthalazine |
| Z1029507712 | 2-({[3-(2H-1,3-benzodioxol-5-yl)-1,2,4-oxadiazol-5-yl]methyl}sulfanyl)-5,6-dimethylthieno[2,3-d]pyrimidin-4-amine |
| Z1029509832 | 4-({[3-(2H-1,3-benzodioxol-5-yl)-1,2,4-oxadiazol-5-yl]methyl}sulfanyl)-2,6-dimethylpyrimidine |
| Z1029509856 | 3-({[3-(2H-1,3-benzodioxol-5-yl)-1,2,4-oxadiazol-5-yl]methyl}sulfanyl)-5,6-dimethylpyridazine-4-carboxamide |
| Z1029510154 | 2-({[3-(2H-1,3-benzodioxol-5-yl)-1,2,4-oxadiazol-5-yl]methyl}sulfanyl)pyridine-4-carbonitrile |
| Z1031201354 | 2-({[3-(2H-1,3-benzodioxol-5-yl)-1,2,4-oxadiazol-5-yl]methyl}sulfanyl)-4,6-dimethylpyridine-3-carboxamide |
| Z1203023499 | 6-({[3-(2H-1,3-benzodioxol-5-yl)-1,2,4-oxadiazol-5-yl]methyl}sulfanyl)-9H-purine |
| Z1222487486 | 2-({[3-(2H-1,3-benzodioxol-5-yl)-1,2,4-oxadiazol-5-yl]methyl}sulfanyl)-5-chloro-7-methyl-1,3-benzoxazole |
| Z1346686154 | 3-(2H-1,3-benzodioxol-5-yl)-5-[(1H-1,2,3-triazol-5-ylsulfanyl)methyl]-1,2,4-oxadiazole |
| Z1607494891 | 2-({[3-(2H-1,3-benzodioxol-5-yl)-1,2,4-oxadiazol-5-yl]methyl}sulfanyl)-5,6-dimethylpyrimidin-4-ol |
| Z1651266851 | 3-(2H-1,3-benzodioxol-5-yl)-5-[({5-methyl-[1,3]oxazolo[4,5-b]pyridin-2-yl}sulfanyl)methyl]-1,2,4-oxadiazole |
| Z1696915398 | 6-methyl-2-{[(5-phenyl-1,3,4-thiadiazol-2-yl)methyl]sulfanyl}pyrimidin-4-amine |
| Z1715553861 | 2-({[3-(2H-1,3-benzodioxol-5-yl)-1,2,4-oxadiazol-5-yl]methyl}sulfanyl)-7-chloro-1,3-benzoxazole |
| Z1892324362 | 3-(2H-1,3-benzodioxol-5-yl)-5-[(1,3-oxazol-2-ylsulfanyl)methyl]-1,2,4-oxadiazole |
| Z2067308590 | 2-({[3-(2H-1,3-benzodioxol-5-yl)-1,2,4-oxadiazol-5-yl]methyl}sulfanyl)-6-tert-butylpyrimidin-4-ol |
| Z2199288132 | 3-(2H-1,3-benzodioxol-5-yl)-5-({1H,4H,5H,6H-cyclopenta[d]imidazol-2-ylsulfanyl}methyl)-1,2,4-oxadiazole |
| Z74373281 | 6-methyl-2-{[(5-phenyl-1,3,4-oxadiazol-2-yl)methyl]sulfanyl}pyrimidin-4-amine |
| Z74373573 | 6-methyl-2-{[(5-phenyl-1,3-oxazol-2-yl)methyl]sulfanyl}pyrimidin-4-amine |
| Z74373706 | 6-methyl-2-{[(2-phenyl-1,3-thiazol-5-yl)methyl]sulfanyl}pyrimidin-4-amine |

Further particularly preferred compounds of the invention are shown in the following Table 2:

**Table 2:**

| **Compound ID** | **Name** |
|---|---|
| Z3400108329 | 4-({[5-(3-chlorophenyl)-1,3-oxazol-2-yl]methyl}sulfanyl)-6-[difluoro(phenyl)methyl]-1,3,5-triazin-2-amine |
| Z3400108333 | 4-({[5-(3-chlorophenyl)-1,3-oxazol-2-yl]methyl}sulfanyl)-6-(1,3-thiazol-4-yl)-1,3,5-triazin-2-amine |
| Z3400108337 | 4-({[5-(3-chlorophenyl)-1,3-oxazol-2-yl]methyl}sulfanyl)-6-(1-methyl-1H-imidazol-2-yl)-1,3,5-triazin-2-amine |
| Z3400108303 | 4-({[5-(3-chlorophenyl)-1,3-oxazol-2-yl]methyl}sulfanyl)-6-(3,3-difluorocyclobutyl)-1,3,5-triazin-2-amine |
| Z3400108326 | 4-[chloro(fluoro)methyl]-6-({[5-(3-chlorophenyl)-1,3-oxazol-2-yl]methyl}sulfanyl)-1,3,5-triazin-2-amine |
| Z3400108331 | 4-({[5-(3-chlorophenyl)-1,3-oxazol-2-yl]methyl}sulfanyl)-6-(thiophen-3-yl)-1,3,5-triazin-2-amine |
| Z3400108302 | 4-({[5-(3-chlorophenyl)-1,3-oxazol-2-yl]methyl}sulfanyl)-6-(1-methoxycyclobutyl)-1,3,5-triazin-2-amine |
| Z3400108300 | 4-({[5-(3-chlorophenyl)-1,3-oxazol-2-yl]methyl}sulfanyl)-6-(1-methylcyclopropyl)-1,3,5-triazin-2-amine |
| Z3400108330 | 4-({[5-(3-chlorophenyl)-1,3-oxazol-2-yl]methyl}sulfanyl)-6-(thiophen-2-yl)-1,3,5-triazin-2-amine |
| Z3400108334 | 4-({[5-(3-chlorophenyl)-1,3-oxazol-2-yl]methyl}sulfanyl)-6-(1-methyl-1H-pyrazol-4-yl)-1,3,5-triazin-2-amine |
| Z3400108335 | 4-({[5-(3-chlorophenyl)-1,3-oxazol-2-yl]methyl}sulfanyl)-6-(1-methyl-1H-pyrazol-5-yl)-1,3,5-triazin-2-amine |
| Z3400108325 | 4-({[5-(3-chlorophenyl)-1,3-oxazol-2-yl]methyl}sulfanyl)-6-(2-methanesulfonylpropan-2-yl)-1,3,5-triazin-2-amine |
| Z3400108341 | 4-({[5-(3-chlorophenyl)-1,3-oxazol-2-yl]methyl}sulfanyl)-6-(pyridin-4-yl)-1,3,5-triazin-2-amine |
| Z3400108339 | 4-({[5-(3-chlorophenyl)-1,3-oxazol-2-yl]methyl}sulfanyl)-6-(pyridin-2-yl)-1,3,5-triazin-2-amine |
| Z3400108304 | 4-({[5-(3-chlorophenyl)-1,3-oxazol-2-yl]methyl}sulfanyl)-6-(methoxymethyl)-1,3,5-triazin-2-amine |
| Z3400108306 | 4-({[5-(3-chlorophenyl)-1,3-oxazol-2-yl]methyl}sulfanyl)-6-(oxolan-2-yl)-1,3,5-triazin-2-amine |
| Z3400108301 | 4-({[5-(3-chlorophenyl)-1,3-oxazol-2-yl]methyl}sulfanyl)-6-cyclobutyl-1,3,5-triazin-2-amine |

Further particularly preferred compounds for use according to the invention as defined in claim 20 are shown in the following Table 3:

**Table 3:**

| **Coumpound ID** | **Name** |
|---|---|
| Z642432840 | 2-{[5-(4-methylphenyl)-1,3,4-oxadiazol-2-yl]sulfanyl}-1,3-benzoxazole |
| Z1340746590 | 4-{[5-(1-benzofuran-2-yl)-1,3,4-oxadiazol-2-yl]sulfanyl}-6-methylpyrimidine |

Further particularly preferred compounds according to the invention are shown in the following Table 4 whereby it is to be understood that 1,2,4-oxadiazolyl compounds presented in Table 4 are only disclosed as far as they relate to the compound for use of claim 20:

**Table 4:**

| **Compound ID** | **Name** |
|---|---|
| Z855788832 | 6-({[5-(4-methoxyphenyl)-1,3-oxazol-2-yl]methyl}sulfanyl)-1,3,5-triazine-2,4-diamine |
| Z1029502468 | 4,6-diamino-2-({[3-(2H-1,3-benzodioxol-5-yl)-1,2,4-oxadiazol-5-yl]methyl}sulfanyl)pyridine-3-carbonitrile |

Further particularly preferred compounds according to the invention are shown in the following Table 5:

**Table 5:**

| **Compound ID** | **Name** |
|---|---|
| Z1029491270 | 2-({[3-(2H-1,3-benzodioxol-5-yl)-1,2,4-oxadiazol-5-yl]methyl}sulfanyl)-1-methyl-1H-1,3-benzodiazole |
| Z1029491492 | 4-({[3-(2H-1,3-benzodioxol-5-yl)-1,2,4-oxadiazol-5-yl]methyl}sulfanyl)-2-methylquinazoline |
| Z1029492422 | 2-({[3-(2H-1,3-benzodioxol-5-yl)-1,2,4-oxadiazol-5-yl]methyl}sulfanyl)-6-chloro-1,3-benzoxazole |
| Z1029493690 | 6-({[3-(2H-1,3-benzodioxol-5-yl)-1,2,4-oxadiazol-5-yl]methyl}sulfanyl)-1,3,5-triazine-2,4-diamine |
| Z1029494394 | 2-({[3-(2H-1,3-benzodioxol-5-yl)-1,2,4-oxadiazol-5-yl]methyl}sulfanyl)quinoline |
| Z1029494414 | 2-({[3-(2H-1,3-benzodioxol-5-yl)-1,2,4-oxadiazol-5-yl]methyl}sulfanyl)-1,3-benzoxazole |
| Z1029494424 | 2-({[3-(2H-1,3-benzodioxol-5-yl)-1,2,4-oxadiazol-5-yl]methyl}sulfanyl)-1,3-benzothiazole |
| Z1029495632 | 4-({[3-(2H-1,3-benzodioxol-5-yl)-1,2,4-oxadiazol-5-yl]methyl}sulfanyl)quinazoline |
| Z1029496466 | 2-({[3-(2H-1,3-benzodioxol-5-yl)-1,2,4-oxadiazol-5-yl]methyl}sulfanyl)-4-methylquinoline |
| Z1029496890 | 2-({[3-(2H-1,3-benzodioxol-5-yl)-1,2,4-oxadiazol-5-yl]methyl}sulfanyl)-1-(propan-2-yl)-1H-1,3-benzodiazole |
| Z1029499752 | 2-({[3-(2H-1,3-benzodioxol-5-yl)-1,2,4-oxadiazol-5-yl]methyl}sulfanyl)-5-cyclohexyl-1,3,4-oxadiazole |
| Z1029500204 | 3-(2H-1,3-benzodioxol-5-yl)-5-({imidazo[1,5-a]pyridin-3-ylsulfanyl}methyl)-1,2,4-oxadiazole |
| Z1029500216 | 3-(2H-1,3-benzodioxol-5-yl)-5-{[(1-propyl-1H-imidazol-2-yl)sulfanyl]methyl}-1,2,4-oxadiazole |
| Z1029500474 | 2-({[3-(2H-1,3-benzodioxol-5-yl)-1,2,4-oxadiazol-5-yl]methyl}sulfanyl)-N-methylquinazolin-4-amine |
| Z1029501304 | 2-({[3-(2H-1,3-benzodioxol-5-yl)-1,2,4-oxadiazol-5-yl]methyl}sulfanyl)-5-(trifluoromethyl)pyridine |
| Z1029501700 | 6-({[3-(2H-1,3-benzodioxol-5-yl)-1,2,4-oxadiazol-5-yl]methyl}sulfanyl)-N, N-dimethylpyridine-3-sulfonamide |
| Z1029504560 | 4-({[3-(2H-1,3-benzodioxol-5-yl)-1,2,4-oxadiazol-5-yl]methyl}sulfanyl)-6-methyl-2-(propan-2-yl)pyrimidine |
| Z1029504794 | 2-({[3-(2H-1,3-benzodioxol-5-yl)-1,2,4-oxadiazol-5-yl]methyl}sulfanyl)-1-(difluoromethyl)-1H-1,3-benzodiazole |
| Z1029505062 | 3-(2H-1,3-benzodioxol-5-yl)-5-({thieno[3,2-d]pyrimidin-4-ylsulfanyl}methyl)-1,2,4-oxadiazole |
| Z1029506108 | 2-({[3-(2H-1,3-benzodioxol-5-yl)-1,2,4-oxadiazol-5-yl]methyl}sulfanyl)-3-chloropyridine |
| Z1029506216 | 3-(2H-1,3-benzodioxol-5-yl)-5-[({1-methyl-1H-pyrazolo[3,4-d]pyrimidin-4-yl}sulfanyl)methyl]-1,2,4-oxadiazole |
| Z1029507034 | 2-({[3-(2H-1,3-benzodioxol-5-yl)-1,2,4-oxadiazol-5-yl]methyl}sulfanyl)-4-(trifluoromethyl)pyrimidine |
| Z1029508010 | 2-({[3-(2H-1,3-benzodioxol-5-yl)-1,2,4-oxadiazol-5-yl]methyl}sulfanyl)-5-chloropyridine |
| Z1029510124 | 2-({[3-(2H-1,3-benzodioxol-5-yl)-1,2,4-oxadiazol-5-yl]methyl}sulfanyl)-5-methylpyridine |
| Z1029510468 | 2-({[3-(2H-1,3-benzodioxol-5-yl)-1,2,4-oxadiazol-5-yl]methyl}sulfanyl)-5-methylpyrimidine |
| Z1029510526 | 3-(2H-1,3-benzodioxol-5-yl)-5-{[(5-tert-butyl-1,3,4-oxadiazol-2-yl)sulfanyl]methyl}-1,2,4-oxadiazole |
| Z1029511174 | 3-(2H-1,3-benzodioxol-5-yl)-5-{[(5-phenyl-1,3-oxazol-2-yl)sulfanyl]methyl}-1,2,4-oxadiazole |
| Z1029519298 | 2-({[3-(2H-1,3-benzodioxol-5-yl)-1,2,4-oxadiazol-5-yl]methyl}sulfanyl)-1-ethyl-1,4-dihydropyrimidin-4-one |
| Z1032412320 | 6-({[3-(2H-1,3-benzodioxol-5-yl)-1,2,4-oxadiazol-5-yl]methyl}sulfanyl)pyridine-3-sulfonamide |
| Z1170218673 | 2-({[3-(2H-1,3-benzodioxol-5-yl)-1,2,4-oxadiazol-5-yl]methyl}sulfanyl)-4,6-dimethylpyrimidine |
| Z1203037016 | 6-methyl-2-({[3-(pyridin-4-yl)-1,2,4-oxadiazol-5-yl]methyl}sulfanyl)pyrimidin-4-amine |
| Z1231014585 | 2-({[3-(2H-1,3-benzodioxol-5-yl)-1,2,4-oxadiazol-5-yl]methyl}sulfanyl)-6-(trifluoromethyl)pyrimidin-4-ol |
| Z1263951070 | 6-methyl-2-({[3-(2,2,2-trifluoroethyl)-1,2,4-oxadiazol-5-yl]methyl}sulfanyl)pyrimidin-4-amine |
| Z1268440212 | 2-({[3-(2H-1,3-benzodioxol-5-yl)-1,2,4-oxadiazol-5-yl]methyl}sulfanyl)-4-methylpyrimidine |
| Z1329003512 | 3-(2H-1,3-benzodioxol-5-yl)-5-({[1-methyl-5-(propan-2-yl)-1H-imidazol-2-yl]sulfanyl}methyl)-1,2,4-oxadiazole |
| Z1425912580 | 3-(2H-1,3-benzodioxol-5-yl)-5-{[(5-tert-butyl-1,3-oxazol-2-yl)sulfanyl]methyl}-1,2,4-oxadiazole |
| Z1497574643 | 2-{[(3-cyclopentyl-1,2,4-oxadiazol-5-yl)methyl]sulfanyl}-6-methylpyrimidin-4-amine |
| Z1509671877 | 2-[({3-[(3,5-dimethylphenoxy)methyl]-1,2,4-oxadiazol-5-yl}methyl)sulfanyl]-6-methylpyrimidin-4-amine |
| Z1518617719 | 3-(2H-1,3-benzodioxol-5-yl)-5-[({1-ethyl-1H-imidazo[4,5-c]pyridin-2-yl}sulfanyl)methyl]-1,2,4-oxadiazole |
| Z153632010 | 6-methyl-2-({[3-(thiophen-2-yl)-1,2,4-oxadiazol-5-yl]methyl}sulfanyl)pyrimidin-4-amine |
| Z1537370343 | 3-(2H-1,3-benzodioxol-5-yl)-5-[({6-methylthieno[2,3-d]pyrimidin-4-yl}sulfanyl)methyl]-1,2,4-oxadiazole |
| Z1606901941 | 2-({[3-(methoxymethyl)-1,2,4-oxadiazo1-5-yl]methyl}sulfanyl)-6-methylpyrimidin-4-amine |
| Z1607523924 | 2-({[3-(2H-1,3-benzodioxol-5-yl)-1,2,4-oxadiazol-5-yl]methyl}sulfanyl)-5H,6H,7H-cyclopenta[d]pyrimidin-4-ol |
| Z1636448291 | 3-({[3-(2H-1,3-benzodioxol-5-yl)-1,2,4-oxadiazol-5-yl]methyl}sulfanyl)pyridazine |
| Z165913012 | 6-methyl-2-{[(5-phenyl-1,2,4-oxadiazol-3-yl)methyl]sulfanyl}pyrimidin-4-amine |
| Z167919650 | 2-({[5-(4-methoxyphenyl)-1,3,4-oxadiazol-2-yl]methyl}sulfanyl)-6-methylpyrimidin-4-amine |
| Z1686590093 | 3-(2H-1,3-benzodioxol-5-yl)-5-{[(5-methyl-1,3,4-oxadiazol-2-yl)sulfanyl]methyl}-1,2,4-oxadiazole |
| Z1737959431 | 3-(2H-1,3-benzodioxol-5-yl)-5-({imidazo[1,2-a]pyrazin-8-ylsulfanyl}methyl)-1,2,4-oxadiazole |
| Z1742797621 | 3-({[3-(2H-1,3-benzodioxol-5-yl)-1,2,4-oxadiazol-5-yl]methyl}sulfanyl)-6-(trifluoromethyl)pyridazine |
| Z1815466224 | 2-({[3-(2H-1,3-benzodioxol-5-yl)-1,2,4-oxadiazol-5-yl]methyl}sulfanyl)-4,6-dimethylpyridine |
| Z1818892697 | 2-({[3-(2H-1,3-benzodioxol-5-yl)-1,2,4-oxadiazol-5-yl]methyl}sulfanyl)-6-cyclopropylpyrimidin-4-ol |
| Z1838461414 | 3-(2H-1,3-benzodioxol-5-yl)-5-{[(4-methyl-1,3-oxazol-2-yl)sulfanyl]methyl}-1,2,4-oxadiazole |
| Z1883024789 | 3-(2H-1,3-benzodioxol-5-yl)-5-{[(4-phenyl-1,3-oxazol-2-yl)sulfanyl]methyl}-1,2,4-oxadiazole |
| Z1913692254 | 3-({[3-(2H-1,3-benzodioxol-5-yl)-1,2,4-oxadiazol-5-yl]methyl}sulfanyl)pyrazin-2-amine |
| Z215143852 | 2-({[3-(4-methoxyphenyl)-1,2-oxazol-5-yl]methyl}sulfanyl)-6-methylpyrimidin-4-amine |
| Z215143872 | 6-methyl-2-{[(3-phenyl-1,2-oxazol-5-yl)methyl]sulfanyl}pyrimidin-4-amine |
| Z2199309979 | 2-({[3-(2H-1,3-benzodioxol-5-yl)-1,2,4-oxadiazol-5-yl]methyl}sulfanyl)-5-fluoro-1,3-benzoxazole |
| Z2217546941 | 3-(2H-1,3-benzodioxol-5-yl)-5-{[(4,5-dimethyl-1H-imidazol-2-yl)sulfanyl]methyl}-1,2,4-oxadiazole |
| Z2234585705 | 2-({[3-(2H-1,3-benzodioxol-5-yl)-1,2,4-oxadiazol-5-yl]methyl}sulfanyl)pyrimidin-4-ol |
| Z225946944 | 6-methyl-2-({[3-(pyridin-3-yl)-1,2,4-oxadiazol-5-yl]methyl}sulfanyl)pyrimidin-4-amine |
| Z236243226 | 6-methyl-2-{[(5-methyl-2-phenyl-1,3-oxazol-4-yl)methyl]sulfanyl}pyrimidin-4-amine |
| Z243492726 | 6-methyl-2-({[3-(2-methylpropyl)-1,2,4-oxadiazol-5-yl]methyl}sulfanyl)pyrimidin-4-amine |
| Z2736147791 | 2-({[3-(2H-1,3-benzodioxol-5-yl)-1,2,4-oxadiazol-5-yl]methyl}sulfanyl)-6-methylpyrimidin-4-ol |
| Z2769120597 | 2-{[(3-cyclobutyl-1,2,4-oxadiazol-5-yl)methyl]sulfanyl}-6-methylpyrimidin-4-amine |
| Z281391700 | 2-({[2-(4-methoxyphenyl)-1,3-oxazol-4-yl]methyl}sulfanyl)-6-methylpyrimidin-4-amine |
| Z285633030 | 2-({[5-(4-methoxyphenyl)-1,2,4-oxadiazol-3-yl]methyl}sulfanyl)-6-methylpyrimidin-4-amine |
| Z285633032 | 2-{[(3-tert-butyl-1,2,4-oxadiazol-5-yl)methyl]sulfanyl}-6-methylpyrimidin-4-amine |
| Z2902738608 | 1-({[3-(2H-1,3-benzodioxol-5-yl)-1,2,4-oxadiazol-5-yl]methyl}sulfanyl)isoquinoline |
| Z2918137285 | 6-methyl-2-[({3-[(propan-2-yloxy)methyl]-1,2,4-oxadiazol-5-yl}methyl)sulfanyl]pyrimidin-4-amine |
| Z3213802691 | 2-({[3-(2H-1,3-benzodioxol-5-yl)-1,2,4-oxadiazol-5-yl]methyl}sulfanyl)-N-phenylquinazolin-4-amine |
| Z3213802693 | 6-amino-2-({[3-(2H-1,3-benzodioxol-5-yl)-1,2,4-oxadiazol-5-yl]methyl}sulfanyl)pyrimidin-4-ol |
| Z3213802695 | 4-({[3-(2H-1,3-benzodioxol-5-yl)-1,2,4-oxadiazol-5-yl]methyl}sulfanyl)-6-methyl-1,3,5-triazin-2-amine |
| Z3213802699 | 2-({[3-(2H-1,3-benzodioxol-5-yl)-1,2,4-oxadiazol-5-yl]methyl}sulfanyl)-5-(4-chlorophenyl)-6-ethylpyrimidin-4-amine |
| Z3213802705 | 2-({[3-(2H-1,3-benzodioxol-5-yl)-1,2,4-oxadiazol-5-yl]methyl}sulfanyl)-4-methylpyridine |
| Z3213802707 | 2-({[3-(2H-1,3-benzodioxol-5-yl)-1,2,4-oxadiazol-5-yl]methyl}sulfanyl)pyridin-3-ol |
| Z3213802708 | 3-({[3-(2H-1,3-benzodioxol-5-yl)-1,2,4-oxadiazol-5-yl]methyl}sulfanyl)-1,2,4-triazin-5-ol |
| Z3213802710 | 4-({[3-(2H-1,3-benzodioxol-5-yl)-1,2,4-oxadiazol-5-yl]methyl}sulfanyl)-1,3,5-triazin-2-amine |
| Z3213802729 | 5-({[3-(2H-1,3-benzodioxol-5-yl)-1,2,4-oxadiazol-5-yl]methyl}sulfanyl)-[1,3]thiazolo[5,4-d]pyrimidin-7-ol |
| Z3213802731 | 2-({[3-(2H-1,3-benzodioxol-5-yl)-1,2,4-oxadiazol-5-yl]methyl}sulfanyl)-4-(trifluoromethyl)pyridine |
| Z3213802738 | 4-({[3-(2H-1,3-benzodioxol-5-yl)-1,2,4-oxadiazol-5-yl]methyl}sulfanyl)-6-(trifluoromethyl)pyrimidine |
| Z3213802739 | 2-({[3-(2H-1,3-benzodioxol-5-yl)-1,2,4-oxadiazol-5-yl]methyl}sulfanyl)-4-tert-butylpyrimidine |
| Z3213802750 | 2-({[3-(2H-1,3-benzodioxol-5-yl)-1,2,4-oxadiazol-5-yl]methyl}sulfanyl)-4-(pyridin-4-yl)pyrimidine |
| Z3213802754 | 3-(2H-1,3-benzodioxol-5-yl)-5-({[1,3]thiazolo[5,4-b]pyridin-2-ylsulfanyl}methyl)-1,2,4-oxadiazole |
| Z3213802764 | 2-({[3-(2H-1,3-benzodioxol-5-yl)-1,2,4-oxadiazol-5-yl]methyl}sulfanyl)-6-fluoro-1,3-benzoxazole |
| Z3213802766 | 3-(2H-1,3-benzodioxol-5-yl)-5-[({1-methyl-1H-imidazo[4,5-c]pyridin-2-yl}sulfanyl)methyl]-1,2,4-oxadiazole |
| Z3213802783 | 2-({[3-(2H-1,3-benzodioxol-5-yl)-1,2,4-oxadiazol-5-yl]methyl}sulfanyl)-5-cyclopropyl-1,3,4-oxadiazole |
| Z3213802789 | 3-(2H-1,3-benzodioxol-5-yl)-5-{[(4-tert-butyl-1,3-oxazol-2-yl)sulfanyl]methyl}-1,2,4-oxadiazole |
| Z3213802799 | 3-(2H-1,3-benzodioxol-5-yl)-5-({1H,4H,6H,7H-pyrano[3,4-d]imidazol-2-ylsulfanyl}methyl)-1,2,4-oxadiazole |
| Z3213805248 | 2-({[3-(2-methoxypropan-2-yl)-1,2,4-oxadiazol-5-yl]methyl}sulfanyl)-6-methylpyrimidin-4-amine |
| Z3213805251 | 6-methyl-2-({[3-(1-methyl-1H-imidazol-5-yl)-1,2,4-oxadiazol-5-yl]methyl}sulfanyl)pyrimidin-4-amine |
| Z3213805252 | 2-({[3-(6-methoxypyridazin-3-yl)-1,2,4-oxadiazol-5-yl]methyl}sulfanyl)-6-methylpyrimidin-4-amine |
| Z326667474 | 2-[({3-[(4-fluorophenoxy)methyl]-1,2,4-oxadiazol-5-yl}methyl)sulfanyl]-6-methylpyrimidin-4-amine |
| Z326667486 | 2-{[(3-cyclopropyl-1,2,4-oxadiazol-5-yl)methyl]sulfanyl}-6-methylpyrimidin-4-amine |
| Z362900180 | 6-methyl-2-({[3-(pyridin-2-yl)-1,2,4-oxadiazol-5-yl]methyl}sulfanyl)pyrimidin-4-amine |
| Z74373912 | 2-({[2-(4-methoxyphenyl)-1,3-thiazol-4-yl]methyl}sulfanyl)-6-methylpyrimidin-4-amine |
| Z74373920 | 6-methyl-2-{[(2-phenyl-1,3-oxazol-4-yl)methyl]sulfanyl}pyrimidin-4-amine |
| Z855791334 | 2-({[5-(4-methoxyphenyl)-1,3-oxazol-2-yl]methyl}sulfanyl)-6-methylpyrimidin-4-amine |
| Z97827862 | 6-methyl-2-{[(5-phenyl-1,2-oxazol-3-yl)methyl]sulfanyl}pyrimidin-4-amine |
| Z990622086 | 6-methyl-2-{[(4-methyl-5-phenyl-4H-1,2,4-triazol-3-yl)methyl]sulfanyl}pyrimidin-4-amine |

Further particularly preferred compounds for use according to the invention as defined in claim 20 are shown in the following Table 6:

**Table 6:**

| **Compound ID** | **Name** |
|---|---|
| Z1137177241 | 2-(((3-(furan-3-yl)-1,2,4-oxadiazol-5-yl)methyl)thio)-6-methylpyrimidin-4-amine |
| Z3214047394 | 6-methyl-2-((2-(3-(p-tolyl)-1,2,4-oxadiazol-5-yl)ethyl)thio)pyrimidin-4(1H)-imine |
| Z1453477070 | 2-((1-(3-(4-methoxyphenyl)-1,2,4-oxadiazol-5-yl)ethyl)thio)-6-methylpyrimidin-4(1H)-imine |

According to the invention compounds disclosed herein are to be understood as also including the respective enantiomers, diastereomers, hydrates, solvates, pharmaceutically acceptable co-crystals or salts and complexes thereof.

Pharmaceutically acceptable salts are typically salts of an organic or inorganic acids generally known in the art as pharmaceutically acceptable, preferably those disclosed in P. H. Stahl and C. G. Wermuth, editors, Handbook of Pharmaceutical Salts: Properties, Selection and Use, Weinheim/Zürich:Wiley-VCH/VHCA, 2002. A preferred salt of the invention is the hydrochloride salt of a compound as disclosed herein.

The compounds as defined herein are particularly useful as agonists of GPR40, wherein, according to preferred embodiments, the compound shows a higher selectivity for GPR40 than for GPR120.

More preferably, the compound as defined herein shows a % activation of GPR40 being at least 3fold higher than the % activation of GPR120, with % activation being the hundredfold ratio of activation of GPR40 or GPR120, respectively, by said compound to the activation of GPR40 or GPR120, respectively, by AMG 837. AMG 837 is a known potent agonist of GPR40 [22].

The compounds of the present invention are potent and selective agonists and are preferably provides as such agonists, of FFAR1 (GPR40]. In particular, the compounds as disclosed herein are more selective and potent agonists of FFAR1 than of FFAR4. In preferred embodiments of the invention, a compound as disclosed herein shows an at least 3 fold %, more preferably a 3 to 5 fold, % activation of FFAR1 in comparison to its % activation of FFAR4 (GPR120), with % activation always being the 100fold ratio of activation of the respective receptor by the compound as disclosed herein and the activation of the respective receptor by AMG 837. According to alternative embodiments of the invention, the % activation as used herein may also be expressed by reference to the activation by TAK-875.

The compounds as disclosed herein are useful as medicaments, preferably for the prevention, improvement of symptoms, suppression of progression or treatment of conditions or diseases in a mammalian (such as, e.g., human, mouse, rat, rabbit, dog, cat, bovine, horse, swine, monkey) subject as further outlined below.

For medical use the compounds of this disclosure may be used as such or as an active ingredient of a pharmaceutical composition comprising at least one compound of general formula (I) and at least one pharmaceutically acceptable carrier.

Useful pharmaceutically acceptable carriers are various organic or inorganic carrier substances which are conventionally used as preparation materials in the pharmaceutical art. These may be incorporated as excipients, lubricants, binders and disintegrants for solid preparations, or solvents, solubilizing agents, suspending agents, isotonicity agents, buffers and soothing agents for liquid preparations, and the like, in the present pharmaceutical composition. Further ingredients are preferably selected from preparation additives such as preservatives, antioxidants, colorants, sweetening agents and the like, which can be added as necessary.

Preferred examples of excipients include, but are not limited to, lactose, sucrose, D-mannitol, D-sorbitol, starch, gelatinated starch, dextrin, crystalline cellulose, low-substituted hydroxypropylcellulose, sodium carboxymethylcellulose, gum arabic, pullulan, light anhydrous silicic acid, synthesis aluminum silicate and magnesium alumino metasilicate.

Preferred examples of lubricants include, but are not limited to, magnesium stearate, calcium stearate, talc and colloidal silica.

Preferred binders include, but are not limited to, gelatinated starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone.

Preferred disintegrants include, but are not limited to, lactose, sucrose, starch, carboxymethylcellulose, calcium carboxymethylcellulose, croscarmellose sodium, sodium carboxymethyl starch, light anhydrous silicic acid and low-substituted hydroxypropylcellulose.

Preferred examples of useful solvents include, but are not limited to, water for injection, physiological brine, Ringer's solution, Ringer lactate, alcohol, propylene glycol, polyethylene glycol, sesame oil, corn oil, olive oil and cottonseed oil.

Preferred solubilizing agents include, but are not limited to, polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferred suspending agents for use in the present inventiuon include, but are not limited to, surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates; and polyoxyethylene hydrogenated castor oil.

Preferred examples of isotonicity agents are sodium chloride, glycerol, D-mannitol, D-sorbitol and glucose.

Preferred buffer substances include buffers such as phosphate, acetate, carbonate, citrate and the like.

A preferred soothing agent in the context of the invention is benzyl alcohol.

Preferred examples of preservatives for use in the pharmaceutical composition include, but are not limited to, p-oxybenzoates, chlorobutanol, benzyl alcohol, phenethyl alcohol, dehydroacetic acid and sorbic acid.

Preferred antioxidants for use in the invention include, but are not limited to, sulfite and ascorbate.

Preferred colorants include, e.g., aqueous water-soluble food tar colors (e.g., food colors such as Food Color Red Nos. 2 and 3, Food Color Yellow Nos. 4 and 5, Food Color Blue Nos. 1 and 2 and the like food colors), water insoluble lake dyes (e.g., aluminum salt of the aforementioned water-soluble food tar color) and natural dyes (e.g., β-carotene, chlorophyll, ferric oxide red).

Preferred sweetening agents are, e.g., saccharin sodium, dipotassium glycyrrhizinate, aspartame and stevia.

Preferred dosage forms of the pharmaceutical composition include oral preparations such as tablet (including sugar-coated tablet, film-coated tablet, sublingual tablet, orally disintegrating tablet), capsules (including soft capsule, microcapsule), granule, powder, troche, syrup, emulsion, suspension, films (e.g., orally disintegrable films) and the like; and parenteral agents such as injection (e.g., subcutaneous injection, intravenous injection, intramuscular injection, intraperitoneal injection, drip infusion), external preparations (e.g., dermal preparation, ointment), suppository (e.g., rectal suppository, vaginal suppository), pellet, nasal preparation, pulmonary preparation (inhalant), eye drop.

Pharmaceutical compositions of the invention may be in the form of release control preparations (e.g., sustained-release microcapsule) such as an immediate-release preparation, a sustained-release preparation and the like

The pharmaceutical composition can be produced according to a method conventionally used in the field of pharmaceutical formulation.

While the content of the compound of the present invention in the pharmaceutical composition varies depending on the dosage form, dose of the compound of the present invention or as disclosed herein and further parameters such as the specific aids as outlined above, it is typically contained in the pharmaceutical composition at about 0.1 to about 100 wt %.

Oral pharmaceutical compositions of the invention may comprise one or more coatings which may be applied as necessary for the purpose of various parameters such as masking of taste, enteric property or durability.

Further specific guidance concerning the ingredients, but also routes for administration, dosage etc., of the pharmaceutical composition can be found in the latest edition of Remington's Pharmaceutical Sciences (Mack Publishing Co., Eastern, PA, USA).

Any references in the present disclosure to methods of treatment refer to the compounds, pharmaceutical compositions and medicaments of the present invention for use in a method for treatment of the human (or animal) body by therapy.

As defined in claim 20, the invention is also directed to a compound of general formula (I)

R¹-S-CH₂-OXA-R ² (I)

including enantiomers, diastereomers, hydrates, solvates, pharmaceutically acceptable cocrystals or salts, and complexes thereof;
wherein
OXA is selected from the group consisting of 1,3-oxazolyl, 1,2,4-oxadiazolyl or 1,3,4-oxadiazolyl;
when OXA is 1,3-oxazolyl, the group R¹-S-CH₂ is bound to C² of the 1,3-oxazolyl and the group R² is bound to C⁴ of the 1,3-oxazolyl;
when OXA is 1,2,4-oxadiazolyl the group R¹-S-CH₂ is bound to C⁵ of the 1,2,4-oxadiazolyl and the group R² is bound to C³ of the 1,2,4-oxadiazolyl;
when OXA is 1,3,4-oxadiazolyl the group R¹-S-CH₂ is bound to C⁵ of the 1,3,4-oxadiazolyl and the group R² is bound to C² of the 1,3,4-oxadiazolyl';
R¹ is a 6 membered heteroaryl group selected from the group consisting of 1,3,5-triazinyl and pyrimidinyl being independently substituted with one or more substituents selected from the group consisting of hydroxyl, amino, C₁-C₆-alkyl, C₃-C₆-cycloalkyl, C₁-C₄-alkoxy, N-mono- or N,N-di-substituted C₁-C₃-alkylamino, non-aromatic 5- to 6-membered heterocyclyl, 6-membered aryl and 5- to 6-membered heteroaryl which substituents may be unsubstituted or substituted with one or more groups selected from the group consisting of halide, cyano and C₁-C₆-alkyl, wherein the 6-membered aryl and 5- to 6-membered heteroaryl group, respectively, may be fused to said 1,3,5-triazinyl or pyrimidyl group, respectively, and
R² is phenyl being unsubstituted or being substituted with one or more substituents selected from the group consisting of halide, cyano, amino, C₁-C₆-alkyl, C₃-C₆-cycloalkyl which may be optionally substituted with one or more halides, C₁-C₄-alkoxy which may optionally substituted with one or more halides, hydroxy-C₁-C₆-alkyl, sulfonyl-C₁-C₆-alkyl, sulphamidyl-N-C₁-C6-alkyl and carboxamidyl-N-mono- or -N,N-di-C₁-C₆-alkyl;
for use as an agonist of GPR40 in the treatment of T2DM and/or a pre-diabetic condition.

The compounds disclosed herein for use in the prevention, improvement of symptoms, suppression of progression or treatment of conditions or diseases involving of conditions or diseases amenable to higher GBR40 (FFAR1) activity, in particular, conditions or diseases involving energy household and metabolism, preferably conditions or diseases involving impaired control of glucose blood levels, more preferably diabetes, most preferred T2DM, and pre-diabetic conditions such as obesity and insulin resistance. The present invention therefore also relates to a method for the prevention, improvement of symptoms, suppression of progression or treatment of diseases involving of conditions or diseases amenable to higher GBR40 (FFAR1) activity, in particular, conditions or diseases involving energy household and metabolism, preferably conditions or diseases involving impaired control of glucose blood levels, more preferably diabetes, most preferred T2DM, and pre-diabetic conditions such as obesity and insulin resistance, which method comprises the step of administering an effective amount of a compound as disclosed herein to a subject, preferably human subject, in need thereof.

The present invention also relates to the use of the disclosed compounds for the preparation of a medicament for the prevention, improvement of symptoms, suppression of progression or treatment of diseases involving of conditions or diseases amenable to higher GBR40 (FFAR1) activity, in particular, conditions or diseases involving energy household and metabolism, preferably conditions or diseases involving impaired control of glucose blood levels, more preferably diabetes, most preferred T2DM, and pre-diabetic conditions such as obesity and insulin resistance.

The administration of the compound disclosed herein is preferably systemic, with oral administration being particularly preferred.

The "effective amount" of the compound disclosed herein varies depending on the administration subject, route of administration, target disease, symptoms, sex of the subject etc. For example, when it is administered orally to an adult patient (body weight 60 kg), its dose is typically about 0.01 to about 100 mg/kg body weight per dose, preferably about 0.05 to about 30 mg/kg body weight per dose, more preferably about 0.1 to about 10 mg/kg body weight per dose, with such exemplary or preferable effective amounts being preferably administered in 1 to 3 doses per day.

When the compound of formula (I) as disclosed herein is applied to a condition or diseases as outlined above, it can be used in an appropriate combination with a medicament or a treatment method generally employed for the condition or disease, respectively, whereby the compound of the invention (or compound being useful in the invention) can administered with the second, third or more medicament simultaneously or non-simultaneously. Preferred combination therapies according to the invention employ at least one compound of formula (1) and one or more selected from biduanides such as preferagly Metformin, SGTL2 inhibitors (gliflozins) such as preferably dapagliflohin, DPP-4 inhibitors such as preferably one or more selected from Sitagliptin, Vildagliptin, Saxagliptin and Linagliptin, α-glucosidase inhibitors such as Acarbose, Miglitol and Voglibose, sulfonylurea compounds such as preferably one or more selected from acetohexamide, carbutamide, chlorpropamide, glycyclamide metahexamide, tolazamide, tolbutamide glibenclamide, glibornuride, gliclazide,^{[} glipizide, gliquidone, glisoxepide, glyclopyramide and glimipiride, glinides such as preferably one or more selected from repaglinide, nateglinide and mitiglinide as well as other agonists of GPR40 and/or GPR120 such as preferably one or more of TAK-875, LY2881835, AMG6837, GW9608, grifolic acid, NCG21, GSK-137677A and TUG391.

It is to be understood that with respect to all uses according to the invention, a "compound as disclosed herein" refers to the compound of general formula (I) as defined in claim 20.

Preferred embodiments of the compounds of the present disclosure wherein OXA in general formula (I) is 1,3-oxazolyl are preferably prepared according to one the following general synthesis schemes: 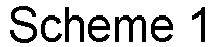 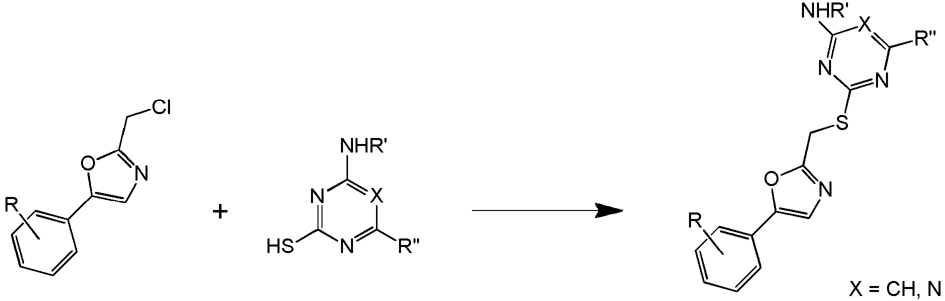 wherein R is as defined as the substituents at the phenyl group as defined in R² according to general formula (I), R' is H or C₁-C₃-alkyl, and R" is a substituent selected from hydroxyl, amino, C₁-C₅-alkyl, C₃-C₅-cycloalkyl, C₁-C₃-alkoxy, , N-mono- or N,N-di-substituted C₁-C₃-alkylamino, non-aromatic 5- to 6-membered heterocyclyl, 6- membered aryl and 5- to 6-membered heteroaryl, optionally substituted with one or more groups selected from halide, cyano and C₁to C₆-alkyl. 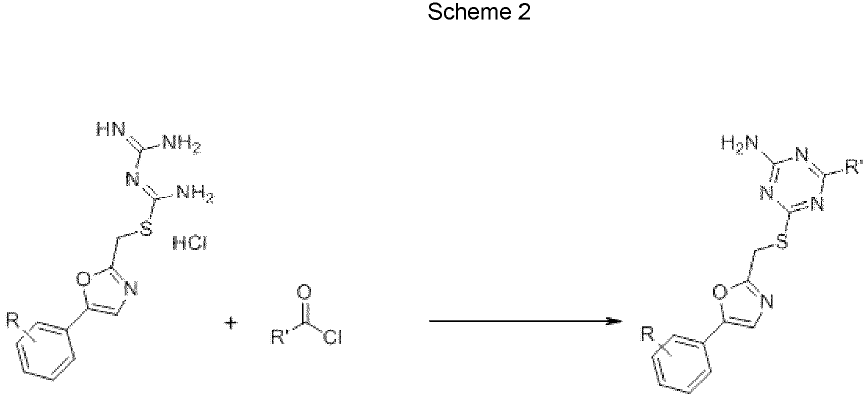 wherein R is as defined as the substituents at the phenyl group as defined in R² according to general formula (I), R' is a substituent selected from hydroxyl, amino, C₁-C₅-alkyl, C₃-C₅-cycloalkyl, C₁-C₃-alkoxy, N-mono- or N,N-di-substituted C₁-C₃-alkylamino, non-aromatic 5- to 6-membered heterocyclyl, 6- membered aryl and 5- to 6-membered heteroaryl,, optionally substituted with one or more groups selected from halide, cyano and C₁to C₆-alkyl. 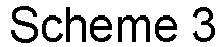 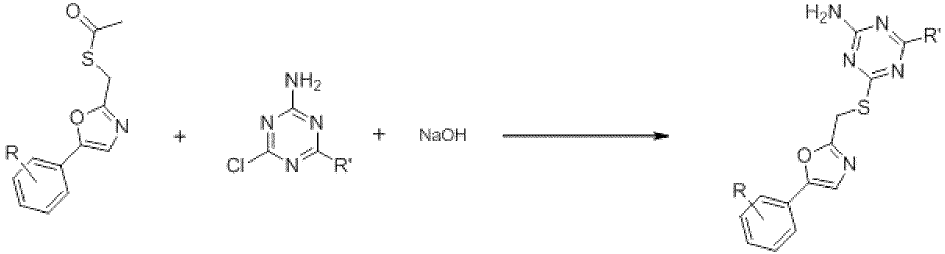 wherein R is as defined as the substituents at the phenyl group as defined in R₂ according to general formula (I), R' is a substituent selected from hydroxyl, amino, C₁-C₅-alkyl, C₃-C₅-cycloalkyl, C₁-C₃-alkoxy, N-mono- or N,N-di-substituted C₁-C₃-alkylamino, non-aromatic 5- to 6-membered heterocyclyl, 6- membered aryl and 5- to 6-membered heteroaryl,, optionally substituted with one or more groups selected from halide, cyano and C₁ to C₆-alkyl.

The Figures show:
- Fig. 1:: shows preferred compounds of the invention. nM EC50, p(EC50,M) and % activation compared to AMG 837 are indicated.
- Fig. 2: shows further compounds for use as GRP40 agonists and for use as medicaments, preferably for the treatment and/or prevention of T2DM. nM EC50 values, p(EC50,M) values and % activation compared to TAK-875 are indicated.
- Fig. 3: shows a graphic representation of glucose levels during oral glucose tolerance test (OGTT) according to the experiment of Example 87. Values are expressed as means of 5 animals tested. For glucose tolerance test (GTT), mice were orally treated with glucose at dose of 2 g/kg at the dose volume of 10 ml/kg after a 6-hour fasting. Glucose measurements were performed immediately before compound treatment (-60 min), at -30 min, 0 min (before glucose administration), and then 15, 30, 60, 90 and 120 min after glucose administration.

The present invention is further illustrated by the following non-limiting examples:
It is to be understood that any 1,2,4 -oxadiazolyl and 1,3,4 oxadiazolyl, respectively, compound referred to in the following examples is disclosed only in the context of the compound for use of the invention as defined in claim 20.

### A. Synthesis of intermediate compounds:

### Example 1: 2-Amino-1-(3-chloro-4-methoxyphenyl)ethanone hydrochloride

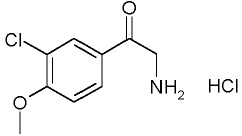

To a stirred solution of 2-bromo-1-(3-chloro-4-methoxyphenyl)ethanone (10.76 g, 40.832 mmol) in anhydrous acetonitrile (100 mL) was added sodium diformylamide (5.432 g, 57.165 mmol). The mixture was stirred at ambient temperature for 14 h. Inorganics was filtered off, and the filtrate was concentrated in vacuo. 5N Hydrochloric acid (100 mL) was added to the dark oily residue, and the resulting mixture was heated to reflux for 3 h. The hot solution was separated from dark viscous oil, and was allowed to cool down to ambient temperature. The precipitate formed was filtered and successively washed with 5N hydrochloric acid and diethyl ether affording 2-amino-1-(3-chloro-4-methoxyphenyl)ethanone hydrochloride (5.193 g, 100% purity, 54% yield) as yellow crystals. ¹H NMR (400 MHz, DMSO-d₆) δ 8.45 (br s, 3H), 8.07 (s, 1H), 8.02 (d, J = 8.7 Hz, 1H), 7.33 (d, J = 8.7 Hz, 1H), 4.55 (m, 2H), 3.98 (s, 3H). MS (CI): m/z = 200 [M+H]⁺.

### Example 2: N-(2-Oxo-2-(3-chloro-4-methoxyphenyl)ethyl)chloroacetamide

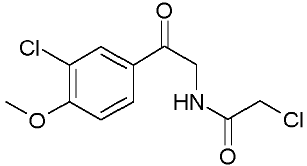

To an ice-chilled stirred slurry of 2-amino-1-(3-chloro-4-methoxyphenyl)ethanone hydrochloride (5.193 g, 21.995 mmol) and sodium hydrocarbonate (4.065 g, 48.389 mmol) in a mixture of ethyl acetate (150 mL) and water (50 mL), chloroacetyl chloride (1.92 mL, 24.194 mmol) was added dropwise. The reaction mixture was stirred at 0-5°C for 1 h 30 min. After the completion of the reaction, the ethyl acetate layer was separated and dried over sodium sulfate. Sodium sulfate was filtered off, and the filtrate was concentrated in vacuo affording *N*-(2-oxo-2-(3-chloro-4-methoxyphenyl)ethyl)chloroacetamide (2.65 g, 100% purity, 44% yield) as a pink solid. ¹H NMR (400 MHz, CDCl₃) δ 8.03 (d, J = 2.1 Hz, 1H), 7.89 (dd, J = 8.7, 2.1 Hz, 1H), 7.61 (br s, 1H), 7.00 (d, J = 8.7 Hz, 1H), 4.72 (d, J = 4.3 Hz, 2H), 4.13 (s, 2H), 3.99 (s, 3H). MS (CI): m/z = 276 [M+H]⁺.

### Example 3: 5-(3-Chloro-4-methoxyphenyl)-2-(chloromethyl)-1,3-oxazole

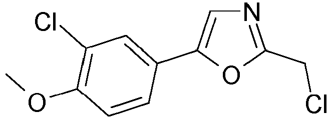

A stirred slurry of *N*-(2-oxo-2-(3-chloro-4-methoxyphenyl)ethyl)chloroacetamide (2.568 g, 9.3 mmol) in phosphoryl chloride (11.7 mL, 125.55 mmol) was heated under reflux for 2 h. Gas evolution was observed. Then, the reaction mixture was allowed to cool down to RT, and was poured into crashed ice (200 g). The precipitate was filtered and washed with water affording 5-(3-chloro-4-methoxyphenyl)-2-(chloromethyl)-1,3-oxazole (1.984 g, 100% purity, 83% yield) as a brown solid. ¹H NMR (400 MHz, CDCl₃) δ 7.65 (s, 1H), 7.49 (d, J = 8.5 Hz, 1H), 7.20 (s, 1H), 6.96 (d, J = 8.5 Hz, 1H), 4.64 (s, 2H), 3.93 (s, 3H). MS (CI): m/z = 258 [M+H]⁺.

### Example 4: N-(2-Oxo-2-(3-trifluoromethylphenyl)ethyl)chloroacetamide

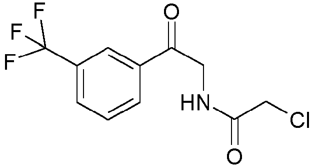

To an ice-chilled stirred slurry of 2-amino-1-(3-trifluoromethylphenyl)ethanone hydrochloride (2.55 g, 10.641 mmol) and sodium hydrocarbonate (1.967 g, 23.41 mmol) in ethyl acetate (75 mL)/water (25 mL) mixture, chloroacetyl chloride (0.93 mL, 11.705 mmol) was added dropwise. The reaction mixture was stirred at 0-5°C for 1 h 30 min. The ethyl acetate layer was separated and dried over sodium sulfate. Sodium sulfate was filtered off, and the filtrate was concentrated in vacuo affording *N*-(2-oxo-2-(3-trifluoromethylphenyl)ethyl)chloroacetamide (2.691 g, 95% purity, 90% yield) as a yellow solid. ¹H NMR (500 MHz, CDCl₃) δ 8.26 (s, 1H), 8.18 (d, J = 7.8 Hz, 1H), 7.91 (d, J = 7.8 Hz, 1H), 7.70 (m, 1H), 7.61 (br s, 1H), 4.84 (d, J = 3.6 Hz, 2H), 4.17 (s, 2H). MS (CI): m/z = 280 [M+H]⁺.

### Example 5: 2-(Chloromethyl)-5-[3-(trifluoromethyl)phenyl]-1,3-oxazole

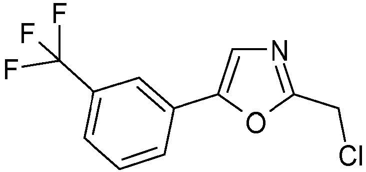

A stirred slurry of *N*-(2-oxo-2-(3-(trifluoromethylphenyl)ethyl)chloroacetamide (2.654 g, 9.49 mmol) in phosphoryl chloride (11.94 mL, 128.115 mmol) was heated to reflux for 2 h. Gas evolution was observed. Then the reaction mixture was allowed to cool down to ambient temperature, and was poured into crashed ice (200 g). The precipitate was filtered and washed with water affording 2-(chloromethyl)-5-[3-(trifluoromethyl)phenyl]-1,3-oxazole (2.308 g, 100% purity, 93% yield) as a brown solid. ¹H NMR (400 MHz, CDCl₃) δ 7.88 (s, 1H), 7.81 (d, J = 7.5 Hz, 1H), 7.66 - 7.51 (m, 2H), 7.39 (s, 1H), 4.67 (d, J = 2.6 Hz, 2H). MS (CI): m/z = 262 [M+H]⁺.

### Example 6: N-(2-Oxo-2-(2,3-dichlorophenyl)ethyl)chloroacetamide

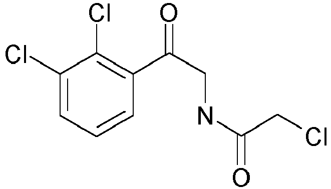

To an ice-chilled stirred slurry of 2-amino-1-(2,3-dichlorophenyl)ethanone hydrochloride (4.68 g, 19.458 mmol) and sodium hydrocarbonate (3.596 g, 42.808 mmol) in ethyl acetate (225 mL)/water (75 mL) mixture, chloroacetyl chloride (1.70 mL, 21.404 mmol) was added dropwise. The reaction mixture was stirred at 0-5°C for 1 h 30 min. The ethyl acetate layer was separated and dried over sodium sulfate. Sodium sulfate was filtered off, and the filtrate was concentrated in vacuo affording *N*-(2-oxo-2-(2,3-dichlorophenyl)ethyl)chloroacetamide (5.0 g, 98% purity, 92% yield) as a colorless solid. ¹H NMR (500 MHz, CDCl₃) δ 7.65 (dd, J = 7.9, 1.6 Hz, 1H), 7.48 (dd, J = 7.9, 1.6 Hz, 1H), 7.44 (br s, 1H), 7.34 (t, J = 7.9 Hz, 1H), 4.70 (d, J = 4.9 Hz, 2H), 4.13 (s, 2H). MS (CI): m/z = 280 [M+H]⁺.

### Example 7: 2-(Chloromethyl)-5-(2,3-dichlorophenyl)-1,3-oxazole

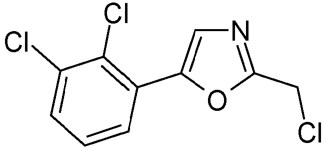

A stirred slurry of *N*-(2-oxo-2-(2,3-dichlorophenyl)ethyl)chloroacetamide (4.943 g, 17.62 mmol) in phosphoryl chloride (25 mL, 268.176 mmol) was heated to reflux for 2 h. Gas evolution was observed. Then the reaction mixture was allowed to cool down to ambient temperature, and was poured into crashed ice (250 g). The precipitate was filtered and washed with water affording 2-(chloromethyl)-5-(2,3-dichlorophenyl)-1,3-oxazole (4.052 g, 99% purity, 88% yield) as a brown solid. ¹H NMR (400 MHz, CDCl₃) δ 7.77 (s, 1H), 7.73 (dd, J = 8.0, 1.5 Hz, 1H), 7.46 (dd, J = 8.0, 1.6 Hz, 1H), 7.29 (t, J = 8.0 Hz, 1H), 4.67 (d, J = 1.3 Hz, 2H). MS (CI): m/z = 262 [M+H]⁺.

### Example 8: 2-Amino-1-(2,5-dichlorophenyl)ethanone hydrochloride

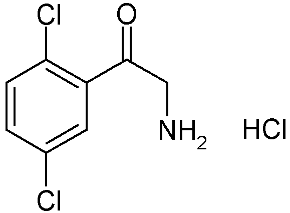

To a stirred solution of 2-bromo-1-(2,5-dichlorophenyl)ethanone (9.045 g, 33.758 mmol) in anhydrous acetonitrile (100 mL) was added sodium diformylamide (8.02 g, 84.395 mmol). The mixture was stirred at ambient temperature for 40 h. Inorganics was filtered off, and the filtrate was concentrated in vacuo. 5N hydrochloric acid (100 mL) was added to the dark oily residue, and the resulting mixture was heated to reflux for 3 h. The hot solution was separated from dark viscous oil, and was allowed to cool down to ambient temperature. The precipitate formed was filtered and successively washed with 5N hydrochloric acid and diethyl ether affording 2-amino-1-(2,5-dichlorophenyl)ethanone hydrochloride (2.973 g, 100% purity, 37% yield) as colorless crystals. ¹H NMR (500 MHz, DMSO-*d₆*) δ 8.55 (br s, 3H), 8.05 (t, J = 2.5 Hz, 1H), 7.72 (dd, J = 8.8, 2.5 Hz, 1H), 7.66 (d, J = 8.8 Hz, 1H), 4.52 (s, 2H). MS (CI): m/z = 204 [M]+.

### Example 9: N-(2-Oxo-2-(2,5-dichlorophenyl)ethyl)chloroacetamide

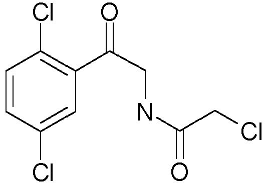

To an ice-chilled stirred slurry of 2-amino-1-(2,5-dichlorophenyl)ethanone hydrochloride (2.976 g, 12.373 mmol) and sodium hydrocarbonate (2.287 g, 27.221 mmol) in ethyl acetate (150 mL)/water (50 mL) mixture, chloroacetyl chloride (1.08 mL, 13.61 mmol) was added dropwise. The reaction mixture was stirred at 0-5°C for 1 h 30 min. The ethyl acetate layer was separated and dried over sodium sulfate. Sodium sulfate was filtered off, and the filtrate was concentrated in vacuo affording *N*-(2-oxo-2-(2,5-dichlorophenyl)ethyl)chloroacetamide (2.9 g, 100% purity, 84% yield) as a yellow solid. ¹H NMR (500 MHz, CDCl₃) δ 7.67 (t, J = 2.3 Hz, 1H), 7.44 (m, 3H), 4.74 (d, J = 4.8, 2H), 4.14 (s, 2H). MS (CI): m/z = 280 [M+H]⁺.

### Example 10: 2-(Chloromethyl)-5-(2,5-dichlorophenyl)-1,3-oxazole

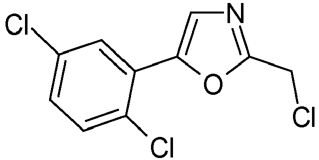

A stirred slurry of *N*-(2-oxo-2-(2,5-dichlorophenyl)ethyl)chloroacetamide (2.675 g, 9.535 mmol) in phosphoryl chloride (20 mL, 214.537 mmol) was heated to reflux for 2 h. Gas evolution was observed. Then the reaction mixture was allowed to cool down to ambient temperature, and was poured into crashed ice (200 g). The precipitate was filtered and washed with water affording 2-(chloromethyl)-5-(2,5-dichlorophenyl)-1,3-oxazole (2.288 g, 100% purity, 91% yield) as a brown solid. ¹H NMR (400 MHz CDCl₃) δ 7.83 - 7.76 (m, 2H), 7.39 (d, J = 8.6 Hz, 1H), 7.26 - 7.20 (m, 1H), 4.67 (s, 2H). MS (CI): m/z = 262 [M+H]⁺.

### Example 11: 2-Amino-1-(4-ethylphenyl)ethanone hydrochloride

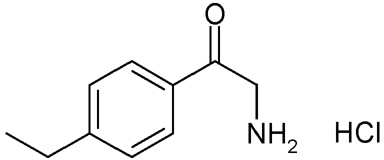

To a stirred solution of 2-bromo-1-(4-ethylphenyl)ethanone (7.95 g, 35.07 mmol) in anhydrous acetonitrile (100 mL) was added sodium diformylamide (8.317 g, 87.517 mmol). The mixture was stirred at ambient temperature for 15 h. Inorganics was filtered off, and the filtrate was concentrated in vacuo. 5N hydrochloric acid (70 mL) was added to the dark oily residue, and the resulting mixture was heated to reflux for 2.5 h. The hot solution was separated from dark viscous oil, and was allowed to cool down to ambient temperature. The reaction mixture was concentrated in vacuo, and the residue was crystallized from 5N hydrochloric acid affording 2-amino-1-(4-ethylphenyl)ethanone hydrochloride (2.848 g, 100% purity, 41% yield) as yellow crystals. 1H NMR (500 MHz, DMSO-*d₆*) δ 8.44 (br s, 3H), 7.94 (d, J = 7.8 Hz, 2H), 7.43 (d, J = 7.8 Hz, 2H), 4.54 (m, 2H), 2.70 (q, J = 7.6 Hz, 2H), 1.20 (t, J = 7.6 Hz, 3H). MS (CI): m/z = 164 [M]+.

### Example 12: N-(2-Oxo-2-(4-ethylphenyl)ethyl)chloroacetamide

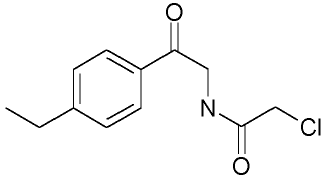

To an ice-chilled stirred slurry of 2-amino-1-(4-ethylphenyl)ethanone hydrochloride (2.848 g, 14.262 mmol) and sodium hydrocarbonate (2.636 g, 31.376 mmol) in ethyl acetate (150 mL)/water (50 mL) mixture, chloroacetyl chloride (1.25 mL, 15.688 mmol) was added dropwise. The reaction mixture was stirred at 0-5°C for 2 h. The ethyl acetate layer was separated and dried over sodium sulfate. Sodium sulfate was filtered off, and the filtrate was concentrated in vacuo affording N-(2-oxo-2-(4-ethylphenyl)ethyl)chloroacetamide (3.091 g, 100% purity, 90% yield) as a beige solid. ¹H NMR (500 MHz, CDCl₃) δ 7.92 (d, J = 8.2, 2H), 7.67( br s, 1H), 7.34 (d, J = 8.2, 2H), 4.77 (d, J = 3.9 Hz, 2H), 4.14 (s, 2H), 2.74 (q, J = 7.7, 2H), 1.28 (t, J = 7.6, 3H). MS (CI): m/z = 240 [M+H]⁺.

### Example 13: 5-(4-Ethylphenyl)-2-(chloromethyl)-1,3-oxazole

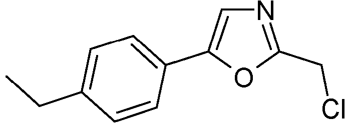

A stirred slurry of *N*-(2-oxo-2-(4-ethylphenyl)ethyl)chloroacetamide (3.062 g, 12.774 mmol) in phosphoryl chloride (22 mL, 236.064 mmol) was heated to reflux for 2 h. Gas evolution was observed. Then the reaction mixture was allowed to cool down to ambient temperature, and was poured into crashed ice (300 g). The precipitate was filtered and washed with water affording 5-(4-ethylphenyl)-2-(chloromethyl)-1,3-oxazole (2.513 g, 100% purity, 89% yield) as a brown solid. ¹H NMR (400 MHz, CDCl₃) δ 7.55 (d, J = 8.0 Hz, 2H), 7.36 - 7.07 (m, 3H), 4.65 (s, 2H), 2.66 (q, J = 7.6 Hz, 2H), 1.24 (t, J = 7.6 Hz, 3H). MS (CI): m/z = 222 [M+H]⁺.

### Example 14: N-(2-Oxo-2-(3-ethoxyphenyl)ethyl)chloroacetamide

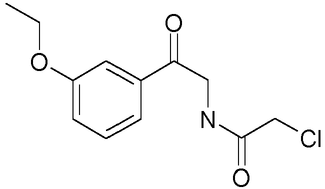

To an ice-chilled stirred slurry of 2-amino-1-(3-ethoxyphenyl)ethanone hydrochloride(2.563 g, 11.883 mmol) and sodium hydrocarbonate (2.196 g, 26.143 mmol) in ethyl acetate (150 mL)/water (50 mL) mixture, chloroacetyl chloride (1.04 mL, 13.071 mmol) was added dropwise. The reaction mixture was stirred at 0-5°C for 2 h. The ethyl acetate layer was separated and dried over sodium sulfate. Sodium sulfate was filtered off, and the filtrate was concentrated in vacuo affording *N*-(2-oxo-2-(3-ethoxyphenyl)ethyl)chloroacetamide (2.79 g, 100% purity, 92% yield) as a yellow solid. ¹H NMR (400 MHz, CDCl₃) δ 7.61 (br s, 1H), 7.52 (d, J = 7.7 Hz, 1H), 7.46 (t, J = 2.0 Hz, 1H), 7.38 (t, J = 8.0 Hz, 1H), 7.14 (dd, J = 8.2, 2.6 Hz, 1H), 4.75 (d, J = 4.4 Hz, 2H), 4.11 (s, 2H), 4.07 (q, J = 7.0 Hz, 2H), 1.42 (t, J = 7.0 Hz, 3H). MS (CI): m/z = 256 [M+H]⁺.

### Example 15: 5-(3-Ethoxyphenyl)-2-(chloromethyl)-1,3-oxazole

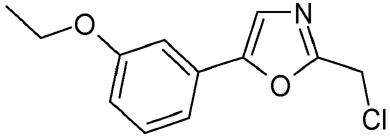

A stirred slurry of *N*-(2-oxo-2-(3-ethoxyphenyl)ethyl)chloroacetamide (2.737 g, 10.704 mmol) in phosphoryl chloride (15 mL, 160.56 mmol) was heated to reflux for 2 h. Gas evolution was observed. Then the reaction mixture was allowed to cool down to ambient temperature, and was poured into crashed ice (200 g). Chloroform (150 mL) was added, and the resulting mixture was stirred for 0.25 h. The organic layer was separated and dried over sodium sulfate. Sodium sulfate was filtered off, and the filtrate was concentrated in vacuo affording 5-(3-ethoxyphenyl)-2-(chloromethyl)-1,3-oxazole (2.418 g, 100% purity, 95% yield) as a brown oil. ¹H NMR (400 MHz, CDCl₃) δ 7.31 (t, J = 8.0 Hz, 1H), 7.25 - 7.18 (m, 2H), 7.15 (t, J = 2.1 Hz, 1H), 6.87 (dd, J = 8.4, 2.5 Hz, 1H), 4.65 (s, 2H), 4.07 (q, J = 7.0 Hz, 2H), 1.42 (t, J = 7.0 Hz, 3H). MS (CI): m/z = 238 [M+H]⁺.

### Example 16: 2-Amino-1-biphenyl-3-ylethanone hydrochloride

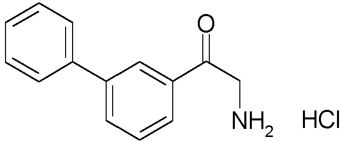

To a stirred solution of 1-biphenyl-3-yl-2-bromoethanone (15.287 g, 38.89 mmol) in anhydrous acetonitrile (100 mL) was added sodium diformylamide (10.163 g, 106.948 mmol). The mixture was stirred at rt for 72 h. Inorganics was filtered off, and the filtrate was concentrated in vacuo. 5N hydrochloric acid (100 mL) was added to the dark oily residue, and the resulting mixture was heated to reflux for 5 h. The hot solution was separated from dark viscous oil, and was allowed to cool down to rt. The precipitate formed was filtered and successively washed with 5N hydrochloric acid and diethyl ether affording 2-amino-1-biphenyl-3-ylethanone hydrochloride (6.35 g, 100% purity, 66% yield) as brown crystals. ¹H NMR (500 MHz, DMSO-*d₆*) δ 8.44 (br s, 3H), 8.25 (s, 1H), 8.07 - 7.96 (m, 2H), 7.78 (d, J = 7.0 Hz, 2H), 7.69 (t, J = 7.6 Hz, 1H), 7.52 (t, J = 7.3 Hz, 2H), 7.44 (t, J = 7.1 Hz, 1H), 4.74 - 4.65 (m, 2H). MS (CI): m/z = 212 [M]+.

### Example 17: N-(2-Oxo-2-biphenyl-3-ylethyl)chloroacetamide

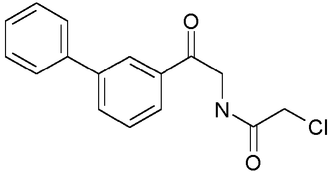

To an ice-chilled stirred slurry of 2-amino-1-biphenyl-3-ylethanone hydrochloride (6.349 g, 25.63 mmol) and sodium hydrocarbonate (4.737 g, 56.386 mmol) in ethyl acetate (150 mL)/water (50 mL) mixture, chloroacetyl chloride (2.31 mL, 28.193 mmol) was added dropwise. The reaction mixture was stirred at 0-5°C for 2 h 15 min. The ethyl acetate layer was separated and dried over sodium sulfate. Sodium sulfate was filtered off, and the filtrate was concentrated in vacuo affording N-(2-oxo-2-biphenyl-3-ylethyl)chloroacetamide (6.858 g, 100% purity, 93% yield) as a yellow solid. ¹H NMR (500 MHz, CDCl₃) δ 8.21 (s, 1H), 7.97 (d, J = 7.6 Hz, 1H), 7.88 (d, J = 7.6 Hz, 1H), 7.68 (br s, 1H), 7.62 (m, 3H), 7.50 (t, J = 7.5 Hz, 2H), 7.42 (t, J = 7.3 Hz, 1H), 4.86 (d, J = 4.4 Hz, 2H), 4.16 (s, 2H). MS (CI): m/z = 288 [M+H]⁺.

### Example 18: 5-Biphenyl-3-yl-2-(chloromethyl)-1,3-oxazole

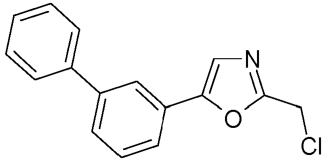

A stirred slurry of *N*-(2-oxo-2-biphenyl-3-ylethyl)chloroacetamide (6.858 g, 23.833 mmol) in phosphoryl chloride (30 mL, 321.746 mmol) was heated to reflux for 2 h. Gas evolution was observed. Then the reaction mixture was allowed to cool down to ambient temperature, and was poured into crashed ice (300 g). The precipitate was filtered and washed with water affording 5-biphenyl-3-yl-2-(chloromethyl)-1,3-oxazole as (6.092 g, 100% purity, 95% yield) a brown solid. ¹H NMR (500 MHz, CDCl₃) δ 7.88 (s, 1H), 7.68 - 7.62 (m, 3H), 7.60 (d, J = 8.3 Hz, 1H), 7.56 - 7.46 (m, 3H), 7.45 - 7.35 (m, 2H), 4.71 (s, 2H). MS (CI): m/z = 270 [M+H]⁺.

### Example 19: 2-Amino-1-(3-dichloro-4-fluorophenyl)ethanone hydrochloride

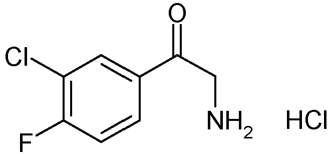

To a stirred solution of 2-bromo-1-(3-chloro-4-fluorophenyl)ethanone (15.215 g, 27.83 mmol) in anhydrous acetonitrile (100 mL) was added sodium diformylamide (8.701 g, 91.561 mmol).

The mixture was stirred at ambient temperature for 72 h. Inorganics was filtered off, and the filtrate was concentrated in vacuo. 5N hydrochloric acid (100 mL) was added to the dark oily residue, and the resulting mixture was heated to reflux for 5 h. The hot solution was separated from dark viscous oil, and was allowed to cool down to ambient temperature. The reaction mixture was concentrated in vacuo, and the residue was crystallized from 5N hydrochloric acid affording 2-amino-1-(3-dichloro-4-fluorophenyl)ethanone hydrochloride (1.683 g, 100% purity, 27% yield) as colorless crystals. ¹H NMR (500 MHz, DMSO-*d₆*) δ 8.41 (br s, 3H), 8.31 - 8.20 (m, 1H), 8.06 (ddt, J = 8.8, 4.8, 2.3 Hz, 1H), 7.66 (t, J = 8.8 Hz, 1H), 4.61 (s, 2H). MS (CI): m/z = 188 [M]+.

### Example 20: N-(2-Oxo-2-(3-chloro-4-fluorophenyl)ethyl)chloroacetamide

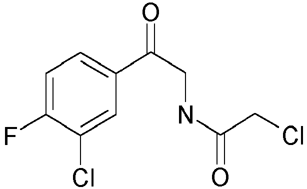

To an ice-chilled stirred slurry of 2-amino-1-(3-chloro-4-fluorophenyl)ethanone hydrochloride (1.71 g, 7.632 mmol) and sodium hydrocarbonate (1.411 g, 16.79 mmol) in ethyl acetate (150 mL)/water (50 mL) mixture, chloroacetyl chloride (0.67 mL, 8.395 mmol) was added dropwise. The reaction mixture was stirred at 0-5°C for 1 h 30 min. The ethyl acetate layer was separated and dried over sodium sulfate. Sodium sulfate was filtered off and the filtrate was concentrated in vacuo affording *N*-(2-oxo-2-(3-chloro-4-fluorophenyl)ethyl)chloroacetamide (1.95 g, 100% purity, 97% yield) as a colorless solid. ¹H NMR (500 MHz, CDCl₃) δ 8.12 - 8.06 (m, 1H), 7.91 (m, 1H), 7.58 (br s, 1H), 7.30 (t, J = 8.8 Hz, 1H), 4.76 (d, J = 4.2 Hz, 2H), 4.16 (s, 2H). MS (CI): m/z = 264 [M+H]⁺.

### Example 21: 5-(3-Chloro-4-fluorophenyl)-2-(chloromethyl)-1,3-oxazole

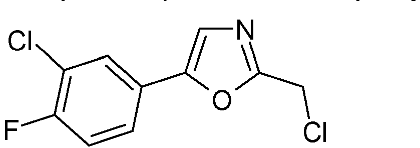

A stirred slurry of *N*-(2-oxo-2-(3-chloro-4-fluorophenyl)ethyl)chloroacetamide (1.946 g, 7.369 mmol) in phosphoryl chloride (16 mL, 171.698 mmol) was heated to reflux for 1.5 h. Gas evolution was observed. Then the reaction mixture was allowed to cool down to ambient temperature and was poured into crashed ice (200 g). The precipitate was filtered and washed with water affording 5-(3-chloro-4-fluorophenyl)-2-(chloromethyl)-1,3-oxazole (1.449 g, 100% purity, 80% yield) as a brown solid. ¹H NMR (400 MHz, CDCl₃) δ 7.69 (dd, J = 6.9, 2.2 Hz, 1H), 7.50 (ddd, J = 8.7, 4.5, 2.2 Hz, 1H), 7.27 (s, 1H), 7.19 (t, J = 8.6 Hz, 1H), 4.64 (s, 2H). MS (CI): m/z = 246 [M+H]⁺.

### Example 22: N-(2-Oxo-2-(1-naphthyl)ethyl)chloroacetamide

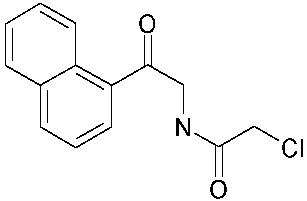

To an ice-chilled stirred slurry of 2-amino-1-(1-naphthyl)ethanone hydrochloride (7.4 g, 33.38 mmol) and sodium hydrocarbonate (6.169 g, 73.436 mmol) in ethyl acetate (300 mL)/water (100 mL) mixture, chloroacetyl chloride (2.92 mL, 36.718 mmol) was added dropwise. The reaction mixture was stirred at 0-5°C for 1 h 30 min. The ethyl acetate layer was separated and dried over sodium sulfate. Sodium sulfate was filtered off and the filtrate was concentrated in vacuo affording *N*-(2-oxo-2-(1-naphthyl)ethyl)chloroacetamide (7.17 g, 100% purity, 82% yield) as a brown solid. m = 7.17 g (yield 82%). ¹H NMR (500 MHz, CDCl₃) δ 8.85 (d, J = 8.7 Hz, 1H), 8.10 (d, J = 8.2 Hz, 1H), 8.03 (d, J = 7.3 Hz, 1H), 7.92 (d, J = 8.2 Hz, 1H), 7.74 (br s, 1H), 7.67 (m, 1H), 7.63 - 7.53 (m, 2H), 4.87 (d, J = 4.6 Hz, 2H), 4.19 (s, 2H). MS (CI): m/z = 262 [M+H]⁺.

### Example 23: 2-(Chloromethyl)-5-(1-naphthyl)-1,3-oxazole

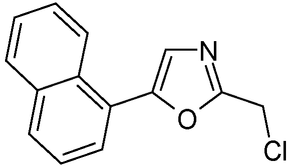

A stirred slurry of *N*-(2-oxo-2-(1-naphthyl)ethyl)chloroacetamide (6.744 g, 25.769 mmol) in phosphoryl chloride (35 mL, 375.454 mmol) was heated to reflux for 2 h. Gas evolution was observed. Then the reaction mixture was allowed to cool down to ambient temperature, and was poured into crashed ice (300 g). Chloroform (300 mL) was added, and the resulting mixture was stirred for 0.5 h. The organic layer was separated and dried over sodium sulfate. Sodium sulfate was filtered off, and the filtrate was concentrated in vacuo affording 2-(chloromethyl)-5-(1-naphthyl)-1,3-oxazole (6.045 g, 100% purity, 96% yield) as a black oil. ¹H NMR (400 MHz, CDCl₃) δ 8.23 (d, J = 7.8 Hz, 1H), 7.90 (m, 2H), 7.74 (dd, J = 7.2, 1.2 Hz, 1H), 7.61 - 7.49 (m, 3H), 7.40 (s, 1H), 4.73 (s, 2H). MS (CI): m/z = 244 [M+H]⁺.

### Example 24: N-(2-Oxo-2-(2-naphthyl)ethyl)chloroacetamide

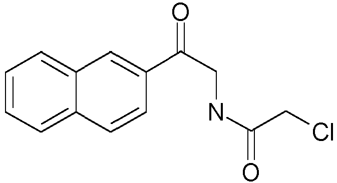

To an ice-chilled stirred slurry of 2-amino-1-(2-naphthyl)ethanone hydrochloride (2.57 g, 11.593 mmol) and sodium hydrocarbonate (2.143 g, 25.505 mmol) in ethyl acetate (150 mL)/water (50 mL) mixture, chloroacetyl chloride (1.02 mL, 12.752 mmol) was added dropwise. The reaction mixture was stirred at 0-5°C for 1 h 30 min. The ethyl acetate layer was separated and dried over sodium sulfate. Sodium sulfate was filtered off, and the filtrate was concentrated in vacuo affording crude *N*-(2-oxo-2-(2-naphthyl)ethyl)chloroacetamide (2.131 g, 96% purity, 67% yield) as a brown solid. ¹H NMR (500 MHz, CDCl₃) δ 8.53 (s, 1H), 8.04 (dd, J = 8.7, 1.9 Hz, 1H), 8.00 (d, J = 8.2 Hz, 1H), 7.95 (d, J = 8.6 Hz, 1H), 7.91 (d, J = 8.2 Hz, 1H), 7.76 (br s, 1H), 7.66 (t, J = 7.5 Hz, 1H), 7.61 (t, J = 7.5 Hz, 1H), 4.95 (d, J = 4.3 Hz, 2H), 4.19 (s, 2H). MS (CI): m/z = 262 [M+H]⁺.

### Example 25: 2-(Chloromethyl)-5-(2-naphthyl)-1,3-oxazole

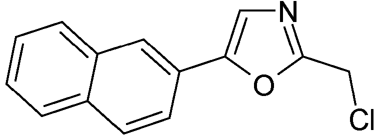

A stirred slurry of *N*-(2-oxo-2-(2-naphthyl)ethyl)chloroacetamide (2.082 g, 7.955 mmol) in phosphoryl chloride (15 mL, 160.93 mmol) was heated to reflux for 3 h. Gas evolution was observed. Then the reaction mixture was allowed to cool down to ambient temperature, and was poured into crashed ice (200 g). The mixture was allowed to warm up to ambient temperature, and was extracted with dichloromethane (3 x 100 mL). The dichloromethane solution was dried over sodium sulfate. Sodium sulfate was filtered off, and the filtrate was concentrated in vacuo affording crude 2-(chloromethyl)-5-(2-naphthyl)-1,3-oxazole as a brown oil. It was subjected to column chromatography on Silicagel (0.040-0.060) (ethyl acetate / hexane) affording 2-(chloromethyl)-5-(2-naphthyl)-1,3-oxazole (1.281 g, 97% purity, 66% yield) as a yellow solid. ¹H NMR (400 MHz, CDCl₃) δ 8.13 (s, 1H), 7.87 (m, 2H), 7.85 - 7.79 (m, 1H), 7.70 (dd, J = 8.6, 1.7 Hz, 1H), 7.50 (tt, J = 7.3, 3.6 Hz, 2H), 7.41 (d, J = 1.3 Hz, 1H), 4.70 (d, J = 1.3 Hz, 2H). MS (CI): m/z = 244 [M+H]⁺.

### Example 26: 2-Amino-1-(3-trifluoromethoxyphenyl)ethanone hydrochloride

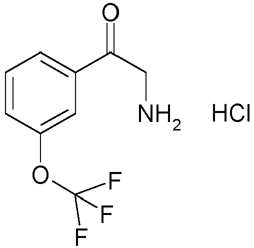

To a stirred solution of 2-bromo-1-(3-trifluoromethoxyphenyl)ethanone (11.45 g, 34.384 mmol) in anhydrous ethyl acetate (100 mL) was added sodium diformylamide (8.169 g, 85.96 mmol). The mixture was stirred at ambient temperature for 96 h. Inorganics was filtered off, and the filtrate was concentrated in vacuo. 5N hydrochloric acid (100 mL) was added to the dark oily residue, and the resulting mixture was heated to reflux for 4 h. The hot solution was separated from dark viscous oil, and was allowed to cool down to 0°C. The precipitate formed was filtered and successively washed with 10N hydrochloric acid and diethyl ether affording 2-amino-1-(3-trifluoromethoxyphenyl)ethanone hydrochloride (2.785 g, 100% purity, 32% yield) as colorless crystals. ¹H NMR (400 MHz, DMSO-*d₆*) δ 8.49 (s, 3H), 8.06 (m, 1H), 7.94 (br s, 1H), 7.75 (m, 2H), 4.62 (s, 2H). ¹⁹F NMR (376 MHz, DMSO-*d₆*) δ -57.34. MS (CI): m/z = 220 [M]⁺.

### Example 27: N-(2-Oxo-2-(3-trifluoromethoxyphenyl)ethyl)chloroacetamide

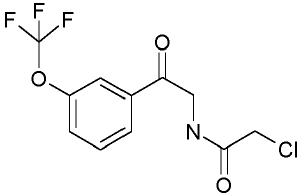

To an ice-chilled stirred slurry of 2-amino-1-(3-trifluoromethoxyphenyl)ethanone hydrochloride (2.785 g, 10.895 mmol) and sodium hydrocarbonate (2.014 g, 23.969 mmol) in ethyl acetate (150 mL)/water (50 mL) mixture, chloroacetyl chloride (0.95 mL, 11.985 mmol) was added dropwise. The reaction mixture was stirred at 0-5°C for 1.5 h. The ethyl acetate layer was separated and dried over sodium sulfate. Sodium sulfate was filtered off, and the filtrate was concentrated in vacuo affording *N*-(2-oxo-2-(3-trifluoromethoxyphenyl)ethyl)chloroacetamide (3.139 g, 94% purity, 92% yield) as a yellow solid. ¹H NMR (500 MHz, CDCl₃) δ 7.96 - 7.89 (d, J = 7.7 Hz, 1H), 7.85 (s, 1H), 7.60 (m, 2H), 7.51 (d, J = 8.0 Hz, 1H), 4.81 (d, J = 4.4 Hz, 2H), 4.16 (s, 2H). ¹⁹F NMR (470 MHz, CDCl₃) δ - 57.94. MS (CI): m/z = 296 [M+H]⁺.

### Example 28: 2-(Chloromethyl)-5-(3-trifluoromethoxyphenyl)-1,3-oxazole

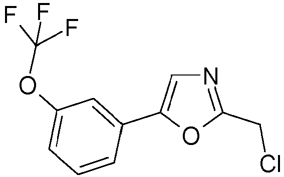

A stirred slurry of *N*-(2-oxo-2-(3-trifluoromethoxyphenyl)ethyl)chloroacetamide (3.139 g, 9.998 mmol) in phosphoryl chloride (22 mL, 236.027 mmol) was heated under reflux for 2 h. Gas evolution was observed. Then, the reaction mixture was allowed to cool down to ambient temperature and was poured into crashed ice (300 g). The mixture was allowed to warm up to ambient temperature and was extracted with chloroform (2 x 75 mL). The dichloromethane solution was dried over sodium sulfate. Sodium sulfate was filtered off and the filtrate was concentrated in vacuo affording brown oil. m = 2.68 g. It was subjected to column chromatography on Silicagel (0.040-0.060) (ethyl acetate/hexane) affording 2-(chloromethyl)-5-(3-trifluoromethoxyphenyl)-1,3-oxazole (1.973 g, 100% purity, 71% yield) as a brown oil. ¹H NMR (400 MHz, CDCl₃) δ 7.57 (dt, J = 7.9, 1.3 Hz, 1H), 7.48 (s, 1H), 7.45 (t, J = 8.1 Hz, 1H), 7.35 (s, 1H), 7.19 (d, J = 8.3 Hz, 1H), 4.66 (s, 2H). ¹⁹F NMR (376 MHz, CDCl₃) δ -58.27. MS (CI): m/z = 278 [M+H]⁺.

### Example 29: 2-Amino-1-(2-chloro-5-methoxyphenyl)ethan-1-one hydrochloride

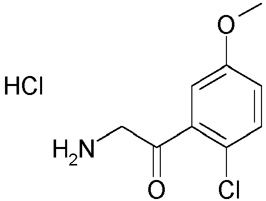

To a stirred solution of 2-bromo-1-(2-chloro-5-methoxyphenyl)ethan-1-one (3.31 g, 12.57 mmol) in anhydrous acetonitrile (70 mL) was added sodium diformylamide (2.99 g, 31.42 mmol) in one portion. The reaction mixture was stirred at ambient temperature for 60 h. The precipitate was filtered off and the filtrate was concentrated in vacuo. Then, 5N hydrochloric acid (50 mL) was added to the dark oily residue, and the resulting mixture was heated under reflux for 3.5 h. The hot solution was separated from dark viscous oil and was allowed to cool down to 0°C. The formed precipitate was filtered and washed with diethyl ether affording 2-amino-1-(2-chloro-5-methoxyphenyl)ethan-1-one hydrochloride (1.25 g, 100% purity, 42% yield) as colorless crystals. ¹H NMR (400 MHz, DMSO-*d₆*) δ 8.37 (br.s, 3H), 7.53 (d, J = 8.9 Hz, 1H), 7.46 (d, J = 3.1 Hz, 1H), 7.23 (dd, J = 8.9, 3.1 Hz, 1H), 4.53 (s, 2H), 3.83 (s, 3H). MS (CI): m/z = 200 [M+H]⁺.

### Example 30: 2-Chloro-N-[2-(2-chloro-5-methoxyphenyl)-2-oxoethyl]acetamide

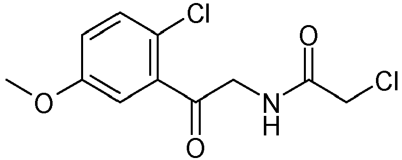

To an ice-chilled stirred slurry of 2-amino-1-(2-chloro-5-methoxyphenyl)ethan-1-one hydrochloride (1.21 g, 5.11 mmol) and sodium hydrogen carbonate (945.1 mg, 11.25 mmol) in a mixture of ethyl acetate (75 mL)/water (25 mL), 2-chloroacetyl chloride (635.32 mg, 5.63 mmol, 450.0 µl) was added dropwise. The reaction mixture was stirred at 0-5°C for 1.5 h. The ethyl acetate layer was separated and dried over sodium sulfate. Sodium sulfate was filtered off, and the filtrate was concentrated in vacuo affording 2-chloro-N-[2-(2-chloro-5-methoxyphenyl)-2-oxoethyl]acetamide (1.2 g, 100% purity, 85% yield) as a beige solid. ¹H NMR (500 MHz, CDCl₃) δ 7.52 (s, 1H), 7.37 (d, J = 8.9 Hz, 1H), 7.19 (d, J = 3.1 Hz, 1H), 7.02 (dd, J = 8.8, 3.1 Hz, 1H), 4.77 (d, J = 4.8 Hz, 2H), 4.14 (s, 2H), 3.84 (s, 3H). MS (CI): m/z = 276 [M+H]⁺.

### Example 31: 5-(2-Chloro-5-methoxyphenyl)-2-(chloromethyl)-1,3-oxazole

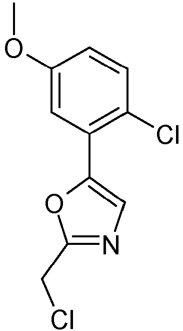

A stirred slurry of 2-chloro-N-[2-(2-chloro-5-methoxyphenyl)-2-oxoethyl]acetamide (1.17 g, 4.24 mmol) in phosphoryl trichloride (24.63 g, 160.6 mmol, 14.97 ml) was heated under reflux for 2 h. Gas evolution was observed. Then, the reaction mixture was allowed to cool down to ambient temperature and was poured into crashed ice (250 g). The precipitate was filtered and washed with water affording 5-(2-chloro-5-methoxyphenyl)-2-(chloromethyl)-1,3-oxazole (980.0 mg, 100% purity, 90% yield) as a beige solid. ¹H NMR (500 MHz, CDCl₃) δ 7.78 (s, 1H), 7.38 (d, J = 8.9 Hz, 1H), 7.34 (d, J = 3.1 Hz, 1H), 6.86 (dd, J = 8.9, 3.1 Hz, 1H), 4.70 (s, 2H), 3.88 (s, 3H). MS (CI): m/z = 258 [M+H]⁺.

### Example 32: 2-Amino-1-(3-chloro-5-methoxyphenyl)ethan-1-one hydrochloride

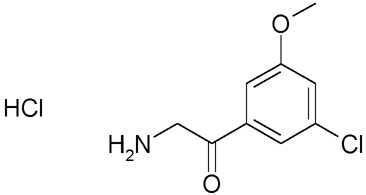

To a stirred solution of 2-bromo-1-(3-chloro-5-methoxyphenyl)ethan-1-one (2.89 g, 10.96 mmol) in anhydrous acetonitrile (56 mL) was added sodium diformylamide (4.01 g, 42.18 mmol) in one portion. The reaction mixture was stirred at rt for 66 h. The precipitate was filtered off and the filtrate was concentrated in vacuo. 5N hydrochloric acid (45 mL) was added to the dark oily residue and the resulting mixture was heated to reflux for 2.5 h. The hot solution was separated from dark viscous oil, and was allowed to cool down to 0°C. The formed precipitate was filtered and washed with diethyl ether affording 2-amino-1-(3-chloro-5-methoxyphenyl)ethan-1-one hydrochloride (1.01 g, 100% purity, 39% yield) as colorless crystals. ¹H NMR (400 MHz, DMSO-*d₆*) δ 8.43 (br.s, 3H), 7.62 (s, 1H), 7.47 (s, 1H), 7.41 (s, 1H), 4.60 (s, 2H), 3.86 (s, 3H). MS (CI): m/z = 200 [M+H]⁺.

### Example 33: 2-Chloro-N-[2-(3-chloro-5-methoxyphenyl)-2-oxoethyl]acetamide

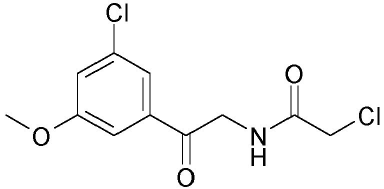

To an ice-chilled stirred slurry of 2-amino-1-(3-chloro-5-methoxyphenyl)ethan-1-one hydrochloride (998.71 mg, 4.23 mmol) and sodium hydrogen carbonate (781.79 mg, 9.31 mmol) in a mixture of ethyl acetate (60 mL)/water (20 mL), 2-chloroacetyl chloride (525.47 mg, 4.65 mmol, 370.0 µl) was added dropwise. The reaction mixture was stirred at 0-5°C for 1.5 h. The ethyl acetate layer was separated and dried over sodium sulfate. Sodium sulfate was filtered off, and the filtrate was concentrated in vacuo affording 2-chloro-N-[2-(3-chloro-5-methoxyphenyl)-2-oxoethyl]acetamide (1.05 g, 100% purity, 90% yield) as a yellow solid. ¹H NMR (500 MHz, CDCl₃) δ 7.58 (br.s, 1H), 7.54 (s, 1H), 7.39 (s, 1H), 7.17 (s, 1H), 4.76 (d, J = 4.2 Hz, 2H), 4.15 (s, 2H), 3.88 (s, 3H). MS (CI): m/z = 276 [M+H]⁺.

### Example 34: 5-(3-Chloro-5-methoxyphenyl)-2-(chloromethyl)-1,3-oxazole

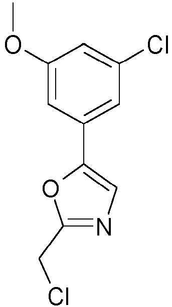

A stirred slurry of 2-chloro-*N*-[2-(3-chloro-5-methoxyphenyl)-2-oxoethyl]acetamide (986.36 mg, 3.57 mmol) in phosphoryl trichloride (19.74 g, 128.74 mmol, 12.0 ml) was heated under reflux for 2.5 h. Gas evolution was observed. Then, the reaction mixture was allowed to cool down to ambient temperature and was poured into crashed ice (250 g). The precipitate was filtered and washed with water affording 5-(3-chloro-5-methoxyphenyl)-2-(chloromethyl)-1,3-oxazole (890.0 mg, 99% purity, 97% yield) as a yellow solid. ¹H NMR (400 MHz, CDCl₃) δ 7.31 (s, 1H), 7.22 (s, 1H), 7.04 (s, 1H), 6.86 (s, 1H), 4.65 (s, 2H), 3.83 (s, 3H). MS (CI): m/z = 258 [M+H]⁺.

### Example 35: 2-Amino-1-(2-chloro-3-methoxyphenyl)ethan-1-one hydrochloride

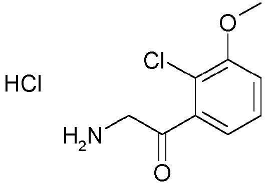

To a stirred solution of 2-bromo-1-(2-chloro-3-methoxyphenyl)ethan-1-one (6.39 g, 24.25 mmol) in anhydrous acetonitrile (70 mL) was added sodium diformylamide (5.18 g, 54.56 mmol) in one portion. The reaction mixture was stirred at ambient temperature for 48 h. The precipitate was filtered off and the filtrate was concentrated in vacuo. 5N hydrochloric acid (50 mL) was added to the dark oily residue and the resulting mixture was heated to reflux for 4 h. The hot solution was separated from dark viscous oil, and was allowed to cool down to 0°C. The formed precipitate was filtered and successively washed with 10N hydrochloric acid and diethyl ether affording 2-amino-1-(2-chloro-3-methoxyphenyl)ethan-1-one hydrochloride (1.82 g, 100% purity, 32% yield) as yellow crystals. ¹H NMR (500 MHz, DMSO-*d₆*) δ 8.50 (s, 3H), 7.49 (t, J = 7.9 Hz, 1H), 7.45 - 7.37 (m, 2H), 4.45 (q, J = 5.5 Hz, 2H), 3.90 (s, 3H). MS (CI): m/z = 200 [M+H]⁺.

### Example 36: 2-Chloro-N-[2-(2-chloro-3-methoxyphenyl)-2-oxoethyl]acetamide

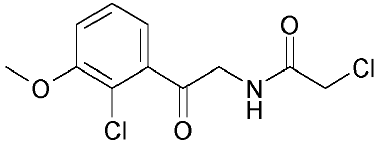

To an ice-chilled stirred slurry of 2-amino-1-(2-chloro-3-methoxyphenyl)ethan-1-one hydrochloride (1.82 g, 7.7 mmol) and sodium hydrogen carbonate (1.42 g, 16.95 mmol) in ethyl acetate (100 mL)/water (30 mL) mixture, 2-chloroacetyl chloride (957.03 mg, 8.47 mmol, 670.0 µl) was added dropwise. The reaction mixture was stirred at 0-5°C for 1.5 h. The ethyl acetate layer was separated and dried over sodium sulfate. Sodium sulfate was filtered off and the filtrate was concentrated in vacuo affording 2-chloro-N-[2-(2-chloro-3-methoxyphenyl)-2-oxoethyl]acetamide (1.77 g, 98% purity, 81% yield) as a beige solid. ¹H NMR (500 MHz, CDCl₃) δ 7.51 (br s, 1H), 7.35 (td, J = 8.0, 2.3 Hz, 1H), 7.20 - 7.14 (m, 1H), 7.10 (dt, J = 8.3, 1.9 Hz, 1H), 4.71 (s, 2H), 4.14 (s, 2H), 3.95 (s, 3H). MS (CI): m/z = 276 [M+H]⁺.

### Example 37: 5-(2-Chloro-3-methoxyphenyl)-2-(chloromethyl)-1,3-oxazole

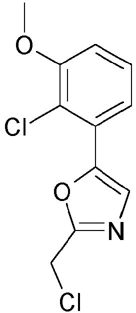

A stirred slurry of 2-chloro-*N*-[2-(2-chloro-3-methoxyphenyl)-2-oxoethyl]acetamide (1.77 g, 6.39 mmol) in phosphoryl trichloride (26.34 g, 171.76 mmol, 16.01 ml) was heated under reflux for 2 h. Gas evolution was observed. Then, the reaction mixture was allowed to cool down to ambient temperature and was poured into crashed ice (300 g). The precipitate was filtered and washed with water affording 5-(2-chloro-3-methoxyphenyl)-2-(chloromethyl)-1,3-oxazole (1.36 g, 100% purity, 83% yield) as a brown solid. ¹H NMR (500 MHz, CDCl₃) δ 7.79 (s, 1H), 7.44 (d, J = 8.0 Hz, 1H), 7.32 (t, J = 8.0 Hz, 1H), 6.95 (d, J = 8.2 Hz, 1H), 4.69 (s, 2H), 3.95 (s, 3H). MS (CI): m/z = 258 [M+H]⁺.

### Example 38: 1-[3-Chloro-4-(3-methanesulfonylpropoxy)phenyl]ethan-1-one

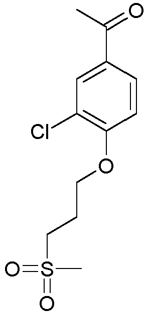

To a stirred solution of 3'-chloro-4'-hydroxyacetophenone (5.5 g, 32.24 mmol) in anhydrous DMF (75 mL) were successively added 1-bromo-3-methanesulfonylpropane (7.78 g, 38.69 mmol) and anhydrous potassium carbonate (8.91 g, 64.48 mmol). The reaction mixture was heated 70°C for 48 h. Inorganics was filtered off and the filtrate was concentrated in vacuo. The residue was dissolved in dichloromethane (200 mL) and was successively washed with water (2 x 200 mL), 1% aq. NaOH (200 mL) and dried over sodium sulfate. Sodium sulfate was filtered off and the filtrate was concentrated in vacuo affording 1-[3-chloro-4-(3-methanesulfonylpropoxy)phenyl]ethan-1-one (9.31 g, 98% purity, 97% yield) as a yellow solid. ¹H NMR (400 MHz, CDCl₃) δ 7.98 (d, J = 2.1 Hz, 1H), 7.83 (dd, J = 8.6, 2.1 Hz, 1H), 6.93 (d, J = 8.6 Hz, 1H), 4.25 (t, J = 5.8 Hz, 2H), 3.30 (t, J = 7.5 Hz, 2H), 2.96 (s, 3H), 2.54 (s, 3H), 2.43 (p, J = 6.2 Hz, 2H). MS (CI): m/z = 291 [M+H]⁺.

### Example 39: 2-Bromo-1-[3-chloro-4-(3-methanesulfonylpropoxy)phenyl]ethan-1-one

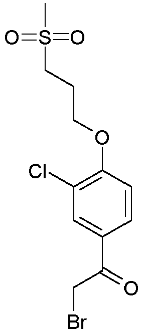

To an ice-chilled solution of 1-[3-chloro-4-(3-methanesulfonylpropoxy)phenyl]ethan-1-one (9.3 g, 31.98 mmol) in anhydrous THF (200 mL) *N,N,N*-trimethylanilinium dibromane bromide (12.62 g, 33.58 mmol) was added portionwise. Then, the reaction mixture was allowed to warm up to ambient temperature and stirred for 18 h. The precipitate was filtered off and the filtrate was concentrated in vacuo affording crude target bromoketone as a dark solid. m = 16.3 g. It was subjected to column chromatography on Silicagel (0.040-0.060) affording 2-bromo-1-[3-chloro-4-(3-methanesulfonylpropoxy)phenyl]ethan-1-one (4.33 g, 85% purity, 31% yield) as a colorless solid. It was used in the next step without further purification. ¹H NMR (400 MHz, CDCl₃) δ 8.06 - 7.98 (m, 1H), 7.87 (d, J = 8.7 Hz, 1H), 7.01 - 6.92 (m, 1H), 4.35 (s, 2H), 4.28 (q, J = 5.9 Hz, 2H), 3.30 (t, J = 7.5 Hz, 2H), 2.97 (s, 3H), 2.44 (t, J = 7.3 Hz, 2H). MS (CI): m/z = 369 [M]⁺.

### Example 40: 2-Amino-1-[3-chloro-4-(3-methanesulfonylpropoxy)phenyl]ethan-1-one hydrochloride

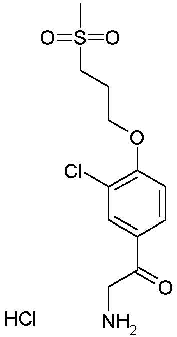

To a stirred solution of 2-bromo-1-[3-chloro-4-(3-methanesulfonylpropoxy)phenyl]ethan-1-one (4.33 g, 11.71 mmol) in anhydrous acetonitrile (110 mL) was added sodium diformylamide (2.84 g, 29.86 mmol) in one portion. The reaction mixture was stirred at ambient temperature for 72 h. The precipitate was filtered off and the filtrate was concentrated in vacuo. 5N Hydrochloric acid (70 mL) was added to the dark oily residue, and the resulting mixture was heated to reflux for 5 h. The hot solution was separated from dark viscous oil and was allowed to cool down to ambient temperature. The precipitate formed was filtered and washed with diethyl ether affording 2-amino-1-[3-chloro-4-(3-methanesulfonylpropoxy)phenyl]ethan-1-one hydrochloride (2.92 g, 90% purity, 77% yield) as a yellow solid. ¹H NMR (500 MHz, DMSO-*d₆*) δ 8.40 (br s, 3H), 8.08 (d, J = 2.3 Hz, 1H), 8.00 (dq, J = 7.3, 5.2, 3.8 Hz, 1H), 7.33 (dd, J = 8.8, 2.0 Hz, 1H), 4.54 (s, 2H), 4.33 (t, J = 6.3 Hz, 2H), 3.32 - 3.27 (m, 2H), 3.03 (s, 3H), 2.22 (dq, J = 12.9, 6.5 Hz, 2H). MS (CI): m/z = 306 [M+H]⁺.

### Example 41: 2-Chloro-N-2-[3-chloro-4-(3-methanesulfonylpropoxy)phenyl]-2-oxoethylacetamide

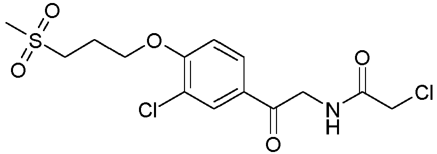

To an ice-chilled stirred slurry of 2-amino-1-[3-chloro-4-(3-methanesulfonylpropoxy)phenyl]ethan-1-one hydrochloride (2.9 g, 8.47 mmol) and sodium hydrogen carbonate (1.57 g, 18.64 mmol) in a mixture of ethyl acetate (120 mL)/water (40 mL), 2-chloroacetyl chloride (1.05 g, 9.32 mmol, 740.0 µl) was added dropwise. The reaction mixture was stirred at 0-5°C for 1.5 h. The precipitate was filtered and washed with diethyl ether affording crude 2-chloro-*N*-2-[3-chloro-4-(3-methanesulfonylpropoxy)phenyl]-2-oxoethylacetamide (2.28 g, 83 % purity, 58% yield) as a beige solid. The product was used in the next step without further purification. ¹H NMR (400 MHz, DMSO-*d₆*) δ 8.53 (s, 1H), 8.28 (s, 1H), 8.12 - 7.93 (m, 1H), 7.29 (d, J = 8.7 Hz, 1H), 4.64 (d, J = 5.5 Hz, 2H), 4.31 (t, J = 6.7 Hz, 2H), 4.19 (s, 2H), 3.27 (m, 2H), 3.03 (s, 3H), 2.23 (d, J = 8.6 Hz, 2H). MS (CI): m/z = 382 [M+H]⁺.

### Example 42: 5-[3-Chloro-4-(3-methanesulfonylpropoxy)phenyl]-2-(chloromethyl)-1,3-oxazole

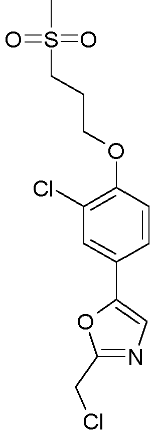

A stirred slurry of 2-chloro-*N*-2-[3-chloro-4-(3-methanesulfonylpropoxy)phenyl]-2-oxoethylacetamide (2.28 g, 5.96 mmol) in phosphoryl trichloride (23.03 g, 150.2 mmol, 14.0 ml) was heated to reflux for 1 h. Gas evolution was observed. Then, the reaction mixture was allowed to cool down to ambient temperature and was poured into crashed ice (250 g). Then, chloroform (200 mL) was added and the resulting mixture was stirred for 30 min. The organic layer was separated and dried over sodium sulfate. Sodium sulfate was filtered off and the filtrate was concentrated in vacuo affording 5-[3-chloro-4-(3-methanesulfonylpropoxy)phenyl]-2-(chloromethyl)-1,3-oxazole (1.64 g, 96% purity, 87% yield) as a brown solid. ¹H NMR (500 MHz, CDCl₃) δ 7.69 (d, J = 2.1 Hz, 1H), 7.52 (dd, J = 8.6, 2.1 Hz, 1H), 7.24 (s, 1H), 6.98 (dd, J = 8.6, 1.7 Hz, 1H), 4.67 (d, J = 1.8 Hz, 2H), 4.25 (t, J = 5.8 Hz, 2H), 3.34 (t, J = 7.6 Hz, 2H), 2.99 (s, 3H), 2.44 (ddd, J = 13.1, 9.5, 5.7 Hz, 2H). MS (CI): m/z = 364 [M+H]⁺.

### Example 43: 4-Methyl-6-(methylamino)pyrimidine-2-thiol

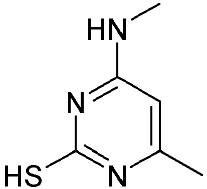

A stirred mixture of 2-chloro-*N*,6-dimethylpyrimidin-4-amine (5.17 g, 32.804 mmol) and thiourea (2.747 g, 36.084 mmol) in anhydrous ethanol (70 mL) was heated under reflux for 15 h. The reaction mixture was concentrated in vacuo and the residue was subjected to column chromatography affording 4-methyl-6-(methylamino)pyrimidine-2-thiol (0.45 g, 92% purity, 9% yield) as a colorless solid. ¹H NMR (500 MHz, DMSO-*d₆*) δ 11.88 (s, 1H), 7.83 - 7.76 (m, 1H), 5.72 (s, 1H), 2.80 (d, J = 3.2 Hz, 3H), 2.05 (s, 3H). MS (CI): m/z = 156 [M+H]⁺.

### Example 44: (E)-N-[Amino([5-(3-methoxyphenyl)-1,3-oxazol-2-yl]methylsulfanyl)methyl-idene]guanidine hydrochloride

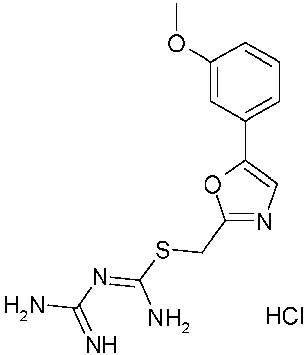

To a stirred solution of 2-(chloromethyl)-5-(3-methoxyphenyl)-1,3-oxazole (420.0 mg, 1.88 mmol) in anhydrous acetonitrile (4 mL) was added of 1-(diaminomethylene)thiourea (221.73 mg, 1.88 mmol). The reaction mixture was heated 60°C for 4 h. The precipitate was filtered and successively washed with acetonitrile (2 x 5 mL) and acetone (3 x 5 mL) affording (*E*)-*N-*[amino([5-(3-methoxyphenyl)-1,3-oxazol-2-yl]methylsulfanyl)methylidene]guanidine hydrochloride (309.0 mg, 75% purity, 40% yield) as a brown solid. It was used in the next step without further purification. ¹H NMR (500 MHz, DMSO-*d₆*) δ 8.17 (s, 2H), 8.10 - 7.71 (m, 4H), 7.66 (s, 1H), 7.38 (t, J = 8.0 Hz, 1H), 7.26 (d, J = 8.0 Hz, 1H), 7.22 (s, 1H), 7.00 - 6.91 (m, 1H), 4.47 (s, 2H), 3.80 (s, 3H). MS (CI): m/z = 306 [M+H]⁺.

### Example 45: N-[Amino([5-(3-chlorophenyl)-1,3-oxazol-2-yl]methylsulfanyl)methylidene]-guanidine hydrochloride

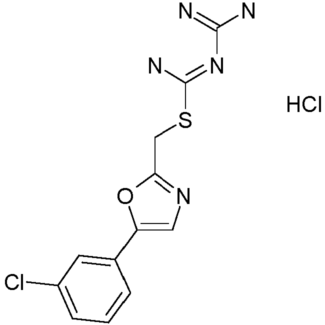

To a stirred solution of 2-(chloromethyl)-5-(3-chlorophenyl)-1,3-oxazole (3.29 g, 14.43 mmol) in anhydrous acetonitrile (100 mL) was added 1-(diaminomethylene)thiourea (1.7 g, 14.42 mmol). The reaction mixture was stirred at ambient temperature for 15 h. The precipitate was filtered off and the filtrate was concentrated in vacuo and treated with acetone (100 mL). The obtained precipitate was filtered and washed with acetone (3 x 50 mL) affording *N*-[amino([5-(3-chlorophenyl)-1,3-oxazol-2-yl]methylsulfanyl)methylidene]guanidine hydrochloride (1.93 g, 95% purity, 37% yield) as a brown solid. ¹H NMR (500 MHz, DMSO-*d₆*) δ 8.20 (s, 2H), 8.12 - 7.86 (m, 4H), 7.77 (s, 2H), 7.65 (d, J = 7.7 Hz, 1H), 7.51 (t, J = 7.9 Hz, 1H), 7.43 (d, J = 8.3 Hz, 1H), 4.48 (s, 2H). MS (CI): m/z = 310 [M+H]⁺.

### Example 46: 1-([5-(3-Chlorophenyl)-1,3-oxazol-2-yl]methylsulfanyl)ethan-1-one

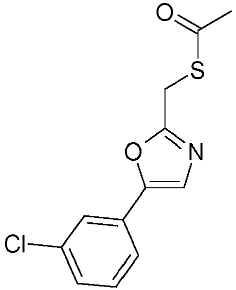

To a stirred solution of 2-(chloromethyl)-5-(3-chlorophenyl)-1,3-oxazole (2.11 g, 9.26 mmol) in anhydrous DMF (55 mL) was added potassium acetylsulfanide (1.16 g, 10.18 mmol). The reaction mixture was stirred at ambient temperature for 22 h. Inorganics was filtered off and the filtrate was concentrated in vacuo. The dark oily residue was dissolved in dichloromethane (150 mL) and washed with water (200 mL). The organic layer was dried over sodium sulfate. Sodium sulfate was filtered off and the filtrate was concentrated in vacuo affording 1-([5-(3-chlorophenyl)-1,3-oxazol-2-yl]methylsulfanyl)ethan-1-one (2.29 g, 100% purity, 92% yield) as a dark oil. ¹H NMR (500 MHz, CDCl₃) δ 7.63 - 7.58 (m, 1H), 7.51 - 7.47 (m, 1H), 7.35 (td, J = 7.9, 2.3 Hz, 1H), 7.32 - 7.29 (m, 1H), 7.29 - 7.25 (m, 1H), 4.32 (s, 2H), 2.44 (s, 3H). MS (CI): m/z = 268 [M+H]⁺.

### Example 47: 1-([5-(2-Chloro-5-methoxyphenyl)-1,3-oxazol-2-yl]methylsulfanyl)ethan-1-one

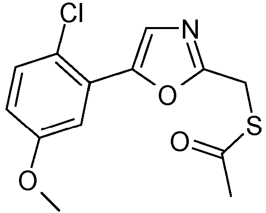

To a stirred solution of 5-(2-chloro-5-methoxyphenyl)-2-(chloromethyl)-1,3-oxazole (0.668 g, 2.588 mmol) in anhydrous DMF (20 mL) was added potassium acetylsulfanide (0.325 g, 2.847 mmol). The reaction mixture was stirred at ambient temperature for 40 h. Inorganics was filtered off and the filtrate was concentrated in vacuo. The dark oily residue was dissolved in dichloromethane (50 mL) and washed with water (150 mL). The organic layer was dried over sodium sulfate. Sodium sulfate was filtered off and the filtrate was concentrated in vacuo affording 1-([5-(2-chloro-5-methoxyphenyl)-1,3-oxazol-2-yl]methylsulfanyl)ethan-1-one (0.617 g, 97% purity, 80% yield) as a dark oil. ¹H NMR (400 MHz, CDCl₃) δ 7.68 (s, 1H), 7.32 (d, J = 8.8 Hz, 1H), 7.26 (d, J = 3.0 Hz, 1H), 6.79 (dd, J = 8.89, 3.0 Hz, 1H), 4.30 (s, 2H), 3.83 (s, 3H), 2.40 (s, 3H). MS (CI): m/z = 298 [M+H]⁺.

### Example 48: 1-([5-(2,5-Dichlorophenyl)-1,3-oxazol-2-yl]methylsulfanyl)ethan-1-one

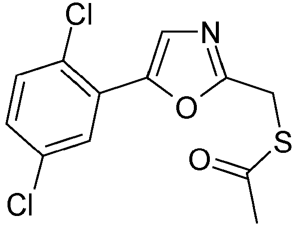

To a stirred solution of 2-(chloromethyl)-5-(2,5-dichlorophenyl)-1,3-oxazole (1.21 g, 4.609 mmol) in anhydrous DMF (40 mL) was added potassium acetylsulfanide (0.579 g, 5.07 mmol). The reaction mixture was stirred at ambient temperature for 40 h. Inorganics was filtered off and the filtrate was concentrated in vacuo. The dark oily residue was dissolved in dichloromethane (100 mL) and washed with water (200 mL). The organic layer was dried over sodium sulfate. Sodium sulfate was filtered off and the filtrate was concentrated in vacuo affording 1-([5-(2,5-dichlorophenyl)-1,3-oxazol-2-yl]methylsulfanyl)ethan-1-one (1.234 g, 100% purity, 89% yield) as a yellow solid. ¹H NMR (400 MHz, CDCl₃) δ 7.74 (d, J = 2.5 Hz, 1H), 7.71 (s, 1H), 7.37 (d, J = 8.6 Hz, 1H), 7.20 (dd, J = 8.6, 2.5 Hz, 1H), 4.30 (s, 2H), 2.41 (s, 3H). MS (CI): m/z = 302 [M+H]⁺.

### Example 49: 2-(Iodomethyl)-6-methylpyrimidin-4(1H)-imine

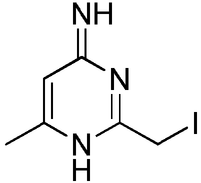

I₂ (19.0 g, 0.15 mmol, 1.5 equiv) and CCl₄ (2.0 mL) were added to the solution of 2,6-dimethylpyrimidin-4-amine (24.6 g, 0.2 mol, 2.0 equiv) in 20 % aqueous solution of H₂SO₄ (75.0 mL). The resulting mixture was heated to reflux and 20 mL of the 20 % aqueous solution of H₂O₂ (11.3 g, 0.1 mol, 1.0 equiv) were added dropwise monitoring that the organic phase was dark red. The resulting mixture was stirred under reflux for 2 h (until the organic phase became yellow). Then, the reaction mixture was cooled to room temperature, aqueous layer was separated and diluted with aqueous solution of NaOH to pH 11. The formed precipitate was filtered on, washed with H₂O (5.0 mL) and dried in vacuo at 70 °C to obtain pure product (10.9 g, 44.0 %): m/z = 249.99 [M+H]⁺.

### Example 50: tert-Butyl 4-((4-amino-6-methylpyrimidin-2-yl)thio)piperidine-1-carboxylate

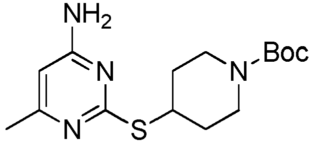

4-Amino-6-methylpyrimidine-2-thiol (1.15 g, 8.16 mmol, 1.15 equiv), NaOH (340.4 mg, 8.51 mmol, 1.2 equiv) and EtOH (20 mL) were mixed together. The resulting mixture was stirred for 10 min at room temperature followed by the dropwise addition of the solution tert-butyl 4-iodopiperidine-1-carboxylate (2.20 g, 7.09 mmol, 1.0 equiv) in hot EtOH (10 mL). Then, the reaction mixture was stirred for 3 h at 70 °C. After all starting material was consumed, as was shown by LCMS, the resulting mixture was allowed to cool down to room temperature and the volatiles were removed under reduced pressure. The obtained residue was subjected to HPLC (Waters Sunfire 19_{*}100 C18 5 mkm column and mixture of H₂O-CH₃OH as a mobile phase) to afford pure product (1.05 g, 43.6 %): m/z = 325.16 [M+H]⁺..

### Example 51: 6-Methyl-2-(piperidin-4-ylthio)pyrimidin-4-amine

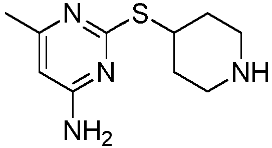

tert-Butyl 4-((4-amino-6-methylpyrimidin-2-yl)thio)piperidine-1-carboxylate (1.0 g, 3.08 mmol, 1.0 equiv) was dissolved in a 4.0 M solution of HCl in dioxane (20.0 mL). The resulting mixture was stirred overnight at room temperature. After the completion of the reaction, monitored by LCMS, the formed precipitate was filtered off, washed wit E₂O (10.0 mL) and air-dried to afford pure product (822.97 mg, 90 %, 2HCl): m/z = 225.11 [M+H]⁺.

### B. Synthesis of final compounds:

### Example 52: 4-([5-(2,5-Dichlorophenyl)-1,3-oxazol-2-yl]methylsulfanyl)-6-ethyl-1,3,5-triazin-2-amine (Z3397119001)

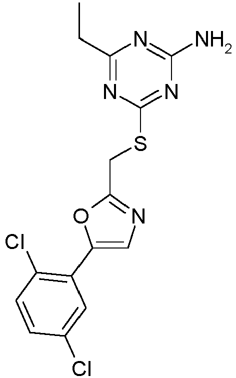

To a stirred solution of 2-(chloromethyl)-5-(2,5-dichlorophenyl)-1,3-oxazole (201.22 mg, 766.48 µmol) in anhydrous DMF (5 mL) were successively added 6-amino-4-ethyl-1,2-dihydro-1,3,5-triazine-2-thione (119.73 mg, 766.48 µmol) and *N,N*-diisopropylethylamine (118.72 mg, 918.58 µmol, 160.0 µl). The resulting mixture was stirred at ambient temperature for 11 h. The volatiles were removed in vacuo. The oily residue was treated with water (5 x 25 mL) and the formed precipitate was filtered affording 4-([5-(2,5-dichlorophenyl)-1,3-oxazol-2-yl]methylsulfanyl)-6-ethyl-1,3,5-triazin-2-amine (260.0 mg, 97% purity, 86% yield) as a beige solid. ¹H NMR (500 MHz, DMSO-*d₆*) δ 7.79 (d, J = 2.6 Hz, 1H), 7.73 (d, J = 2.7 Hz, 1H), 7.62 (dd, J = 8.6, 2.6 Hz, 1H), 7.57 - 7.40 (m, 3H), 4.59 (s, 2H), 3.29 - 3.14 (m, 2H), 1.12 (t, J = 7.6 Hz, 3H). MS (CI): m/z = 382 [M+H]⁺.

### Example 53: 2-([5-(3-Methoxyphenyl)-1,3-oxazol-2-yl]methylsulfanyl)-N,6-dimethylpyrimidin-4-amine (Z3375851526)

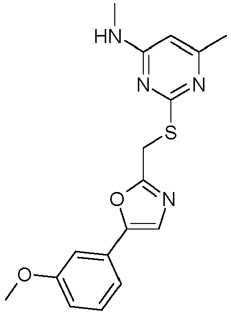

To a stirred solution of 4-methyl-6-(methylamino)pyrimidine-2-thiol (83.28 mg, 536.54 µmol) in anhydrous DMF (2 mL) were successively added 2-(chloromethyl)-5-(3-methoxyphenyl)-1,3-oxazole (100.0 mg, 447.12 µmol) and *N,N*-diisopropylethylamine (86.68 mg, 670.68 µmol, 120.0 µl). The resulting mixture was stirred at ambient temperature for 18 h. The precipitate was filtered off and the filtrate was concentrated in vacuo The dark oily residue was purified by HPLC (eluent MeCN/H₂O 30% => 40%) affording 2-([5-(3-methoxyphenyl)-1,3-oxazol-2-yl]methylsulfanyl)-*N*,6-dimethylpyrimidin-4-amine (66.0 mg, 96% purity, 41% yield) as a beige solid. ¹H NMR (400 MHz, DMSO-*d₆*) δ 7.60 (s, 1H), 7.40 - 7.29 (m, 2H), 7.22 (d, J = 7.7 Hz, 1H), 7.17 (t, J = 2.0 Hz, 1H), 6.95 - 6.89 (m, 1H), 6.03 (s, 1H), 4.52 (s, 2H), 3.79 (s, 3H), 2.76 (d, J = 4.6 Hz, 3H), 2.13 (s, 3H). MS (CI): m/z = 343 [M+H]⁺.

### Example 54: 6-([5-(3-Chlorophenyl)-1,3-oxazol-2-yl]methylsulfanyl)-2-methylpyrimidin-4-amine (Z3402157929)

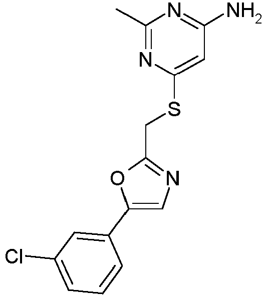

To a stirred solution of 6-amino-2-methylpyrimidine-4-thiol (81.87 mg, 579.82 µmol) in anhydrous DMF (3 mL) were successively added 2-(chloromethyl)-5-(3-chlorophenyl)-1,3-oxazole (115.0 mg, 504.22 µmol) and *N,N*-diisopropylethylamine (97.75 mg, 756.29 µmol, 130.0 µl). The resulted mixture was stirred at ambient temperature for 15 h. The precipitate was filtered off and the filtrate was concentrated in vacuo. The dark oily residue was purified by HPLC (eluent MeCN/H₂O 45% => 55%) affording 6-([5-(3-chlorophenyl)-1,3-oxazol-2-yl]methylsulfanyl)-2-methylpyrimidin-4-amine (12.0 mg, 100% purity, 7% yield) as a grey solid. ¹H NMR (400 MHz, DMSO-*d₆*) δ 7.77 - 7.73 (m, 2H), 7.63 (d, J = 7.7 Hz, 1H), 7.55 (br.s, 2H), 7.50 (t, J = 7.9 Hz, 1H), 7.43 (d, J = 8.0 Hz, 1H), 6.43 (s, 1H), 4.63 (s, 2H), 2.33 (s, 3H). MS (CI): m/z = 333 [M+H]⁺.

### Example 55: 4-([5-(2,5-Dichlorophenyl)-1,3-oxazol-2-yl]methylsulfanyl)-6-methyl-1,3,5-triazin-2-amine (Z3485538332)

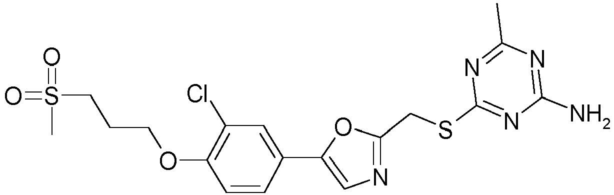

To a stirred solution of 5-[3-chloro-4-(3-methanesulfonylpropoxy)phenyl]-2-(chloromethyl)-1,3-oxazole (100 mg, 275 µmol) in anhydrous DMF (1 mL) were successively added 6-amino-4-methyl-1,2-dihydro-1,3,5-triazine-2-thione (39 mg, 275 µmol) and *N,N-*diisopropylethylamine (43 mg, 330 µmol). The resulting mixture was stirred at ambient temperature for 12 h. The volatiles were removed in vacuo. The oily residue was treated with water (5 x 10 mL), and the precipitate formed was filtered affording 4-([5-(2,5-dichlorophenyl)-1,3-oxazol-2-yl]methylsulfanyl)-6-methyl-1,3,5-triazin-2-amine (104 mg, 100% purity, 80% yield) as a beige solid. ¹H NMR (400 MHz, DMSO-*d₆*) δ 7.76 (s, 1H), 7.63 - 7.47 (m, 4H), 7.25 (d, J = 8.7 Hz, 1H), 4.56 (s, 2H), 4.22 (t, J = 6.3 Hz, 2H), 3.30 - 3.18 (m, 2H), 3.03 (s, 3H), 2.30 - 2.09 (m, 5H). MS (CI): m/z = 470 [M+H]⁺.

### Example 56: 4-([5-(2,5-Dichlorophenyl)-1,3-oxazol-2-yl]methylsulfanyl)-6-ethyl-1,3,5-triazin-2-amine (Z3485538333)

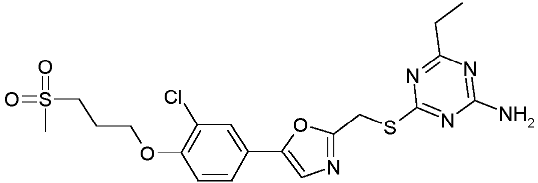

To a stirred solution of 5-[3-chloro-4-(3-methanesulfonylpropoxy)phenyl]-2-(chloromethyl)-1,3-oxazole (100 mg, 275 µmol) in anhydrous DMF (1 mL) were successively added 6-amino-4-ethyl-1,2-dihydro-1,3,5-triazine-2-thione (43 mg, 275 µmol) and *N,N-*diisopropylethylamine (43 mg, 330 µmol). The resulting mixture was stirred at ambient temperature for 12 h. The volatiles were removed in vacuo. The oily residue was treated with water (5 x 10 mL) and the precipitate formed was filtered affording 4-([5-(2,5-dichlorophenyl)-1,3-oxazol-2-yl]methylsulfanyl)-6-ethyl-1,3,5-triazin-2-amine (80 mg, 100% purity, 60% yield) as a beige solid. ¹H NMR (400 MHz, DMSO-*d₆*) δ 7.75 (s, 1H), 7.69 - 7.43 (m, 4H), 7.32 - 7.20 (m, 1H), 4.56 (s, 2H), 4.22 (s, 2H), 3.36 - 3.17 (m, 4H), 3.03 (s, 3H), 2.30 - 2.07 (m, 2H), 1.27 - 0.98 (m, 3H). MS (CI): m/z = 484 [M+H]⁺.

### Example 57: 2-[({5-[3-Chloro-4-(3-methanesulfonylpropoxy)phenyl]-1,3-oxazol-2-yl}methyl)sulfanyl]-6-methylpyrimidin-4-amine (Z3485538331)

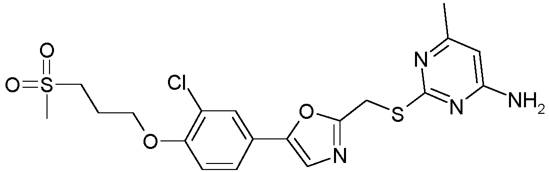

To a stirred solution of 5-[3-chloro-4-(3-methanesulfonylpropoxy)phenyl]-2-(chloromethyl)-1,3-oxazole (100 mg, 275 µmol) in anhydrous DMF (1 mL) were successively added 4-amino-6-methylpyrimidine-2-thiol (39 mg, 275 µmol) and *N,N*-diisopropylethylamine (43 mg, 330 µmol). The resulting mixture was stirred at ambient temperature for 12 h. The volatiles were removed in vacuo. The oily residue was treated with water (5 x 10 mL) and the formed precipitate was filtered affording 2-[({5-[3-chloro-4-(3-methanesulfonylpropoxy)phenyl]-1,3-oxazol-2-yl}methyl)sulfanyl]-6-methylpyrimidin-4-amine (102 mg, 100% purity, 79% yield) as a beige solid. ¹H NMR (400 MHz, DMSO-*d₆*) δ 7.74 (s, 1H), 7.65 - 7.45 (m, 2H), 7.24 (s, 1H), 7.00 - 6.76 (m, 2H), 6.02 (s, 1H), 4.52 (s, 2H), 4.22 (s, 2H), 3.26(m, 2H), 3.03 (s, 3H), 2.34 - 1.89 (m, 5H). MS (CI): m/z = 469 [M+H]⁺.

### Example 58: 2-[({5-[3-Chloro-4-(3-methanesulfonylpropoxy)phenyl]-1,3-oxazol-2-yl}methyl)sulfanyl]-6-(trifluoromethyl)pyrimidin-4-amine (Z3485538334)

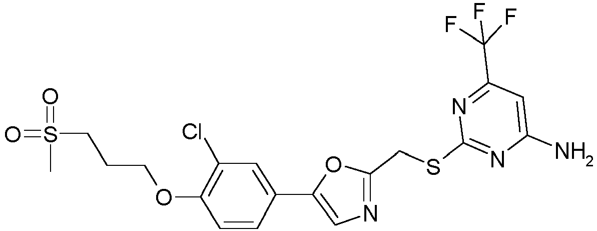

To a stirred solution of 5-[3-chloro-4-(3-methanesulfonylpropoxy)phenyl]-2-(chloromethyl)-1,3-oxazole (60 mg, 165 µmol) in anhydrous DMF (1 mL) were successively added 4-amino-6-(trifluoromethyl)pyrimidine-2-thiol (32 mg, 165 µmol) and *N,N*-diisopropylethylamine (26 mg, 198 µmol, 4 µl). The resulting mixture was stirred at ambient temperature for 12 h. The volatiles were removed in vacuo and the oily residue was subjected to HPLC (eluent MeCN/H₂O) to afford 2-[({5-[3-chloro-4-(3-methanesulfonylpropoxy)phenyl]-1,3-oxazol-2-yl}methyl)sulfanyl]-6-(trifluoromethyl)pyrimidin-4-amine (21 mg, 100% purity, 24% yield) as a solid. ¹H NMR (400 MHz, DMSO-*d₆*) 7.70-7.78 (br.s 2H), δ 7.73 (d, J = 2.2 Hz, 1H), 7.60 - 7.55 (m, 2H), 7.25 (d, J = 8.7 Hz, 1H), 6.57 (s, 1H), 4.54 (s, 2H), 4.23 (t, J = 6.3 Hz, 2H), 3.31 - 3.27 (m, 2H), 3.03 (s, 3H), 2.19 (t, J = 7.7 Hz, 2H). MS (CI): m/z = 523 [M+H]⁺.

### Example 59: 4-Cyclopropyl-6-([5-(3-methoxyphenyl)-1,3-oxazol-2-yl]methylsulfanyl)-1,3,5-triazin-2-amine (Z3399992230)

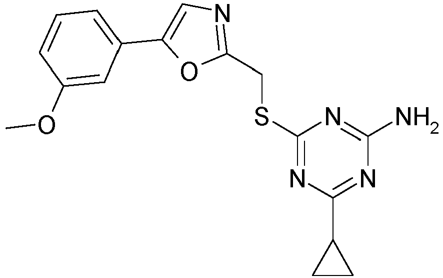

To a stirred slurry of (*E*)-*N*-[amino([5-(3-methoxyphenyl)-1,3-oxazol-2-yl]methylsulfanyl)methylidene]guanidine hydrochloride (70.0 mg, 204.79 µmol) in anhydrous THF (1 mL) were successively added cyclopropanecarbonyl chloride (29.98 mg, 286.76 µmol, 30.0 µl), triethylamine (51.82 mg, 512.08 µmol, 70.0 µl) and sodium sulfate (87.28 mg, 614.49 µmol). The reaction mixture was stirred 45 min at ambient temperature and then was heated 55°C for 5.5 h. The precipitate was filtered off and the filtrate was concentrated in vacuo. The dark oily residue was purified by HPLC (eluent MeCN/_{H2O} 30% => 45%) affording 4-cyclopropyl-6-([5-(3-methoxyphenyl)-1,3-oxazol-2-yl]methylsulfanyl)-1,3,5-triazin-2-amine (11.0 mg, 100% purity, 15% yield) as a yellow oil. ¹H NMR (400 MHz, CDCl₃) δ 7.29 (t, J = 8.0 Hz, 1H), 7.23 (s, 1H), 7.17 (d, J = 7.7 Hz, 1H), 7.10 (t, J = 2.1 Hz, 1H), 6.84 (dd, J = 8.4, 2.6 Hz, 1H), 5.32 (s, 2H), 4.47 (s, 2H), 3.82 (s, 3H), 1.84 (tt, J = 8.4, 4.6 Hz, 1H), 1.13 (dt, J = 6.6, 3.4 Hz, 2H), 0.97 (dq, J = 7.3, 3.8 Hz, 2H). MS (CI): m/z = 356 [M+H]⁺.

### Example 60: 4-([5-(3-Chlorophenyl)-1,3-oxazol-2-yl]methylsulfanyl)-6-(trifluoromethyl)-1,3,5-triazin-2-amine (Z3400108327)

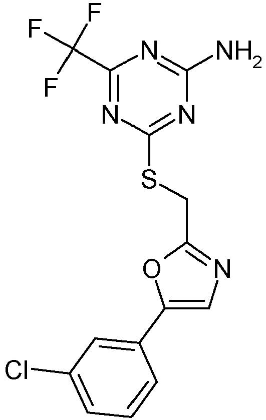

To a stirred slurry of *N*-[amino([5-(3-chlorophenyl)-1,3-oxazol-2-yl]methylsulfanyl)methylidene]guanidine hydrochloride (83.0 mg, 239.72 µmol) in anhydrous THF (1 mL) were successively added trifluoroacetyl 2,2,2-trifluoroacetate (51.38 mg, 244.63 µmol, 30.0 µl), triethylamine (92.22 mg, 911.37 µmol, 130.0 µl) and sodium sulfate (102.2 mg, 719.5 µmol). The reaction mixture was stirred 10 min at ambient temperature, and then was heated 50°C for 6.5 h. The precipitate was filtered off and the filtrate was concentrated in vacuo. The dark oily residue was purified by HPLC (eluent MeCN/H₂O 30% => 40%) affording 4-([5-(3-chlorophenyl)-1,3-oxazol-2-yl]methylsulfanyl)-6-(trifluoromethyl)-1,3,5-triazin-2-amine (2.38 mg, 95% purity, 2% yield) as a grey solid. ¹H NMR (400 MHz, DMSO-*d₆*) δ 8.44 (s, 1H), 8.35 (s, 1H), 7.75 (d, J = 2.0 Hz, 2H), 7.63 (dt, J = 7.8, 1.4 Hz, 1H), 7.49 (t, J = 7.9 Hz, 1H), 7.45 - 7.38 (m, 1H), 4.63 (s, 2H). MS (CI): m/z = 388 [M+H]⁺.

### Example 61: 4-([5-(3-Chlorophenyl)-1,3-oxazol-2-yl]methylsulfanyl)-6-(fluoromethyl)-1,3,5-triazin-2-amine (Z3481547512)

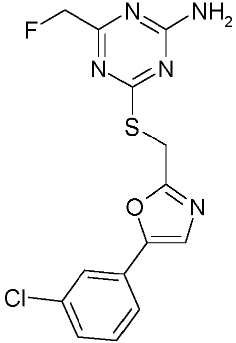

To a stirred under argon solution of 1-([5-(3-chlorophenyl)-1,3-oxazol-2-yl]methylsulfanyl)ethan-1-one (364 mg, 1360 µmol) in DMSO (3 mL) were successively added 4-chloro-6-(fluoromethyl)-1,3,5-triazin-2-amine (221 mg, 1360 µmol) and a solution of potassium hydroxide (114 mg, 2040 µmol) in water (0.3 mL). The mixture was stirred at ambient temperature for 16 h. The precipitate was filtered off, and the filtrate was subjected to HPLC (eluent MeCN/H₂O 40% => 50%) affording 4-([5-(3-chlorophenyl)-1,3-oxazol-2-yl]methylsulfanyl)-6-(fluoromethyl)-1,3,5-triazin-2-amine (21 mg, 100% purity, 4% yield) as a brown solid. ¹H NMR (400 MHz, DMSO-*d₆*) δ 7.90 (s, 1H), 7.80 (s, 1H), 7.75 (s, 1H), 7.74 (s, 1H), 7.63 (d, J = 7.7 Hz, 1H), 7.49 (t, J = 7.9 Hz, 1H), 7.42 (d, J = 8.1 Hz, 1H), 5.18 (d, J = 46.5 Hz, 2H), 4.60 (s, 2H). MS (CI): m/z = 352 [M+H]⁺.

### Example 62: 4-([5-(3-Chlorophenyl)-1,3-oxazol-2-yl]methylsulfanyl)-6-(difluoromethyl)-1,3,5-triazin-2-amine (Z3400108328)

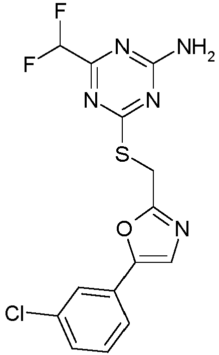

To a stirred under argon solution of 1-([5-(3-chlorophenyl)-1,3-oxazol-2-yl]methylsulfanyl)ethan-1-one (142.35 mg, 531.67 µmol) in DMSO (1 mL) were successively added 4-chloro-6-(difluoromethyl)-1,3,5-triazin-2-amine (96.0 mg, 531.73 µmol) and a solution of potassium hydroxide (44.74 mg, 797.51 µmol) in water (0.1 mL). The mixture was stirred at ambient temperature for 16 h. The precipitate was filtered off and the filtrate was subjected to HPLC (eluent MeCN/H₂O 40% => 50%) affording 4-([5-(3-chlorophenyl)-1,3-oxazol-2-yl]methylsulfanyl)-6-(difluoromethyl)-1,3,5-triazin-2-amine (21.7 mg, 100% purity, 11% yield) as a brown solid. ¹H NMR (400 MHz, DMSO-*d₆*) δ 8.19 (s, 1H), 8.10 (s, 1H), 7.78 - 7.72 (m, 2H), 7.63 (d, J = 7.8 Hz, 1H), 7.49 (t, J = 7.8 Hz, 1H), 7.42 (d, J = 8.0 Hz, 1H), 6.56 (t, J = 53.7 Hz, 1H), 4.62 (s, 2H). MS (CI): m/z = 370 [M+H]⁺.

### Example 63: 4-([5-(3-Chlorophenyl)-1,3-oxazol-2-yl]methylsulfanyl)-6-(4-methylpiperazin-1-yl)-1,3,5-triazin-2-amine (Z3400108321)

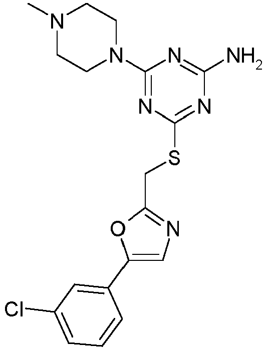

To a stirred under argon solution of 1-([5-(3-chlorophenyl)-1,3-oxazol-2-yl]methylsulfanyl)ethan-1-one (107.0 mg, 399.65 µmol) in DMSO (1 mL) were successively added 4-chloro-6-(4-methylpiperazin-1-yl)-1,3,5-triazin-2-amine (91.38 mg, 399.6 µmol) and a solution of potassium hydroxide (33.63 mg, 599.4 µmol) in water (0.1 mL). The mixture was stirred at ambient temperature for 12 h. The precipitate was filtered off and the filtrate was subjected to HPLC (eluent MeCN/H₂O 30% => 50%) affording 4-([5-(3-chlorophenyl)-1,3-oxazol-2-yl]methylsulfanyl)-6-(4-methylpiperazin-1-yl)-1,3,5-triazin-2-amine (34.9 mg, 100% purity, 21% yield) as a colorless solid. ¹H NMR (400 MHz, DMSO-*d₆*) δ 7.72 (s, 2H), 7.62 (d, J = 7.8 Hz, 1H), 7.49 (t, J = 7.9 Hz, 1H), 7.42 (d, J = 8.1 Hz, 1H), 6.92 (br.s, 2H), 4.46 (s, 2H), 3.75 - 3.58 (m, 4H), 2.32 - 2.14 (m, 4H), 2.12 (s, 3H). MS (CI): m/z = 418 [M+H]⁺.

### Example 64: 2-[4-Amino-6-([5-(3-chlorophenyl)-1,3-oxazol-2-yl]methylsulfanyl)-1,3,5-triazin-2-yl]aminoethan-1-ol (Z3446839933)

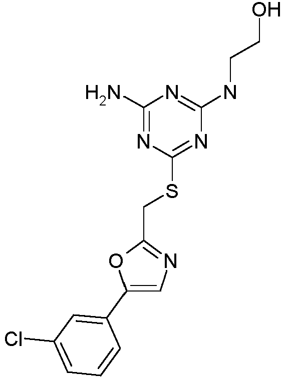

To a stirred under argon solution of 1-([5-(3-chlorophenyl)-1,3-oxazol-2-yl]methylsulfanyl)ethan-1-one (100.0 mg, 373.51 µmol) in DMSO (1 mL) were successively added 2-[(4-amino-6-chloro-1,3,5-triazin-2-yl)amino]ethan-1-ol (70.57 mg, 372.19 µmol) and a solution of potassium hydroxide (31.32 mg, 558.29 µmol) in water (0.1 mL). The mixture was stirred at ambient temperature for 12 h. The precipitate was filtered off, and the filtrate was subjected to HPLC (eluent MeCN/H₂O 30% => 45%) affording 2-[4-amino-6-([5-(3-chlorophenyl)-1,3-oxazol-2-yl]methylsulfanyl)-1,3,5-triazin-2-yl]aminoethan-1-ol (18.4 mg, 100% purity, 13% yield) as a colorless solid. ¹H NMR (400 MHz, DMSO-*d₆*) δ 7.75 (s, 1H), 7.73 (s, 1H), 7.63 (d, J = 7.7 Hz, 1H), 7.49 (t, J = 7.8 Hz, 1H), 7.42 (d, J = 7.8 Hz, 1H), 7.24 - 7.04 (m, 1H), 6.93 (s, 1H), 6.75 (s, 1H), 4.67 - 4.57 (m, 1H), 4.55 (s, 1H), 4.51 (s, 1H), 3.51 - 3.39 (m, 2H), 3.30 - 3.21 (m, 2H). MS (CI): m/z = 379 [M+H]⁺.

### Example 65: 4-([5-(3-Chlorophenyl)-1,3-oxazol-2-yl]methylsulfanyl)-6-(morpholin-4-yl)-1,3,5-triazin-2-amine (Z3400108313)

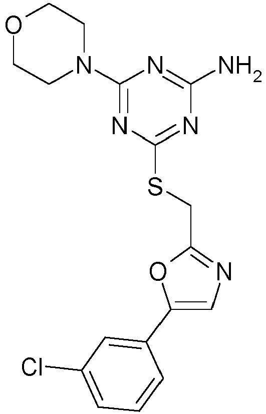

To a stirred under argon solution of 1-([5-(3-chlorophenyl)-1,3-oxazol-2-yl]methylsulfanyl)ethan-1-one (100.0 mg, 373.51 µmol) in DMSO (1 mL) were successively added 4-chloro-6-(morpholin-4-yl)-1,3,5-triazin-2-amine (80.53 mg, 373.45 µmol) and a solution of potassium hydroxide (31.43 mg, 560.17 µmol) in water (0.1 mL). The mixture was stirred at ambient temperature for 12 h. The precipitate was filtered off, and the filtrate was subjected to HPLC (eluent MeCN/H₂O 30% => 50%) affording 4-([5-(3-chlorophenyl)-1,3-oxazol-2-yl]methylsulfanyl)-6-(morpholin-4-yl)-1,3,5-triazin-2-amine (25.2 mg, 100% purity, 17% yield) as a colorless solid. ¹H NMR (400 MHz, DMSO-*d₆*) δ 7.73 (s, 2H), 7.62 (d, J = 7.8 Hz, 1H), 7.50 (t, J = 7.9 Hz, 1H), 7.42 (d, J = 8.2 Hz, 1H), 6.97 (br s, 2H), 4.47 (s, 2H), 3.63 (s, 4H), 3.57 - 3.39 (m, 4H). MS (CI): m/z = 405 [M+H]⁺.

### Example 66: 4-([5-(3-Chlorophenyl)-1,3-oxazol-2-yl]methylsulfanyl)-6-methoxy-1,3,5-triazin-2-amine (Z3400108307)

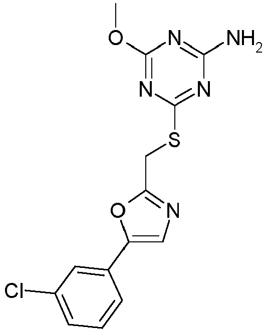

To a stirred under argon solution of 1-([5-(3-chlorophenyl)-1,3-oxazol-2-yl]methylsulfanyl)ethan-1-one (278.6 mg, 1.04 mmol) in methanol (2 mL) was added a solution of potassium hydroxide (80.0 mg, 1.43 mmol) in water (1 mL). The mixture was stirred at rt for 1.5 h. Then a solution of 4-chloro-6-methoxy-1,3,5-triazin-2-amine (173.0 mg, 1.08 mmol) in DMSO (4 mL) was added via a syringe. The resulted mixture was stirred at ambient temperature for additional 12 h. The precipitate was filtered off and washed with methanol, and the filtrate was concentrated in vacuo. The dark oily residue was subjected to HPLC (eluent MeCN/H₂O 30% => 45%) affording 4-([5-(3-chlorophenyl)-1,3-oxazol-2-yl]methylsulfanyl)-6-methoxy-1,3,5-triazin-2-amine (103.0 mg, 100% purity, 28% yield) as a colorless solid. ¹H NMR (400 MHz, DMSO-*d₆*) δ 7.78 - 7.69 (m, 2H), 7.62 (dt, J = 7.8, 1.4 Hz, 1H), 7.57 (s, 2H), 7.49 (t, J = 7.9 Hz, 1H), 7.41 (dt, J = 7.9, 1.6 Hz, 1H), 4.57 (s, 2H), 3.81 (s, 3H). MS (CI): m/z = 350 [M+H]⁺.

### Example 67: 6-({[5-(2,5-Dichlorophenyl)-1,3-oxazol-2-yl]methyl}thio)-N,N-dimethyl-1,3,5-triazine-2,4-diamine (Z3485538342)

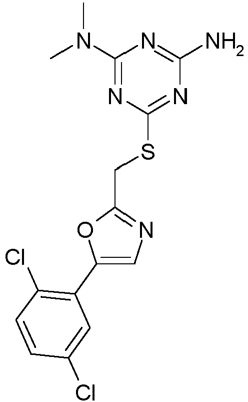

To a stirred under argon solution of 1-([5-(2,5-dichlorophenyl)-1,3-oxazol-2-yl]methylsulfanyl)ethan-1-one (100.0 mg, 331 µmol) in DMSO (1 mL) were successively added 6-chloro-*N*²,*N*²-dimethyl-1,3,5-triazine-2,4-diamine (57 mg, 331 µmol) and a solution of potassium hydroxide (28 mg, 496 µmol) in water (0.1 mL). The mixture was stirred at ambient temperature for 12 h. The precipitate was filtered off and the filtrate was subjected to HPLC (eluent MeCN/H₂O) affording 6-({[5-(2,5-dichlorophenyl)-1,3-oxazol-2-yl]methyl}thio)-N,N-dimethyl-1,3,5-triazine-2,4-diamine (24 mg, 97% purity, 18% yield) as a solid. ¹H NMR (400 MHz, CDCl₃) δ 7.72 (s, 2H), 7.36 (d, J = 8.6 Hz, 1H), 7.19 (dd, J = 8.7, 2.5 Hz, 1H), 4.90 (br.s, 2H), 4.48 (s, 2H), 3.15 (s, 3H), 3.07 (s, 3H). MS (CI): m/z = 397 [M+H]⁺.

### Example 68: 4-([5-(2,5-Dichlorophenyl)-1,3-oxazol-2-yl]methylsulfanyl)-6-(morpholin-4-yl)-1,3,5-triazin-2-amine (Z3485538340)

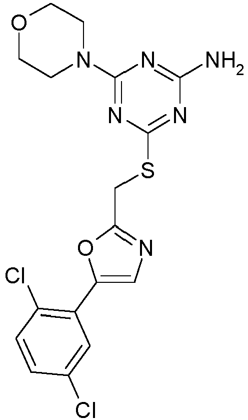

To a stirred under argon solution of 1-([5-(2,5-dichlorophenyl)-1,3-oxazol-2-yl]methylsulfanyl)ethan-1-one (100.0 mg, 331 µmol) in DMSO (1 mL) were successively added 4-chloro-6-(morpholin-4-yl)-1,3,5-triazin-2-amine (71 mg, 331 µmol) and a solution of potassium hydroxide (28 mg, 496 µmol) in water (0.1 mL). The mixture was stirred at ambient temperature for 12 h. The precipitate was filtered off, and the filtrate was subjected to HPLC (eluent MeCN/H₂O) affording 4-([5-(2,5-dichlorophenyl)-1,3-oxazol-2-yl]methylsulfanyl)-6-(morpholin-4-yl)-1,3,5-triazin-2-amine (11 mg, 100% purity, 8% yield) as a solid. ¹H NMR (400 MHz, CDCl₃) δ 7.71 (s, 2H), 7.36 (d, J = 8.8 Hz, 1H), 7.19 (d, J = 9.2 Hz, 1H), 4.95 (s, 2H), 4.43 (s, 2H), 3.76 (s, 4H), 3.64 (s, 4H). MS (CI): m/z = 439 [M+H]⁺.

### Example 69: 4-([5-(2,5-Dichlorophenyl)-1,3-oxazol-2-yl]methylsulfanyl)-6-(4-methylpiperazin-1-yl)-1,3,5-triazin-2-amine (Z3485538341)

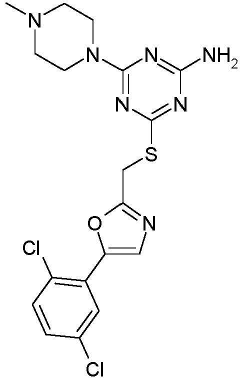

To a stirred under argon solution of 1-([5-(2,5-dichlorophenyl)-1,3-oxazol-2-yl]methylsulfanyl)ethan-1-one (100.0 mg, 331 µmol) in DMSO (1 mL) were successively added 4-chloro-6-(4-methylpiperazin-1-yl)-1,3,5-triazin-2-amine (76 mg, 331 µmol) and a solution of potassium hydroxide (28 mg, 496 µmol) in water (0.1 mL). The mixture was stirred at ambient temperature for 12 h. The precipitate was filtered off, and the filtrate was subjected to HPLC (eluent MeCN/H₂O) affording 4-([5-(2,5-dichlorophenyl)-1,3-oxazol-2-yl]methylsulfanyl)-6-(4-methylpiperazin-1-yl)-1,3,5-triazin-2-amine (5 mg, 100% purity, 3% yield) as a solid. ¹H NMR (400 MHz, CDCl₃) δ 7.73 - 7.68 (m, 2H), 7.36 (d, J = 8.5 Hz, 1H), 7.19 (dd, J = 8.5, 2.5 Hz, 1H), 4.96 (s, 2H), 4.43 (s, 2H), 3.97 - 3.76 (m, 4H), 2.56 - 2.43 (m, 4H), 2.37 (s, 3H). MS (CI): m/z = 452 [M+H]⁺.

### Example 70: 4-([5-(2,5-Dichlorophenyl)-1,3-oxazol-2-yl]methylsulfanyl)-6-(difluoromethyl)-1,3,5-triazin-2-amine (Z3485538343)

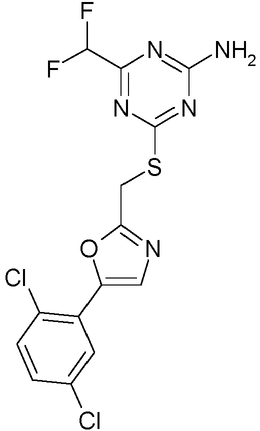

To a stirred under argon solution of 1-([5-(2,5-dichlorophenyl)-1,3-oxazol-2-yl]methylsulfanyl)ethan-1-one (100.0 mg, 331 µmol) in DMSO (1 mL) were successively added 4-chloro-6-(difluoromethyl)-1,3,5-triazin-2-amine (57 mg, 331 µmol) and a solution of potassium hydroxide (28 mg, 496 µmol) in water (0.1 mL). The mixture was stirred at ambient temperature for 12 h. The precipitate was filtered off and the filtrate was subjected to HPLC (eluent MeCN/H₂O) affording 4-([5-(2,5-dichlorophenyl)-1,3-oxazol-2-yl]methylsulfanyl)-6-(difluoromethyl)-1,3,5-triazin-2-amine (17 mg, 95% purity, 13% yield) as a solid. ¹H NMR (400 MHz, CDCl₃) δ 7.72 (s, 2H), 7.37 (d, J = 8.5 Hz, 1H), 7.21 (dd, J = 9.1, 2.4 Hz, 1H), 6.24 (t, J = 54.3 Hz, 1H), 5.67 (br s, 2H), 4.54 (s, 2H). MS (CI): m/z = 404 [M+H]⁺.

### Example 71: 4-([5-(2,5-Dichlorophenyl)-1,3-oxazol-2-yl]methylsulfanyl)-6-(fluoromethyl)-1,3,5-triazin-2-amine (Z3485538344)

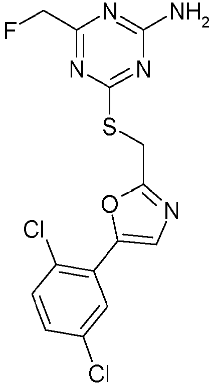

To a stirred under argon solution of 1-([5-(2,5-dichlorophenyl)-1,3-oxazol-2-yl]methylsulfanyl)ethan-1-one (100.0 mg, 331 µmol) in DMSO (1 mL) were successively added 4-chloro-6-(fluoromethyl)-1,3,5-triazin-2-amine (54 mg, 331 µmol) and a solution of potassium hydroxide (28 mg, 496 µmol) in water (0.1 mL). The mixture was stirred at ambient temperature for 12 h. The precipitate was filtered off, and the filtrate was subjected to HPLC (eluent MeCN/H₂O) affording 4-([5-(2,5-dichlorophenyl)-1,3-oxazol-2-yl]methylsulfanyl)-6-(fluoromethyl)-1,3,5-triazin-2-amine (21 mg, 100% purity, 16% yield) as a solid. ¹H NMR (400 MHz, CDCl₃) δ 7.72 (s, 2H), 7.37 (d, J = 8.6 Hz, 1H), 7.20 (dd, J = 8.3, 2.3 Hz, 1H), 6.00 (br s, 1H), 5.61 (br s, 1H), 5.20 (d, J = 46.7 Hz, 2H), 4.53 (s, 2H). MS (CI): m/z = 386 [M+H]⁺.

### Example 72: 4-(([5-(2-Chloro-5-methoxyphenyl)-1,3-oxazol-2-yl]methyl)sulfanyl)-6-(fluoromethyl)-1,3,5-triazin-2-amine (Z3485538337)

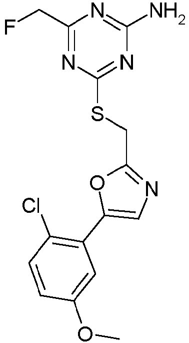

To a stirred under argon solution of 1-([5-(2-chloro-5-methoxyphenyl)-1,3-oxazol-2-yl]methylsulfanyl)ethan-1-one (121.0 mg, 406 µmol) in DMSO (1.2 mL) were successively added 4-chloro-6-(fluoromethyl)-1,3,5-triazin-2-amine (66 mg, 406 µmol) and a solution of potassium hydroxide (34 mg, 609 µmol) in water (0.12 mL). The mixture was stirred at ambient temperature for 12 h. The precipitate was filtered off, and the filtrate was subjected to HPLC (eluent MeCN/H₂O) affording 4-({[5-(2-chloro-5-methoxyphenyl)-1,3-oxazol-2-yl]methyl}sulfanyl)-6-(fluoromethyl)-1,3,5-triazin-2-amine (27 mg, 100% purity, 17% yield) as a solid. ¹H NMR (400 MHz, CDCl₃) δ 7.69 (s, 1H), 7.33 (d, J = 8.7 Hz, 1H), 7.24 (s, 1H), 6.79 (dd, J = 8.9, 3.2 Hz, 1H), 5.52 (br s, 2H), 5.19 (d, J = 46.6 Hz, 2H), 4.54 (s, 2H), 3.82 (s, 3H). MS (CI): m/z = 382 [M+H]⁺.

### Example 73: 4-(([5-(2-Chloro-5-methoxyphenyl)-1,3-oxazol-2-yl]methyl)sulfanyl)-6-(4-methylpiperazin-1-yl)-1,3,5-triazin-2-amine (Z3485538338)

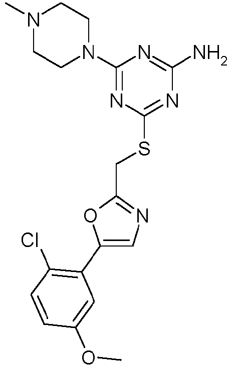

To a stirred under argon solution of 1-([5-(2-chloro-5-methoxyphenyl)-1,3-oxazol-2-yl]methylsulfanyl)ethan-1-one (242.0 mg, 812 µmol) in DMSO (2.4 mL) were successively added 4-chloro-6-(4-methylpiperazin-1-yl)-1,3,5-triazin-2-amine (186 mg, 812 µmol) and a solution of potassium hydroxide (68 mg, 1218 µmol) in water (0.25 mL). The mixture was stirred at ambient temperature for 12 h. The precipitate was filtered off, and the filtrate was subjected to HPLC (eluent MeCN/H₂O) affording 4-({[5-(2-chloro-5-methoxyphenyl)-1,3-oxazol-2-yl]methyl}sulfanyl)-6-(4-methylpiperazin-1-yl)-1,3,5-triazin-2-amine (24 mg, 95% purity, 7% yield) as a solid. ¹H NMR (400 MHz, CDCl₃) δ 7.68 (s, 1H), 7.32 (d, J = 8.9 Hz, 1H), 7.23 (m, 1H), 7.65 (dd, J = 8.5, 2.5 Hz, 1H), 4.87 (s, 2H), 4.44 (s, 2H), 3.81 (s, 3H), 3.46-3.59 (m, 4H), 2.35-2.31 (m, 4H), 2.27 (s, 3H). MS (CI): m/z = 248 [M+H]⁺.

### Example 74: 4-(([5-(2-Chloro-5-methoxyphenyl)-1,3-oxazol-2-yl]methyl}sulfanyl)-6-(morpholin-4-yl)-1,3,5-triazin-2-amine (Z3485538336)

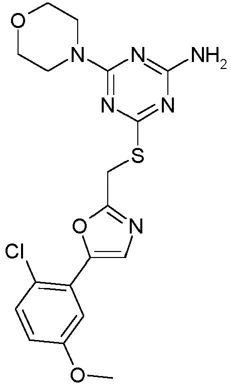

To a stirred under argon solution of 1-([5-(2-chloro-5-methoxyphenyl)-1,3-oxazol-2-yl]methylsulfanyl)ethan-1-one (121.0 mg, 406 µmol) in DMSO (1.2 mL) were successively added 4-chloro-6-(morpholin-4-yl)-1,3,5-triazin-2-amine (88 mg, 406 µmol) and a solution of potassium hydroxide (34 mg, 609 µmol) in water (0.12 mL). The mixture was stirred at ambient temperature for 12 h. The precipitate was filtered off, and the filtrate was subjected to HPLC (eluent MeCN/H₂O) affording 4-({[5-(2-chloro-5-methoxyphenyl)-1,3-oxazol-2-yl]methyl}sulfanyl)-6-(morpholin-4-yl)-1,3,5-triazin-2-amine (30 mg, 100% purity, 17% yield) as a solid. ¹H NMR (400 MHz, CDCl₃) δ 7.67 (s, 1H), 7.32 (d, J = 8.9 Hz, 1H), 7.23 (s, 1H), 6.78 (dd, J = 8.9, 3.1 Hz, 1H), 4.96 (s, 2H), 4.43 (s, 2H), 3.81 (s, 3H), 3.79 - 3.68 (m, 4H), 3.68 - 3.52 (m, 4H). MS (CI): m/z = 435 [M+H]⁺.

### Example 75: 4-({[5-(2-Chloro-5-methoxyphenyl)-1,3-oxazol-2-yl]methyl}sulfanyl)-6-(difluoromethyl)-1,3,5-triazin-2-amine (Z3485538339)

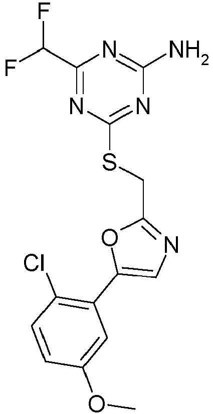

To a stirred under argon solution of 1-([5-(2-chloro-5-methoxyphenyl)-1,3-oxazol-2-yl]methylsulfanyl)ethan-1-one (121.0 mg, 406 µmol) in DMSO (1.2 mL) were successively added 4-chloro-6-(difluoromethyl)-1,3,5-triazin-2-amine (73 mg, 406 µmol) and a solution of potassium hydroxide (34 mg, 609 µmol) in water (0.12 mL). The mixture was stirred at ambient temperature for 12 h. The precipitate was filtered off, and the filtrate was subjected to HPLC (eluent MeCN/H₂O) affording 4-({[5-(2-chloro-5-methoxyphenyl)-1,3-oxazol-2-yl]methyl}sulfanyl)-6-(difluoromethyl)-1,3,5-triazin-2-amine (24 mg, 100% purity, 15% yield) as a solid. ¹H NMR (400 MHz, CDCl₃) δ 7.69 (s, 1H), 7.33 (d, J = 9.1 Hz, 1H), 7.24 - 7.23 (m, 1H), 6.80 (dd, J = 8.3, 2.3 Hz, 1H), 6.23 (t, J = 54.1 Hz, 1H), 5.61 (br s, 2H), 4.56 (s, 2H), 3.81 (s, 3H). MS (CI): m/z = 400 [M+H]⁺.

### Example 76: 6-Methyl-2-((2-(3-(p-tolyl)-1,2,4-oxadiazol-5-yl)ethyl)thio)pyrimidin-4(1H)-imine (Z3214047394)

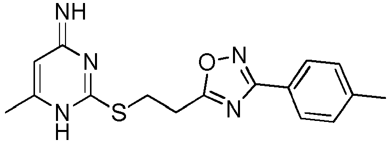

4-Imino-6-methyl-1,4-dihydropyrimidine-2-thiol (28.59 mg, 0.203 mmol, 1.15 equiv), NaOH (8.48 mg, 0.212 mmol, 1.2 equiv) and EtOH (5 mL) were mixed together. The resulting mixture was stirred for 10 min at room temperature followed by the dropwise addition of the solution of 5-(2-chloroethyl)-3-(p-tolyl)-1,2,4-oxadiazole (39.3 mg, 0.176 mmol, 1.0 equiv) in hot EtOH (3 mL). Then, the reaction mixture was stirred for 3 h at 70 °C. After all starting material was consumed, as was shown by LCMS, the resulting mixture was allowed to cool down to room temperature and the volatiles were removed under reduced pressure. The obtained residue was subjected to HPLC (Waters Sunfire 19_{*}100 C18 5 mkm column and mixture of H₂O-CH₃OH as a mobile phase) to afford pure product (36.2 mg, 24.0 %).: m/z = 290 [M+H]⁺.

Following the above procedure all compounds of the series were obtained. The molar ratio of the reagents and reaction conditions were kept the same in each reaction of the series. The exact amounts of the reagents and the yields of the products are in the attached Excel file.

### Example 77: 6-Methyl-2-((3-phenyl-1,2,4-oxadiazol-5-yl)methoxy)pyrimidin-4-amine (Z3214047395)

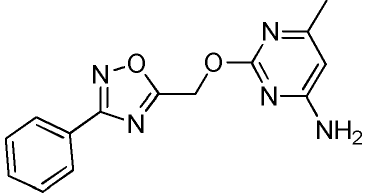

DMF (3.0 mL), NaH (88.8 mg, 3.7 mmol, 1.05 equiv) and (3-phenyl-1,2,4-oxadiazol-5-yl)methanol (650.0 mg, 3.7 mmol, 1.05 equiv) were mixed together in a round-bottom flask. After the completion of gas evolution, 2-chloro-6-methylpyrimidin-4-amine (500.0 mg, 3.5 mmol, 1.0 equiv) was added and the resulting mixture was stirred at room temperature overnight. Then, the reaction mixture was diluted with a mixture of EtOAc (30.0 mL) and H₂O (50.0 mL). The organic phase was separated, dried over Na₂SO₄, filtered off and concentrated under reduced pressure. The residue was purified using HPLC (Waters Sunfire 19_{*}100 C18 5 mkm column and mixture of H₂O-CH₃OH as a mobile phase) to obtain pure product (180.6 mg, 18.23 %): m/z = 284,20 [M+H]⁺.

### Example 78: 2-(((3-(4-Ethylphenyl)-1,2,4-oxadiazol-5-yl)thio)methyl)-6-methylpyrimidin-4-amine (Z3214047396)

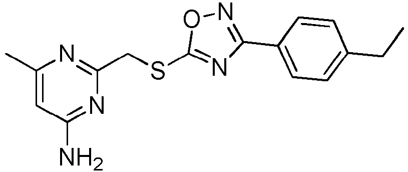

3-(4-Ethylphenyl)-1,2,4-oxadiazole-5-thiol (466.7 mg, 2.26 mmol, 1.15 equiv), NaOH (94 mg, 2.36 mmol, 1.2 equiv) and EtOH (20 mL) were mixed together. The resulting mixture was stirred for 10 min at room temperature followed by the dropwise addition of the solution 2-(iodomethyl)-6-methylpyrimidin-4(1*H*)-imine (492.3 mg, 1.97 mmol, 1.0 equiv) in hot EtOH (10 mL). Then, the reaction mixture was stirred for 3 h at 70 °C. After all starting material was consumed, as was shown by LCMS, the resulting mixture was allowed to cool down to room temperature and the volatiles were removed under reduced pressure. The obtained residue was subjected to HPLC (Waters Sunfire 19_{*}100 C18 5 mkm column and mixture of H₂O-CH₃OH as a mobile phase) to afford pure product (174.5 mg, 23.6 %): m/z = 328,20 [M+H]⁺.

### Example 79: 2-((1-((3-(Benzo[d][1,3]dioxol-5-yl)-1,2,4-oxadiazol-5-yl)methyl)piperidin-4-yl)thio)-6-methylpyrimidin-4-amine (Z3214047397)

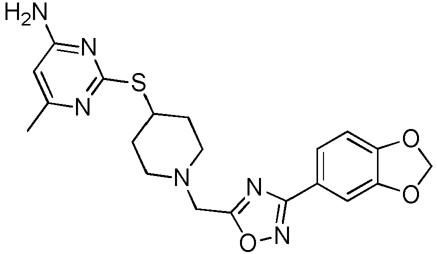

TEA (1.56 g, 15.4 mmol, 5.5 equiv) and 3-(benzo[d][1,3]dioxol-5-yl)-5-(chloromethyl)-1,2,4-oxadiazole (667.4 mg, 2.8 mmol, 1.0 equiv) were added to the solution of 26-methyl-2-(piperidin-4-ylthio)pyrimidin-4-amine (831.9 g, 2.8 mmol, 1.0 equiv, 2HCl) in DMF (10.0 mL). The resulting mixture was stirred at 40 °C for 16 h. After LCMS showed full conversion of starting material, the reaction mixture was allowed to cool down to room temperature and diluted with a mixture of EtOAc (80.0 mL) and H₂O (100.0 mL). The organic phase was separated, dried over Na₂SO₄, filtered off and concentrated in vacuo. The obtained solid was subjected to HPLC (Waters Sunfire 19_{*}100 C18 5 mkm column and mixture of H₂O-CH₃OH as a mobile phase) to afford pure product (174.5 mg, 14.62 %). ^{:} m/z = 427,20 [M+H]⁺.

### Parallel Syntheses:

### Example 80: 2-(((5-(3-Chloro-4-methoxyphenyl)oxazol-2-yl)methyl)thio)-6-methylpyrimidin-4(3H)-one (Z3325085942):

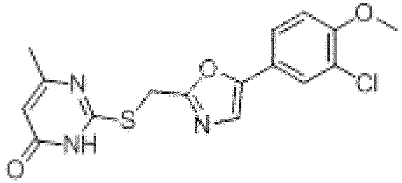

5-(3-Chloro-4-methoxyphenyl)-2-(chloromethyl)oxazole (72.61 mg, 0.282 mmol, 1.0 equiv) and 2-mercapto-6-methylpyrimidin-4(3*H*)-one (40.0 mg, 0.282 mmol, 1.0 equiv) were mixed together in anhydrous DMF (1.5 mL). The resulting mixture was stirred for 5 min followed by the addition of DIPEA (43.63 mg, 0.337 mmol, 2.0 equiv). Then, the reaction mixture was heated 90 °C in sealed vial for 12 h. After the completion of the reaction, monitored by LCMS, the volatiles were removed under reduced pressure. The residue was subjected to HPLC (Waters Sunfire 19_{*}100 C18 5 mkm column and mixture of H₂O-CH₃OH as a mobile phase) to afford pure product (57.7 mg, 56.37 % yield) :): m/z = 364,00 [M+H]⁺.

### Example 81: 4-(((5-(3-Chlorophenyl)oxazol-2-yl)methyl)thio)-6-(thiophen-3-yl)-1,3,5-triazin-2-amine (Z3400108331):

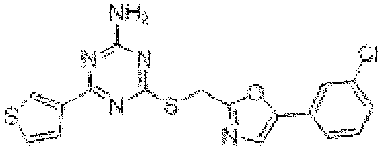

DIPEA (51.70 mg, 04 mmol, 2.0 equiv) was added to a stirred slurry of *N*-[amino({[5-(3-chlorophenyl)-1,3-oxazol-2-yl]methyl}sulfanyl)methylidene]guanidine (69.25 mg, 0.2 mmol, 1.0 equiv, HCl) and thiophene-3-carbonyl chloride (29.3 mg, 0.2 mmol, 1.0 equiv) in anhydrous THF (1.5 mL). The reaction mixture was heated 70 °C in sealed vial for 12 h. After all starting material was consumed, as was shown by LCMS, the solvent was removed in vacuo. The obtained solid was dissolved in DMSO (1.0 mL) and filtered off. The filtrate was subjected to HPLC (Waters Sunfire 19_{*}100 C18 5 mkm column and mixture of H₂O-CH₃OH as a mobile phase) to afford pure product (6.4 mg, 7.18 % yield): m/z = 402,00 [M+H]⁺.

### Example 82: 2-((5-(p-Tolyl)-1,3,4-oxadiazol-2-yl)thio)benzo[d]oxazole (Z642432840)

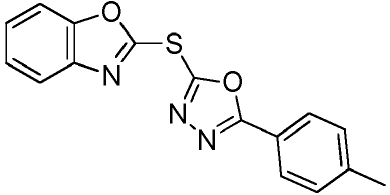

5-(*p*-Tolyl)-1,3,4-oxadiazole-2-thiol (372.0 mg, 1.93 mmol, 1.0 equiv), DMSO (0.5 mL) and TEA (235.0 mg, 2.32 mmol, 1.2 equiv) were mixed together in an 8 mL vessel. The resulting mixture was stirred for 20 min followed by the addition of 2-chlorobenzo[d]oxazole (297.0 mg, 1.93 mmol, 1.0 equiv). The resulting mixture was stirred at 100 °C for 9 h. After the completion of the reaction, monitored by LCMS, the resulting suspension was filtered off and the obtained filtrate was subjected to HPLC (Waters Sunfire 19_{*}100 C18 5 mkm column and mixture of H₂O-CH₃OH as a mobile phase) to afford pure product (34.0 mg, 6.80 %).: ^{:}m/z = 310,00 [M+H]⁺.

### Example 83: 6-(((5-(4-Methoxyphenyl)oxazol-2-yl)methyl)thio)-1,3,5-triazine-2,4-diamine (Z855788832)

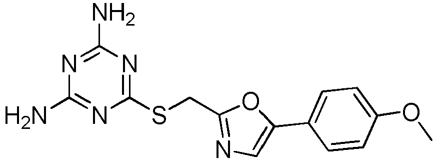

4,6-Diamino-1,3,5-triazine-2-thiol (45.0 mg, 0.314 mmol, 1.0 equiv), DMSO (0.5 mL) and 2-(chloromethyl)-5-(4-methoxyphenyl)oxazole (70.3 mg, 0.314 mmol, 1.0 equiv) were mixed together and stirred for 20 min at room temperature. Then, the 4.0 M solution of KOH (91.7 mg, 1.63 mmol, 5.2 equiv) was added and the reaction mixture was stirred at 100 °C for 8 h. The obtained mixture was placed in autoclave and CO₂ was blown in. The resulting mixture was stirred for 6 h in the autoclave. After the completion of the reaction, the reaction mixture was filtered off and the obtained filtrate was subjected to HPLC (Waters Sunfire 19_{*}100 C18 5 mkm column and mixture of H₂O-CH₃OH as a mobile phase) to afford pure product (17.1 mg, 17.1 %):m/z = 331,00 [M+H]⁺.

### Example 84: 3-(Benzo[d][1,3]dioxol-5-yl)-5-(((1-methyl-1H-benzo[d]imidazol-2-yl)thio)methyl)-1,2,4-oxadiazole (Z1029491270)

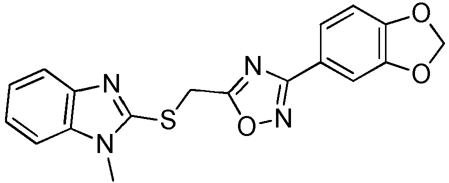

DIPEA (61.7 mg, 0.477 mmol, 3.5 equiv) was added to the solution of 1-methyl-1*H-*benzo[*d*]imidazole-2-thiol (22.4 mg, 0.136 mmol, 1.0 equiv) and 3-(benzo[*d*][1,3]dioxol-5-yl)-5-(chloromethyl)-1,2,4-oxadiazole (39.1 mg, 0.163 mmol, 1.2 equiv) in DMF (1.0 mL). The resulting mixture was stirred at 90 °C overnight. After the completion of the reaction, monitored by LCMS, the reaction mixture was concentrated under reduced pressure. The residue was dissolved in DMSO (1.0 mL) and filtered off. The filtrate was subjected to HPLC (Waters Sunfire 19_{*}100 C18 5 mkm column and mixture of H₂O-CH₃OH as a mobile phase) to afford pure product (24.6 mg, 49.2 %): m/z = 367,00 [M+H]⁺.

### Example 85: 4-(((5-(3-chlorophenyl)oxazol-2-yl)methyl)thio)-6-ethyl-1,3,5-triazin-2-amine (Z1558775684)

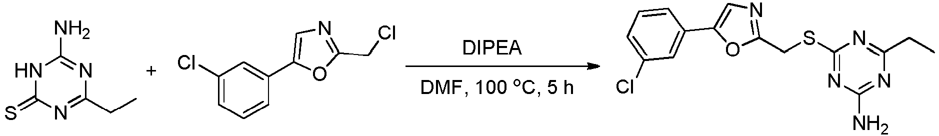

DIPEA (44.7 mg, 345.8 mmol) was added to the solution of 6-amino-4-ethyl-1,3,5-triazine-2(1*H*)-thione (45.0 mg, 288.1 mmol) in DMSO (0.5 mL). The resulting mixture was stirred for 30 min followed by the addition of 2-(chloromethyl)-5-(3-chlorophenyl)oxazole (69.5 mg, 304.7 mmol). The reaction mixture was stirred at room temperature for 1 h and then for 5 h at 100 °C. After all starting material was consumed, as was shown by LCMS, the resulting mixture was filtered off and the filtrate was subjected to HPLC (Waters SunFire C18 19-100 5 mkm column and H₂O-MeCN as a mobile phase, Run Time = 5 min) to afford pure product (22.3 mg, 22.3%); LCMS : 348 (M+1)

### Example 86: Measurement of agonistic activity of compounds of the invention on GPR40

Assays for agonistic activity of compounds as decribed herein on GPR40 are carried out in mammalian cells which provide a readout for GPR40 activation. Primarily stable cell lines of HEK cells, which measure B-arrestin recruitment to heterologously expressed GPR40 via BRET readout. This involves co-expressing in one cell two components, namely a B-arrestin tagged with one element of the BRET assay (fluorescent protein or Luciferase) and the GPR40 tagged with the other element of the assay (fluorescent protein or Luciferase). A fluorescent protein (FP) was fused to the C-terminus of the receptor and was co-expressed in HEK293T cells with luciferase (Luc) fused the N-terminus of G-protein subunit G gamma 2 (Gγ). Furthermore, a fluorescent protein (FP) was fused to the C-terminus of the receptor and was co-expressed in HEK293T cells with luciferase (Luc) fused to the N-terminus of beta-Arrestin-2. 48h after transfections, cells were incubated with increasing doses of GPR40 reference ligand AMG 837 or a compound as disclosed herein and changes in BRET were measured.

pEC50 data and % activation (% activation = (activation of GPR40 by compound of invention/activation of GPR40 by AMG-837)*100) data of preferred compounds of the invention are shown in Fig. 1 and of preferred compounds for use in the invention are shown in Fig. 2.

### Example 87: OGTT testing in C57BI/6N male mice

### 1. Materials

### 1.1. Test substances.

Compound Z1558775684 was synthesized according to Example 85. Metformin was acquired from Teva Pharmaceutical Industries Ltd., Petach Tikwa, Israel, Lot 78133817 03 2020.

### 1.2. Reagents used for formulation of the test substances

METHOCEL F4M Hydroxypropyl methylcellulose (Dow Chemical Company, Midland, USA).

### 1.4. Other Reagents and Materials

Syringes for injection, 1 ml without needle, Medicare S-3SI1, (Dopomoga-1 Ltd, Ukraine). Stainless steel animal feeding tube 20ga x 38 mm (Intech Solomon, USA) D-(+)-Glucose monohydrate (Sigma, 16301-250G). On Call Plus glucometer (Acon Laboratories, Inc., USA) and specific test strips (REF G133-111). Sterile plastic tubes different volumes (Falcon, Eppendorf). Ethanol, 96 % (Ukrorgsynthesis Ltd, Ukraine) Stainless Steel Scissors for microsurgery.

### 1.5. Equipment

Balance Sartorius LE225D, d = 0.01 mg. Water purification system NANOpure Diamond D11911 (Thermo Scientific Barnstead, USA). Ultrasonic bath (Daihan, Korea; WUC-A03H). Micropipettes 0.5-5 µL, 2-20 µL, 20-200, 100-1000 µL (Eppendorf, Hamburg, Germany)

### 2. Test systems

### 2.1. Animals

### Species/strain Mice/C57BL/6N: All Males

Age at arrival 5 months
Number of animals in the study 25
Animal care: Study design, animal selection, handling and treatment were all in accordance with the Bienta toxicity study protocols and animal care guidelines.
Breeders: The animals were obtained from the animal facility of the Institute of Pharmacology and Toxicology, National Academy of Medical Sciences of Ukraine.
Hygienic class. Conventional at arrival. The animals were kept in good conventional conditions during the study.

### 2.2. Reason for animal species and strain selection

C57BL/6N is a mouse strain commonly used in glucose metabolism studies.

### 2.3. Animal identification

The animals were individually identified by earmarking. The cages were labeled with tags indicating the ID numbers and earmarks of mice, the study code, sex, and route of administration, start and end date of the experimental period.

### 2.4. Housing conditions

| | |
|---|---|
| Hygienic level: | Good conventional |
| Type of animal cages: | Polycarbonate bottoms with stainless steel wire mesh lids |
| Cage size: | H × W × D: 12.0 × 17.5 × 28.0 cm |
| Cleaning: | By changing the bedding twice a week |
| Number of animals per cage: | 3-5 |
| Environmental conditions: | |
| Air exchange: | 15-20 times/hour |
| Temperature: | 22 ± 3C |
| Relative humidity: | 40 - 60 % |
| Lighting: | 12-hour light/dark cycles. |
| Feed. | Mice were given free access to standardized rodent diet. |
| Drinking. | Mice had free access to acidified boiled tap water. |

### 2.5. Acclimatization period

During the acclimatization period (7 days) 3-5 animals were kept in each cage. All animals were monitored daily. Animals free from any clinical symptoms of sickness were used in the study.

### 2.6. Randomization

Animals were randomly assigned to groups according to the standard procedure 7 days prior to the starting day of the study. Each cage contained animals from a uniform experimental group.

### 3. Study design

### 3.1. Dose levels, group division, and sampling

Each experimental group consisted of five male C57BI/6N mice. Animals were dosed once perorally with 20 mg/kg of tested compound Z1558775684. A control group was dosed with vehicle on the same schedule. Metformin at the dosage of 300 mg/kg was used as reference compound. All mice were observed for clinical signs of gross toxicity before administration.

### 3.2. Groups characteristic

Mice were 5 months old, body weight ranged from 24.0 g to 32.2 g at arrival. Average body weight across all experimental groups was 28.0 g (SD = 2.12 g, CV = 7.6 %).

### 4. Compound administration

### 4.1. Drug formulation, route and volume of administration

Compound Z1558775684 was dissolved/suspended in DMSO - PEG400 - physiological saline (20%:50%:30%) at concentration 4 mg/ml. Metformin was dissolved in the same vehicle at concentration 60 mg/ml. The test samples were administered *per* os in the volume corresponding to 5 ml/kg body weight.

### 4.2. Frequency and duration of application

Single doses of test samples were administered 60 min before glucose treatment at 3 p.m.

### 4.3. Duration of experimental period

Seven days of acclimatization, 1 treatment day, 1 day for data analysis.

### 5. Observations, examinations

### 5.1. Determination of blood glucose level

The blood glucose level was measured after 6-hour fasting using Call Plus glucometer and specific test strips. Blood was obtained from the tail vein by incision of the tail tip, 5-6 µl of blood was used for each assay.

### 6. Statistical analysis

Non-parametric statistical analysis (criteria of Wilcoxon-Mann-Whitney U) for independent samples was used to calculate the significance. The experiments were performed according to the Bienta Standard Operating Procedures/Manuals.

### 7. Results

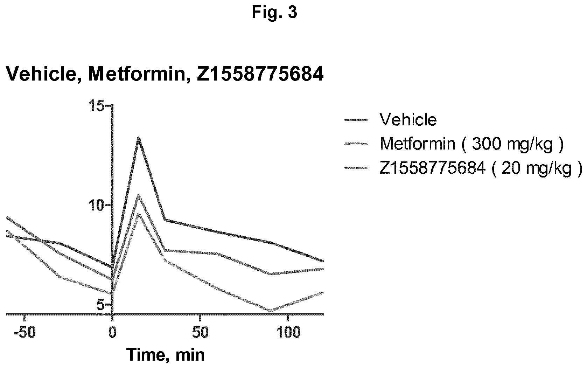

The results are presented in Figure 3. Statistically significant decrease in glucose level was observed in the Metformin-, and Z1558775684-treated groups compared with the Vehicle-treated group.

### References

1 Covington et al.(2006) Biochem. Soc. Trans. 34 (Pt 5), 770-773
2 Ang et al. (2017) The FASEB Journal. 32, 201700252RR. doi:10.1096/fj
3 Davenport et al. (2013) International Union of Basic and Clinical Pharmacology. LXXXVIII. G protein-coupled receptor list: recommendations for new pairings with cognate ligands. Pharmacol. Rev., 65 (3), 967-86
4 Stoddart et al. (2008) International Union of Pharmacology. LXXI. Free fatty acid receptors FFA1, -2, and -3: pharmacology and pathophysiological functions. Pharmacol. Rev., 60 (4): 405-17
5 Briscoe et al. (2003) J Biol Chem 278, 11303-11311
6 Itoh et al. (2003) Nature 422, 173-176
7 Kotarsky et al. (2003) Biochem Biophys Res Commun 301, 406-410
8 Hirasawa et al. (2005) Nat Med 11, 90-94
9 Ichimura et al. (2012) Nature, 483 (7389) 350-354
10 Oh et al. (2010) Cell, 142 (5), 687-98
11 Brown et al. (2003) J Biol Chem 278, 11312-11319
12 Le Poul et al. (2003) J Biol Chem 278, 25481-25489
13 Nilsson et al. (2003) Biochem Biophys Res Commun 303, 1047-1052
14 Srivastava et al. (2014) Nature 513 (7516), 124-127
15 Burant et al. (2012) Lancet 379, 1403-1411
16 Houze et al. (2012) Bioorg Med Chem Lett 22, 1267-1270
17 Briscoe et al. (2006) British Journal of Pharmacology 148, 619-628
18 Hara et al. (2009) Naunyn Schmiedebergs Arch Pharmacol 380, 247-255
19 Sun et al. (2010) Mol Pharmacol 78, 804-810
20 Martin et al. (2012) J Lipid Res 53, 2256-2265
21 Shimpukade et al. (2012) J Med Chem 55, 4511-4515
22 Houze et al. (2012) Bioorg Med Chem Lett. 22 (2),1267-70

## Claims

1. A compound of general formula (I)
R¹-S-CH₂-OXA-R² (I)
including enantiomers, diastereomers, hydrates, solvates, pharmaceutically acceptable cocrystals or salts, and complexes thereof;
wherein
OXA is 1,3-oxazolyl;
the group R¹-S-CH₂ is bound to C² of the 1,3-oxazolyl and the group R² is bound to C⁴ of the 1,3-oxazolyl;
R¹ is a 6 membered heteroaryl group selected from the group consisting of 1,3,5-triazinyl and pyrimidinyl being independently substituted with one or more substituents selected from the group consisting of hydroxyl, amino, C₁-C₆-alkyl, C₃-C₆-cycloalkyl, C₁-C₄-alkoxy, N-mono- or N,N-di-substituted C₁-C₃-alkylamino, nonaromatic 5- to 6-membered heterocyclyl, 6-membered aryl and 5- to 6-membered heteroaryl which substituents may be unsubstituted or substituted with one or more groups selected from the group consisting of halide, cyano and C₁-C₆-alkyl, wherein the 6-membered aryl and 5- to 6-membered heteroaryl group, respectively, may be fused to said 1,3,5-triazinyl or pyrimidyl group, respectively, and
R² is phenyl being unsubstituted or being substituted with one or more substituents selected from the group consisting of halide, cyano, amino, C₁-C₆-alkyl, C₃-C₆-cycloalkyl which may be optionally substituted with one or more halides, C₁-C₄-alkoxy which may optionally substituted with one or more halides, hydroxy-C₁-C₆-alkyl, sulfonyl-C₁-C₆-alkyl, sulphamidyl-N-C₁-C6-alkyl and carboxamidyl-N-mono- or - N,N-di-C₁-C₆-alkyl;
with the proviso that the following compounds are excluded:

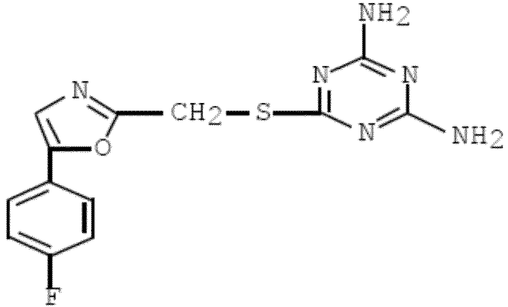

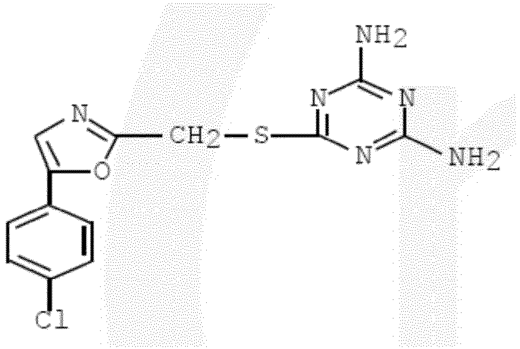

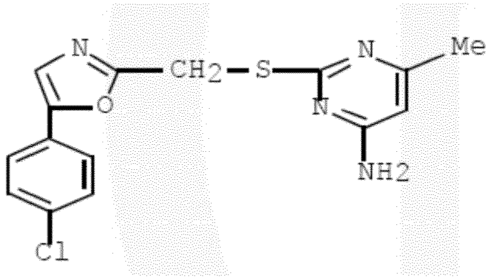

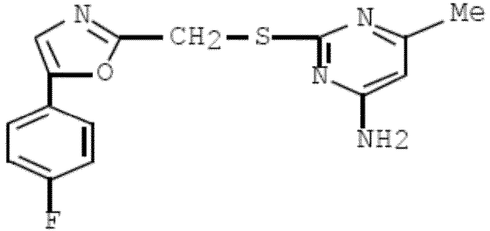

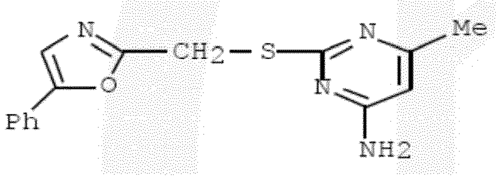

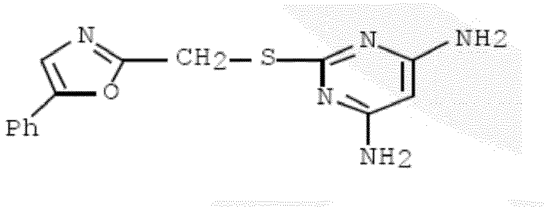

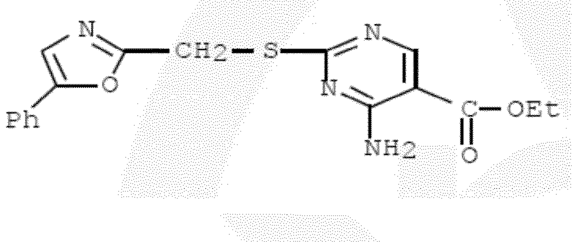

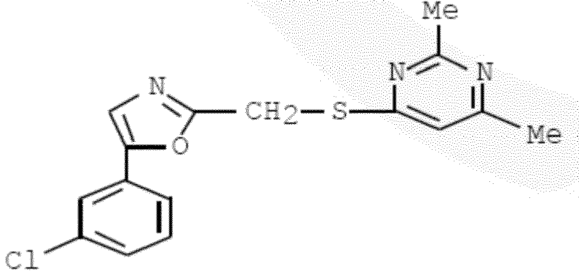

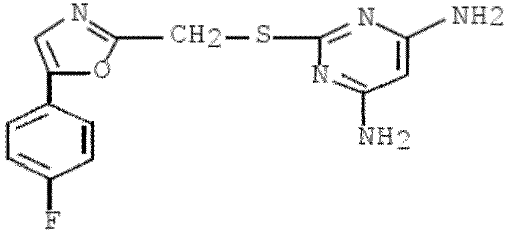

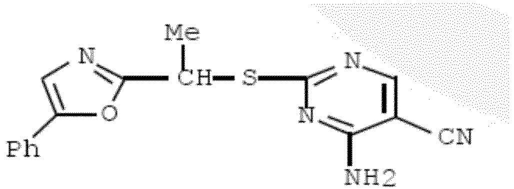

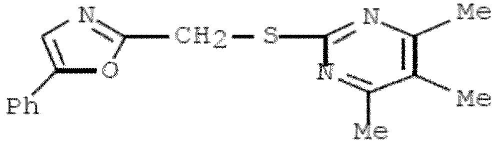

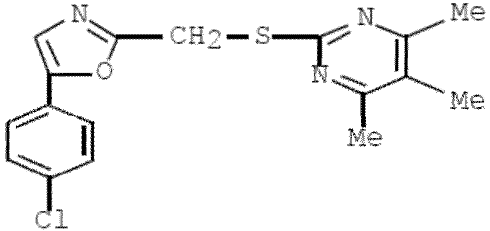

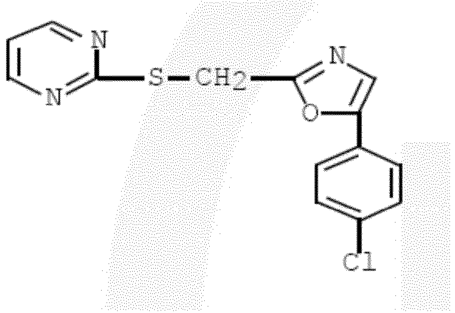

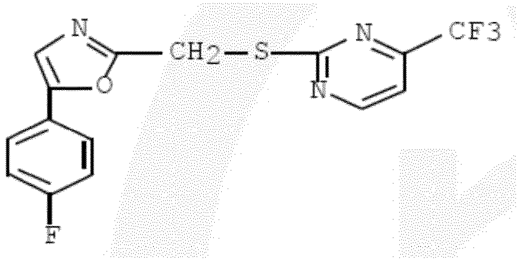

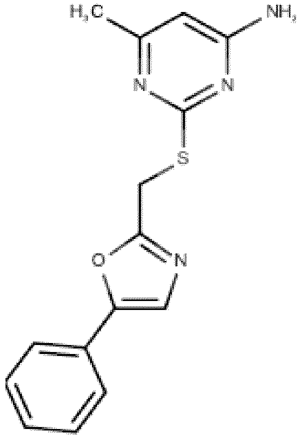

2. The compound of claim 1 wherein halide is selected from CI, Br and F.

3. The compound of claim 1 or 2 wherein R¹ is 1,3,5-triazinyl substituted as defined in claim 1.

4. The compound of claim 3 wherein, if the 1,3,5-triazinyl group is substituted with more than one substituent, the substituents are different.

5. The compound of claim 3 or 4 wherein the 1,3,5 triazinyl group is independently substituted with one or two substituents selected from the group consisting of amino, methyl, ethyl, isopropyl and tert.-butyl, which substituent(s) is/are optionally substituted with one or more halide.

6. The compound of claim 3 wherein R¹ is selected from the group consisting of 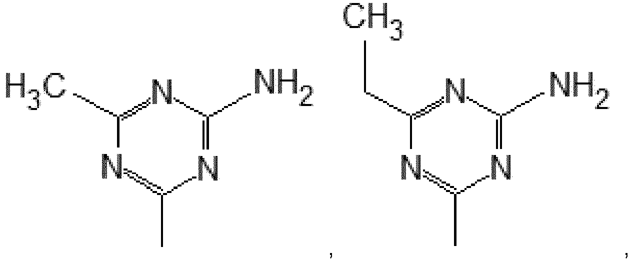 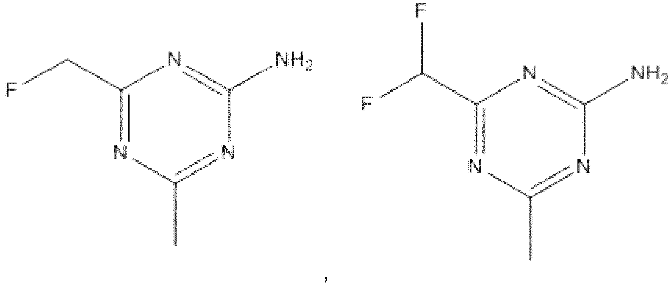 and

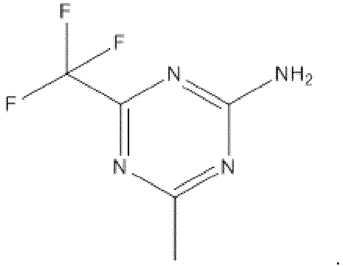

7. The compound according to any one of claims 1 or 2 wherein R¹ is pyrimidinyl substituted as defined in claim 1.

8. The compound of claim 7 wherein, if the pyrimidinyl group is substituted with more than one substituent, the substituents are different.

9. The compound of claim 7 or 8 wherein the pyrimidinyl group is independently substituted by one or more substituents selected from the group consisting of amino, methyl and ethyl.

10. The compound of claim 9 wherein the amino group is substituted with methyl.

11. The compound of claim 9 or 10 wherein the methyl or ethyl group is substituted with one or more halides as defined in claim 2.

12. The compound of claim 7 wherein R¹ is selected from the group consisting of

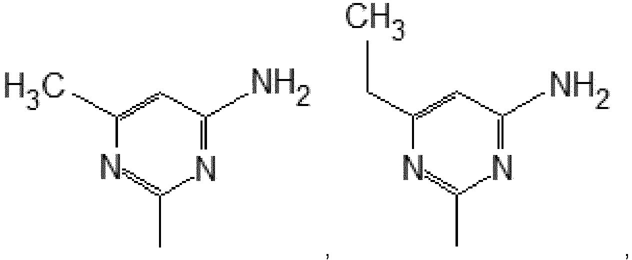

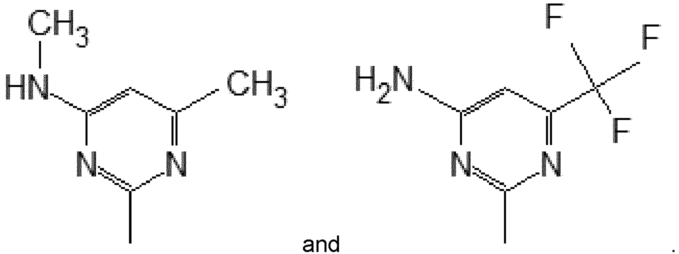

13. The compound according to any one of the preceding claims wherein R² is independently substituted with one or more substituents selected from the group consisting of Cl, Br, F, methyl, triflourmethyl, methoxy and ethoxy.

14. The compound according to any one of the preceding claims wherein R² is substituted in at least one meta position and/or at least one ortho position.

15. The compound of claim 14 wherein R² is selected from the group consisting of

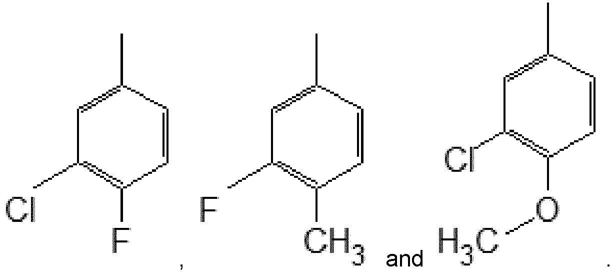

16. The compound of claim 13 or 14 wherein R² is not substituted in the para position.

17. The compound of claim 16 wherein R² is selected from the group consisting of 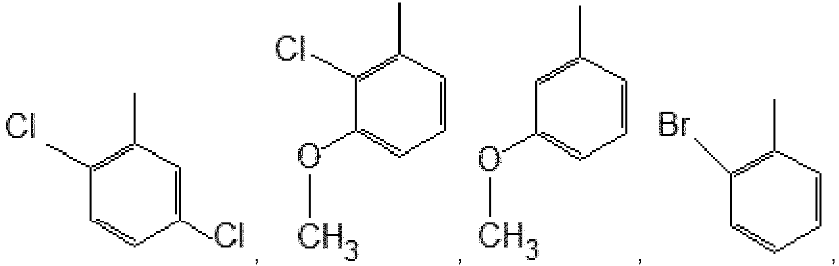 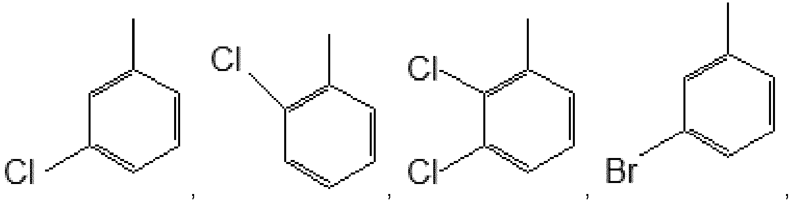 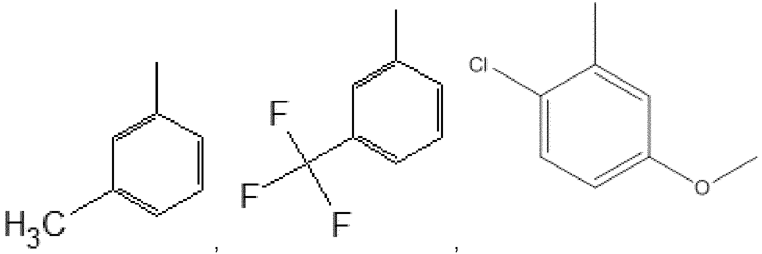 and

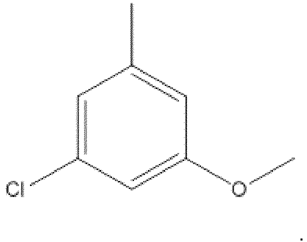

18. The compound of claim 1 being selected from the group consisting of the following compounds:
2-({[5-(2-chloro-5-methoxyphenyl)-1,3-oxazol-2-yl]methyl}sulfanyl)-6-methylpyrimidin-4-amine
4-({[5-(2-chloro-5-methoxyphenyl)-1,3-oxazol-2-yl]methyl}sulfanyl)-6-(difluoromethyl)-1,3,5-triazin-2-amine
4-([5-(2,5-dichlorophenyl)-1,3-oxazol-2-yl]methylsulfanyl)-6-(difluoromethyl)-1,3,5-triazin-2-amine
4-({[5-(2-chloro-5-methoxyphenyl)-1,3-oxazol-2-yl]methyl}sulfanyl)-6-(fluoromethyl)-1,3,5-triazin-2-amine
2-({[5-(2-chloro-3-methoxyphenyl)-1,3-oxazol-2-yl]methyl}sulfanyl)-6-methylpyrimidin-4-amine
2-({[5-(2-chloro-3-methoxyphenyl)-1,3-oxazol-2-yl]methyl}sulfanyl)-6-methylpyrimidin-4-amine
4-({[5-(2,5-dichlorophenyl)-1,3-oxazol-2-yl]methyl}sulfanyl)-6-methyl-1,3,5-triazin-2-amine
4-({[5-(2-chloro-5-methoxyphenyl)-1,3-oxazol-2-yl]methyl}sulfanyl)-6-ethyl-1,3,5-triazin-2-amine
4-({[5-(2-chloro-5-methoxyphenyl)-1,3-oxazol-2-yl]methyl}sulfanyl)-6-methyl-1,3,5-triazin-2-amine
4-({[5-(3-chloro-4-methoxyphenyl)-1,3-oxazol-2-yl]methyl}sulfanyl)-6-methyl-1,3,5-triazin-2-amine
2-({[5-(2-chloro-5-methoxyphenyl)-1,3-oxazol-2-yl]methyl}sulfanyl)-6-(trifluoromethyl)pyrimidin-4-amine
4-([5-(2,5-dichlorophenyl)-1,3-oxazol-2-yl]methylsulfanyl)-6-(fluoromethyl)-1,3,5-triazin-2-amine
4-({[5-(2,5-dichlorophenyl)-1,3-oxazol-2-yl]methyl}sulfanyl)- 6-methylpyrimidin-4-amine
4-([5-(2,5-dichlorophenyl)-1,3-oxazol-2-yl]methylsulfanyl)-6-ethyl-1,3,5-triazin-2-amine
2-({[5-(3-chloro-4-methoxyphenyl)-1,3-oxazol-2-yl]methyl}sulfanyl)-6-methylpyrimidin-4-amine
4-({[5-(2-chloro-3-methoxyphenyl)-1,3-oxazol-2-yl]methyl}sulfanyl)-6-methyl-1,3,5-triazin-2-amine
4-({[5-(3-chloro-4-methoxyphenyl)-1,3-oxazol-2-yl]methyl}sulfanyl)-6-ethyl-1,3,5-triazin-2-amine
4-ethyl-6-({[5-(3-methoxyphenyl)-1,3-oxazol-2-yl]methyl}sulfanyl)-1,3,5-triazin-2-amine
2-({[5-(2-bromophenyl)-1,3-oxazol-2-yl]methyl}sulfanyl)-6-methylpyrimidin-4-amine
2-({[5-(3-methoxyphenyl)-1,3-oxazol-2-yl]methyl}sulfanyl)-6-methylpyrimidin-4-amine
4-({[5-(3-chlorophenyl)-1,3-oxazol-2-yl]methyl}sulfanyl)-6-(pyridin-4-yl)-1,3,5-triazin-2-amine
4-({[5-(3-chloro-5-methoxyphenyl)-1,3-oxazol-2-yl]methyl}sulfanyl)-6-ethyl-1,3,5-triazin-2-amine
4-({[5-(3-chlorophenyl)-1,3-oxazol-2-yl]methyl}sulfanyl)-6-ethyl-1,3,5-triazin-2-amine
4-({[5-(3-chloro-4-fluorophenyl)-1,3-oxazol-2-yl]methyl}sulfanyl)-6-methyl-1,3,5-triazin-2-amine
6-methyl-2-({[5-(4-methylphenyl)-1,3-oxazol-2-yl]methyl}sulfanyl)pyrimidin-4-amine
2-({[5-(3-chlorophenyl)-1,3-oxazol-2-yl]methyl}sulfanyl)-6-methylpyrimidin-4-amine
4-({[5-(3-chlorophenyl)-1,3-oxazol-2-yl]methyl}sulfanyl)-6-(1-methyl-1H-pyrazol-5-yl)-1,3,5-triazin-2-amine
2-[({5-[3-chloro-4-(3-methanesulfonylpropoxy)phenyl]-1,3-oxazol-2-yl}methyl)sulfanyl]-6-methylpyrimidin-4-amine
2-({[5-(3-ethoxyphenyl)-1,3-oxazol-2-yl]methyl}sulfanyl)-6-methylpyrimidin-4-amine
2-({[5-(3-chloro-4-fluorophenyl)-1,3-oxazol-2-yl]methyl}sulfanyl)-6-methylpyrimidin-4-amine
4-({[5-(3-chlorophenyl)-1,3-oxazol-2-yl]methyl}sulfanyl)-6-methyl-1,3,5-triazin-2-amine
4-({[5-(2-chlorophenyl)-1,3-oxazol-2-yl]methyl}sulfanyl)-6-methyl-1,3,5-triazin-2-amine
4-({[5-(3-methoxyphenyl)-1,3-oxazol-2-yl]methyl}sulfanyl)-6-methyl-1,3,5-triazin-2-amine
2-({[5-(3-chloro-5-methoxyphenyl)-1,3-oxazol-2-yl]methyl}sulfanyl)-6-methylpyrimidin-4-amine
4-({[5-(2,3-dichlorophenyl)-1,3-oxazol-2-yl]methyl}sulfanyl)-6-ethyl-1,3,5-triazin-2-amine
6-methyl-2-[({5-[3-(trifluoromethyl)phenyl]-1,3-oxazol-2-yl}methyl)sulfanyl]pyrimidin-4-amine
2-({[5-(2,3-dichlorophenyl)-1,3-oxazol-2-yl]methyl}sulfanyl)-6-methylpyrimidin-4-amine
4-methyl-6-[({5-[3-(trifluoromethyl)phenyl]-1,3-oxazol-2-yl}methyl)sulfanyl]-1,3,5-triazin-2-amine
4-({[5-(3-bromophenyl)-1,3-oxazol-2-yl]methyl}sulfanyl)-6-methyl-1,3,5-triazin-2-amine
4-({[5-(3-ethoxyphenyl)-1,3-oxazol-2-yl]methyl}sulfanyl)-6-methyl-1,3,5-triazin-2-amine
4-({[5-(2,3-dichlorophenyl)-1,3-oxazol-2-yl]methyl}sulfanyl)-6-methyl-1,3,5-triazin-2-amine
2-({[5-(2,5-dichlorophenyl)-1,3-oxazol-2-yl]methyl}sulfanyl)-6-(trifluoromethyl)pyrimidin-4-amine
4-[chloro(fluoro)methyl]-6-({[5-(3-chlorophenyl)-1,3-oxazol-2-yl]methyl}sulfanyl)-1,3,5-triazin-2-amine
4-({[5-(3-chloro-5-methoxyphenyl)-1,3-oxazol-2-yl]methyl}sulfanyl)-6-methyl-1,3,5-triazin-2-amine
6-({[5-(2,5-dichlorophenyl)-1,3-oxazol-2-yl]methyl}thio)-N,N-dimethyl-1,3,5-triazine-2,4-diamine
4-methyl-6-({[5-(4-methylphenyl)-1,3-oxazol-2-yl]methyl}sulfanyl)-1,3,5-triazin-2-amine
2-({[5-(2-chlorophenyl)-1,3-oxazol-2-yl]methyl}sulfanyl)-6-methylpyrimidin-4-amine
4-methyl-6-({[5-(naphthalen-2-yl)-1,3-oxazol-2-yl]methyl}sulfanyl)-1,3,5-triazin-2-amine
2-({[5-(3-methoxyphenyl)-1,3-oxazol-2-yl]methyl}sulfanyl)-6-(trifluoromethyl)pyrimidin-4-amine
4-methyl-6-[({5-[3-(trifluoromethoxy)phenyl]-1,3-oxazol-2-yl}methyl)sulfanyl]-1,3,5-triazin-2-amine
2-({[5-(4-methoxyphenyl)-1,3-oxazol-2-yl]methyl}sulfanyl)-6-methylpyrimidin-4-amine
4-({[5-(4-methoxyphenyl)-1,3-oxazol-2-yl]methyl}sulfanyl)-6-methyl-1,3,5-triazin-2-amine
6-methyl-2-({[5-(naphthalen-2-yl)-1,3-oxazol-2-yl]methyl}sulfanyl)pyrimidin-4-amine
6-methyl-2-({[5-(2-methylphenyl)-1,3-oxazol-2-yl]methyl}sulfanyl)pyrimidin-4-amine
4-([5-(3-chlorophenyl)-1,3-oxazol-2-yl]methylsulfanyl)-6-(trifluoromethyl)-1,3,5-triazin-2-amine
4-({[5-(2-chloro-5-methoxyphenyl)-1,3-oxazol-2-yl]methyl}sulfanyl)-6-(morpholin-4-yl)-1,3,5-triazin-2-amine
2-([5-(3-methoxyphenyl)-1,3-oxazol-2-yl]methylsulfanyl)-N,6-dimethylpyrimidin-4-amine
4-({[5-(4-ethylphenyl)-1,3-oxazol-2-yl]methyl}sulfanyl)-6-methyl-1,3,5-triazin-2-amine
2-({[5-(2-chlorophenyl)-1,3-oxazol-2-yl]methyl}sulfanyl)-6-(trifluoromethyl)pyrimidin-4-amine
4-({[5-(3-chlorophenyl)-1,3-oxazol-2-yl]methyl}sulfanyl)-6-phenyl-1,3,5-triazin-2-amine
4-([5-(2,5-dichlorophenyl)-1,3-oxazol-2-yl]methylsulfanyl)-6-(morpholin-4-yl)-1,3,5-triazin-2-amine
2-({[5-(3-chlorophenyl)-1,3-oxazol-2-yl]methyl}sulfanyl)-6-(trifluoromethyl)pyrimidin-4-amine
4-cyclopropyl-6-([5-(3-methoxyphenyl)-1,3-oxazol-2-yl]methylsulfanyl)-1,3,5-triazin-2-amine
2-({[5-(3-chloro-5-methoxyphenyl)-1,3-oxazol-2-yl]methyl}sulfanyl)-6-(trifluoromethyl)pyrimidin-4-amine
2-({[5-(2,3-dichlorophenyl)-1,3-oxazol-2-yl]methyl}sulfanyl)-6-(trifluoromethyl)pyrimidin-4-amine
4-({[5-(3-chlorophenyl)-1,3-oxazol-2-yl]methyl}sulfanyl)-6-(1,3-thiazol-4-yl)-1,3,5-triazin-2-amine
4-({[5-(3-chlorophenyl)-1,3-oxazol-2-yl]methyl}sulfanyl)-6-(1-methyl-1H-pyrazol-4-yl)-1,3,5-triazin-2-amine
4-({[5-(4-fluorophenyl)-1,3-oxazol-2-yl]methyl}sulfanyl)-6-methyl-1,3,5-triazin-2-amine
2-({thieno[2,3-d]pyrimidin-4-ylsulfanyl}methyl)-5-[3-(trifluoromethyl)phenyl]-1,3-oxazole
4-({[5-(3-chlorophenyl)-1,3-oxazol-2-yl]methyl}sulfanyl)-6-(1-methylcyclopropyl)-1,3,5-triazin-2-amine
4-({[5-(3-chlorophenyl)-1,3-oxazol-2-yl]methyl}sulfanyl)-6-(3,3-difluorocyclobutyl)-1,3,5-triazin-2-amine
6-methyl-2-[({5-[3-(trifluoromethoxy)phenyl]-1,3-oxazol-2-yl}methyl) sulfanyl]pyrimidin-4-amine
2-[({5-[3-(trifluoromethoxy)phenyl]-1,3-oxazol-2-yl}methyl)sulfanyl]-6-(trifluoromethyl)pyrimidin-4-amine
2-({[5-(2-methoxyphenyl)-1,3-oxazol-2-yl]methyl}sulfanyl)-6-methylpyrimidin-4-amine
5-(2,5-dichlorophenyl)-2-[({2-methylpyrido[2,3-d]pyrimidin-4-yl}sulfanyl)methyl]-1,3-oxazole
5-(3-methoxyphenyl)-2-({thieno[2,3-d]pyrimidin-4-ylsulfanyl}methyl)-1,3-oxazole
2-({[5-(4-methylphenyl)-1,3-oxazol-2-yl]methyl}sulfanyl)-6-(trifluoromethyl)pyrimidin-4-amine
4-([5-(3-chlorophenyl)-1,3-oxazol-2-yl]methylsulfanyl)-6-methoxy-1,3,5-triazin-2-amine
4-ethyl-6-[({5-[3-(trifluoromethoxy)phenyl]-1,3-oxazol-2-yl}methyl)sulfanyl]-1,3,5-triazin-2-amine
2-({[5-(3-chlorophenyl)-1,3-oxazol-2-yl]methyl}sulfanyl)-N-methylquinazolin-4-amine
4-({[5-(3-chlorophenyl)-1,3-oxazol-2-yl]methyl}sulfanyl)-6-(methoxymethyl)-1,3,5-triazin-2-amine
6-({[5-(2-chlorophenyl)-1,3-oxazol-2-yl]methyl}sulfanyl)-1,3,5-triazine-2,4-diamine
2-({[5-(3-chloro-4-fluorophenyl)-1,3-oxazol-2-yl]methyl}sulfanyl)-6-(trifluoromethyl)pyrimidin-4-amine
5-(3-methoxyphenyl)-2-[({2-methylpyrido[2,3-d]pyrimidin-4-yl}sulfanyl)methyl]-1,3-oxazole

19. A pharmaceutical composition comprising at least one compound according to any one of the preceding claims and at least one pharmaceutical carrier.

20. A compound of general formula (I)
R¹-S-CH₂-OXA-R² (I)
including enantiomers, diastereomers, hydrates, solvates, pharmaceutically acceptable cocrystals or salts, and complexes thereof;
wherein
OXA is selected from the group consisting of 1,3-oxazolyl, 1,2,4-oxadiazolyl or 1,3,4-oxadiazolyl;
when OXA is 1,3-oxazolyl, the group R¹-S-CH₂ is bound to C² of the 1,3-oxazolyl and the group R² is bound to C⁴ of the 1,3-oxazolyl;
when OXA is 1,2,4-oxadiazolyl the group R¹-S-CH₂ is bound to C⁵ of the 1,2,4-oxadiazolyl and the group R² is bound to C³ of the 1,2,4-oxadiazolyl;
when OXA is 1,3,4-oxadiazolyl the group R¹-S-CH₂ is bound to C⁵ of the 1,3,4-oxadiazolyl and the group R² is bound to C² of the 1,3,4-oxadiazolyl';
R¹ is a 6 membered heteroaryl group selected from the group consisting of 1,3,5-triazinyl and pyrimidinyl being independently substituted with one or more substituents selected from the group consisting of hydroxyl, amino, C₁-C₆-alkyl, C₃-C₆-cycloalkyl, C₁-C₄-alkoxy, N-mono- or N,N-di-substituted C₁-C₃-alkylamino, nonaromatic 5- to 6-membered heterocyclyl, 6-membered aryl and 5- to 6-membered heteroaryl which substituents may be unsubstituted or substituted with one or more groups selected from the group consisting of halide, cyano and C₁-C₆-alkyl, wherein the 6-membered aryl and 5- to 6-membered heteroaryl group, respectively, may be fused to said 1,3,5-triazinyl or pyrimidyl group, respectively, and
R² is phenyl being unsubstituted or being substituted with one or more substituents selected from the group consisting of halide, cyano, amino, C₁-C₆-alkyl, C₃-C₆-cycloalkyl which may be optionally substituted with one or more halides, C₁-C₄-alkoxy which may optionally substituted with one or more halides, hydroxy-C₁-C₆-alkyl, sulfonyl-C₁-C₆-alkyl, sulphamidyl-N-C₁-C6-alkyl and carboxamidyl-N-mono- or - N,N-di-C₁-C₆-alkyl;
for use as an agonist of GPR40 in the treatment and/or prevention of type 2 diabetes mellitus and/or a pre-diabetic condition.

21. The compound for use of claim 20 wherein the pre-diabetic condition is selected from the group consisting of obesity and insulin resistance.

22. The compound for use of claim 20 or 21 wherein the compound shows a higher selectivity for GPR40 than for GPR120.

23. The compound for use of claim 22 wherein the compound shows a % activation of GPR40 being at least 3fold higher than the % activation of GPR120, with % activation being the hundredfold ratio of activation of GPR40 or GPR120, respectively, by said compound to the activation of GPR40 or GPR120, respectively, by AMG 837.

24. The compound for use according to any one of claims 20 to 23 wherein halide is selected from CI, Br and F.

25. The compound for use according to any one of claim 20 to 24 wherein R¹ is defined as in any one of claims 3 to 12.

26. The compound for use according to any one of claims 20 to 25 wherein R² is defined as in any one of claims 13 to 17.

27. The compound for use according to any one of claims 20 to 26 wherein OXA is 1,3-oxazolyl.

28. The compound for use of claim 20 wherein the compound is selected from the group consisting of the following compounds:
2-({[5-(2-chloro-5-methoxyphenyl)-1,3-oxazol-2-yl]methyl}sulfanyl)-6-methylpyrimidin-4-amine
4-({[5-(2-chloro-5-methoxyphenyl)-1,3-oxazol-2-yl]methyl}sulfanyl)-6-(difluoromethyl)-1,3,5-triazin-2-amine
4-([5-(2,5-dichlorophenyl)-1,3-oxazol-2-yl]methylsulfanyl)-6-(difluoromethyl)-1,3,5-triazin-2-amine
4-({[5-(2-chloro-5-methoxyphenyl)-1,3-oxazol-2-yl]methyl}sulfanyl)-6-(fluoromethyl)-1,3,5-triazin-2-amine
2-({[5-(2-chloro-3-methoxyphenyl)-1,3-oxazol-2-yl]methyl}sulfanyl)-6-methylpyrimidin-4-amine
2-({[5-(2-chloro-3-methoxyphenyl)-1,3-oxazol-2-yl]methyl}sulfanyl)-6-methylpyrimidin-4-amine
4-({[5-(2,5-dichlorophenyl)-1,3-oxazol-2-yl]methyl}sulfanyl)-6-methyl-1,3,5-triazin-2-amine
4-({[5-(2-chloro-5-methoxyphenyl)-1,3-oxazol-2-yl]methyl}sulfanyl)-6-ethyl-1,3,5-triazin-2-amine
4-({[5-(2-chloro-5-methoxyphenyl)-1,3-oxazol-2-yl]methyl}sulfanyl)-6-methyl-1,3,5-triazin-2-amine
4-({[5-(3-ch loro-4-methoxyphenyl)-1, 3-oxazol-2-yl] methyl}sulfanyl)-6-methyl-1, 3, 5-triazin-2-amine
2-({[5-(2-chloro-5-methoxyphenyl)-1,3-oxazol-2-yl]methyl}sulfanyl)-6-(trifluoromethyl)pyrimidin-4-amine
4-([5-(2,5-dichlorophenyl)-1,3-oxazol-2-yl]methylsulfanyl)-6-(fluoromethyl)-1,3,5-triazin-2-amine
4-({[5-(2,5-dichlorophenyl)-1,3-oxazol-2-yl]methyl}sulfanyl)- 6-methylpyrimidin-4-amine
4-([5-(2,5-dichlorophenyl)-1,3-oxazol-2-yl]methylsulfanyl)-6-ethyl-1,3,5-triazin-2-amine
2-({[5-(3-chloro-4-methoxyphenyl)-1,3-oxazol-2-yl]methyl}sulfanyl)-6-methylpyrimidin-4-amine
4-({[5-(2-chloro-3-methoxyphenyl)-1,3-oxazol-2-yl]methyl}sulfanyl)-6-methyl-1,3,5-triazin-2-amine
4-({[5-(3-chloro-4-methoxyphenyl)-1,3-oxazol-2-yl]methyl}sulfanyl)-6-ethyl-1,3,5-triazin-2-amine
4-ethyl-6-({[5-(3-methoxyphenyl)-1,3-oxazol-2-yl]methyl}sulfanyl)-1,3,5-triazin-2-amine
2-({[5-(2-bromophenyl)-1,3-oxazol-2-yl]methyl}sulfanyl)-6-methylpyrimidin-4-amine
2-({[5-(3-methoxyphenyl)-1,3-oxazol-2-yl]methyl}sulfanyl)-6-methylpyrimidin-4-amine
4-({[5-(3-chlorophenyl)-1,3-oxazol-2-yl]methyl}sulfanyl)-6-(pyridin-4-yl)-1,3,5-triazin-2-amine
4-({[5-(3-chloro-5-methoxyphenyl)-1,3-oxazol-2-yl]methyl}sulfanyl)-6-ethyl-1,3,5-triazin-2-amine
4-({[5-(3-chlorophenyl)-1,3-oxazol-2-yl]methyl}sulfanyl)-6-ethyl-1,3,5-triazin-2-amine
4-({[5-(3-chloro-4-fluorophenyl)-1,3-oxazol-2-yl]methyl}sulfanyl)-6-methyl-1,3,5-triazin-2-amine
6-methyl-2-({[5-(4-methylphenyl)-1,3-oxazol-2-yl]methyl}sulfanyl)pyrimidin-4-amine
2-({[5-(3-chlorophenyl)-1,3-oxazol-2-yl]methyl}sulfanyl)-6-methylpyrimidin-4-amine
4-({[5-(3-chlorophenyl)-1,3-oxazol-2-yl]methyl}sulfanyl)-6-(1-methyl-1H-pyrazol-5-yl)-1,3,5-triazin-2-amine
2-[({5-[3-chloro-4-(3-methanesulfonylpropoxy)phenyl]-1,3-oxazol-2-yl}methyl)sulfanyl]-6-methylpyrimidin-4-amine
2-({[5-(3-ethoxyphenyl)-1,3-oxazol-2-yl]methyl}sulfanyl)-6-methylpyrimidin-4-amine
2-({[5-(3-chloro-4-fluorophenyl)-1,3-oxazol-2-yl]methyl}sulfanyl)-6-methylpyrimidin-4-amine
4-({[5-(3-chlorophenyl)-1,3-oxazol-2-yl]methyl}sulfanyl)-6-methyl-1,3,5-triazin-2-amine
4-({[5-(2-chlorophenyl)-1,3-oxazol-2-yl]methyl}sulfanyl)-6-methyl-1,3,5-triazin-2-amine
4-({[5-(3-methoxyphenyl)-1,3-oxazol-2-yl]methyl}sulfanyl)-6-methyl-1,3,5-triazin-2-amine
2-({[5-(3-chloro-5-methoxyphenyl)-1,3-oxazol-2-yl]methyl}sulfanyl)-6-methylpyrimidin-4-amine
4-({[5-(2,3-dichlorophenyl)-1,3-oxazol-2-yl]methyl}sulfanyl)-6-ethyl-1,3,5-triazin-2-amine
6-methyl-2-[({5-[3-(trifluoromethyl)phenyl]-1,3-oxazol-2-yl}methyl)sulfanyl]pyrimidin-4-amine
2-({[5-(2,3-dichlorophenyl)-1,3-oxazol-2-yl]methyl}sulfanyl)-6-methylpyrimidin-4-amine
4-methyl-6-[({5-[3-(trifluoromethyl)phenyl]-1,3-oxazol-2-yl}methyl)sulfanyl]-1,3,5-triazin-2-amine
4-({[5-(3-bromophenyl)-1,3-oxazol-2-yl]methyl}sulfanyl)-6-methyl-1,3,5-triazin-2-amine
4-({[5-(3-ethoxyphenyl)-1,3-oxazol-2-yl]methyl}sulfanyl)-6-methyl-1,3,5-triazin-2-amine
4-({[5-(2,3-dichlorophenyl)-1,3-oxazol-2-yl]methyl}sulfanyl)-6-methyl-1,3,5-triazin-2-amine
2-({[5-(2,5-dichlorophenyl)-1,3-oxazol-2-yl]methyl}sulfanyl)-6-(trifluoromethyl)pyrimidin-4-amine
4-[chloro(fluoro)methyl]-6-({[5-(3-chlorophenyl)-1,3-oxazol-2-yl]methyl}sulfanyl)-1,3,5-triazin-2-amine
4-({[5-(3-chloro-5-methoxyphenyl)-1,3-oxazol-2-yl]methyl}sulfanyl)-6-methyl-1,3,5-triazin-2-amine
6-({[5-(2,5-dichlorophenyl)-1,3-oxazol-2-yl]methyl}thio)-N,N-dimethyl-1,3,5-triazine-2,4-diamine
4-methyl-6-({[5-(4-methylphenyl)-1,3-oxazol-2-yl]methyl}sulfanyl)-1,3,5-triazin-2-amine
2-({[5-(2-chlorophenyl)-1,3-oxazol-2-yl]methyl}sulfanyl)-6-methylpyrimidin-4-amine
4-methyl-6-({[5-(naphthalen-2-yl)-1,3-oxazol-2-yl]methyl}sulfanyl)-1,3,5-triazin-2-amine
2-({[5-(3-methoxyphenyl)-1,3-oxazol-2-yl]methyl}sulfanyl)-6-(trifluoromethyl)pyrimidin-4-amine
4-methyl-6-[({5-[3-(trifluoromethoxy)phenyl]-1,3-oxazol-2-yl}methyl)sulfanyl]-1,3,5-triazin-2-amine
2-({[5-(4-methoxyphenyl)-1,3-oxazol-2-yl]methyl}sulfanyl)-6-methylpyrimidin-4-amine
4-({[5-(4-methoxyphenyl)-1,3-oxazol-2-yl]methyl}sulfanyl)-6-methyl-1,3,5-triazin-2-amine
6-methyl-2-({[5-(naphthalen-2-yl)-1,3-oxazol-2-yl]methyl}sulfanyl)pyrimidin-4-amine
6-methyl-2-({[5-(2-methylphenyl)-1,3-oxazol-2-yl]methyl}sulfanyl)pyrimidin-4-amine
4-([5-(3-chlorophenyl)-1,3-oxazol-2-yl]methylsulfanyl)-6-(trifluoromethyl)-1,3,5-triazin-2-amine
4-({[5-(2-chloro-5-methoxyphenyl)-1,3-oxazol-2-yl]methyl}sulfanyl)-6-(morpholin-4-yl)-1,3,5-triazin-2-amine
2-([5-(3-methoxyphenyl)-1,3-oxazol-2-yl]methylsulfanyl)-N,6-dimethylpyrimidin-4-amine
4-({[5-(4-ethylphenyl)-1,3-oxazol-2-yl]methyl}sulfanyl)-6-methyl-1,3,5-triazin-2-amine
2-({[5-(2-chlorophenyl)-1,3-oxazol-2-yl]methyl}sulfanyl)-6-(trifluoromethyl)pyrimidin-4-amine
4-({[5-(3-chlorophenyl)-1,3-oxazol-2-yl]methyl}sulfanyl)-6-phenyl-1,3,5-triazin-2-amine
4-([5-(2,5-dichlorophenyl)-1,3-oxazol-2-yl]methylsulfanyl)-6-(morpholin-4-yl)-1,3,5-triazin-2-amine
2-({[5-(3-chlorophenyl)-1,3-oxazol-2-yl]methyl}sulfanyl)-6-(trifluoromethyl)pyrimidin-4-amine
4-cyclopropyl-6-([5-(3-methoxyphenyl)-1,3-oxazol-2-yl]methylsulfanyl)-1,3,5-triazin-2-amine
2-({[5-(3-chloro-5-methoxyphenyl)-1,3-oxazol-2-yl]methyl}sulfanyl)-6-(trifluoromethyl)pyrimidin-4-amine
2-({[5-(2,3-dichlorophenyl)-1,3-oxazol-2-yl]methyl}sulfanyl)-6-(trifluoromethyl)pyrimidin-4-amine
4-({[5-(3-chlorophenyl)-1,3-oxazol-2-yl]methyl}sulfanyl)-6-(1,3-thiazol-4-yl)-1,3,5-triazin-2-amine
4-({[5-(3-chlorophenyl)-1,3-oxazol-2-yl]methyl}sulfanyl)-6-(1-methyl-1H-pyrazol-4-yl)-1,3,5-triazin-2-amine
4-({[5-(4-fluorophenyl)-1,3-oxazol-2-yl]methyl}sulfanyl)-6-methyl-1,3,5-triazin-2-amine
2-({thieno[2,3-d]pyrimidin-4-ylsulfanyl}methyl)-5-[3-(trifluoromethyl)phenyl]-1,3-oxazole
4-({[5-(3-chlorophenyl)-1,3-oxazol-2-yl]methyl}sulfanyl)-6-(1-methylcyclopropyl)-1,3,5-triazin-2-amine
4-({[5-(3-chlorophenyl)-1,3-oxazol-2-yl]methyl}sulfanyl)-6-(3,3-difluorocyclobutyl)-1,3,5-triazin-2-amine
6-methyl-2-[({5-[3-(trifluoromethoxy)phenyl]-1,3-oxazol-2-yl}methyl) sulfanyl]pyrimidin-4-amine
2-[({5-[3-(trifluoromethoxy)phenyl]-1,3-oxazol-2-yl}methyl)sulfanyl]-6-(trifluoromethyl)pyrimidin-4-amine
2-({[5-(2-methoxyphenyl)-1,3-oxazol-2-yl]methyl}sulfanyl)-6-methylpyrimidin-4-amine
5-(2,5-dichlorophenyl)-2-[({2-methylpyrido[2,3-d]pyrimidin-4-yl}sulfanyl)methyl]-1,3-oxazole
5-(3-methoxyphenyl)-2-({thieno[2,3-d]pyrimidin-4-ylsulfanyl}methyl)-1,3-oxazole
2-({[5-(4-methylphenyl)-1,3-oxazol-2-yl]methyl}sulfanyl)-6-(trifluoromethyl)pyrimidin-4-amine
4-([5-(3-chlorophenyl)-1,3-oxazol-2-yl]methylsulfanyl)-6-methoxy-1,3,5-triazin-2-amine
4-ethyl-6-[({5-[3-(trifluoromethoxy)phenyl]-1,3-oxazol-2-yl}methyl)sulfanyl]-1,3,5-triazin-2-amine
2-({[5-(3-chlorophenyl)-1,3-oxazol-2-yl]methyl}sulfanyl)-N-methylquinazolin-4-amine
4-({[5-(3-chlorophenyl)-1,3-oxazol-2-yl]methyl}sulfanyl)-6-(methoxymethyl)-1,3,5-triazin-2-amine
6-({[5-(2-chlorophenyl)-1,3-oxazol-2-yl]methyl}sulfanyl)-1,3,5-triazine-2,4-diamine
2-({[5-(3-chlorophenyl)-1,3,4-oxadiazol-2-yl]methyl}sulfanyl)-6-methylpyrimidin-4-amine
2-({[5-(3-chloro-4-fluorophenyl)-1,3-oxazol-2-yl]methyl}sulfanyl)-6-(trifluoromethyl)pyrimidin-4-amine
2-({[5-(3-chlorophenyl)-1,3,4-oxadiazol-2-yl]methyl}sulfanyl)-6-(trifluoromethyl)pyrimidin-4-amine
5-(3-methoxyphenyl)-2-[({2-methylpyrido[2,3-d]pyrimidin-4-yl}sulfanyl)methyl]-1,3-oxazole
2-({[5-(4-fluorophenyl)-1,3-oxazol-2-yl]methyl}sulfanyl)-6-methylpyrimidin-4-amine
2-({[5-(4-chlorophenyl)-1,3-oxazol-2-yl]methyl}sulfanyl)-6-methylpyrimidin-4-amine
6-methyl-2-{[(5-phenyl-1,3-oxazol-2-yl)methyl]sulfanyl}pyrimidin-4-amine.

## Patentansprüche

1. Verbindung der allgemeinen Formel (I)
R¹-S-CH₂-OXA-R² (I)
einschließlich Enantiomere, Diastereomere, Hydrate, Solvate, pharmazeutisch verträgliche Cokristalle oder Salze und Komplexe davon;
wobei
OXA ist 1,3-Oxazolyl;
die Gruppe R¹-S-CH₂ an C² des 1,3-Oxazolyls gebunden und die Gruppe R² ist an C⁴ des 1,3-Oxazolyls gebunden ist;
R¹ eine 6-gliedrige Heteroarylgruppe ist, die aus der Gruppe ausgewählt ist, die aus 1,3,5-Triazinyl und Pyrimidinyl besteht, die unabhängig voneinander mit einem oder mehreren Substituenten substituiert sind, die aus der Gruppe ausgewählt sind, die aus Hydroxyl, Amino, C₁-C₆-Alkyl, C₃-C₆-Cycloalkyl, C₁-C₄-Alkoxy, N-Mono- oder N,N-Di-substituiertem C₁-C₃-Alkylamino, nicht-aromatischem 5- bis 6-gliedrigem Heterocyclyl,, 6-gliedrigem Aryl und 5- bis 6-gliedrigem Heteroaryl besteht, wobei die Substituenten unsubstituiert oder mit einer oder mehreren Gruppen, ausgewählt aus der Gruppe bestehend aus Halogenid, Cyano und C₁-C₆-Alkyl, substituiert sein können, wobei die 6-gliedrige Aryl- bzw. 5- bis 6-gliedrige Heteroarylgruppe mit der 1,3,5-Triazinyl- bzw. Pyrimidylgruppe kondensiert sein kann, und
R² Phenyl ist, das unsubstituiert ist oder mit einem oder mehreren Substituenten substituiert ist, die ausgewählt sind aus der Gruppe bestehend aus Halogenid, Cyano, Amino, C₁-C₆-Alkyl, C₃-C₆-Cycloalkyl, das gegebenenfalls mit einem oder mehreren Halogeniden substituiert sein kann, C₁-C₄-Alkoxy, das gegebenenfalls mit einem oder mehreren Halogeniden substituiert sein kann, Hydroxy-C₁-C₆-alkyl, Sulfonyl-C₁-C₆-alkyl, Sulfamidyl-N-C₁-C6-alkyl und Carboxamidyl-N-mono- oder -N,N-di-C₁-C₆-alkyl;
mit der Maßgabe, dass die folgenden Verbindungen ausgeschlossen sind:

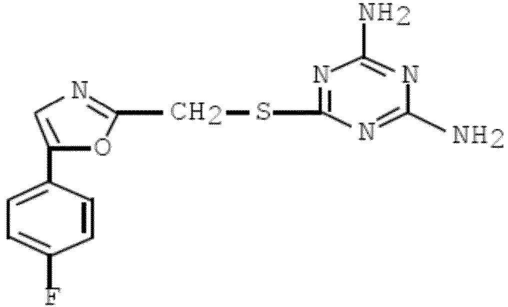

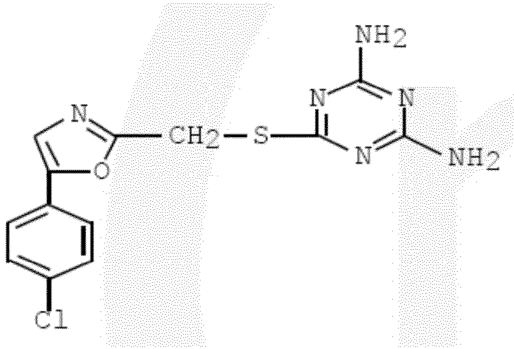

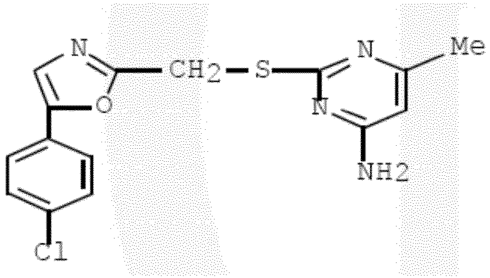

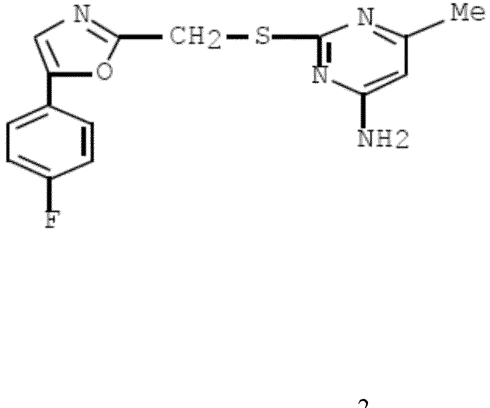

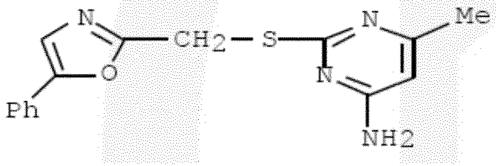

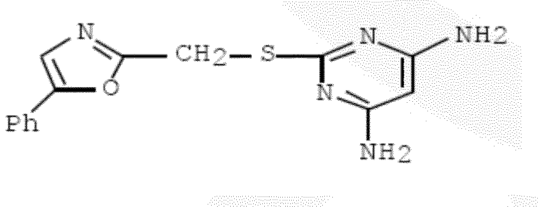

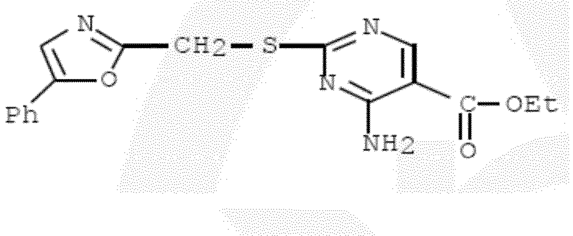

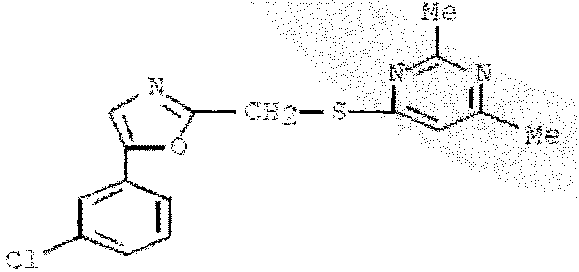

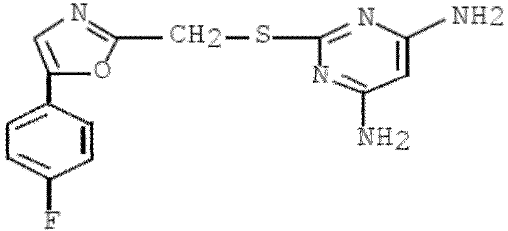

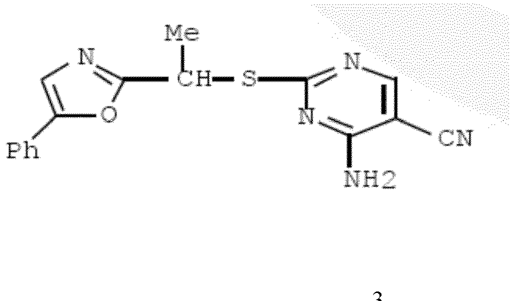

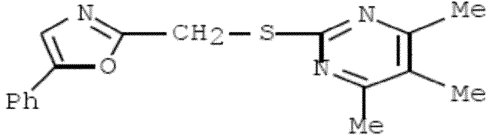

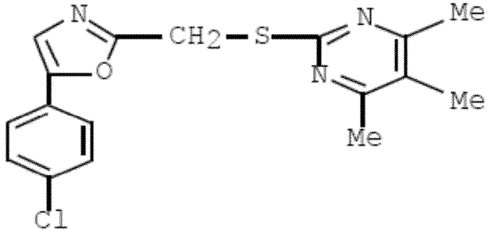

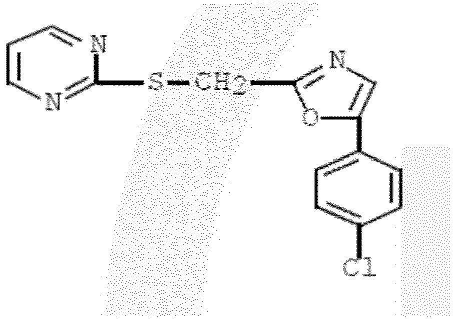

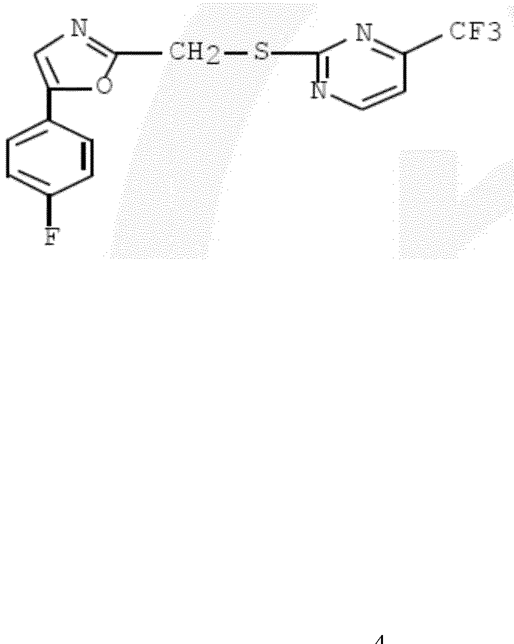

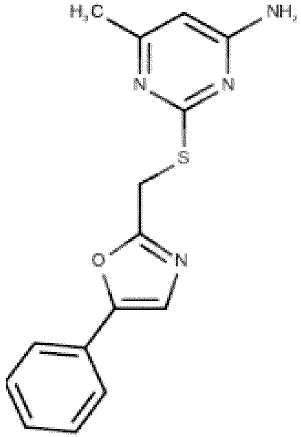

2. Verbindung nach Anspruch 1, wobei das Halogenid ausgewählt ist aus Cl, Br und F.

3. Verbindung nach Anspruch 1 oder 2, woei R¹ 1,3,5-Triazinyl ist, das wie in Anspruch 1 definiert substituiert ist.

4. Verbindung nach Anspruch 3, wobei, wenn die 1,3,5-Triazinylgruppe mit mehr als einem Substituenten substituiert ist, die Substituenten unterschiedlich sind.

5. Verbindung nach Anspruch 3 oder 4, wobei die 1,3,5-Triazinylgruppe unabhängig voneinander mit einem oder zwei Substituenten, ausgewählt aus der Gruppe bestehend aus Amino, Methyl, Ethyl, Isopropyl und tert.-Butyl, substituiert ist, wobei der/die Substituent(en) gegebenenfalls mit einem oder mehreren Halogeniden substituiert ist/sind.

6. Verbindung nach Anspruch 3, wobei R¹ ausgewählt ist aus der Gruppe bestehend aus 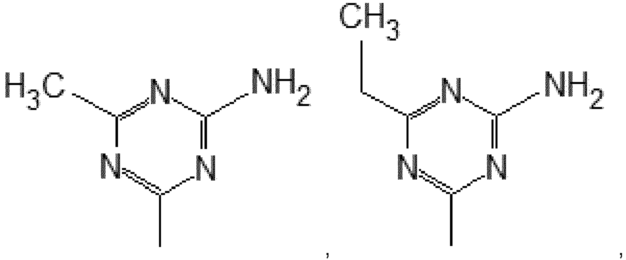 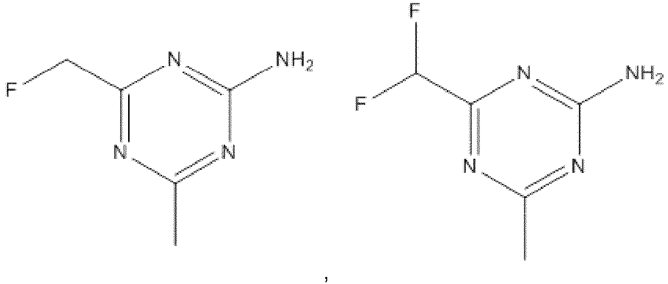 und

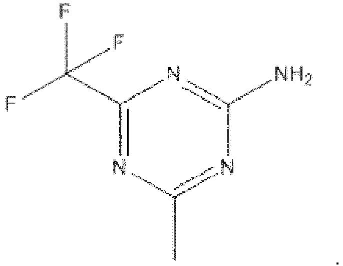

7. Verbindung nach einem der Ansprüche 1 oder 2, wobei R¹ ein wie in Anspruch 1 definiertes substituiertes Pyrimidinyl ist.

8. Verbindung nach Anspruch 7, wobei, wenn die Pyrimidinylgruppe mit mehr als einem Substituenten substituiert ist, die Substituenten unterschiedlich sind.

9. Verbindung nach Anspruch 7 oder 8, wobei die Pyrimidinylgruppe unabhängig voneinander durch einen oder mehrere Substituenten, ausgewählt aus der Gruppe bestehend aus Amino, Methyl und Ethyl, substituiert ist.

10. Verbindung nach Anspruch 9, wobei die Aminogruppe mit Methyl substituiert ist.

11. Verbindung nach Anspruch 9 oder 10, wobei die Methyl- oder Ethylgruppe mit einem oder mehreren Halogeniden gemäß der Definition im Anspruch 2 substituiert ist.

12. Verbindung nach Anspruch 7, wobei R¹ ausgewählt ist aus der Gruppe bestehend aus

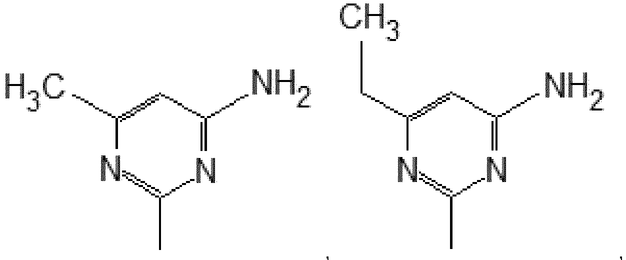

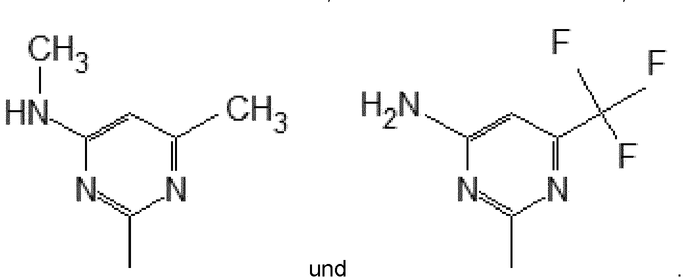

13. Verbindung nach einem der vorhergehenden Ansprüche, wobei R² unabhängig voneinander mit einem oder mehreren Substituenten, ausgewählt aus der Gruppe bestehend aus Cl, Br, F, Methyl, Triflourmethyl, Methoxy und Ethoxy, substituiert ist.

14. Verbindung nach einem der vorhergehenden Ansprüche, wobei R² in mindestens einer meta-Position und/oder mindestens einer ortho-Position substituiert ist.

15. Verbindung nach Anspruch 14, wobei R² ausgewählt ist aus der Gruppe bestehend aus

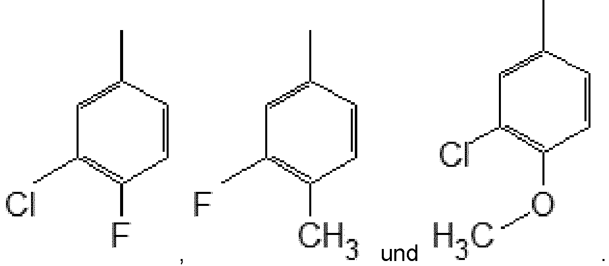

16. Verbindung nach Anspruch 13 oder 14, wobei R² in para-Stellung nicht substituiert ist.

17. Verbindung nach Anspruch 16, wobei R² ausgewählt ist aus der Gruppe bestehend aus 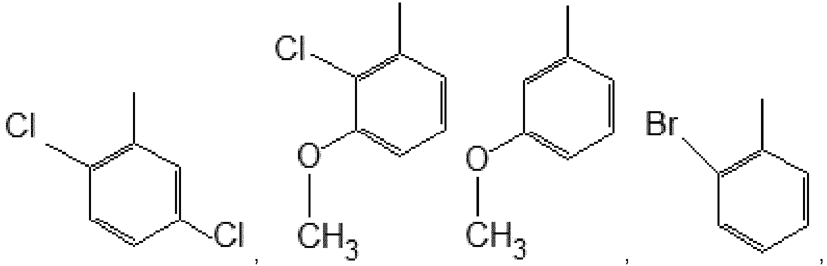 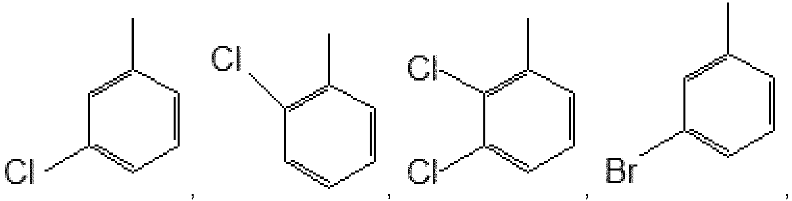 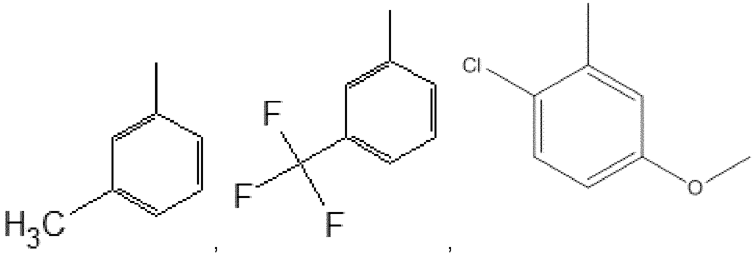 und.

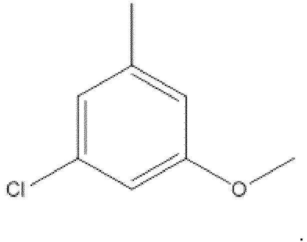

18. Die Verbindung nach Anspruch 1, ausgewählt aus der Gruppe bestehend aus den folgenden Verbindungen:
2-({[5-(2-Chlor-5-methoxyphenyl)-1,3-oxazol-2-yl]methyl}sulfanyl)-6-methylpyrimidin-4-amin
4-({[5-(2-Chlor-5-methoxyphenyl)-1,3-oxazol-2-yl]methyl}sulfanyl)-6-(diflourmethyl)-1,3,5-triazin-2-amin
4-([5-(2,5-Dichlorphenyl)-1,3-oxazol-2-yl]methylsulfanyl)-6-(difluormethyl)-1,3,5-triazin-2-amin
4-({[5-(2-chlor-5-Methoxyphenyl)-1,3-oxazol-2-yl]methyl}sulfanyl)-6-(flourmethyl)-1,3,5-triazin-2-amin
2-({[5-(2-Chlor-3-Methoxyphenyl)-1,3-oxazol-2-yl]methyl}sulfanyl)-6-methylpyrimidin-4-amin
2-({[5-(2-Chlor-3-Methoxyphenyl)-1,3-oxazol-2-yl]methyl}sulfanyl)-6-methylpyrimidin-4-amin
4-({[5-(2,5-Dichlorphenyl)-1,3-oxazol-2-yl]methyl}sulfanyl)-6-methyl-1,3,5-triazin-2-amin
4-({[5-(2-Chlor-5-methoxyphenyl)-1,3-oxazol-2-yl]methyl}sulfanyl)-6-ethyl-1,3,5-triazin-2-amin
4-({[5-(2-Chlor-5-methoxyphenyl)-1,3-oxazol-2-yl]methyl}sulfanyl)-6-methyl-1,3,5-triazin-2-amin
4-({[5-(3-Chlor-4-methoxyphenyl)-1,3-oxazol-2-yl]methyl}sulfanyl)-6-methyl-1,3,5-triazin-2-amin
2-({[5-(2-Chlor-5-methoxyphenyl)-1,3-oxazol-2-yl]methyl}sulfanyl)-6-(trifluormethyl)pyrimidin-4-amin
4-([5-(2,5-Dichlorphenyl)-1,3-oxazol-2-yl]methylsulfanyl)-6-(fluormethyl)-1,3,5-triazin-2-amin
4-({[5-(2,5-Dichlorphenyl)-1,3-oxazol-2-yl]methyl}sulfanyl)-6-methylpyrimidin-4-amin
4-([5-(2,5-Dichlorphenyl)-1,3-oxazol-2-yl]methylsulfanyl)-6-ethyl-1,3,5-triazin-2-amin
2-({[5-(3-Chlor-4-methoxyphenyl)-1,3-oxazol-2-yl]methyl}sulfanyl)-6-methylpyrimidin-4-amin
4-({[5-(2-Chlor-3-methoxyphenyl)-1,3-oxazol-2-yl]methyl}sulfanyl)-6-methyl-1,3,5-triazin-2-amin
4-({[5-(3-Chlor-4-methoxyphenyl)-1,3-oxazol-2-yl]methyl}sulfanyl)-6-ethyl-1,3,5-triazin-2-amin
4-Ethyl-6-({[5-(3-methoxyphenyl)-1,3-oxazol-2-yl]methyl}sulfanyl)-1,3,5-triazin-2-amin
2-({[5-(2-Bromphenyl)-1,3-oxazol-2-yl]methyl}sulfanyl)-6-methylpyrimidin-4-amin
2-({[5-(3-Methoxyphenyl)-1,3-oxazol-2-yl]methyl}sulfanyl)-6-methylpyrimidin-4-amin
4-({[5-(3-Chlorphenyl)-1,3-oxazol-2-yl]methyl}sulfanyl)-6-(pyridin-4-yl)-1,3,5-triazin-2-amin
4-({[5-(3-Chlor-5-methoxyphenyl)-1,3-oxazol-2-yl]methyl}sulfanyl)-6-ethyl-1,3,5-triazin-2-amin
4-({[5-(3-Chlorphenyl)-1,3-oxazol-2-yl]methyl}sulfanyl)-6-ethyl-1,3,5-triazin-2-amin
4-({[5-(3-Chlor-4-flourphenyl)-1,3-oxazol-2-yl]methyl}sulfanyl)-6-methyl-1,3,5-triazin-2-amin
6-Methyl-2-({[5-(4-methylphenyl)-1,3-oxazol-2-yl]methyl}sulfanyl)pyrimidin-4-amin
2-({[5-(3-Chlorphenyl)-1,3-oxazol-2-yl]methyl}sulfanyl)-6-methylpyrimidin-4-amin
4-({[5-(3-Chlorphenyl)-1,3-oxazol-2-yl]methyl}sulfanyl)-6-(1-methyl-1H-pyrazol-5-yl)-1,3,5-triazin-2-amin
2-[({5-[3-Chlor-4-(3-methansulfonylpropoxy)phenyl]-1,3-oxazol-2-yl}methyl)sulfanyl]-6-methylpyrimidin-4-amin
2-({[5-(3-Ethoxyphenyl)-1,3-oxazol-2-yl]methyl}sulfanyl)-6-methylpyrimidin-4-amin
2-({[5-(3-Chlor-4-fluorphenyl)-1,3-oxazol-2-yl]methyl}sulfanyl)-6-methylpyrimidin-4-amin
4-({[5-(3-Chlorphenyl)-1,3-oxazol-2-yl]methyl}sulfanyl)-6-methyl-1,3,5-triazin-2-amin
4-({[5-(2-Chlorphenyl)-1,3-oxazol-2-yl]methyl}sulfanyl)-6-methyl-1,3,5-triazin-2-amin
4-({[5-(3-Methoxyphenyl)-1,3-oxazol-2-yl]methyl}sulfanyl)-6-methyl-1,3,5-triazin-2-amin
2-({[5-(3-Chlor-5-methoxyphenyl)-1,3-oxazol-2-yl]methyl}sulfanyl)-6-methylpyrimidin-4-amin
4-({[5-(2,3-Dichlorphenyl)-1,3-oxazol-2-yl]methyl}sulfanyl)-6-ethyl-1,3,5-triazin-2-amin
6-Methyl-2-[({5-[3-(trifluormethyl)phenyl]-1,3-oxazol-2-yl}methyl)sulfanyl]pyrimidin-4-amin
2-({[5-(2,3-Dichlorphenyl)-1,3-oxazol-2-yl]methyl}sulfanyl)-6-methylpyrimidin-4-amin
4-Methyl-6-[({5-[3-(triflourmethyl)phenyl]-1,3-oxazol-2-yl}methyl)sulfanyl]-1,3,5-triazin-2-amin
4-({[5-(3-Bromphenyl)-1,3-oxazol-2-yl]methyl}sulfanyl)-6-methyl-1,3,5-triazin-2-amin
4-({[5-(3-eEhoxyphenyl)-1,3-oxazol-2-yl]methyl}sulfanyl)-6-methyl-1,3,5-triazin-2-amin
4-({[5-(2,3-Dichlorphenyl)-1,3-oxazol-2-yl]methyl}sulfanyl)-6-methyl-1,3,5-triazin-2-amin
2-({[5-(2,5-Dichlorphenyl)-1,3-oxazol-2-yl]methyl}sulfanyl)-6-(trifluormethyl)pyrimidin-4-amin
4-[Chlor(flour)methyl]-6-({[5-(3-chlorphenyl)-1,3-oxazol-2-yl]methyl}sulfanyl)-1,3,5-triazin-2-amin
4-({[5-(3-Chlor-5-methoxyphenyl)-1,3-oxazol-2-yl]methyl}sulfanyl)-6-methyl-1,3,5-triazin-2-amin
6-({[5-(2,5-Dichlorphenyl)-1,3-oxazol-2-yl]methyl}thio)-N,N-dimethyl-1,3,5-triazin-2,4-diamin
4-Methyl-6-({[5-(4-methylphenyl)-1,3-oxazol-2-yl]methyl}sulfanyl)-1,3,5-triazin-2-amin
2-({[5-(2-Chlorphenyl)-1,3-oxazol-2-yl]methyl}sulfanyl)-6-methylpyrimidin-4-amin
4-Methyl-6-({[5-(naphthalen-2-yl)-1,3-oxazol-2-yl]methyl}sulfanyl)-1,3,5-triazin-2-amin
2-({[5-(3-Methoxyphenyl)-1,3-oxazol-2-yl]methyl}sulfanyl)-6-(trifluormethyl)pyrimidin-4-amin
4-Methyl-6-[({5-[3-(triflourmethoxy)phenyl]-1,3-oxazol-2-yl}methyl)sulfanyl]-1,3,5-triazin-2-amin
2-({[5-(4-Methoxyphenyl)-1,3-oxazol-2-yl]methyl}sulfanyl)-6-methylpyrimidin-4-amin
4-({[5-(4-Methoxyphenyl)-1,3-oxazol-2-yl]methyl}sulfanyl)-6-methyl-1,3,5-triazin-2-amin
6-Methyl-2-({[5-(naphthalen-2-yl)-1,3-oxazol-2-yl]methyl}sulfanyl)pyrimidin-4-amin
6-Methyl-2-({[5-(2-methylphenyl)-1,3-oxazol-2-yl]methyl}sulfanyl)pyrimidin-4-amin
4-([5-(3-Chlorphenyl)-1,3-oxazol-2-yl]methylsulfanyl)-6-(trifluormethyl)-1,3,5-triazin-2-amin
4-({[5-(2-Chlor-5-methoxyphenyl)-1,3-oxazol-2-yl]methyl}sulfanyl)-6-(morpholin-4-yl)-1,3,5-triazin-2-amin
2-([5-(3-Methoxyphenyl)-1,3-oxazol-2-yl]methylsulfanyl)-*N*,6-dimethylpyrimidin-4-amin
4-({[5-(4-Ethylphenyl)-1,3-oxazol-2-yl]methyl}sulfanyl)-6-methyl-1,3,5-triazin-2-amin
2-({[5-(2-Chlorphenyl)-1,3-oxazol-2-yl]methyl}sulfanyl)-6-(trifluormethyl)pyrimidin-4-amin
4-({[5-(3-Chlorphenyl)-1,3-oxazol-2-yl]methyl}sulfanyl)-6-phenyl-1,3,5-triazin-2-amin
4-([5-(2,5-dichlorphenyl)-1,3-oxazol-2-yl]methylsulfanyl)-6-(morpholin-4-yl)-1,3,5-triazin-2-amin
2-({[5-(3-Chlorphenyl)-1,3-oxazol-2-yl]methyl}sulfanyl)-6-(trifluormethyl)pyrimidin-4-amin
4-Cyclopropyl-6-([5-(3-methoxyphenyl)-1,3-oxazol-2-yl]methylsulfanyl)-1,3,5-triazin-2-amin
2-({[5-(3-Chlor-5-methoxyphenyl)-1,3-oxazol-2-yl]methyl}sulfanyl)-6-(trifluormethyl)pyrimidin-4-amin
2-({[5-(2,3-Dichlorphenyl)-1,3-oxazol-2-yl]methyl}sulfanyl)-6-(trifluormethyl)pyrimidin-4-amin
4-({[5-(3-Chlorphenyl)-1,3-oxazol-2-yl]methyl}sulfanyl)-6-(1,3-thiazol-4-yl)-1,3,5-triazin-2-amin
4-({[5-(3-Dichlorphenyl)-1,3-oxazol-2-yl]methyl}sulfanyl)-6-(1-methyl-1H-pyrazol-4-yl)-1,3,5-triazin-2-amin
4-({[5-(4-Flourphenyl)-1,3-oxazol-2-yl]methyl}sulfanyl)-6-methyl-1,3,5-triazin-2-amin
2-({Thieno[2,3-d]pyrimidin-4-ylsulfanyl}methyl)-5-[3-(trifluormethyl)phenyl]-1,3-oxazol
4-({[5-(3-Chlorphenyl)-1,3-oxazol-2-yl]methyl}sulfanyl)-6-(1-methylcyclopropyl)-1,3,5-triazin-2-amin
4-({[5-(3-Dichlorphenyl)-1,3-oxazol-2-yl]methyl}sulfanyl)-6-(3,3-diflourcyclobutyl)-1,3,5-triazin-2-amin
6-Methyl-2-[({5-[3-(trifluormethoxy)phenyl]-1,3-oxazol-2-yl}methyl)sulfanyl]pyrimidin-4-amin
2-[({5-[3-(Trifluormethoxy)phenyl]-1,3-oxazol-2-yl}methyl)sulfanyl]-6-(trifluormethyl)pyrimidin-4-amin
2-({[5-(2-Methoxyphenyl)-1,3-oxazol-2-yl]methyl}sulfanyl)-6-methylpyrimidin-4-amin
5-(2,5-Dichlorphenyl)-2-[({2-methylpyrido[2,3-d]pyrimidin-4-yl}sulfanyl)methyl]-1,3-oxazol
5-(3-Methoxyphenyl)-2-({thieno[2,3-d]pyrimidin-4-ylsulfanyl}methyl)-1,3-oxazol
2-({[5-(4-Methylphenyl)-1,3-oxazol-2-yl]methyl}sulfanyl)-6-(trifluormethyl)pyrimidin-4-amin
4-([5-(3-Chlorphenyl)-1,3-oxazol-2-yl]methylsulfanyl)-6-methoxy-1,3,5-triazin-2-amin
4-Ethyl-6-[({5-[3-(triflourmethoxy)phenyl]-1,3-oxazol-2-yl}methyl)sulfanyl]-1,3,5-triazin-2-amin
2-({[5-(3-Chlorphenyl)-1,3-oxazol-2-yl]methyl}sulfanyl)-N-methylchinazolin-4-amin
4-({[5-(3-Chlorphenyl)-1,3-oxazol-2-yl]methyl}sulfanyl)-6-(methoxymethyl)-1,3,5-triazin-2-amin
6-({[5-(2-Chlorphenyl)-1,3-oxazol-2-yl]methyl}sulfanyl)-1,3,5-triazin-2,4-diamin
2-({[5-(3-Chlor-4-fluorphenyl)-1,3-oxazol-2-yl]methyl}sulfanyl)-6-(trifluormethyl)pyrimidin-4-amin
5-(3-Methoxyphenyl)-2-[({2-methylpyrido[2,3-d]pyrimidin-4-yl}sulfanyl)methyl]-1,3-oxazol

19. Pharmazeutische Zusammensetzung, umfassend mindestens eine Verbindung nach einem der vorhergehenden Ansprüche und mindestens einen pharmazeutischen Träger.

20. Eine Verbindung der allgemeinen Formel (I)
R¹-S-CH₂-OXA-R² (I)
einschließlich Enantiomere, Diastereomere, Hydrate, Solvate, pharmazeutisch verträgliche Cokristalle oder Salze und Komplexe davon;
wobei
OXA ausgewählt ist aus der Gruppe bestehend aus 1,3-Oxazolyl, 1,2,4-Oxadiazolyl oder 1,3,4-Oxadiazolyl;
wenn OXA 1,3-Oxazolyl ist, ist die Gruppe R¹-S-CH₂ an C² des 1,3-Oxazolyls gebunden und die Gruppe R² ist an C⁴ des 1,3-Oxazolyls gebunden;
wenn OXA 1,2,4-Oxadiazolyl ist, ist die Gruppe R¹-S-CH₂ an C⁵ des 1,2,4-Oxadiazolyls gebunden und die Gruppe R² ist an C³ des 1,2,4-Oxadiazolyls gebunden;
wenn OXA 1,3,4-Oxadiazolyl ist, ist die Gruppe R¹-S-CH₂ an C⁵ des 1,3,4-Oxadiazolyls gebunden und die Gruppe R² ist an C² des 1,3,4-Oxadiazolyls' gebunden;
R¹ eine 6-gliedrige Heteroarylgruppe ist, die aus der Gruppe ausgewählt ist, die aus 1,3,5-Triazinyl und Pyrimidinyl besteht, die unabhängig voneinander mit einem oder mehreren Substituenten substituiert sind, die aus der Gruppe ausgewählt sind, die aus Hydroxyl, Amino, C₁-C₆-Alkyl, C₃-C₆-Cycloalkyl, C₁-C₄-Alkoxy, N-Mono- oder N, N-Di-substituiertem C₁-C₃-Alkylamino, nicht-aromatischem 5- bis 6-gliedrigem Heterocyclyl besteht, 6-gliedriges Aryl und 5- bis 6-gliedriges Heteroaryl, wobei die Substituenten unsubstituiert oder mit einer oder mehreren Gruppen, ausgewählt aus der Gruppe bestehend aus Halogenid, Cyano und C₁-C₆-Alkyl, substituiert sein können, wobei die 6-gliedrige Aryl- bzw. 5- bis 6-gliedrige Heteroarylgruppe an die 1,3,5-Triazinyl- bzw. Pyrimidylgruppe kondensiert sein kann, und
R² Phenyl ist, das unsubstituiert ist oder mit einem oder mehreren Substituenten substituiert ist, die ausgewählt sind aus der Gruppe bestehend aus Halogenid, Cyano, Amino, C₁-C₆-Alkyl, C₃-C₆-Cycloalkyl, das gegebenenfalls mit einem oder mehreren Halogeniden substituiert sein kann, C₁-C₄-Alkoxy, das gegebenenfalls mit einem oder mehreren Halogeniden substituiert sein kann, Hydroxy-C₁-C₆-alkyl, Sulfonyl-C₁-C₆-alkyl, Sulfamidyl-N-C₁-C6-alkyl und Carboxamidyl-N-mono- oder -N,N-di-C₁-C₆-alkyl;
zur Verwendung als Agonist von GPR40 bei der Behandlung und/oder Vorbeugung von Typ-2-Diabetes mellitus und/oder einer prädiabetischen Erkrankung.

21. Verbindung zur Verwendung nach Anspruch 20, wobei der prädiabetische Zustand ausgewählt ist aus der Gruppe bestehend aus Fettleibigkeit und Insulinresistenz.

22. Die Verbindung zur Verwendung nach Anspruch 20 oder 21, wobei die Verbindung eine höhere Selektivität für GPR40 als für GPR120 aufweist.

23. Verbindung zur Verwendung nach Anspruch 22, wobei die Verbindung eine prozentuale Aktivierung von GPR40 zeigt, die mindestens dreifach höher ist als die prozentuale Aktivierung von GPR120, wobei die prozentuale Aktivierung das hundertfache Verhältnis der Aktivierung von GPR40 bzw. GPR120 durch die Verbindung zur Aktivierung von GPR40 bzw. GPR120 durch AMG 837 ist.

24. Verbindung zur Verwendung nach einem der Ansprüche 20 bis 23, wobei das Halogenid ausgewählt ist aus Cl, Br und F.

25. Verbindung zur Verwendung nach einem der Ansprüche 20 bis 24, worin R¹ wie in einem der Ansprüche 3 bis 12 definiert ist.

26. Verbindung zur Verwendung nach einem der Ansprüche 20 bis 25, worin R² wie in einem der Ansprüche 13 bis 17 definiert ist.

27. Verbindung zur Verwendung nach einem der Ansprüche 20 bis 26, wobei OXA 1,3-Oxazolyl ist.

28. Verbindung zur Verwendung nach Anspruch 20, wobei die Verbindung ausgewählt ist aus der Gruppe bestehend aus den folgenden Verbindungen:
2-({[5-(2-Chlor-5-methoxyphenyl)-1,3-oxazol-2-yl]methyl}sulfanyl)-6-methylpyrimidin-4-amin
4-({[5-(2-Chlor-5-methoxyphenyl)-1,3-oxazol-2-yl]methyl}sulfanyl)-6-(diflourmethyl)-1,3,5-triazin-2-amin
4-([5-(2,5-Dichlorphenyl)-1,3-oxazol-2-yl]methylsulfanyl)-6-(difluormethyl)-1,3,5-triazin-2-amin
4-({[5-(2-Chlor-5-methoxyphenyl)-1,3-oxazol-2-yl]methyl}sulfanyl)-6-(flourmethyl)-1,3,5-triazin-2-amin
2-({[5-(2-Chlor-3-methoxyphenyl)-1,3-oxazol-2-yl]methyl}sulfanyl)-6-methylpyrimidin-4-amin
2-({[5-(2-Chlor-3-methoxyphenyl)-1,3-oxazol-2-yl]methyl}sulfanyl)-6-methylpyrimidin-4-amin
4-({[5-(2,5-Dichlorphenyl)-1,3-oxazol-2-yl]methyl}sulfanyl)-6-methyl-1,3,5-triazin-2-amin
4-({[5-(2-Chlor-5-methoxyphenyl)-1,3-oxazol-2-yl]methyl}sulfanyl)-6-ethyl-1,3,5-triazin-2-amin
4-({[5-(2-Chlor-5-methoxyphenyl)-1,3-oxazol-2-yl]methyl}sulfanyl)-6-methyl-1,3,5-triazin-2-amin
4-({[5-(3-Chlor-4-methoxyphenyl)-1,3-oxazol-2-yl]methyl}sulfanyl)-6-methyl-1,3,5-triazin-2-amin
2-({[5-(2-Chlor-5-methoxyphenyl)-1,3-oxazol-2-yl]methyl}sulfanyl)-6-(trifluormethyl)pyrimidin-4-amin
4-([5-(2,5-Dichlorphenyl)-1,3-oxazol-2-yl]methylsulfanyl)-6-(fluormethyl)-1,3,5-triazin-2-amin
4-({[5-(2,5-Dichlorphenyl)-1,3-oxazol-2-yl]methyl}sulfanyl)-6-methylpyrimidin-4-amin
4-([5-(2,5-Dichlorphenyl)-1,3-oxazol-2-yl]methylsulfanyl)-6-ethyl-1,3,5-triazin-2-amin
2-({[5-(3-Chlor-4-methoxyphenyl)-1,3-oxazol-2-yl]methyl}sulfanyl)-6-methylpyrimidin-4-amin
4-({[5-(2-Chlor-3-methoxyphenyl)-1,3-oxazol-2-yl]methyl}sulfanyl)-6-methyl-1,3,5-triazin-2-amin
4-({[5-(3-Chlor-4-methoxyphenyl)-1,3-oxazol-2-yl]methyl}sulfanyl)-6-ethyl-1,3,5-triazin-2-amin
4-Ethyl-6-({[5-(3-methoxyphenyl)-1,3-oxazol-2-yl]methyl}sulfanyl)-1,3,5-triazin-2-amin
2-({[5-(2-Bromphenyl)-1,3-oxazol-2-yl]methyl}sulfanyl)-6-methylpyrimidin-4-amin
2-({[5-(3-Methoxyphenyl)-1,3-oxazol-2-yl]methyl}sulfanyl)-6-methylpyrimidin-4-amin
4-({[5-(3-Chlorphenyl)-1,3-oxazol-2-yl]methyl}sulfanyl)-6-(pyridin-4-yl)-1,3,5-triazin-2-amin
4-({[5-(3-Chlor-5-methoxyphenyl)-1,3-oxazol-2-yl]methyl}sulfanyl)-6-ethyl-1,3,5-triazin-2-amin
4-({[5-(3-Chlorphenyl)-1,3-oxazol-2-yl]methyl}sulfanyl)-6-ethyl-1,3,5-triazin-2-amin
4-({[5-(3-Chlor-4-flourphenyl)-1,3-oxazol-2-yl]methyl}sulfanyl)-6-methyl-1,3,5-triazin-2-amin
6-Methyl-2-({[5-(4-methylphenyl)-1,3-oxazol-2-yl]methyl}sulfanyl)pyrimidin-4-amin
2-({[5-(3-Chlorphenyl)-1,3-oxazol-2-yl]methyl}sulfanyl)-6-methylpyrimidin-4-amin
4-({[5-(3-chlorphenyl)-1,3-oxazol-2-yl]methyl}sulfanyl)-6-(1-methyl-1H-pyrazol-5-yl)-1,3,5-triazin-2-amin
2-[({5-[3-Chlor-4-(3-methansulfonylpropoxy)phenyl]-1,3-oxazol-2-yl}methyl)sulfanyl]-6-methylpyrimidin-4-amin
2-({[5-(3-Ethoxyphenyl)-1,3-oxazol-2-yl]methyl}sulfanyl)-6-methylpyrimidin-4-amin
2-({[5-(3-Chlor-4-fluorphenyl)-1,3-oxazol-2-yl]methyl}sulfanyl)-6-methylpyrimidin-4-amin
4-({[5-(3-Chlorphenyl)-1,3-oxazol-2-yl]methyl}sulfanyl)-6-methyl-1,3,5-triazin-2-amin
4-({[5-(2-Chlorphenyl)-1,3-oxazol-2-yl]methyl}sulfanyl)-6-methyl-1,3,5-triazin-2-amin
4-({[5-(3-Methoxyphenyl)-1,3-oxazol-2-yl]methyl}sulfanyl)-6-methyl-1,3,5-triazin-2-amin
2-({[5-(3-Chlor-5-methoxyphenyl)-1,3-oxazol-2-yl]methyl}sulfanyl)-6-methylpyrimidin-4-amin
4-({[5-(2,3-Dichlorphenyl)-1,3-oxazol-2-yl]methyl}sulfanyl)-6-ethyl-1,3,5-triazin-2-amin
6-Methyl-2-[({5-[3-(trifluormethyl)phenyl]-1,3-oxazol-2-yl}methyl)sulfanyl]pyrimidin-4-amin
2-({[5-(2,3-Dichlorphenyl)-1,3-oxazol-2-yl]methyl}sulfanyl)-6-methylpyrimidin-4-amin
4Methyl-6-[({5-[3-(triflourmethyl)phenyl]-1,3-oxazol-2-yl}methyl)sulfanyl]-1,3,5-triazin-2-amin
4-({[5-(3-Bromphenyl)-1,3-oxazol-2-yl]methyl}sulfanyl)-6-methyl-1,3,5-triazin-2-amin
4-({[5-(3-Ethoxyphenyl)-1,3-oxazol-2-yl]methyl}sulfanyl)-6-methyl-1,3,5-triazin-2-amin
4-({[5-(2,3-Cichlorphenyl)-1,3-oxazol-2-yl]methyl}sulfanyl)-6-methyl-1,3,5-triazin-2-amin
2-({[5-(2,5-Dichlorphenyl)-1,3-oxazol-2-yl]methyl}sulfanyl)-6-(trifluormethyl)pyrimidin-4-amin
4-[Chlor(flour)methyl]-6-({[5-(3-chlorphenyl)-1,3-oxazol-2-yl]methyl}sulfanyl)-1,3,5-triazin-2-amin
4-({[5-(3-Chlor-5-methoxyphenyl)-1,3-oxazol-2-yl]methyl}sulfanyl)-6-methyl-1,3,5-triazin-2-amin
6-({[5-(2,5-Dichlorphenyl)-1,3-oxazol-2-yl]methyl}thio)-*N,*N-dimethyl-1,3,5-triazin-2,4-diamin
4-Methyl-6-({[5-(4-methylphenyl)-1,3-oxazol-2-yl]methyl}sulfanyl)-1,3,5-triazin-2-amin
2-({[5-(2-Chlorphenyl)-1,3-oxazol-2-yl]methyl}sulfanyl)-6-methylpyrimidin-4-amin
4-Methyl-6-({[5-(naphthalen-2-yl)-1,3-oxazol-2-yl]methyl}sulfanyl)-1,3,5-triazin-2-amin
2-({[5-(3-Methoxyphenyl)-1,3-oxazol-2-yl]methyl}sulfanyl)-6-(trifluormethyl)pyrimidin-4-amin
4-Methyl-6-[({5-[3-(triflourmethoxy)phenyl]-1,3-oxazol-2-yl}methyl)sulfanyl]-1,3,5-triazin-2-amin
2-({[5-(4-Methoxyphenyl)-1,3-oxazol-2-yl]methyl}sulfanyl)-6-methylpyrimidin-4-amin
4-({[5-(4-Methoxyphenyl)-1,3-oxazol-2-yl]methyl}sulfanyl)-6-methyl-1,3,5-triazin-2-amin
6-Methyl-2-({[5-(naphthalen-2-yl)-1,3-oxazol-2-yl]methyl}sulfanyl)pyrimidin-4-amin
6-Methyl-2-({[5-(2-methylphenyl)-1,3-oxazol-2-yl]methyl}sulfanyl)pyrimidin-4-amin
4-([5-(3-Chlorphenyl)-1,3-oxazol-2-yl]methylsulfanyl)-6-(trifluormethyl)-1,3,5-triazin-2-amin
4-({[5-(2-Chlor-5-methoxyphenyl)-1,3-oxazol-2-yl]methyl}sulfanyl)-6-(morpholin-4-yl)-1,3,5-triazin-2-amin
2-([5-(3-Methoxyphenyl)-1,3-oxazol-2-yl]methylsulfanyl)-*N*,6-dimethylpyrimidin-4-amin
4-({[5-(4-Ethylphenyl)-1,3-oxazol-2-yl]methyl}sulfanyl)-6-methyl-1,3,5-triazin-2-amin
2-({[5-(2-Chlorphenyl)-1,3-oxazol-2-yl]methyl}sulfanyl)-6-(trifluormethyl)pyrimidin-4-amin
4-({[5-(3-Chlorphenyl)-1,3-oxazol-2-yl]methyl}sulfanyl)-6-phenyl-1,3,5-triazin-2-amin
4-([5-(2,5-Dichlorphenyl)-1,3-oxazol-2-yl]methylsulfanyl)-6-(morpholin-4-yl)-1,3,5-triazin-2-amin
2-({[5-(3-Chlorphenyl)-1,3-oxazol-2-yl]methyl}sulfanyl)-6-(trifluormethyl)pyrimidin-4-amin
4-Cyclopropyl-6-([5-(3-methoxyphenyl)-1,3-oxazol-2-yl]methylsulfanyl)-1,3,5-triazin-2-amin
2-({[5-(3-Chlor-5-methoxyphenyl)-1,3-oxazol-2-yl]methyl}sulfanyl)-6-(trifluormethyl)pyrimidin-4-amin
2-({[5-(2,3-Dichlorphenyl)-1,3-oxazol-2-yl]methyl}sulfanyl)-6-(trifluormethyl)pyrimidin-4-amin
4-({[5-(3-Chlorphenyl)-1,3-oxazol-2-yl]methyl}sulfanyl)-6-(1,3-thiazol-4-yl)-1,3,5-triazin-2-amin
4-({[5-(3-Chlorphenyl)-1,3-oxazol-2-yl]methyl}sulfanyl)-6-(1-methyl-1H-pyrazol-4-yl)-1,3,5-triazin-2-amin
4-({[5-(4-Flourphenyl)-1,3-oxazol-2-yl]methyl}sulfanyl)-6-methyl-1,3,5-triazin-2-amin
2-({Thieno[2,3-d]pyrimidin-4-ylsulfanyl}methyl)-5-[3-(Trifluormethyl)phenyl]-1,3-oxazol
4-({[5-(3-Chlorphenyl)-1,3-oxazol-2-yl]methyl}sulfanyl)-6-(1-methylcyclopropyl)-1,3,5-triazin-2-amin
4-({[5-(3-Chlorphenyl)-1,3-oxazol-2-yl]methyl}sulfanyl)-6-(3,3-diflourcyclobutyl)-1,3,5-triazin-2-amin
6-Methyl-2-[({5-[3-(trifluormethoxy)phenyl]-1,3-oxazol-2-yl}methyl)sulfanyl]pyrimidin-4-amin
2-[({5-[3-(Trifluormethoxy)phenyl]-1,3-oxazol-2-yl}methyl)sulfanyl]-6-(trifluormethyl)pyrimidin-4-amin
2-({[5-(2-Methoxyphenyl)-1,3-oxazol-2-yl]methyl}sulfanyl)-6-methylpyrimidin-4-amin
5-(2,5-Dichlorphenyl)-2-[({2-methylpyrido[2,3-d]pyrimidin-4-yl}sulfanyl)methyl]-1,3-oxazol
5-(3-Methoxyphenyl)-2-({thieno[2,3-d]pyrimidin-4-ylsulfanyl}methyl)-1,3-oxazol
2-({[5-(4-Methylphenyl)-1,3-oxazol-2-yl]methyl}sulfanyl)-6-(trifluormethyl)pyrimidin-4-amin
4-([5-(3-Chlorphenyl)-1,3-oxazol-2-yl]methylsulfanyl)-6-methoxy-1,3,5-triazin-2-amin
4-Ethyl-6-[({5-[3-(triflourmethoxy)phenyl]-1,3-oxazol-2-yl}methyl)sulfanyl]-1,3,5-triazin-2-amin
2-({[5-(3-Chlorphenyl)-1,3-oxazol-2-yl]methyl}sulfanyl)-N-methylchinazolin-4-amin
4-({[5-(3-Chlorphenyl)-1,3-oxazol-2-yl]methyl}sulfanyl)-6-(methoxymethyl)-1,3,5-triazin-2-amin
6-({[5-(2-Chlorphenyl)-1,3-oxazol-2-yl]methyl}sulfanyl)-1,3,5-triazin-2,4-diamin
2-({[5-(3-Chlorphenyl)-1,3,4-oxadiazol-2-yl]methyl}sulfanyl)-6-methylpyrimidin-4-amin
2-({[5-(3-Chlor-4-fluorphenyl)-1,3-oxazol-2-yl]methyl}sulfanyl)-6-(trifluormethyl)pyrimidin-4-amin
2-({[5-(3-Chlorphenyl)-1,3,4-oxadiazol-2-yl]methyl}sulfanyl)-6-(trifluormethyl)pyrimidin-4-amin
5-(3-Methoxyphenyl)-2-[({2-methylpyrido[2,3-d]pyrimidin-4-yl}sulfanyl)methyl]-1,3-oxazol
2-({[5-(4-Fluorphenyl)-1,3-oxazol-2-yl]methyl}sulfanyl)-6-methylpyrimidin-4-amin
2-({[5-(4-Chlorphenyl)-1,3-oxazol-2-yl]methyl}sulfanyl)-6-methylpyrimidin-4-amin
6-Methyl-2-{[(5-phenyl-1,3-oxazol-2-yl)methyl]sulfanyl}pyrimidin-4-amin.

## Revendications

1. Composé de formule générale (I)
R¹-S-CH₂-OXA-R² (I)
y compris les énantiomères, les diastéréomères, les hydrates, les solvates, les cristaux ou les sels pharmaceutiquement acceptables et leurs complexes ;
dans lequel
OXA est un 1,3-oxazolyle ;
le groupe R¹-S-CH₂ est lié à C² du 1,3-oxazolyle et le groupe R² est lié à C⁴ du 1,3-oxazolyle ;
R¹ est un groupe hétéroaryle à 6 membres choisi dans le groupe constitué de 1,3,5-triazinyl et pyrimidinyl indépendamment substitué par un ou plusieurs substituants choisis dans le groupe constitué d'hydroxyl, amino, C₁-C₆-alkyl, C₃-C₆-cycloalkyl, C₁-C₄-alkoxy, N-mono- ou N,N-di-substitué C₁-C₃-alkylamino, hétérocyclyle non aromatique à 5 à 6 membres, aryle à 6 membres et hétéroaryle à 5 à 6 membres dont les substituants peuvent être non substitués ou substitués par un ou plusieurs groupes choisis dans le groupe constitué d'halogénure, de cyano et de cyano, aryle à 6 chaînons et hétéroaryle à 5 à 6 chaînons dont les substituants peuvent être non substitués ou substitués par un ou plusieurs groupes choisis dans le groupe consistant en halogénure, cyano et C₁-C₆-alkyl, dans lequel le groupe aryle à 6 chaînons et le groupe hétéroaryle à 5 à 6 chaînons, respectivement, peuvent être fusionnés avec ledit groupe 1,3,5-triazinyle ou pyrimidyle, respectivement, et
R² est un phényle non substitué ou substitué par un ou plusieurs substituants choisis dans le groupe constitué par les halogénures, les cyanos, les amines, les C₁-C₆-alkyl, les C₃-C₆-cycloalkyl qui peuvent être éventuellement substitués par un ou plusieurs halogénures, C₁-C₄-alkoxy éventuellement substitué par un ou plusieurs halogénures, hydroxy-C₁-C(_{6)-akyl}, sulfonyl-C₁-C(₆)-alkyl, sulfamidyl-N-C₁-C6-alkyl et carboxamidyl-N-mono- ou -N,N-di-C₁-C(_{6)-alkyl} ;
à l'exclusion des composés suivants :

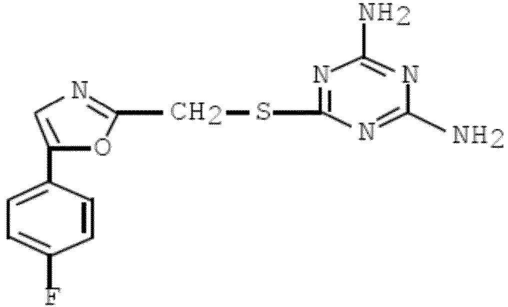

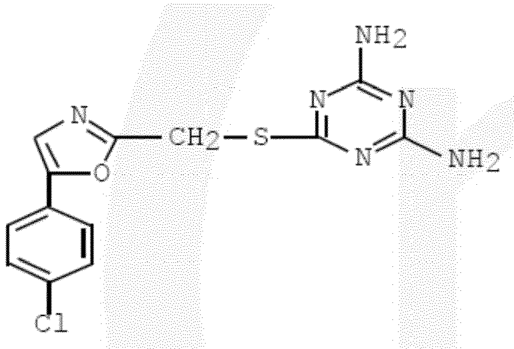

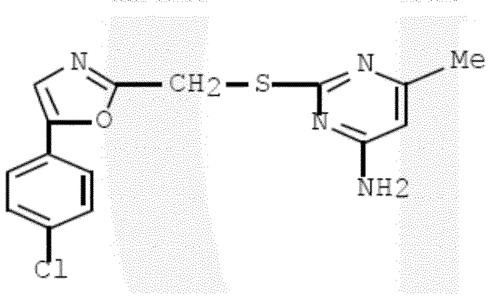

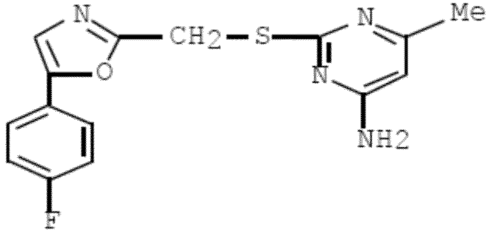

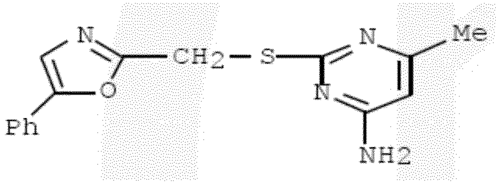

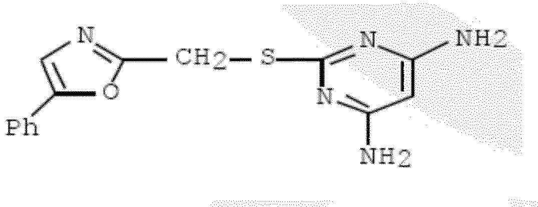

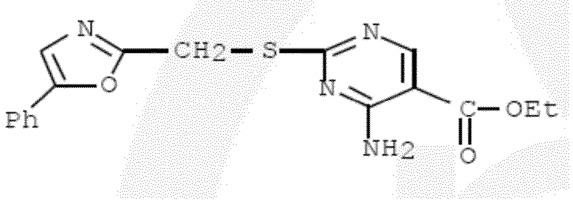

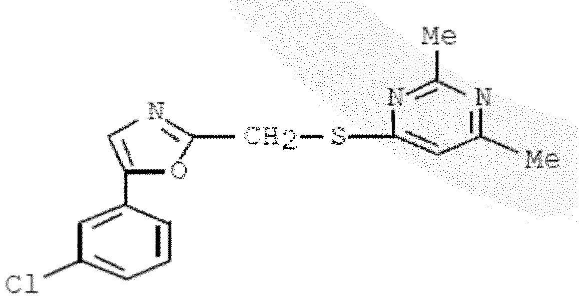

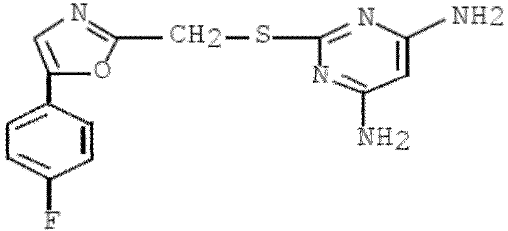

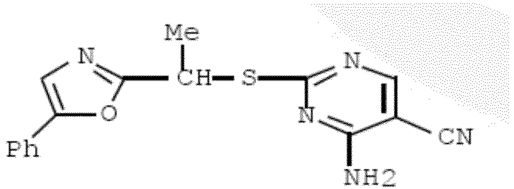

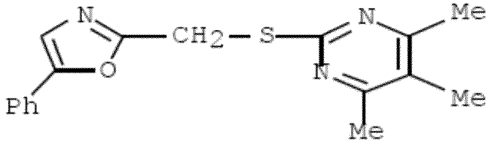

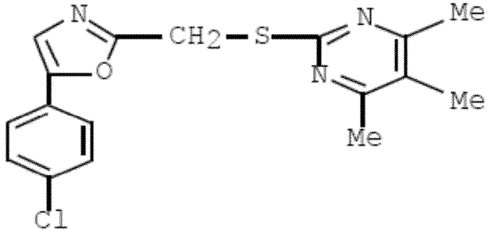

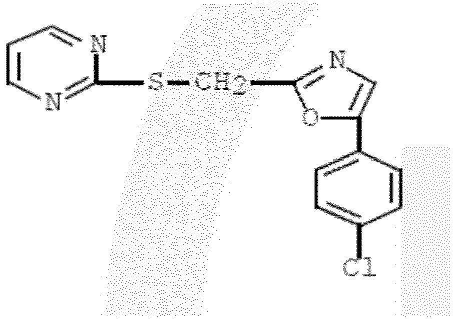

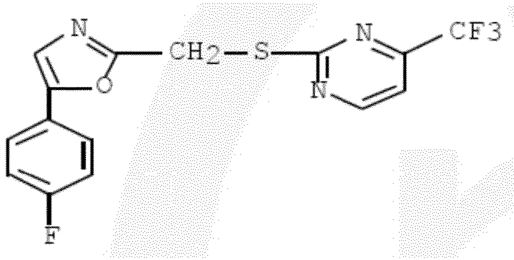

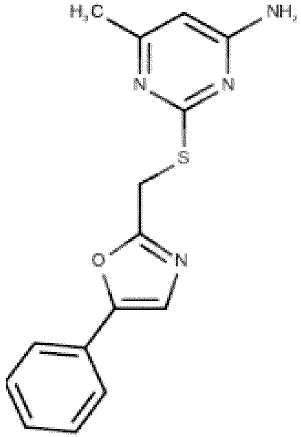

2. Composé selon la revendication 1 dans lequel l'halogénure est choisi parmi CI, Br et F.

3. Composé selon la revendication 1 ou 2 dans lequel R¹ est un 1,3,5-triazinyl substitué comme défini dans la revendication 1.

4. Composé selon la revendication 3 dans lequel, si le groupe 1,3,5-triazinyl est substitué par plus d'un substituant, les substituants sont différents.

5. Composé selon la revendication 3 ou 4, dans lequel le groupe 1,3,5 triazinyle est indépendamment substitué par un ou deux substituants choisis dans le groupe consistant en amino, méthyl, éthyl, isopropyl et tert.-butyl, ce(s) substituant(s) étant éventuellement substitué(s) par un ou plusieurs halogénures.

6. Composé selon la revendication 3 dans lequel R¹ est choisi dans le groupe constitué de 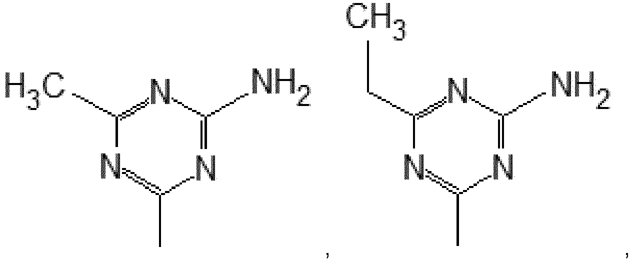 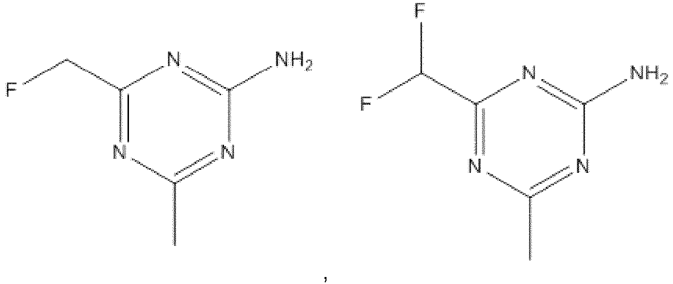 et

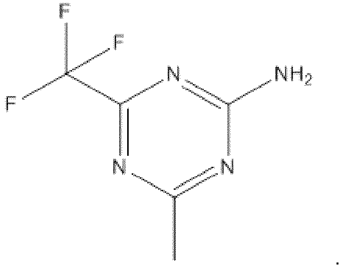

7. Composé selon l'une quelconque des revendications 1 ou 2, dans lequel R¹ est un pyrimidinyle substitué comme défini dans la revendication 1.

8. Composé selon la revendication 7 dans lequel, si le groupe pyrimidinyle est substitué par plus d'un substituant, les substituants sont différents.

9. Composé selon la revendication 7 ou 8, dans lequel le groupe pyrimidinyle est indépendamment substitué par un ou plusieurs substituants choisis dans le groupe constitué par l'amino, le méthyle et l'éthyle.

10. Composé selon la revendication 9, dans lequel le groupe amino est substitué par un méthyle.

11. Composé selon la revendication 9 ou 10 dans lequel le groupe méthyle ou éthyle est substitué par un ou plusieurs halogénures tels que définis dans la revendication 2.

12. Composé selon la revendication 7 dans lequel R¹ est choisi dans le groupe constitué de

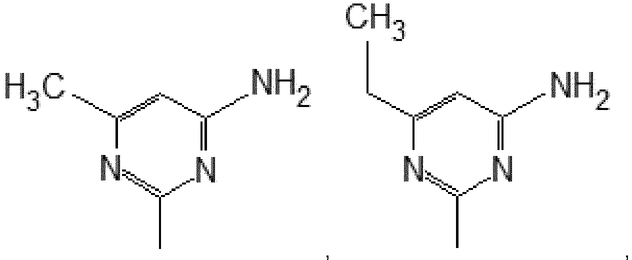

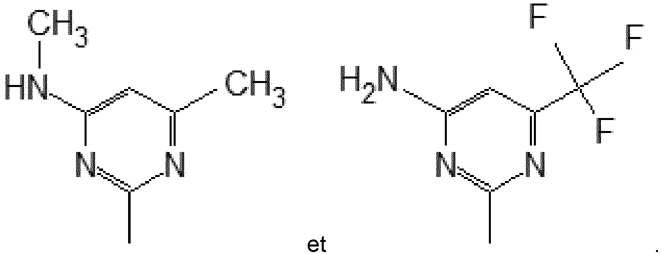

13. Composé selon l'une quelconque des revendications précédentes, dans lequel R² est indépendamment substitué par un ou plusieurs substituants choisis dans le groupe constitué de Cl, Br, F, méthyle, triflourméthyle, méthoxy et éthoxy.

14. Composé selon l'une quelconque des revendications précédentes, dans lequel R² est substitué en au moins une position méta et/ou au moins une position ortho.

15. Composé selon la revendication 14 dans lequel R² est choisi dans le groupe constitué de

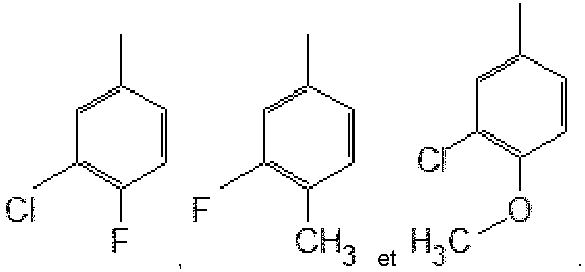

16. Composé selon la revendication 13 ou 14 dans lequel R² n'est pas substitué en position para.

17. Composé selon la revendication 16 dans lequel R² est choisi dans le groupe constitué de 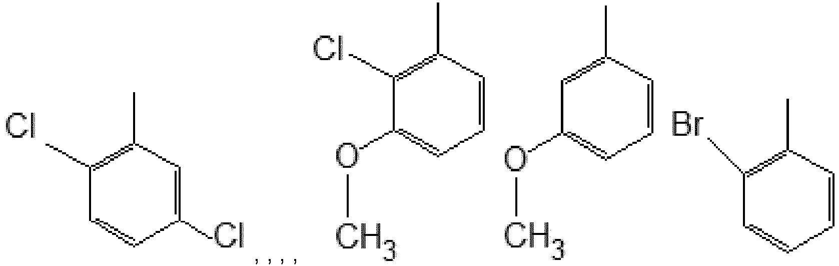 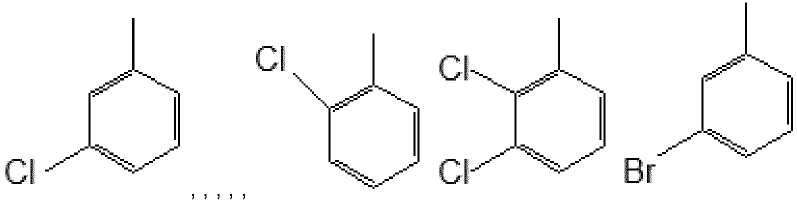 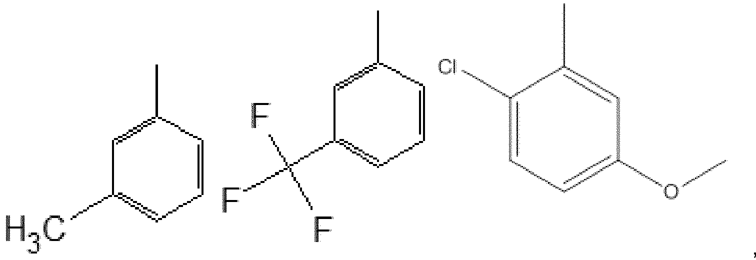 et .

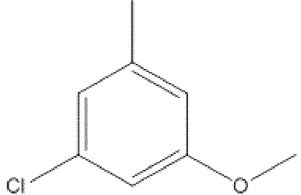

18. Le composé selon la revendication 1 est choisi dans le groupe constitué des composés suivants :
2-({[5-(2-chloro-5-méthoxyphényl)-1,3-oxazol-2-yl]méthyl}sulfanyl)-6-méthylpyrimidine-4-amine
4-({[5-(2-chloro-5-methoxyphényl)-1,3-oxazol-2-yl]méthyl}sulfanyl)-6-(difluorométhyl)-1,3,5-triazin-2-amine
4-([5-(2,5-dichlorophényl)-1,3-oxazol-2-yl]méthylsulfanyl)-6-(difluorométhyl)-1,3,5-triazin-2-amine
4-({[5-(2-chloro-5-methoxyphényl)-1,3-oxazol-2-yl]méthyl}sulfanyl)-6-(fluorométhyl)-1,3,5-triazin-2-amine
2-({[5-(2-chloro-3-méthoxyphényl)-1,3-oxazol-2-yl]méthyl}sulfanyl)-6-méthylpyrimidine-4-amine
2-({[5-(2-chloro-3-méthoxyphényl)-1,3-oxazol-2-yl]méthyl}sulfanyl)-6-méthylpyrimidine-4-amine
4-({[5-(2,5-dichlorophényl)-1,3-oxazol-2-yl]méthyl}sulfanyl)-6-méthyl-1,3,5-triazin-2-amine
4-({[5-(2-chloro-5-methoxyphényl)-1,3-oxazol-2-yl]méthyl}sulfanyl)-6-éthyl-1,3,5-triazin-2-amine
4-({[5-(2-chloro-5-methoxyphényl)-1,3-oxazol-2-yl]méthyl}sulfanyl)-6-méthyl-1,3,5-triazin-2-amine
4-({[5-(3-chloro-4-methoxyphényl)-1,3-oxazol-2-yl]méthyl}sulfanyl)-6-méthyl-1,3,5-triazin-2-amine
2-({[5-(2-chloro-5-méthoxyphényl)-1,3-oxazol-2-yl]méthyl}sulfanyl)-6-(trifluorométhyl)pyrimidin-4-amine
4-([5-(2,5-dichlorophényl)-1,3-oxazol-2-yl]méthylsulfanyl)-6-(fluorométhyl)-1,3,5-triazin-2-amine
4-({[5-(2,5-dichlorophényl)-1,3-oxazol-2-yl]méthyl}sulfanyl)- 6-méthylpyrimidine-4-amine
4-([5-(2,5-dichlorophényl)-1,3-oxazol-2-yl]méthylsulfanyl)-6-éthyl-1,3,5-triazin-2-amine
2-({[5-(3-chloro-4-méthoxyphényl)-1,3-oxazol-2-yl]méthyl}sulfanyl)-6-méthylpyrimidin-4-amine
4-({[5-(2-chloro-3-methoxyphényl)-1,3-oxazol-2-yl]méthyl}sulfanyl)-6-méthyl-1,3,5-triazin-2-amine
4-({[5-(3-chloro-4-methoxyphényl)-1,3-oxazol-2-yl]méthyl}sulfanyl)-6-éthyl-1,3,5-triazin-2-amine
4-éthyl-6-({[5-(3-methoxyphényl)-1,3-oxazol-2-yl]méthyl}sulfanyl)-1,3,5-triazin-2-amine
2-({[5-(2-bromophényl)-1,3-oxazol-2-yl]méthyl}sulfanyl)-6-méthylpyrimidine-4-amine
2-({[5-(3-méthoxyphényl)-1,3-oxazol-2-yl]méthyl}sulfanyl)-6-méthylpyrimidine-4-amine
4-({[5-(3-chlorophényl)-1,3-oxazol-2-yl]méthyl}sulfanyl)-6-(pyridin-4-yl)-1,3,5-triazin-2-amine
4-({[5-(3-chloro-5-methoxyphényl)-1,3-oxazol-2-yl]méthyl}sulfanyl)-6-éthyl-1,3,5-triazin-2-amine
4-({[5-(3-chlorophényl)-1,3-oxazol-2-yl]méthyl}sulfanyl)-6-éthyl-1,3,5-triazin-2-amine
4-({[5-(3-chloro-4-fluorophényl)-1,3-oxazol-2-yl]méthyl}sulfanyl)-6-méthyl-1,3,5-triazin-2-amine
6-méthyl-2-({[5-(4-méthylphényl)-1,3-oxazol-2-yl]méthyl}sulfanyl)pyrimidine-4-amine
2-({[5-(3-chlorophényl)-1,3-oxazol-2-yl]méthyl}sulfanyl)-6-méthylpyrimidine-4-amine
4-({[5-(3-chlorophényl)-1,3-oxazol-2-yl]méthyl}sulfanyl)-6-(1-méthyl-1H-pyrazol-5-yl)-1,3,5-triazin-2-amine
2-[({5-[3-chloro-4-(3-méthanesulfonylpropoxy)phényl]-1,3-oxazol-2-yl}méthyl)sulfanyl]-6-méthylpyrimidine-4-amine
2-({[5-(3-éthoxyphényl)-1,3-oxazol-2-yl]méthyl}sulfanyl)-6-méthylpyrimidine-4-amine
2-({[5-(3-chloro-4-fluorophényl)-1,3-oxazol-2-yl]méthyl}sulfanyl)-6-méthylpyrimidine-4-amine
4-({[5-(3-chlorophényl)-1,3-oxazol-2-yl]méthyl}sulfanyl)-6-méthyl-1,3,5-triazin-2-amine
4-({[5-(2-chlorophényl)-1,3-oxazol-2-yl]méthyl}sulfanyl)-6-méthyl-1,3,5-triazin-2-amine
4-({[5-(3-methoxyphényl)-1,3-oxazol-2-yl]méthyl}sulfanyl)-6-méthyl-1,3,5-triazin-2-amine
2-({[5-(3-chloro-5-méthoxyphényl)-1,3-oxazol-2-yl]méthyl}sulfanyl)-6-méthylpyrimidine-4-amine
4-({[5-(2,3-dichlorophényl)-1,3-oxazol-2-yl]méthyl}sulfanyl)-6-éthyl-1,3,5-triazin-2-amine
6-méthyl-2-[({5-[3-(trifluorométhyl)phényl]-1,3-oxazol-2-yl}méthyl)sulfanyl]pyrimidine-4-amine
2-({[5-(2,3-dichlorophényl)-1,3-oxazol-2-yl]méthyl}sulfanyl)-6-méthylpyrimidine-4-amine
4-méthyl-6-[({5-[3-(trifluorométhyl)phényl]-1,3-oxazol-2-yl}méthyl)sulfanyl]-1,3,5-triazin-2-amine
4-({[5-(3-bromophényl)-1,3-oxazol-2-yl]méthyl}sulfanyl)-6-méthyl-1,3,5-triazin-2-amine
4-({[5-(3-ethoxyphényl)-1,3-oxazol-2-yl]méthyl}sulfanyl)-6-méthyl-1,3,5-triazin-2-amine
4-({[5-(2,3-dichlorophényl)-1,3-oxazol-2-yl]méthyl}sulfanyl)-6-méthyl-1,3,5-triazin-2-amine
2-({[5-(2,5-dichlorophényl)-1,3-oxazol-2-yl]méthyl}sulfanyl)-6-(trifluorométhyl)pyrimidin-4-amine
4-[chloro(fluoro)méthyl]-6-({[5-(3-chlorophényl)-1,3-oxazol-2-yl]méthyl}sulfanyl)-1,3,5-triazin-2-amine
4-({[5-(3-chloro-5-methoxyphényl)-1,3-oxazol-2-yl]méthyl}sulfanyl)-6-méthyl-1,3,5-triazin-2-amine
6-({[5-(2,5-dichlorophényl)-1,3-oxazol-2-yl]méthyl}thio)-*N*,N-diméthyl-1,3,5-triazine-2,4-diamine
4-méthyl-6-({[5-(4-méthylphényl)-1,3-oxazol-2-yl]méthyl}sulfanyl)-1,3,5-triazin-2-amine
2-({[5-(2-chlorophényl)-1,3-oxazol-2-yl]méthyl}sulfanyl)-6-méthylpyrimidine-4-amine
4-méthyl-6-({[5-(naphthalen-2-yl)-1,3-oxazol-2-yl]méthyl}sulfanyl)-1,3,5-triazin-2-amine
2-({[5-(3-méthoxyphényl)-1,3-oxazol-2-yl]méthyl}sulfanyl)-6-(trifluorométhyl)pyrimidin-4-amine
4-méthyl-6-[({5-[3-(trifluoromethoxy)phényl]-1,3-oxazol-2-yl}méthyl)sulfanyl]-1,3,5-triazin-2-amine
2-({[5-(4-méthoxyphényl)-1,3-oxazol-2-yl]méthyl}sulfanyl)-6-méthylpyrimidine-4-amine
4-({[5-(4-methoxyphényl)-1,3-oxazol-2-yl]méthyl}sulfanyl)-6-méthyl-1,3,5-triazin-2-amine
6-méthyl-2-({[5-(naphthalen-2-yl)-1,3-oxazol-2-yl]méthyl}sulfanyl)pyrimidin-4-amine
6-méthyl-2-({[5-(2-méthylphényl)-1,3-oxazol-2-yl]méthyl}sulfanyl)pyrimidine-4-amine
4-([5-(3-chlorophényl)-1,3-oxazol-2-yl]méthylsulfanyl)-6-(trifluorométhyl)-1,3,5-triazin-2-amine
4-({[5-(2-chloro-5-methoxyphényl)-1,3-oxazol-2-yl]méthyl}sulfanyl)-6-(morpholin-4-yl)-1,3,5-triazin-2-amine
2-([5-(3-méthoxyphényl)-1,3-oxazol-2-yl]méthylsulfanyl)-*N*,6-diméthylpyrimidine-4-amine
4-({[5-(4-éthylphényl)-1,3-oxazol-2-yl]méthyl}sulfanyl)-6-méthyl-1,3,5-triazin-2-amine
2-({[5-(2-chlorophényl)-1,3-oxazol-2-yl]méthyl}sulfanyl)-6-(trifluorométhyl)pyrimidine-4-amine
4-({[5-(3-chlorophényl)-1,3-oxazol-2-yl]méthyl}sulfanyl)-6-phényl-1,3,5-triazin-2-amine
4-([5-(2,5-dichlorophényl)-1,3-oxazol-2-yl]méthylsulfanyl)-6-(morpholin-4-yl)-1,3,5-triazin-2-amine
2-({[5-(3-chlorophényl)-1,3-oxazol-2-yl]méthyl}sulfanyl)-6-(trifluorométhyl)pyrimidine-4-amine
4-cyclopropyl-6-([5-(3-méthoxyphényl)-1,3-oxazol-2-yl]méthylsulfanyl)-1,3,5-triazine-2-amine
2-({[5-(3-chloro-5-méthoxyphényl)-1,3-oxazol-2-yl]méthyl}sulfanyl)-6-(trifluorométhyl)pyrimidin-4-amine
2-({[5-(2,3-dichlorophényl)-1,3-oxazol-2-yl]méthyl}sulfanyl)-6-(trifluorométhyl)pyrimidin-4-amine
4-({[5-(3-chlorophényl)-1,3-oxazol-2-yl]méthyl}sulfanyl)-6-(1,3-thiazol-4-yl)-1,3,5-triazin-2-amine
4-({[5-(3-chlorophényl)-1,3-oxazol-2-yl]méthyl}sulfanyl)-6-(1-méthyl-1H-pyrazol-4-yl)-1,3,5-triazin-2-amine
4-({[5-(4-fluorophényl)-1,3-oxazol-2-yl]méthyl}sulfanyl)-6-méthyl-1,3,5-triazin-2-amine 2-({thiéno[2,3-d]pyrimidine-4-ylsulfanyl}méthyle)-5-[3-(trifluorométhyl)phényl]-1,3-oxazole
4-({[5-(3-chlorophényl)-1,3-oxazol-2-yl]méthyl}sulfanyl)-6-(1-méthylcyclopropyl)-1,3,5-triazin-2-amine
4-({[5-(3-chlorophényl)-1,3-oxazol-2-yl]méthyl}sulfanyl)-6-(3,3-difluorocyclobutyl)-1,3,5-triazin-2-amine
6-méthyl-2-[({5-[3-(trifluorométhoxy)phényl]-1,3-oxazol-2-yl}méthyl)sulfanyl]pyrimidine-4-amine
2-[({5-[3-(trifluorométhoxy)phényl]-1,3-oxazol-2-yl}méthyl)sulfanyl]-6-(trifluorométhyl)pyrimidine-4-amine
2-({[5-(2-méthoxyphényl)-1,3-oxazol-2-yl]méthyl}sulfanyl)-6-méthylpyrimidine-4-amine
5-(2,5-dichlorophényl)-2-[({2-méthylpyrido[2,3-d]pyrimidin-4-yl}sulfanyl)méthyl]-1,3-oxazole
5-(3-méthoxyphényl)-2-({thiéno[2,3-d]pyrimidine-4-ylsulfanyl}méthyl)-1,3-oxazole
2-({[5-(4-méthylphényl)-1,3-oxazol-2-yl]méthyl}sulfanyl)-6-(trifluorométhyl)pyrimidine-4-amine
4-([5-(3-chlorophényl)-1,3-oxazol-2-yl]méthylsulfanyl)-6-methoxy-1,3,5-triazin-2-amine
4-éthyl-6-[({5-[3-(trifluoromethoxy)phényl]-1,3-oxazol-2-yl}méthyl)sulfanyl]-1,3,5-triazin-2-amine
2-({[5-(3-chlorophényl)-1,3-oxazol-2-yl]méthyl}sulfanyl)-N-méthylquinazoline-4-amine
4-({[5-(3-chlorophényl)-1,3-oxazol-2-yl]méthyl}sulfanyl)-6-(methoxyméthyl)-1,3,5-triazin-2-amine
6-({[5-(2-chlorophényl)-1,3-oxazol-2-yl]méthyl}sulfanyl)-1,3,5-triazine-2,4-diamine
2-({[5-(3-chloro-4-fluorophényl)-1,3-oxazol-2-yl]méthyl}sulfanyl)-6-(trifluorométhyl)pyrimidine-4-amine
5-(3-méthoxyphényl)-2-[({2-méthylpyrido[2,3-d]pyrimidin-4-yl}sulfanyl)méthyl]-1,3-oxazole

19. Composition pharmaceutique comprenant au moins un composé selon l'une des revendications précédentes et au moins un support pharmaceutique.

20. Composé de formule générale (I)
R¹-S-CH₂-OXA-R² (I)
y compris les énantiomères, les diastéréomères, les hydrates, les solvates, les cristaux ou les sels pharmaceutiquement acceptables et leurs complexes ; dans lequel
OXA est choisi dans le groupe constitué par le 1,3-oxazolyle, le 1,2,4-oxadiazolyle ou le 1,3,4-oxadiazolyle ;
lorsque OXA est un 1,3-oxazolyle, le groupe R¹-S-CH₂ est lié à C² du 1,3-oxazolyle et le groupe R² est lié à C⁴ du 1,3-oxazolyle ;
lorsque OXA est un 1,2,4-oxadiazolyle, le groupe R¹-S-CH₂ est lié à C⁵ du 1,2,4-oxadiazolyle et le groupe R² est lié à C³ du 1,2,4-oxadiazolyle ;
lorsque OXA est un 1,3,4-oxadiazolyle, le groupe R¹-S-CH₂ est lié à C⁵ du 1,3,4-oxadiazolyle et le groupe R² est lié à C² du 1,3,4-oxadiazolyl' ;
R¹ est un groupe hétéroaryle à 6 membres choisi dans le groupe constitué de 1,3,5-triazinyl et pyrimidinyl indépendamment substitué par un ou plusieurs substituants choisis dans le groupe constitué d'hydroxyl, amino, C₁-C₆-alkyl, C₃-C₆-cycloalkyl, C₁-C₄-alkoxy, N-mono- ou N,N-di-substitué C₁-C₃-alkylamino, hétérocyclyle non aromatique à 5-6 chaînons, aryle à 6 chaînons et hétéroaryle à 5 à 6 chaînons dont les substituants peuvent être non substitués ou substitués par un ou plusieurs groupes choisis dans le groupe consistant en halogénure, cyano et C₁-C₆-alkyl, dans lequel le groupe aryle à 6 chaînons et le groupe hétéroaryle à 5 à 6 chaînons, respectivement, peuvent être fusionnés avec ledit groupe 1,3,5-triazinyle ou pyrimidyle, respectivement, et
R² est un phényle non substitué ou substitué par un ou plusieurs substituants choisis dans le groupe constitué par les halogénures, les cyanos, les amines, les C₁-C₆-alkyl, les C₃-C₆-cycloalkyl qui peuvent être éventuellement substitués par un ou plusieurs halogénures, C₁-C₄-alkoxy éventuellement substitué par un ou plusieurs halogénures, hydroxy-C₁-C(_{6)-akyl}, sulfonyl-C₁-C(₆)-alkyl, sulfamidyl-N-C₁-C6-alkyl et carboxamidyl-N-mono- ou -N,N-di-C₁-C(_{6)-alkyl} ;
pour une utilisation en tant qu'agoniste du GPR40 dans le traitement et/ou la prévention du diabète sucré de type 2 et/ou d'un état prédiabétique.

21. Composé utilisé selon la revendication 20, dans lequel l'état prédiabétique est choisi dans le groupe constitué par l'obésité et la résistance à l'insuline.

22. Composé utilisé selon la revendication 20 ou 21, dans lequel le composé présente une sélectivité plus élevée pour le GPR40 que pour le GPR120.

23. Composé utilisé selon la revendication 22, dans lequel le composé présente un % d'activation du GPR40 au moins 3 fois supérieur au % d'activation du GPR120, le % d'activation étant le rapport de cent pour cent de l'activation du GPR40 ou du GPR120, respectivement, par ledit composé à l'activation du GPR40 ou du GPR120, respectivement, par l'AMG 837.

24. Composé utilisable selon l'une des revendications 20 à 23, dans lequel l'halogénure est choisi parmi CI, Br et F.

25. Composé utilisable selon l'une des revendications 20 à 24, dans lequel R¹ est défini comme dans l'une des revendications 3 à 12.

26. Composé utilisable selon l'une quelconque des revendications 20 à 25, dans lequel R² est défini comme dans l'une quelconque des revendications 13 à 17.

27. Composé utilisable selon l'une des revendications 20 à 26, dans lequel OXA est un 1,3-oxazolyle.

28. Composé à utiliser selon la revendication 20, dans lequel le composé est choisi dans le groupe constitué des composés suivants :
2-({[5-(2-chloro-5-méthoxyphényl)-1,3-oxazol-2-yl]méthyl}sulfanyl)-6-méthylpyrimidin-4-amine
4-({[5-(2-chloro-5-methoxyphényl)-1,3-oxazol-2-yl]méthyl}sulfanyl)-6-(difluorométhyl)-1,3,5-triazin-2-amine
4-([5-(2,5-dichlorophényl)-1,3-oxazol-2-yl]méthylsulfanyl)-6-(difluorométhyl)-1,3,5-triazin-2-amine
4-({[5-(2-chloro-5-methoxyphényl)-1,3-oxazol-2-yl]méthyl}sulfanyl)-6-(fluorométhyl)-1,3,5-triazin-2-amine
2-({[5-(2-chloro-3-méthoxyphényl)-1,3-oxazol-2-yl]méthyl}sulfanyl)-6-méthylpyrimidin-4-amine
2-({[5-(2-chloro-3-méthoxyphényl)-1,3-oxazol-2-yl]méthyl}sulfanyl)-6-méthylpyrimidin-4-amine
4-({[5-(2,5-dichlorophényl)-1,3-oxazol-2-yl]méthyl}sulfanyl)-6-méthyl-1,3,5-triazin-2-amine
4-({[5-(2-chloro-5-methoxyphényl)-1,3-oxazol-2-yl]méthyl}sulfanyl)-6-éthyl-1,3,5-triazin-2-amine
4-({[5-(2-chloro-5-methoxyphényl)-1,3-oxazol-2-yl]méthyl}sulfanyl)-6-méthyl-1,3,5-triazin-2-amine
4-({[5-(3-chloro-4-methoxyphényl)-1,3-oxazol-2-yl]méthyl}sulfanyl)-6-méthyl-1,3,5-triazin-2-amine
2-({[5-(2-chloro-5-méthoxyphényl)-1,3-oxazol-2-yl]méthyl}sulfanyl)-6-(trifluorométhyl)pyrimidin-4-amine
4-([5-(2,5-dichlorophényl)-1,3-oxazol-2-yl]méthylsulfanyl)-6-(fluorométhyl)-1,3,5-triazin-2-amine
4-({[5-(2,5-dichlorophényl)-1,3-oxazol-2-yl]méthyl}sulfanyl)- 6-méthylpyrimidine-4-amine
4-([5-(2,5-dichlorophényl)-1,3-oxazol-2-yl]méthylsulfanyl)-6-éthyl-1,3,5-triazin-2-amine
2-({[5-(3-chloro-4-méthoxyphényl)-1,3-oxazol-2-yl]méthyl}sulfanyl)-6-méthylpyrimidine-4-amine
4-({[5-(2-chloro-3-methoxyphényl)-1,3-oxazol-2-yl]méthyl}sulfanyl)-6-méthyl-1,3,5-triazin-2-amine
4-({[5-(3-chloro-4-methoxyphényl)-1,3-oxazol-2-yl]méthyl}sulfanyl)-6-éthyl-1,3,5-triazin-2-amine
4-éthyl-6-({[5-(3-methoxyphényl)-1,3-oxazol-2-yl]méthyl}sulfanyl)-1,3,5-triazin-2-amine
2-({[5-(2-bromophényl)-1,3-oxazol-2-yl]méthyl}sulfanyl)-6-méthylpyrimidine-4-amine
2-({[5-(3-méthoxyphényl)-1,3-oxazol-2-yl]méthyl}sulfanyl)-6-méthylpyrimidine-4-amine
4-({[5-(3-chlorophényl)-1,3-oxazol-2-yl]méthyl}sulfanyl)-6-(pyridin-4-yl)-1,3,5-triazin-2-amine
4-({[5-(3-chloro-5-methoxyphényl)-1,3-oxazol-2-yl]méthyl}sulfanyl)-6-éthyl-1,3,5-triazin-2-amine
4-({[5-(3-chlorophényl)-1,3-oxazol-2-yl]méthyl}sulfanyl)-6-éthyl-1,3,5-triazin-2-amine
4-({[5-(3-chloro-4-fluorophényl)-1,3-oxazol-2-yl]méthyl}sulfanyl)-6-méthyl-1,3,5-triazin-2-amine
6-méthyl-2-({[5-(4-méthylphényl)-1,3-oxazol-2-yl]méthyl}sulfanyl)pyrimidine-4-amine
2-({[5-(3-chlorophényl)-1,3-oxazol-2-yl]méthyl}sulfanyl)-6-méthylpyrimidine-4-amine
4-({[5-(3-chlorophényl)-1,3-oxazol-2-yl]méthyl}sulfanyl)-6-(1-méthyl-1H-pyrazol-5-yl)-1,3,5-triazin-2-amine
2-[({5-[3-chloro-4-(3-méthanesulfonylpropoxy)phényl]-1,3-oxazol-2-yl}méthyl)sulfanyl]-6-méthylpyrimidine-4-amine
2-({[5-(3-éthoxyphényl)-1,3-oxazol-2-yl]méthyl}sulfanyl)-6-méthylpyrimidine-4-amine
2-({[5-(3-chloro-4-fluorophényl)-1,3-oxazol-2-yl]méthyl}sulfanyl)-6-méthylpyrimidine-4-amine
4-({[5-(3-chlorophényl)-1,3-oxazol-2-yl]méthyl}sulfanyl)-6-méthyl-1,3,5-triazin-2-amine
4-({[5-(2-chlorophényl)-1,3-oxazol-2-yl]méthyl}sulfanyl)-6-méthyl-1,3,5-triazin-2-amine
4-({[5-(3-methoxyphényl)-1,3-oxazol-2-yl]méthyl}sulfanyl)-6-méthyl-1,3,5-triazin-2-amine
2-({[5-(3-chloro-5-méthoxyphényl)-1,3-oxazol-2-yl]méthyl}sulfanyl)-6-méthylpyrimidine-4-amine
4-({[5-(2,3-dichlorophényl)-1,3-oxazol-2-yl]méthyl}sulfanyl)-6-éthyl-1,3,5-triazin-2-amine
6-méthyl-2-[({5-[3-(trifluorométhyl)phényl]-1,3-oxazol-2-yl}méthyl)sulfanyl]pyrimidine-4-amine
2-({[5-(2,3-dichlorophényl)-1,3-oxazol-2-yl]méthyl}sulfanyl)-6-méthylpyrimidine-4-amine
4-méthyl-6-[({5-[3-(trifluorométhyl)phényl]-1,3-oxazol-2-yl}méthyl)sulfanyl]-1,3,5-triazin-2-amine
4-({[5-(3-bromophényl)-1,3-oxazol-2-yl]méthyl}sulfanyl)-6-méthyl-1,3,5-triazin-2-amine
4-({[5-(3-ethoxyphényl)-1,3-oxazol-2-yl]méthyl}sulfanyl)-6-méthyl-1,3,5-triazin-2-amine
4-({[5-(2,3-dichlorophényl)-1,3-oxazol-2-yl]méthyl}sulfanyl)-6-méthyl-1,3,5-triazin-2-amine
2-({[5-(2,5-dichlorophényl)-1,3-oxazol-2-yl]méthyl}sulfanyl)-6-(trifluorométhyl)pyrimidin-4-amine
4-[chloro(fluoro)méthyl]-6-({[5-(3-chlorophényl)-1,3-oxazol-2-yl]méthyl}sulfanyl)-1,3,5-triazin-2-amine
4-({[5-(3-chloro-5-methoxyphényl)-1,3-oxazol-2-yl]méthyl}sulfanyl)-6-méthyl-1,3,5-triazin-2-amine
6-({[5-(2,5-dichlorophényl)-1,3-oxazol-2-yl]méthyl}thio)-*N*,N-diméthyl-1,3,5-triazine-2,4-diamine
4-méthyl-6-({[5-(4-méthylphényl)-1,3-oxazol-2-yl]méthyl}sulfanyl)-1,3,5-triazin-2-amine
2-({[5-(2-chlorophényl)-1,3-oxazol-2-yl]méthyl}sulfanyl)-6-méthylpyrimidine-4-amine
4-méthyl-6-({[5-(naphthalen-2-yl)-1,3-oxazol-2-yl]méthyl}sulfanyl)-1,3,5-triazin-2-amine
2-({[5-(3-méthoxyphényl)-1,3-oxazol-2-yl]méthyl}sulfanyl)-6-(trifluorométhyl)pyrimidin-4-amine
4-méthyl-6-[({5-[3-(trifluoromethoxy)phényl]-1,3-oxazol-2-yl}méthyl)sulfanyl]-1,3,5-triazin-2-amine
2-({[5-(4-méthoxyphényl)-1,3-oxazol-2-yl]méthyl}sulfanyl)-6-méthylpyrimidine-4-amine
4-({[5-(4-methoxyphényl)-1,3-oxazol-2-yl]méthyl}sulfanyl)-6-méthyl-1,3,5-triazin-2-amine
6-méthyl-2-({[5-(naphthalen-2-yl)-1,3-oxazol-2-yl]méthyl}sulfanyl)pyrimidin-4-amine
6-méthyl-2-({[5-(2-méthylphényl)-1,3-oxazol-2-yl]méthyl}sulfanyl)pyrimidine-4-amine
4-([5-(3-chlorophényl)-1,3-oxazol-2-yl]méthylsulfanyl)-6-(trifluorométhyl)-1,3,5-triazin-2-amine
4-({[5-(2-chloro-5-methoxyphényl)-1,3-oxazol-2-yl]méthyl}sulfanyl)-6-(morpholin-4-yl)-1,3,5-triazin-2-amine
2-([5-(3-méthoxyphényl)-1,3-oxazol-2-yl]méthylsulfanyl)-*N*,6-diméthylpyrimidine-4-amine
4-({[5-(4-éthylphényl)-1,3-oxazol-2-yl]méthyl}sulfanyl)-6-méthyl-1,3,5-triazin-2-amine
2-({[5-(2-chlorophényl)-1,3-oxazol-2-yl]méthyl}sulfanyl)-6-(trifluorométhyl)pyrimidine-4-amine
4-({[5-(3-chlorophényl)-1,3-oxazol-2-yl]méthyl}sulfanyl)-6-phényl-1,3,5-triazin-2-amine
4-([5-(2,5-dichlorophényl)-1,3-oxazol-2-yl]méthylsulfanyl)-6-(morpholin-4-yl)-1,3,5-triazin-2-amine
2-({[5-(3-chlorophényl)-1,3-oxazol-2-yl]méthyl}sulfanyl)-6-(trifluorométhyl)pyrimidine-4-amine
4-cyclopropyl-6-([5-(3-méthoxyphényl)-1,3-oxazol-2-yl]méthylsulfanyl)-1,3,5-triazine-2-amine
2-({[5-(3-chloro-5-méthoxyphényl)-1,3-oxazol-2-yl]méthyl}sulfanyl)-6-(trifluorométhyl)pyrimidin-4-amine
2-({[5-(2,3-dichlorophényl)-1,3-oxazol-2-yl]méthyl}sulfanyl)-6-(trifluorométhyl)pyrimidin-4-amine
4-({[5-(3-chlorophényl)-1,3-oxazol-2-yl]méthyl}sulfanyl)-6-(1,3-thiazol-4-yl)-1,3,5-triazin-2-amine
4-({[5-(3-chlorophényl)-1,3-oxazol-2-yl]méthyl}sulfanyl)-6-(1-méthyl-1H-pyrazol-4-yl)-1,3,5-triazin-2-amine
4-({[5-(4-fluorophényl)-1,3-oxazol-2-yl]méthyl}sulfanyl)-6-méthyl-1,3,5-triazin-2-amine
2-({thiéno[2,3-d]pyrimidine-4-ylsulfanyl}méthyle)-5-[3-(trifluorométhyl)phényl]-1,3-oxazole
4-({[5-(3-chlorophényl)-1,3-oxazol-2-yl]méthyl}sulfanyl)-6-(1-méthylcyclopropyl)-1,3,5-triazin-2-amine
4-({[5-(3-chlorophényl)-1,3-oxazol-2-yl]méthyl}sulfanyl)-6-(3,3-difluorocyclobutyl)-1,3,5-triazin-2-amine
6-méthyl-2-[({5-[3-(trifluorométhoxy)phényl]-1,3-oxazol-2-yl}méthyl)sulfanyl]pyrimidine-4-amine
2-[({5-[3-(trifluorométhoxy)phényl]-1,3-oxazol-2-yl}méthyl)sulfanyl]-6-(trifluorométhyl)pyrimidine-4-amine
2-({[5-(2-méthoxyphényl)-1,3-oxazol-2-yl]méthyl}sulfanyl)-6-méthylpyrimidine-4-amine
5-(2,5-dichlorophényl)-2-[({2-méthylpyrido[2,3-d]pyrimidin-4-yl}sulfanyl)méthyl]-1,3-oxazole
5-(3-méthoxyphényl)-2-({thiéno[2,3-d]pyrimidine-4-ylsulfanyl}méthyl)-1,3-oxazole
2-({[5-(4-méthylphényl)-1,3-oxazol-2-yl]méthyl}sulfanyl)-6-(trifluorométhyl)pyrimidine-4-amine
4-([5-(3-chlorophényl)-1,3-oxazol-2-yl]méthylsulfanyl)-6-methoxy-1,3,5-triazin-2-amine
4-éthyl-6-[({5-[3-(trifluoromethoxy)phényl]-1,3-oxazol-2-yl}méthyl)sulfanyl]-1,3,5-triazin-2-amine
2-({[5-(3-chlorophényl)-1,3-oxazol-2-yl]méthyl}sulfanyl)-N-méthylquinazoline-4-amine
4-({[5-(3-chlorophényl)-1,3-oxazol-2-yl]méthyl}sulfanyl)-6-(methoxyméthyl)-1,3,5-triazin-2-amine
6-({[5-(2-chlorophényl)-1,3-oxazol-2-yl]méthyl}sulfanyl)-1,3,5-triazine-2,4-diamine
2-({[5-(3-chlorophényl)-1,3,4-oxadiazol-2-yl]méthyl}sulfanyl)-6-méthylpyrimidine-4-amine
2-({[5-(3-chloro-4-fluorophényl)-1,3-oxazol-2-yl]méthyl}sulfanyl)-6-(trifluorométhyl)pyrimidin-4-amine
2-({[5-(3-chlorophényl)-1,3,4-oxadiazol-2-yl]méthyl}sulfanyl)-6-(trifluorométhyl)pyrimidin-4-amine
5-(3-méthoxyphényl)-2-[({2-méthylpyrido[2,3-d]pyrimidin-4-yl}sulfanyl)méthyl]-1,3-oxazole
2-({[5-(4-fluorophényl)-1,3-oxazol-2-yl]méthyl}sulfanyl)-6-méthylpyrimidine-4-amine
2-({[5-(4-chlorophényl)-1,3-oxazol-2-yl]méthyl}sulfanyl)-6-méthylpyrimidine-4-amine
6-méthyl-2-{[(5-phényl-1,3-oxazol-2-yl)méthyl]sulfanyl}pyrimidin-4-amine.
